(19) Europäisches Patentamt
European Patent Office
Office européen des brevets

(11) **EP 2 971 164 B1**

(12) **EUROPEAN PATENT SPECIFICATION**

(45) Date of publication and mention
of the grant of the patent:
**26.07.2023 Bulletin 2023/30**

(21) Application number: **14770813.5**

(22) Date of filing: **13.03.2014**

(51) International Patent Classification (IPC):
*C12Q 1/6886* (2018.01)   *G16B 25/00* (2019.01)
*G16B 20/20* (2019.01)   *G16B 20/30* (2019.01)
*G16B 25/10* (2019.01)   *G16B 40/20* (2019.01)

(52) Cooperative Patent Classification (CPC):
**C12Q 1/6886; G16B 20/20; G16B 20/30;**
**G16B 25/00; G16B 25/10; G16B 40/20;**
C12Q 2600/156; C12Q 2600/158; G16B 20/00;
G16B 40/00

(86) International application number:
**PCT/US2014/026411**

(87) International publication number:
**WO 2014/151764 (25.09.2014 Gazette 2014/39)**

(54) **METHODS AND COMPOSITIONS FOR CLASSIFICATION OF SAMPLES**

VERFAHREN UND ZUSAMMENSETZUNGEN ZUR KLASSIFIZIERUNG VON PROBEN

PROCÉDÉS ET COMPOSITIONS POUR CLASSIFICATION D'ÉCHANTILLONS

(84) Designated Contracting States:
**AL AT BE BG CH CY CZ DE DK EE ES FI FR GR**
**HR HU IE IS IT LI LT LU LV MC MK MT NL NO PL**
**PT RO RS SE SI SK SM TR**

(30) Priority: **15.03.2013 US 201361798941 P**

(43) Date of publication of application:
**20.01.2016 Bulletin 2016/03**

(73) Proprietor: **Veracyte, Inc.**
**South San Francisco, CA 94080 (US)**

(72) Inventors:
• **KENNEDY, Giulia, C.**
**San Francisco, CA 94116 (US)**
• **WILDE, Jonathan, I.**
**Burlingame, CA 94010 (US)**
• **CHUDOVA, Darya**
**San Jose, CA 95123 (US)**
• **PANKRATZ, Daniel**
**Los Gatos, CA 95030 (US)**
• **BARBACIORU, Catalin**
**Fremont, CA 94536 (US)**
• **WALSH, P., Sean**
**Danville, CA 94506 (US)**
• **PAGAN, Moraima**
**San Francisco, CA 94117 (US)**

(74) Representative: **Avidity IP**
**Broers Building**
**Hauser Forum**
**21 JJ Thomson Avenue**
**Cambridge CB3 0FA (GB)**

(56) References cited:
WO-A2-2010/056374   WO-A2-2011/143361
US-A1- 2002 076 735   US-A1- 2012 220 474

• **GRIFFITH O L ET AL: "Meta-analysis and meta-review of thyroid cancer gene expression profiling studies identifies important diagnostic biomarkers", JOURNAL OF CLINICAL ONCOLOGY, AMERICAN SOCIETY OF CLINICAL ONCOLOGY, US, vol. 24, no. 31, 1 November 2006 (2006-11-01), pages 5043-5051, XP002499803, ISSN: 0732-183X, DOI: 10.1200/JCO.2006.06.7330**
• **DURAND S ET AL: "Evaluation of gene expression profiles in thyroid nodule biopsy material to diagnose thyroid cancer", JOURNAL OF CLINICAL ENDOCRINOLOGY AND METABOLISM, THE ENDOCRINE SOCIETY, US, vol. 93, no. 4, 1 April 2008 (2008-04-01), pages 1195-1202, XP002558430, ISSN: 0021-972X, DOI: 10.1210/JC.2007-1571 [retrieved on 2008-01-22]**

- **KEBEBEW ET AL.: 'The prevalence and prognostic value of BRAF mutation in thyroid cancer' ANN SURG. vol. 246, 01 September 2007, pages 466 - 471, XP055179786**
- **ZHANG ET AL.: 'Association between single-nucleotide polymorphisms of BRAF and papillary thyroid carcinoma in a Chinese population' THYROID vol. 23, no. 1, 2013, pages 38 - 44, XP055286235**
- **HADD ET AL.: 'Targeted, high-depth, next-generation sequencing of cancer genes in formalin-fixed, paraffin-embedded and fine-needle aspiration tumor specimens' J MOL DIAGN. vol. 15, 13 January 2013, pages 234 - 247, XP055286254**

**Description**

**CROSS REFERENCE**

This application claims priority to U.S. Provisional Patent Application No. 61/798,941, filed on March 15, 2013.

**BACKGROUND**

Cancer is one of the leading causes of mortality worldwide; yet for many patients, the process of simply clearing the first step of obtaining an accurate diagnosis is often a frustrating and time-consuming experience. This is true of many cancers, including thyroid cancer. This is also particularly true of relatively rare diseases, such as Hurthle cell adenomas and carcinomas, which account for approximately 5% of thyroid neoplasms.

An inaccurate diagnosis of cancer can lead to unnecessary follow-up procedures, including costly surgical procedures, not to mention unnecessary emotional distress to the patient. In the case of thyroid cancer, it is estimated that out of the approximately 130,000 thyroid removal surgeries performed each year due to suspected malignancy in the United States, only about 54,000 are necessary; therefore, tens of thousands of unnecessary thyroid removal surgeries are performed annually. Continued treatment costs and complications due to the need for lifelong drug therapy to replace the lost thyroid function can cause further economic and physical harm.

**SUMMARY**

The invention provides a method of identifying a thyroid cancer in a subject by identifying a BRAFV600E mutation in a fine needle aspiration sample of a subject using a trained algorithm, wherein said trained algorithm was trained by a process comprising:

(i) assaying a plurality of training samples for expression levels of a plurality of biomarkers comprising at least 5 biomarkers selected from Table 27 to obtain a training set comprising expression data, wherein said plurality of training samples comprises a first set of samples containing said BRAFV600E mutation and a second set of samples not containing said BRAFV600E mutation, wherein said plurality of training samples comprise fine needle aspirate (FNA) samples, and

(ii) performing supervised learning on said expression data to yield said trained algorithm that is capable of classifying said fine needle aspiration sample as containing or not containing said BRAFV600E mutation at a statistical confidence level of at least about 95%;

the method comprising:

(a) assaying an expression level of a plurality of biomarkers in said fine needle aspiration sample from said subject, wherein said plurality of biomarkers comprises said at least 5 biomarkers selected from Table 27, wherein said fine needle aspiration sample is independent of said plurality of training samples; and

(b) applying said trained algorithm to said expression level of (a) to classify said fine needle aspiration sample as containing or not containing said BRAFV600E mutation at a statistical confidence level of at least about 95%, to thereby predict a presence or absence of said thyroid cancer.

We disclose a method comprises isolating ribonucleic acid (RNA) from a biological sample obtained from the subject; identifying one or more mutations within a first region of interest in the RNA sample; comparing a frequency of variation for each base pair position in the first region of interest of the RNA sample to one or more references to identify one or more mutations that are correlated with the cancer; comparing the one or more mutations identified to the one or more mutations identified, to identify the presence of absence of at least one mutation; repeating the previous steps for a second region of interest of the RNA sample to generate a mutation profile for the RNA, wherein the second region of interest is different from the first region of interest; and diagnosing and/or treating the subject based on the mutation profile. The steps may be repeated at least 2, 10 or 100 times.

One or more references may comprise frequencies of variation for single base pairs in a reference sequence, wherein the frequencies of variation in the reference sequence are derived from at least 1000 individuals. In some embodiments one or more references of comprise frequencies of variation for single base pairs in a reference sequence, wherein the frequencies of variation in the reference sequence are derived from a known cancer. In some embodiments one or more references comprise frequencies of variation for single base pairs in a reference sequence, wherein the frequencies of variation in the reference sequence are derived from at least 40 samples.

A call score may be assigned to each mutation identified in the RNA. In some embodiments, a mutation profile of is generated using the COSMIC database of known sites of somatic variations in cancer.

In some embodiments The identification of the presence or absence of one or more mutations may be at least 90%, 95%, or 100% accurate.

This disclosure, but not being part of the invention, also describes a method for detecting and normalizing 3'-5' amplification bias in microarray sample data generated from a nucleic acid sample from a subject, the method comprising obtaining a biological sample from a subject, wherein the biological sample comprises a nucleic acid sample; amplifying the nucleic acid sample to generate one or more amplicons, wherein the nucleic acid sample is amplified with the aid of one or more probes; generating a nucleic acid sequence read for an individual amplicon among the one or more amplicons; for each individual amplicon among the one or more amplicons, calculating, with the aid of a computer processor, the extent of a 3' bias for a given probe among the one or more probes upon a comparison of a nucleic acid sequence of the given probe to a nucleic acid sequence of the individual amplicon generated in (c); and applying a normalization procedure to correct for the 3' bias for a given probe.

The nucleic acid may be an mRNA transcript. In some embodiments calculating the extent of the 3' bias further comprises determining the effective distance from the 3' end of the mRNA transcript and the given probe. In some embodiments calculating the extent of the 3' bias further comprises determining the effective distance from one or more sites or sequences in the mRNA transcript and the given probe. In some embodiments calculating the extent of the 3' bias further comprises calculating a distance or median weighted distance between the given probe and one or more downstream polyA sites or sequences within the mRNA transcript, wherein the weighted distance is determined by read counts associated with each polyA site in the mRNA transcript. In some embodiments calculating the extent of the 3' bias further comprises comparing variability of paired intensity profiles of two or more identical probes, wherein the intensity profiles are obtained from two or more independent sets of microarray data, wherein each microarray data set is generated from an identical biological sample.

Comparing variability of paired intensity profiles of two or more identical probes may further comprise performing a per-transcript alignment of probes within the mRNA transcript to calculate the effective distance. In some embodiments the normalization procedure further comprises generating a normalization target distribution. In some embodiments the normalization procedure further comprises quantile normalization, wherein probes are grouped into bins, and a quantile normalization is applied to each probe within each bin to normalize the median intensity of probes across a bin. In some embodiments the normalization procedure removes application bias from sample data.

Summarization methods may be applied to normalized probe intensities and used to improve detection of differential gene expression in the microarray sample data.

This disclosure, but not being part of the invention, also describes a method for the detection of heterogeneity present in microarray data, the method comprising generating hypothetical microarray data from a mixture of one or more samples in silico; generating one or models from the hypothetical microarray data; obtaining microarray data from a mixture of one or more samples performed in vitro; comparing the one or more models of (b) to the data obtained in and based upon the comparison, assessing the strength of the one or more models.

The strength of the one or more models may be determined by comparing mean squared error between the model generated and the data obtained. In some embodiments selecting a model generated determined by a predictive ability of the model. In some embodiments, the predictive ability is determined by comparing the model to experimental data. In some embodiments one or more models are used to improve selectivity and/ or sensitivity in the detection of heterogeneity in a sample.

This disclosure, but not being part of the invention, also provides for a method to identify a cancer in a biological sample from a subject, the method comprising obtaining a biological sample from the subject; assaying an expression level of one or more gene expression products in the biological sample; using one or more clinical classifiers to compare the expression level to a reference expression level of a plurality of genes of Table 1, Table 2, Table 3, Table 4, Table 5, Table 9, Table 10, Table 11, Table 12, Table 14, Table 15, Table 18, Table 19, Table 23, Table 24, Table 25, Table 26 and/or Table 27 to generate a comparison of expression levels, wherein the comparison is performed using an algorithm; classifying the biological sample as containing or not containing cancer and/or a specific tissue type based upon the comparison of the one or more clinical classifiers to yield a classification of the biological sample; and diagnosing and/or treating the subject based upon the classification.

This disclosure, but not being part of the invention, also provides for a method to identify a cancer in a biological sample from a subject, the method comprising obtaining a biological sample from the subject; assaying an expression level of one or more gene expression products in the biological sample; using one or more clinical classifiers to compare the expression level to a reference expression level of a plurality of genes of Table 24, Table 25, Table 26 and/or Table 27 to generate a comparison of expression levels, wherein the comparison is performed using an algorithm; classifying the biological sample as containing or not containing cancer and/or a specific tissue type based upon the comparison of the one or more clinical classifiers to yield a classification of the biological sample; and diagnosing and/or treating the subject based upon the classification.

In some embodiments the biological sample is classified as containing or not containing cancer further comprises a prediction of the presence or absence of a mutation associated with the cancer. In some embodiments the algorithm is trained or the algorithm comprises a linear SVM classifier.

In some embodiments the trained algorithm is trained using tissue samples, fine needle aspirations, or a combination thereof.

In some embodiments the biological sample is classified as containing or not containing whole blood using a clinical classifier comprising a plurality of genes selected from Table 11 or Table 12.

In some embodiments the cancer is thyroid cancer or lymphoma.

In some embodiments the cancer is thyroid cancer or lymphoma and the mutation associated with thyroid cancer or lymphoma is a BRAFV600E mutation. In some embodiments, the method further comprises classifying the sample as having an aggressive prognosis based upon said comparison.

In some embodiments the biological sample is classified as containing or not containing follicular tissue or cells using a clinical classifier comprising a plurality of genes selected from Table 14 or Table 15.

In some embodiments the biological sample is classified as containing or not containing thyroid cancer using a clinical classifier comprising a plurality of genes selected from Table 2, Table 9 or Table 10.

In some embodiments the biological sample is classified as containing or not containing cancer and/or a specific tissue type based upon the comparisons of the one or more clinical classifiers further provides an estimate of the proportion of cancer and/or specific tissue type in the sample.

In some embodiments the biological sample is obtained by needle aspiration, fine needle aspiration, core needle biopsy, vacuum assisted biopsy, large core biopsy, incisional biopsy, excisional biopsy, punch biopsy, shave biopsy, or skin biopsy. In some embodiments the biological sample is a fine needle aspiration of thyroid tissue.

In some embodiments the expression level is assayed by microarray, SAGE, blotting, RT-PCR, sequencing, and/or quantitative PCR.

In some embodiments the gene expression product is RNA, mRNA, rRNA, tRNA, or miRNA.

In some embodiments at least one of the gene expression product corresponds to a gene over-expressed in the cancer.

In some embodiments method differentiates cancer containing samples from non cancer containing samples with at least 95%, 99%, or 100% accuracy.

In some embodiments, the method is used to pre-screen biological samples prior to classifying with one or more clinical classifiers.

In some embodiments the method reduces the rate of false positives returned by the clinical classifiers.

In some embodiments one or more clinical classifiers are used as a diagnostic for thyroid cancer.

In some embodiments the method subject is treated with surgery.

In some embodiments the biological sample is classified as containing or not containing lymphoma using a clinical classifier comprising a plurality of genes selected from Table 1.

In some embodiments the biological sample is classified by two or more the clinical classifiers above or elsewhere herein, which are used for classifying the biological sample as containing or not containing a disease (e.g., cancer) and/or a specific tissue type.

This disclosure, but not being part of the invention, also provides a method to identify blood or follicular tissue in a biological sample from a subject, the method comprising obtaining a biological sample from the subject; assaying an expression level of one or more gene expression products in the biological sample; using one or more clinical statistics to compare the expression level to a reference expression level of a plurality of genes of Table 11 or Table 12, Table 14 or Table 15 to generate a comparison of expression levels, wherein the comparison is performed using an algorithm; classifying the biological sample as containing blood or follicular tissue or not containing blood or follicular tissue based upon the comparison of the one or more clinical statistics to yield a classification of the biological sample; and diagnosing and/or treating the subject based upon the classification.

In some embodiments the biological sample is obtained by needle aspiration, fine needle aspiration, core needle biopsy, vacuum assisted biopsy, large core biopsy, incisional biopsy, excisional biopsy, punch biopsy, shave biopsy, or skin biopsy.

In some embodiments the biological sample is a fine needle aspiration of thyroid tissue. In some embodiments the expression level is assayed by microarray, SAGE, blotting, RT-PCR, sequencing, and/or quantitative PCR.

In some embodiments the gene expression product is RNA, mRNA, rRNA, tRNA, or miRNA.

In some embodiments the method differentiates blood or follicular tissue containing samples from non blood containing samples with at least 95%, 99%, or 100% accuracy.

In some embodiments the method reduces the rate of false positive identification of non-blood tissue.

Another aspect of the present disclosure, but not being part of the invention, describes machine executable code that, upon execution by one or more computer processors, implements any of the methods above or elsewhere herein.

Another aspect of the present disclosure, but not being part of the invention, describes a computer system comprising one or more computer processors and a memory location coupled to the one or more computer processors.

The memory location comprises machine executable code that, upon execution by the one or more computer processors, implements any of the methods above or elsewhere herein.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

## BRIEF DESCRIPTION OF THE DRAWINGS

**Figure 1 (A-C)** are flow charts illustrating exemplary embodiments (A&B) and an exemplary system architecture (C).

**Figure 2** is a table that lists 16 biomarker panels that can be used to diagnose a thyroid condition.

**Figure 3** is a table that lists 7 classification panels that can be used to diagnose a thyroid condition. Classifier 7 is at times herein referred to as "main classifier."

**Figure 4 (A-H)** is a table that lists biomarkers that can be assigned to the indicated classification panel.

**Figure 5** illustrates an exemplary kit.

**Figure 6** depicts a computer useful for displaying, storing, retrieving, or calculating diagnostic results from the methods disclosed herein; displaying, storing, retrieving, or calculating raw data from genomic or nucleic acid expression analysis; or displaying, storing, retrieving, or calculating any sample or customer information.

**Figure 7** depicts a computer control systems that are programmed or configured to implement methods of the application.

**Figure 8** is a chart representing expression of SLC4A1. Benign (B-RNA) and malignant (M-RNA) thyroid tissue RNA or whole blood (SC-001 - SC-009).

**Figure 9** is a chart representing Expression of FPR2. Benign (B-RNA) and malignant (M-RNA) thyroid tissue RNA or whole blood (SC-001 - SC-009).

**Figure 10** is a chart representing expression of EMR3. Benign (B-RNA) and malignant (M-RNA) thyroid tissue RNA or whole blood (SC-001 - SC-009).

**Figure 11** is a chart representing intensity signals for marker FPR2 (pure and mixed). Observed in vitro (dots) and simulated mixture values (line) for marker FPR2 in thyroid and blood mixture experiments.

**Figure 12** is a chart representing comparison of estimated and in vitro designed proportions in the blood mixing study.

**Figure 13** is a chart representing distribution of estimated blood proportions in a cohort of 265 cytology indeterminate thyroid samples.

**Figure 14A** is a chart representing a distribution of the standardized residuals between model predictions and observed intensity values across all *in vitro* mixture samples for 142 classifier markers. Top panel (A) represents $M_0$ model predictions;

**Figure 14B** is a chart representing $M_1$ model predictions. Dashed gray lines are (-1,1) lines, which should contain ~65% of residuals if they are normally distributed.

**Figure 15** is a chart representing in silico simulations indicating approximate linear classifier scores with precision.

**Figure 16** is a chart representing in silico simulations indicating simulations are not linear in the space of classifier scores, yet approximate observed GEC scores with precision.

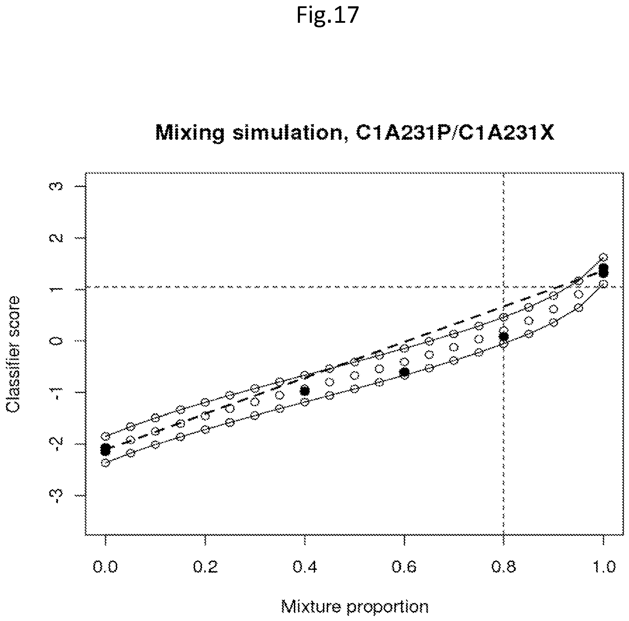

**Figure 17** is a chart representing in silico and in vitro GEC scores of pure thyroid PTC or mixtures using RNA from adjacent normal tissue. The blue dashed line represents classifier score predictions implied by the $M_1$ model (linear in log-2), black circles represent classifier score predictions implied by the $M_0$ model (linear in raw intensity space). Observed results for the mixed samples are shown as red dots. Although the predicted classifier scores for the mixtures using $M_0$ model are not linearly explained by the mixture proportion, *in silico* simulations using this model approximate the in vitro GEC scores with precision.

**Figure 18** reflects charts showing that unknown mixing proportions can be inferred by the invention. Prior (dotted lines) and posterior (solid lines) distributions of the mixing proportions estimated from observed data using the model $M_0$ and a beta prior for mixing proportion. While this is shown to work here in a study where the mixing proportion is known, it may be inferred that it is based on the observed data when the mixing proportion is not known.

**Figure 19** is a chart representing Frequency of known mutations (COSMIC database) across thyroid RNA-seq samples. Some samples are represented by multiple alignment files that have not been aggregated during data processing. It is preferred to aggregate prior to launching the mutation calling method. Except for BRAF, most mutations are detected in only a single sample (y-axis= number of alignment files with a mutation, x-axis= genes in which mutations are detected).

**Figure 20** is a chart representing distribution of COSMIC mutations detected per sample in thyroid PTC RNA-seq.

Mutations are detected using the methods of the invention and a cohort of benign (B) and malignant (M) thyroid samples that had already been characterized with respect to their BRAF V600E mutation status (BRAF Positive, or BRAF negative). The genes listed within the bars of the graph are as follows: RB1, FT140, RB1, FTM3, DYNCIH1, ITM2C, ILRRN3, MFAP1A, SHPRH, TP53, TRIM24, VLDLR, PCYT1A, AP1M1, POLR2I, SUPT5H, BRAF, EGFR, FITM3, ZNF507, IFITM3, PIK3CA, HIST1H4B, MDN1, RIN2, ACADSB, BAP1, PDPK1, APC, PTCH1, STAMBP, PRRG1, APBB1IP, C6ORF106, GALNT12, ATP9B, IFT122, FXYD6, FXYD6-FXYD2, LRRK1, ASXL1, ATM, BRPF3, LAMC1, CAD, EPS8, GGA3, SENP3, CCDC132, NF2, SENP3, TRRAP, C18ORF1, LRP1, and OTX1

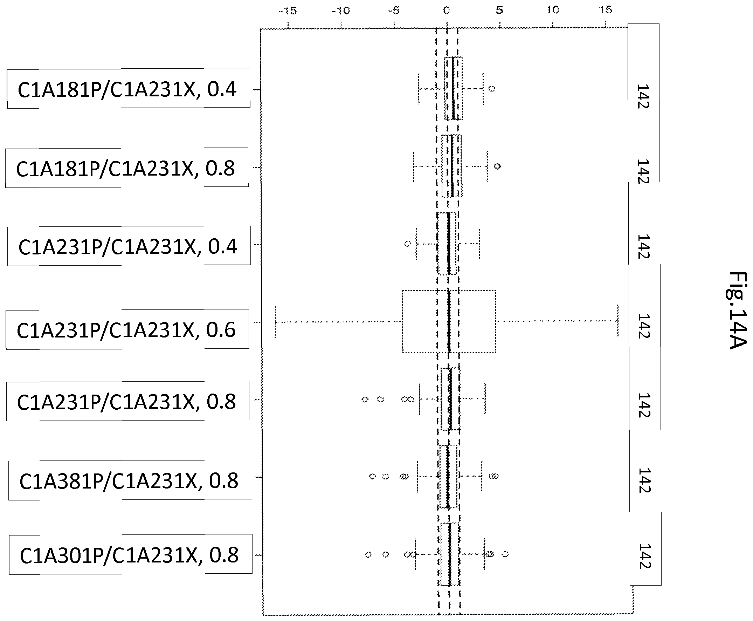

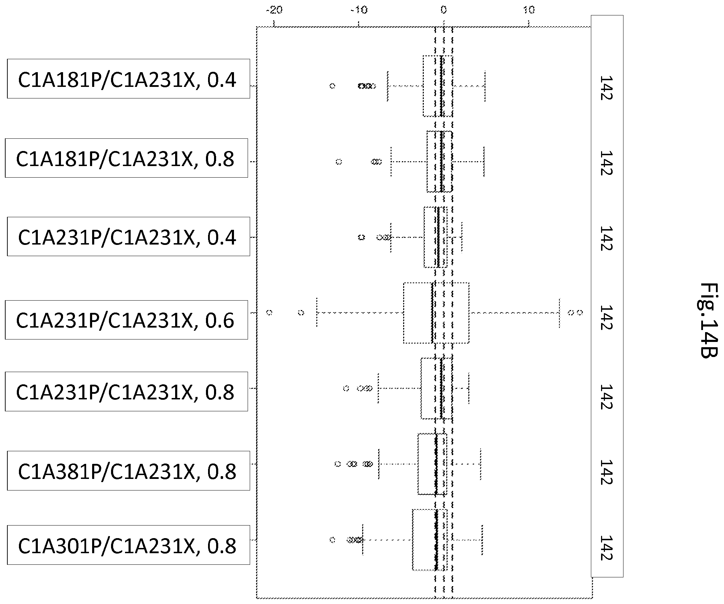

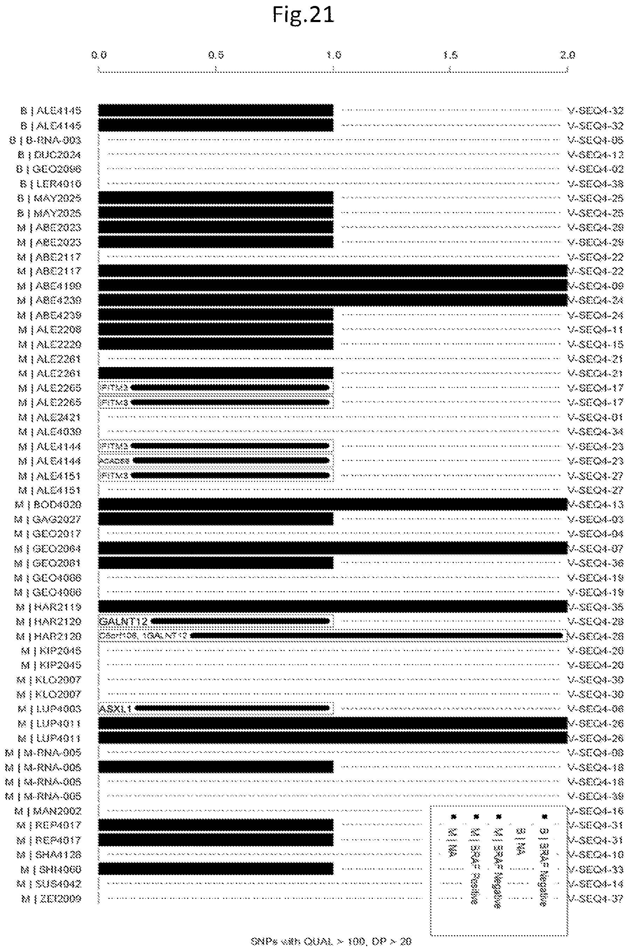

**Figure 21** is a chart representing distribution of COSMIC mutations detected per sample in thyroid PTC using RNA-seq. The distribution is similar as in figure 14, except more stringent data quality requirements are applied. The genes listed within the bars of the graph are as follows: RB1, IFITM3, PCYT1A, BRAF, EGFR, ACADSB, PDPK1, STAMBP, APBB1IP, GALN12, C6ORF106, ASXL1, BRPF3, and EPS8.

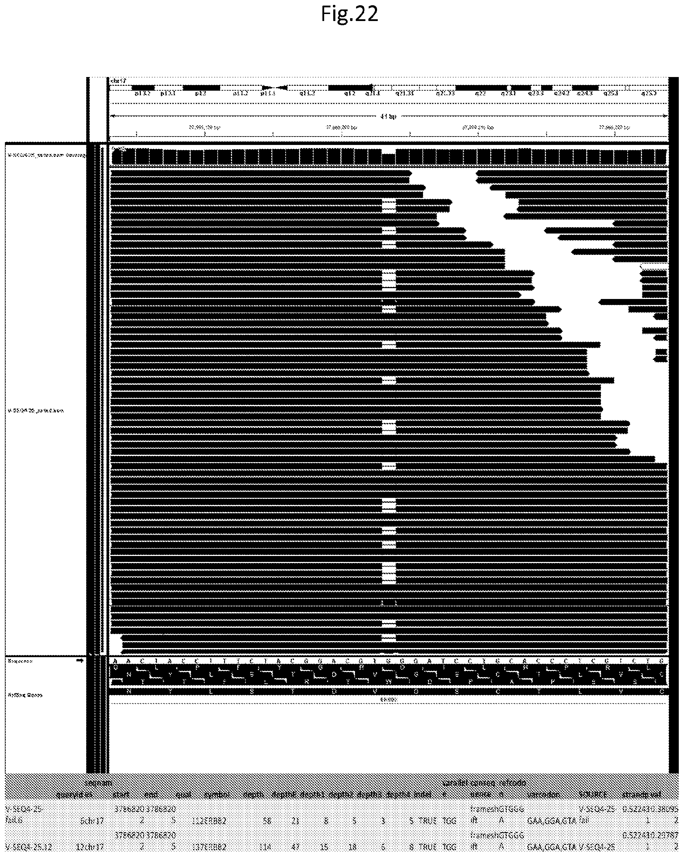

**Figure 22** is a chart representing a ERBB2 deletion (white gap within highlighted column) in a BRAF- sample.

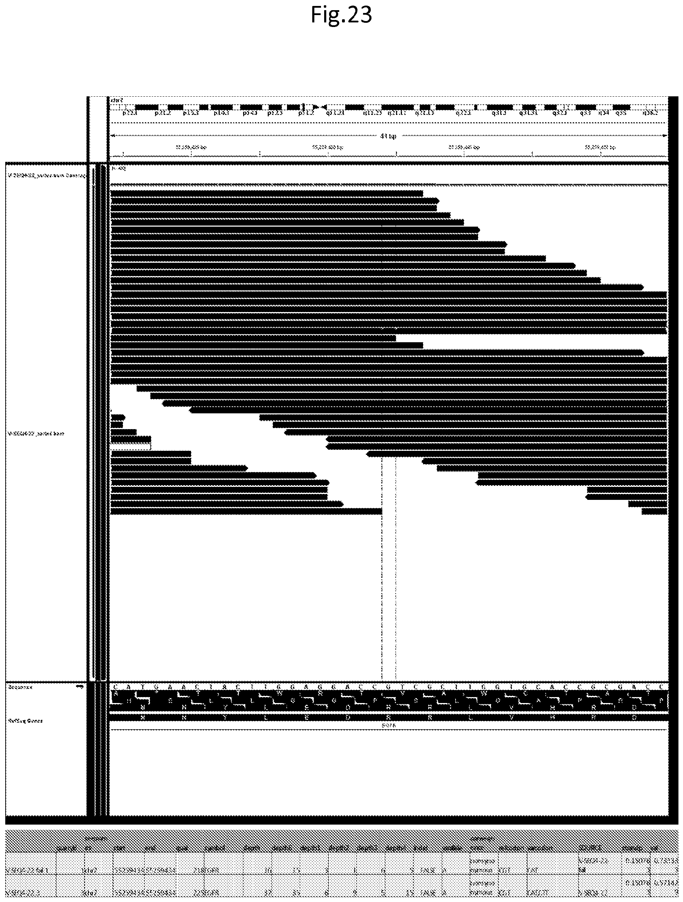

**Figure 23** is chart representing a EGFR point mutation in a BRAF+ thyroid PTC sample.

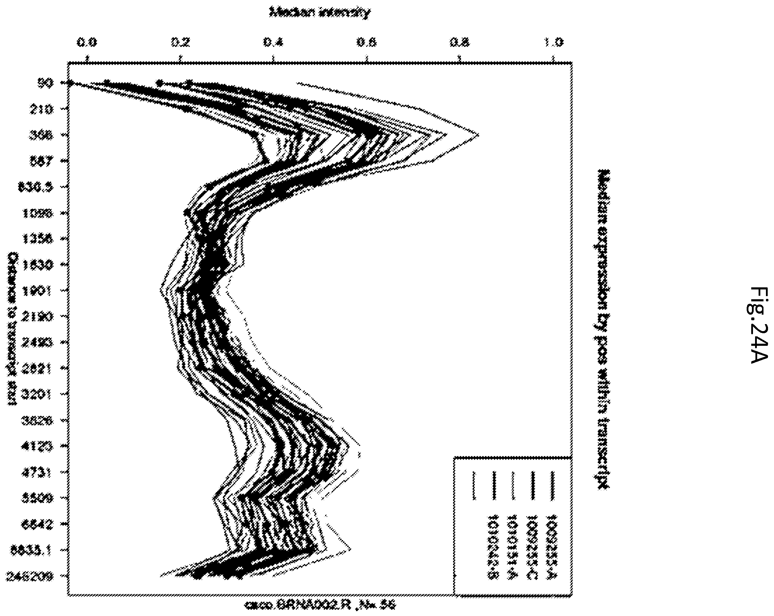

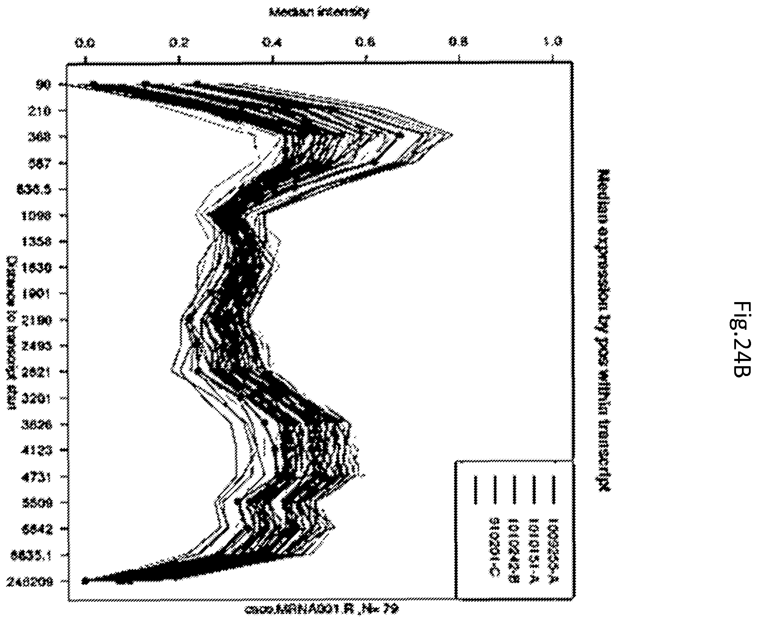

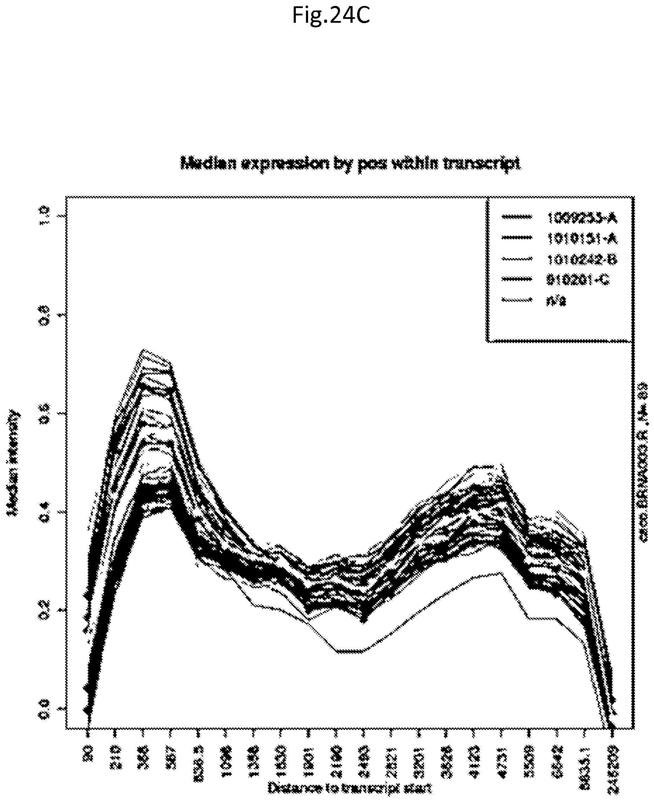

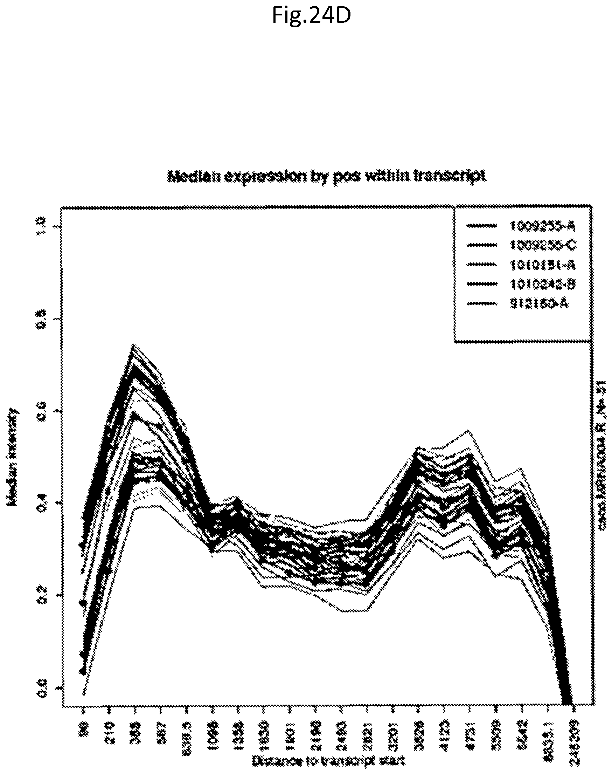

**Figure 24** represents charts indicating median probe intensity signals which show reagent-specific batch effects and vary systematically as a function of the distance of the probe from the transcript start. Each panel represents control RNA from a single sample tested multiple times using different reagent lots with a whole transcriptome amplification system and the extent of the variation observed even within the same lot of reagents. All control RNAs shown are prepared from a single source of frozen human thyroid tissue blocks (shown are control samples from two benign and two malignant nodules). RNA extractions for each control sample are performed at one time, and multiple batches of eluted RNA are immediately pooled, mixed, and then aliquoted into single use vials.

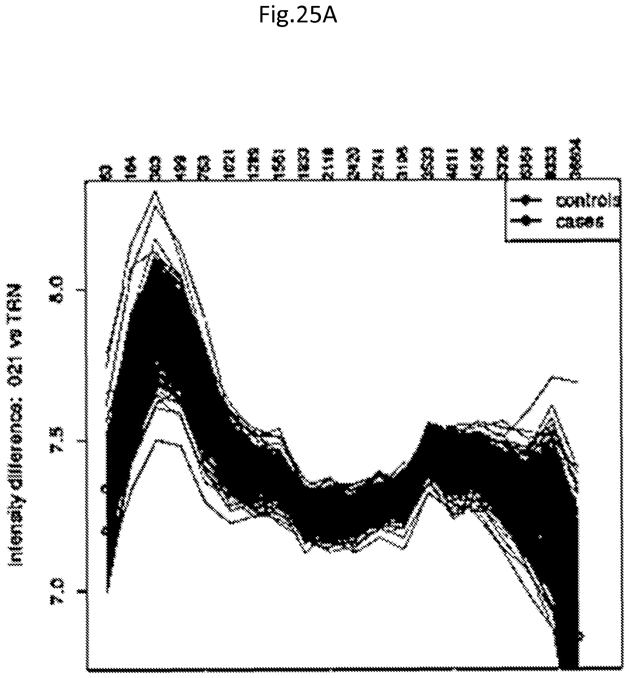

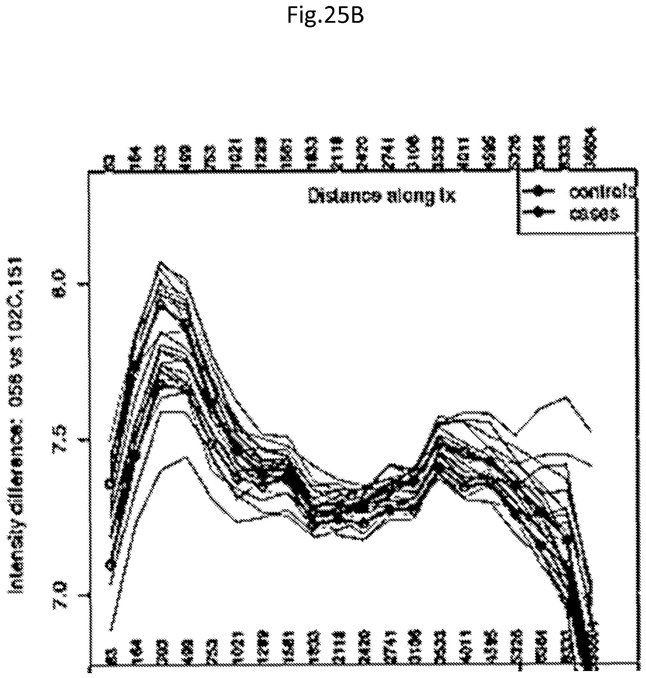

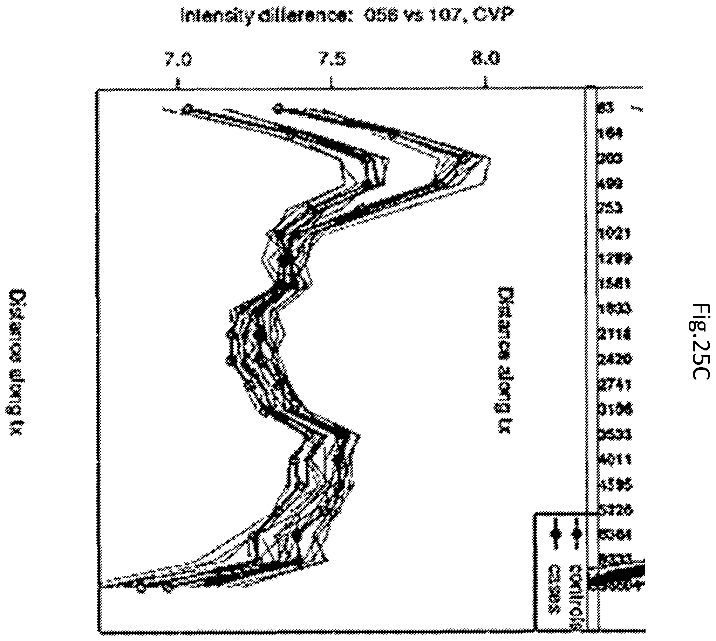

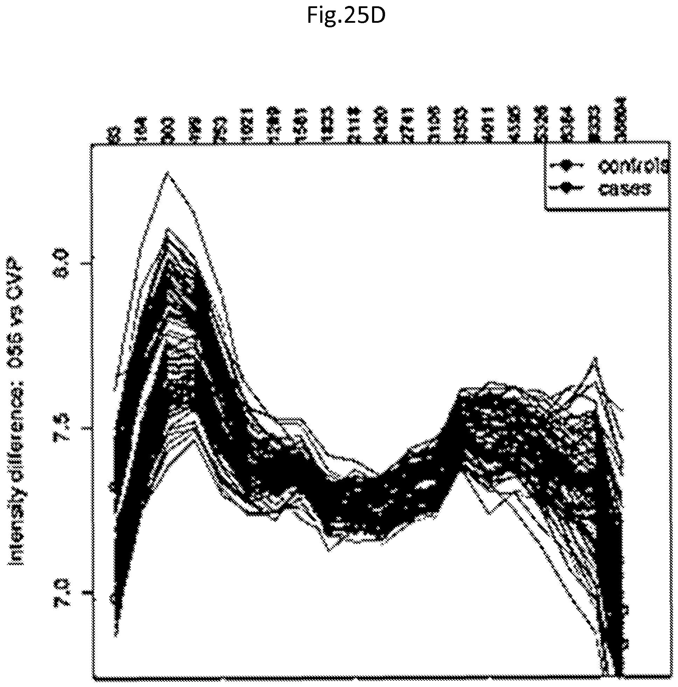

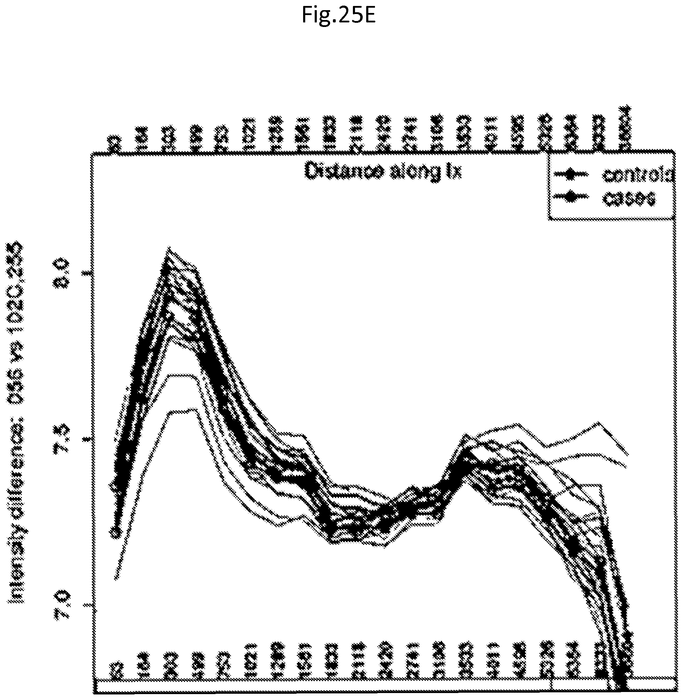

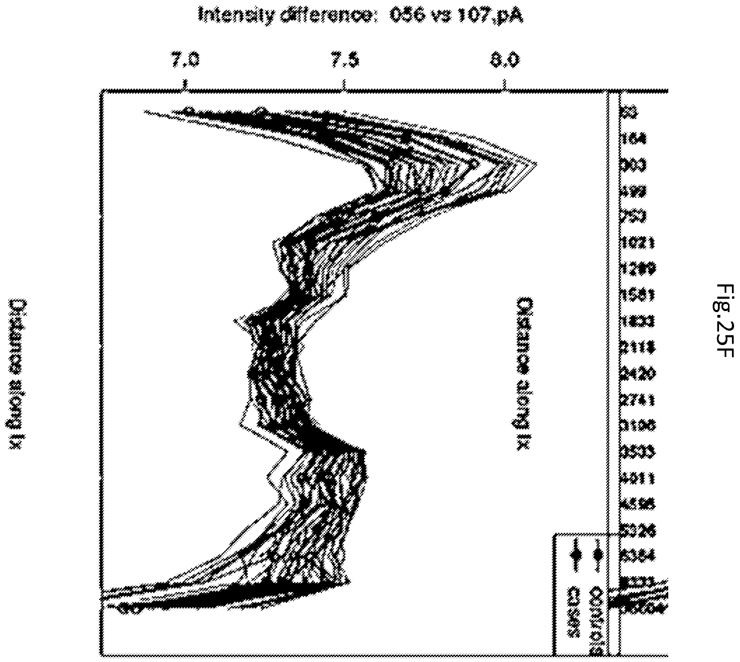

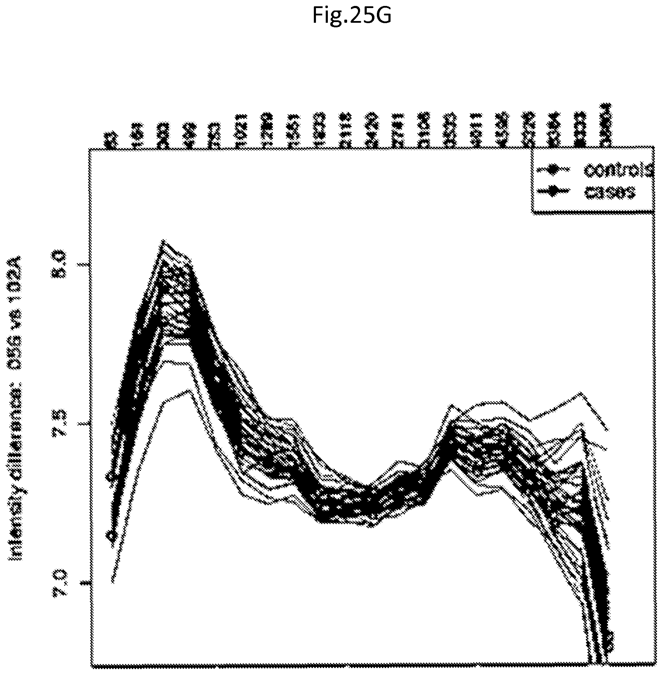

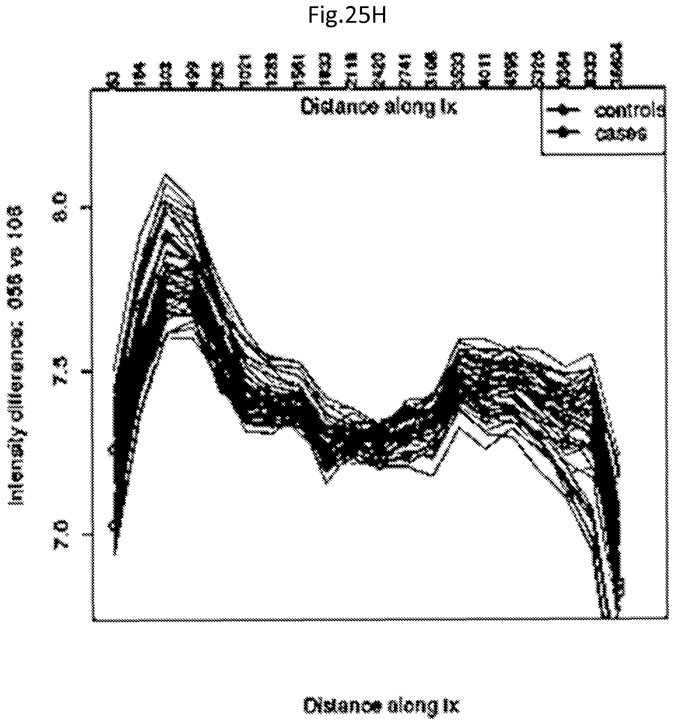

**Figure 25** represents charts indicating signals differ as a function of probe distance from transcript start. Probe intensity signals differ for any given cohort of samples tested in two separate experiments and show reagent-specific batch effects that vary systematically as a function of the distance of the probe from the transcript start. Each panel represents the difference in intensity signals observed for a cohort of samples that is tested using a single lot of whole-transcriptome amplification reagents compared to the same cohort of samples tested using a different lot of these reagents.

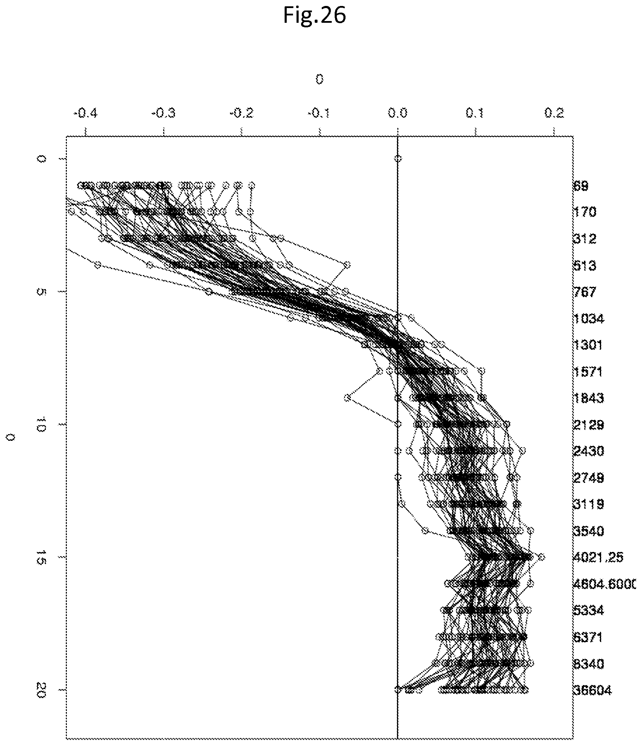

**Figure 26** represents a chart indicating normalized probe intensity residuals. Examples of the residuals of normalized probe intensities stratified by distance to the 3' end of gene transcripts. The residuals are defined as pair-wise differences for each probe's intensity values obtained for the same biological sample in two different experimental batches. Not every transcript shows this distribution of values. Each line represents probe intensities from a unique patient sample; each dot represents median residuals for all probes falling into a specific bin of 3' distances. Distance from the 3' end has been grouped into bins of varying number of nucleotides (x-axis), each containing 5% of all probes on the array.

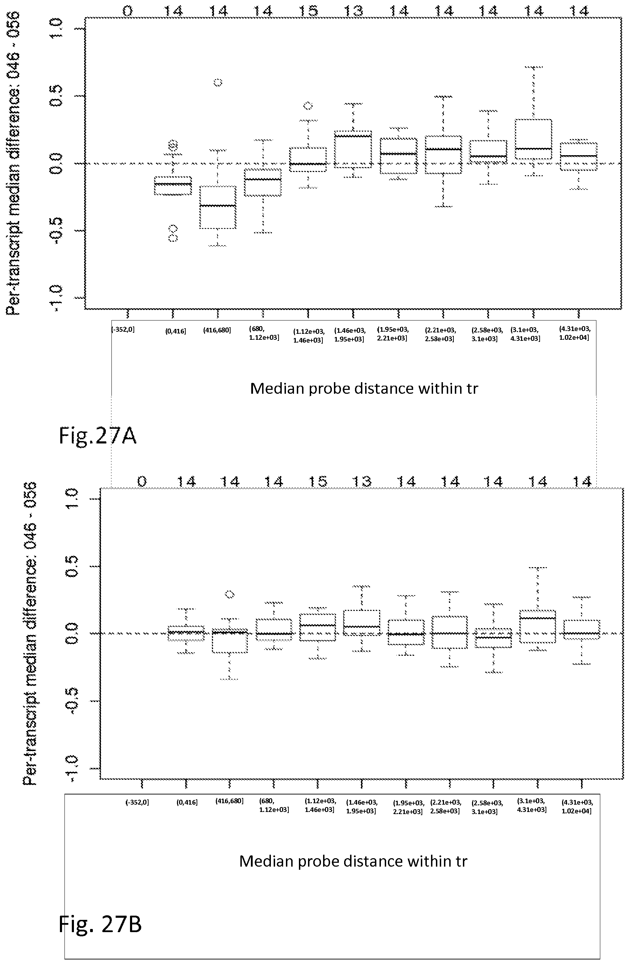

**Figures 27A** and **27B** represent probe position affects probe intensity residuals. Magnitude of median residuals is shown by median probe position within a transcript before data transformation (Fig. 27 A), and after transformation (Fig. 27 B). Applying a data-derived correction factor normalizes the 3' bias effect and results in enhanced reproducibility between experiments.

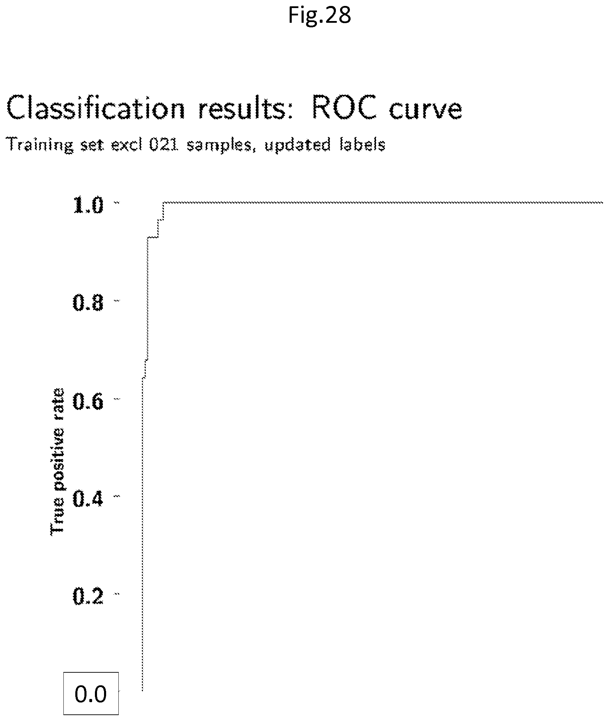

**Figure 28** represents a chart of classification performance using a BRAF mRNA signature spanning multiple thyroid subtypes. ROC curve using the top 30 genes (ranked by FDR p-value) in BRAF+ vs. BRAF- comparison.

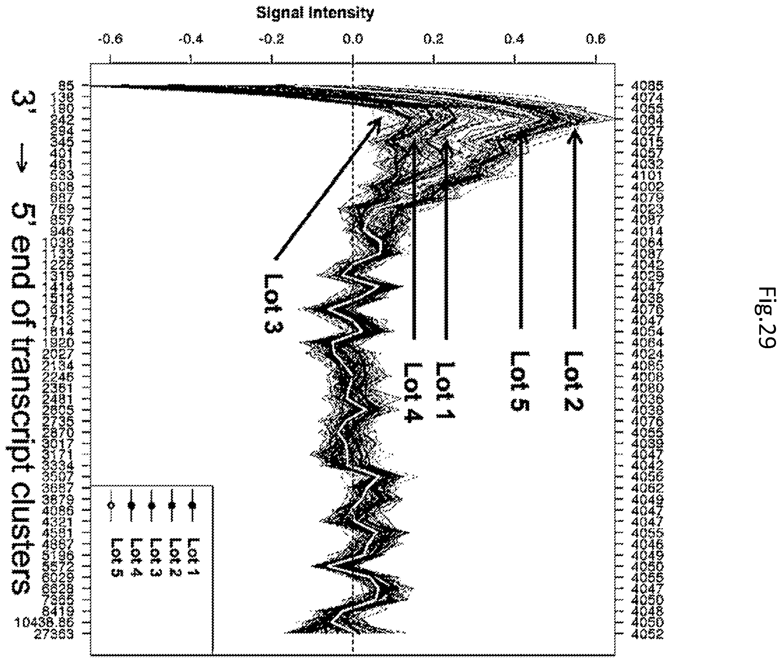

**Figure 29** represents a chart of probeset intensity values that vary along transcripts in a reagent lot dependent manner. The chart provides an example of normalized intensity values for a cohort of samples averaged across multiple transcript clusters. Five distinct WTA and microarray reagent lots are used. The lot-to-lot differences are primarily situated at the 3-prime ends of transcripts.

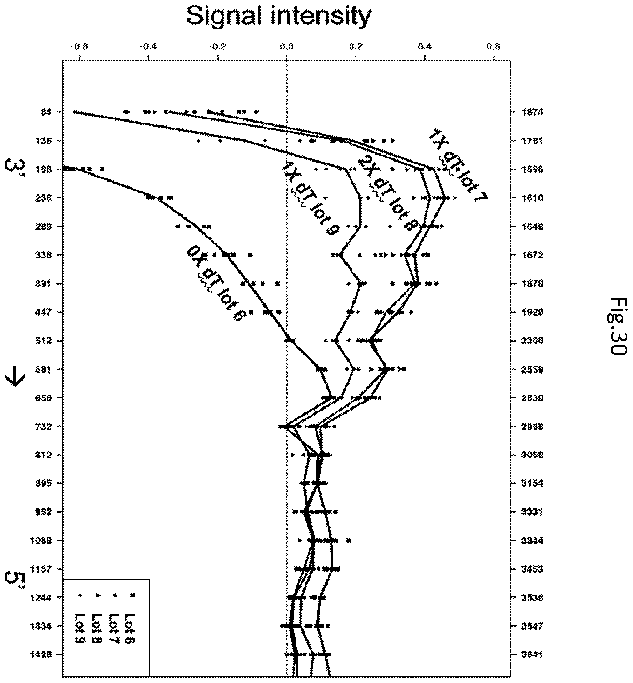

**Figure 30** represents a chart of dose response of signal intensity profiles to poly-dT primer concentration. The chart provides an example of signal intensity values averaged across multiple transcript clusters. When the relative concentration of poly-dT in the WTA kit is increased (2x dT, lot 8) or eliminated (0x dT lot 6), relative to the normal/control condition (1x dT lot 7) using custom formulated primer mixes, differences in signal intensity at the 3' end, but not 5' end, of transcripts is observed. While the poly-dT components in both 1x dT lot 7 and 1x dT lot 9 have identical formulations, each amplification batch gave rise to distinct results.

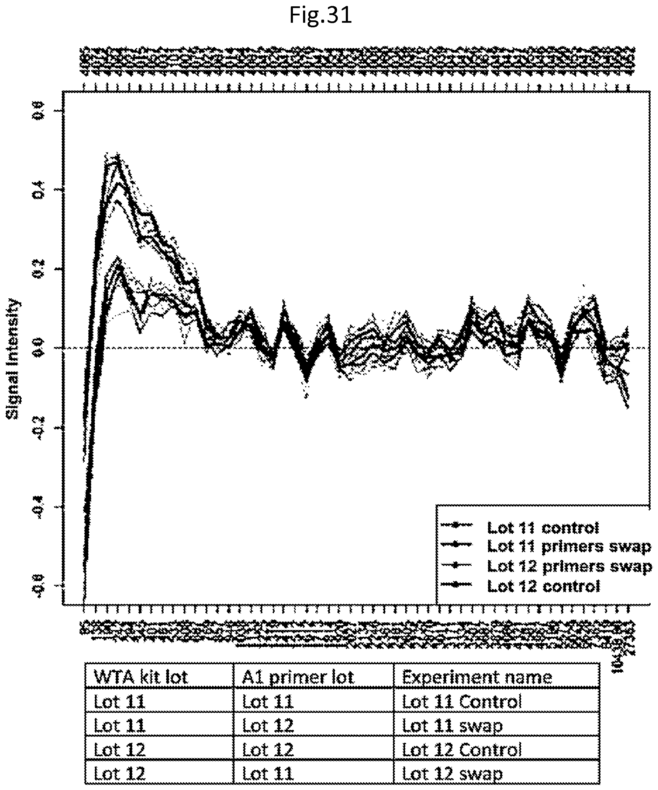

**Figure 31** represents a chart results of a poly-dT primer swap experiment. The poly-dT primers from two WTA kit lots previously showing different 3-prime bias profiles (black and blue lines) are swapped (red and green lines) and control RNA reprocessed in order to assess whether this specific reagent is responsible for the observed variation. The results clearly show that the A1 poly-dT primer component in each kit accounted for most of the observed variation in these experiments.

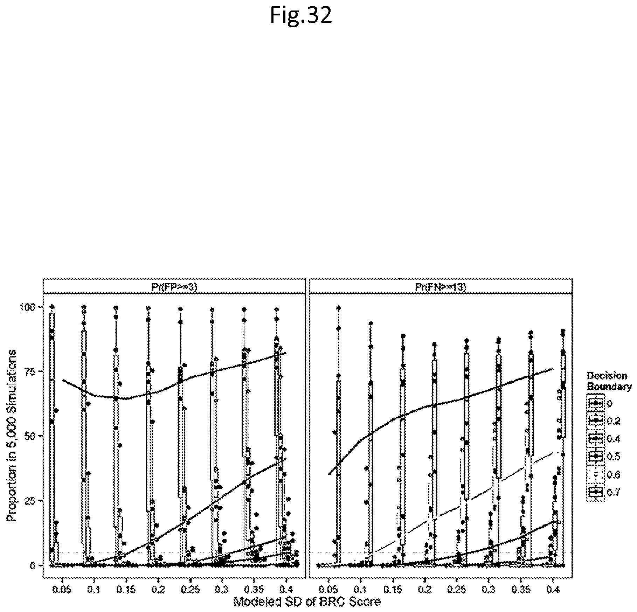

**Figure 32** represents a chart of the distribution of the proportion of simulations (y-axis) at varying levels of score reproducibility (x-axis) with more than three false positives (left) and more than 13 false negatives (right) at each of several candidate decision boundary values. The horizontal dotted line indicates a risk threshold of 5%.

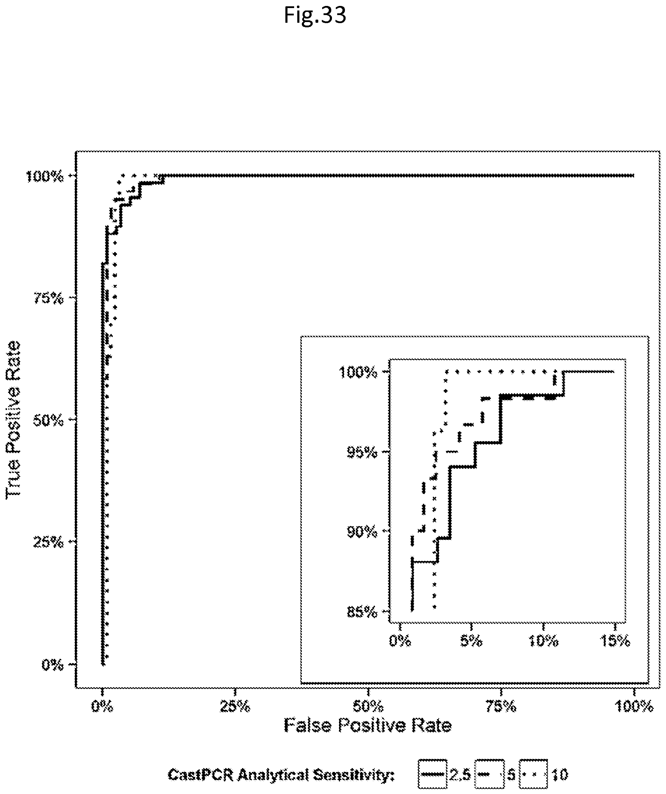

**Figure 33** represents a chart of receiver operator characteristic curves for Afirma BRAF classifier on training data under 10-fold cross-validation at three different thresholds for BRAF V600E-positivity by castPCR. Inset plot shows more detail of the upper-left hand corner of the ROC curve indicating a relative lack of separation between ROC curves depending upon castPCR threshold used.

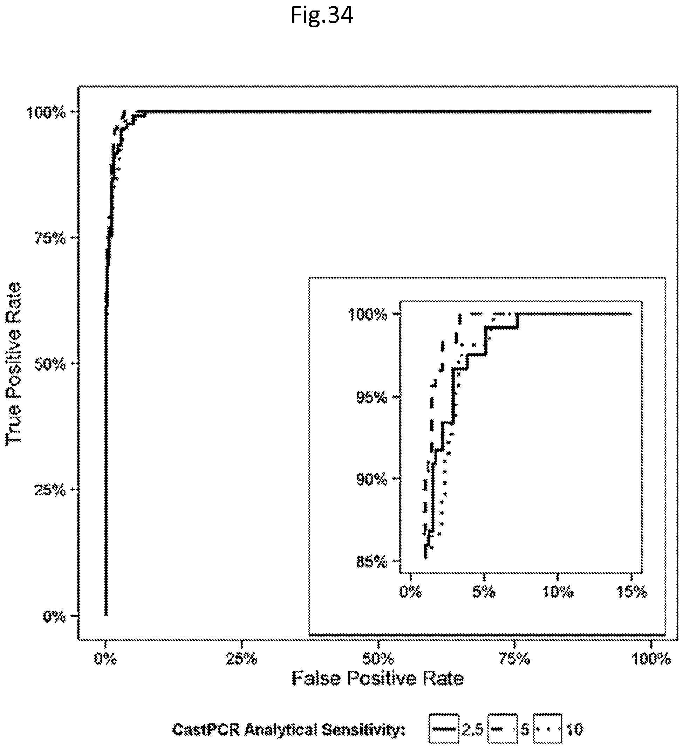

**Figure 34** represents a chart of ROC curves for Afirma BRAF performance on the test set at three different thresholds for BRAF V600E-positivity by castPCR. Inset plot shows more detail of the upper-left hand corner of the ROC curve indicating a relative lack of separation between ROC curves depending upon castPCR threshold used.

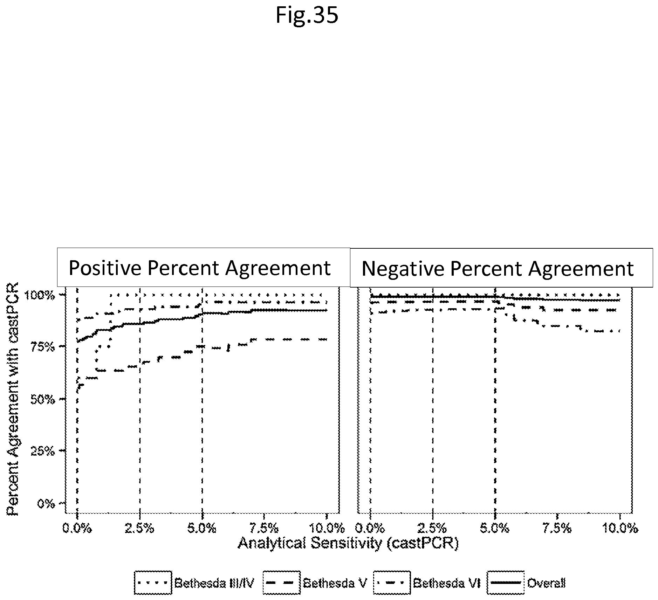

**Figure 35** represents a chart result of positive percent agreement (left, PPA) and negative percent agreement (right, NPA) by cytology category of Afirma BRAF calls with castPCR calls, varying the castPCR % MUT threshold.

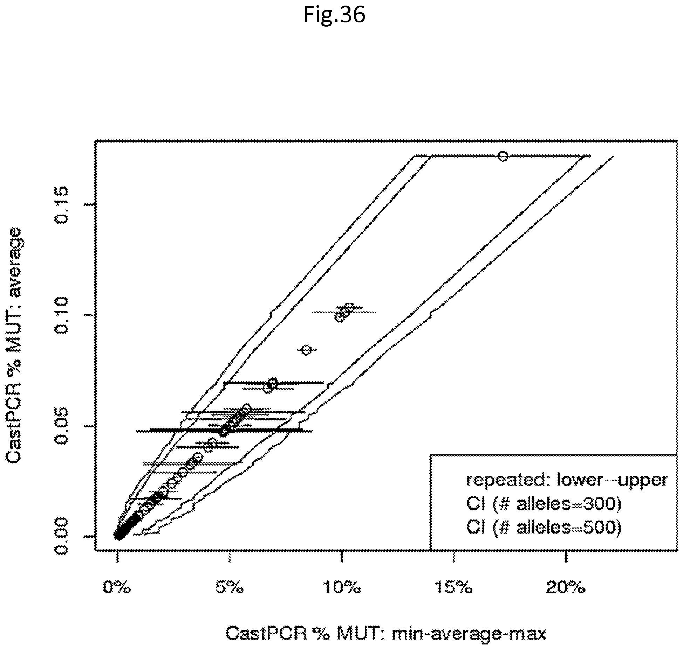

**Figure 36** represents a chart of minimum, average and maximum values (x-axis) for samples with castPCR results between 0% and 10% by average castPCR result (y-axis). Blue and green lines denote binomial confidence intervals bounding expected variability at various inferred underlying sample allele counts.

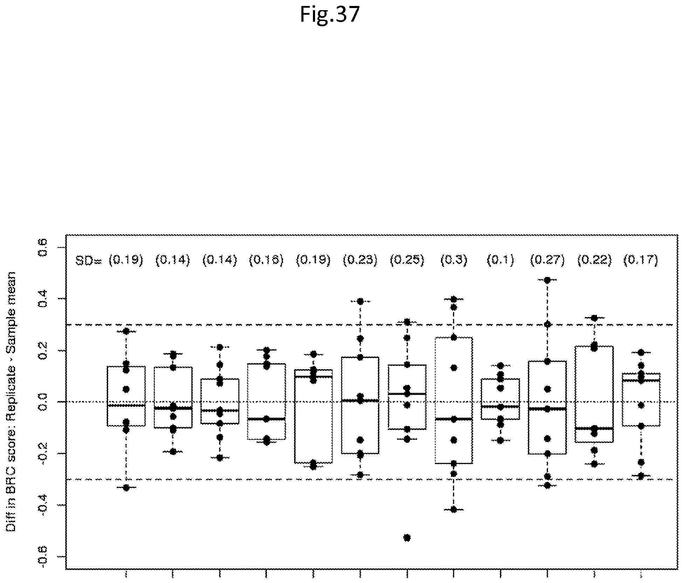

**Figure 37** represents a chart result of differences in Afirma BRAF scores from each sample's mean score (y-axis) for three tissue controls (first three boxplots) and nine FNABs (last 9 boxplots, x-axis).

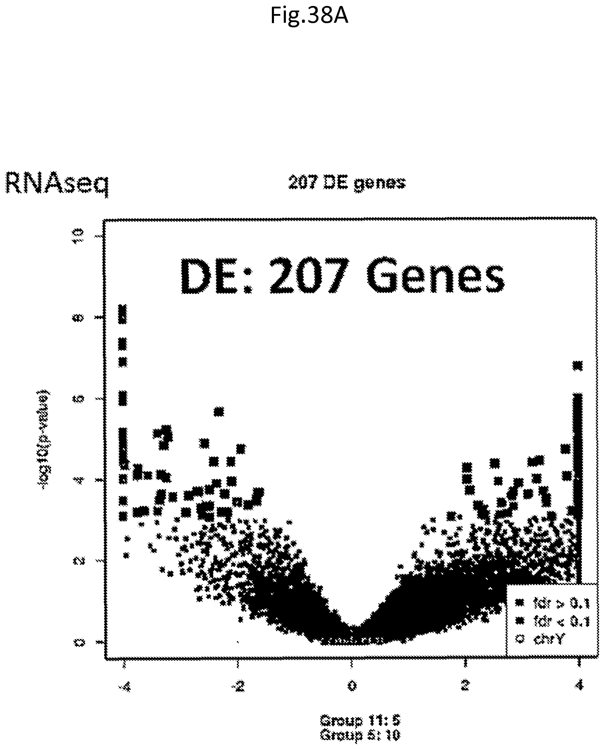

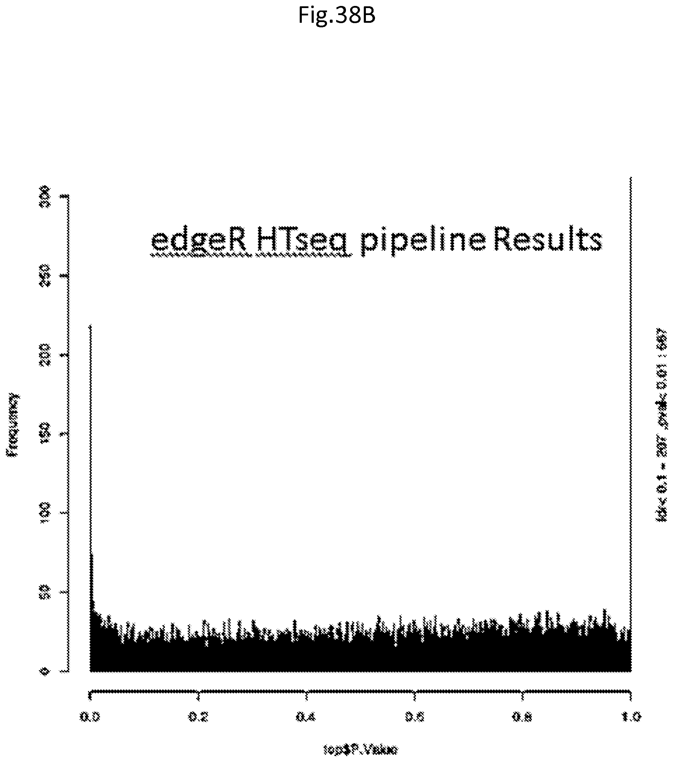

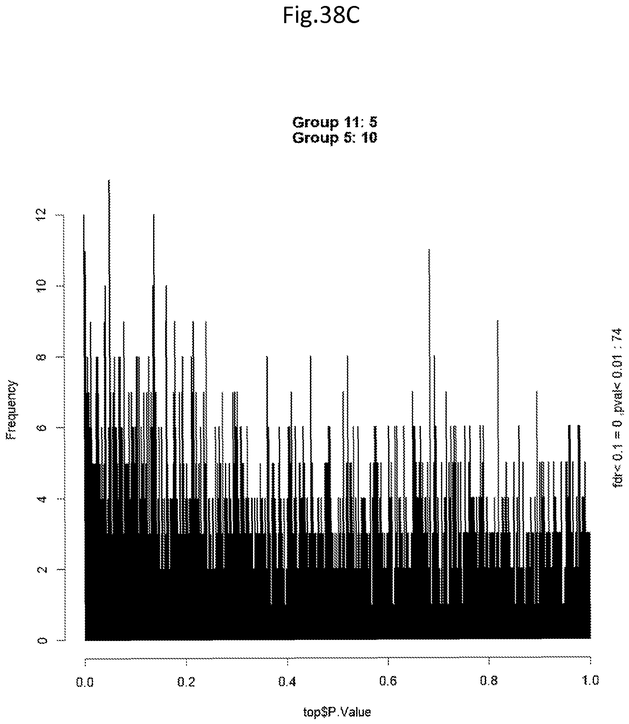

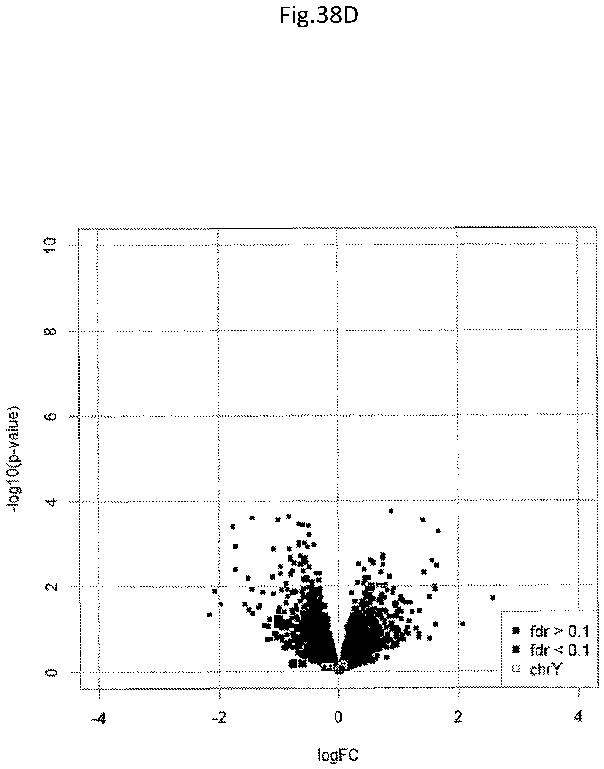

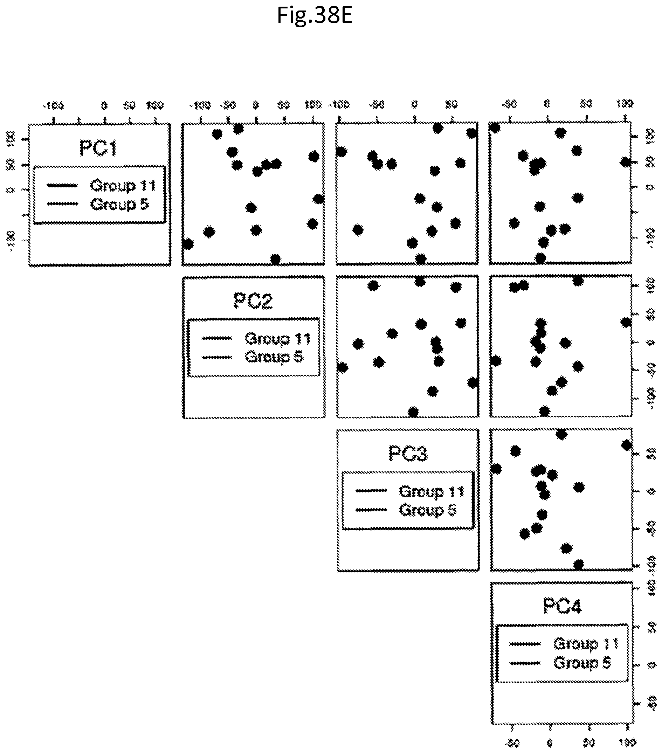

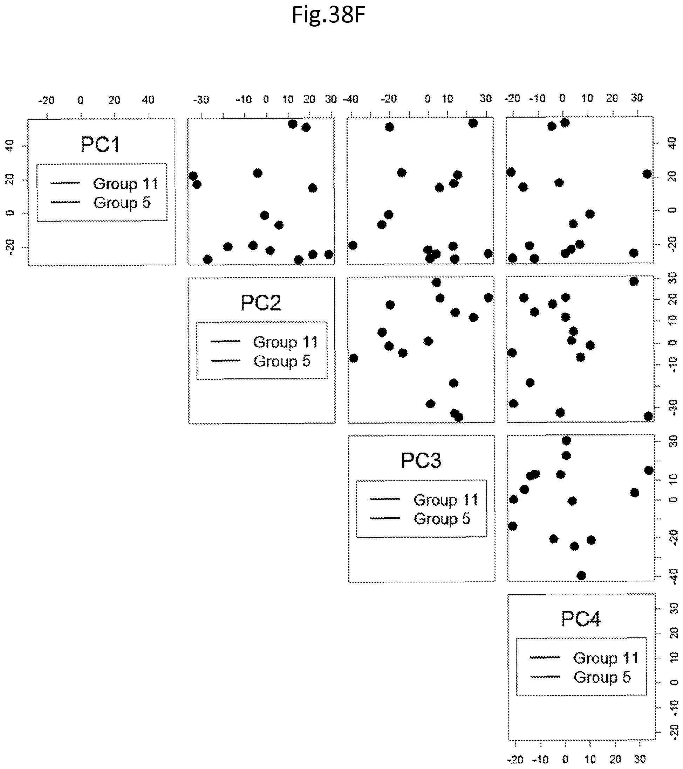

**Figure 38** represents charts of RNAseq, and Microarray results of BRAF+ aggressive or non-aggressive PTC samples, analyzed for differential expression using EdgeR and significance was established with an FDR p-value <0.1. 207 genes are differentially expressed between Aggressive and Not Aggressive BRAF+ samples.

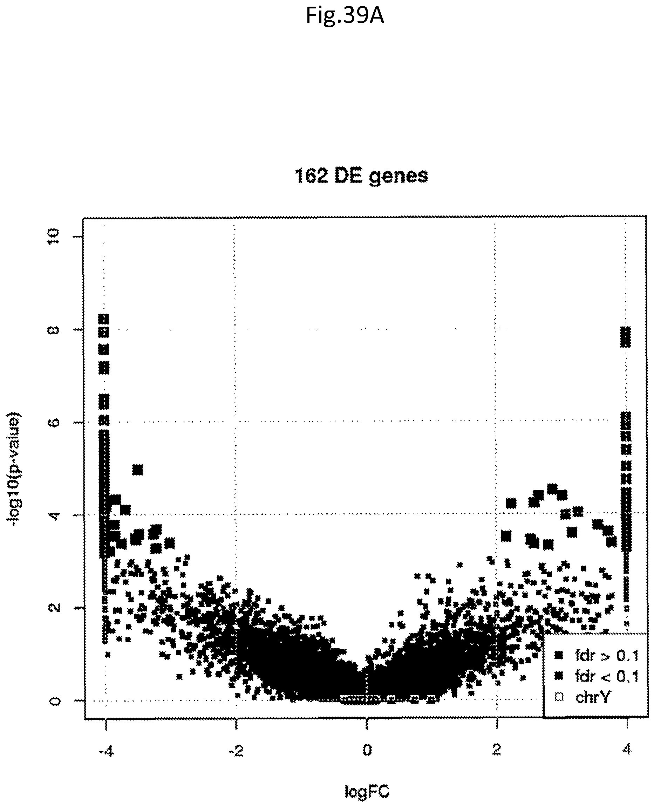

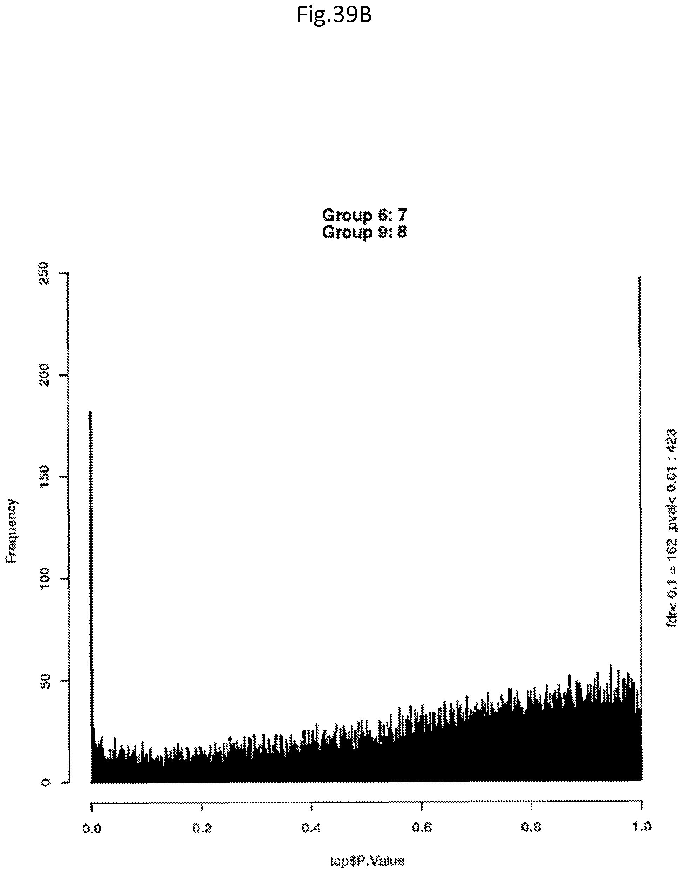

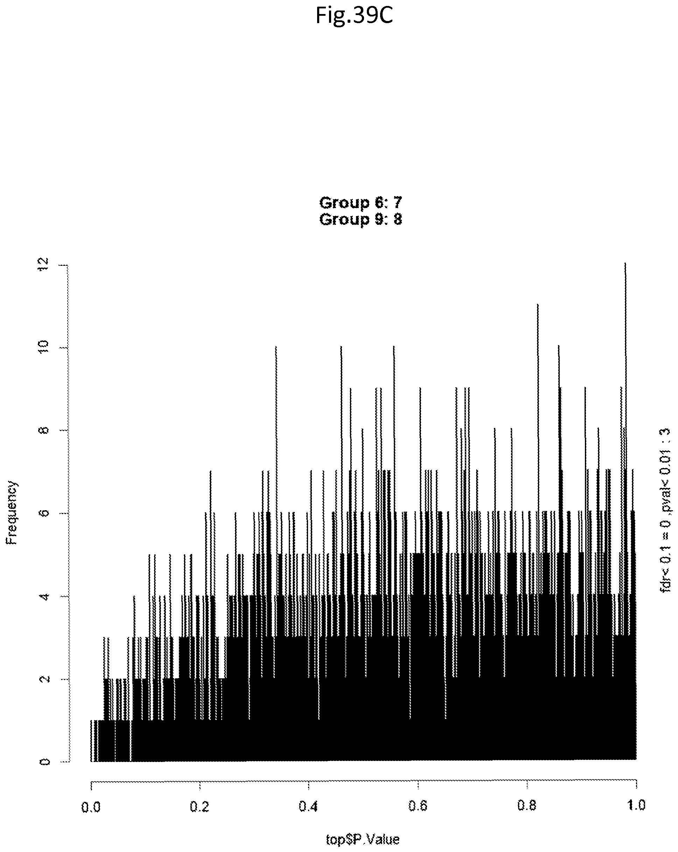

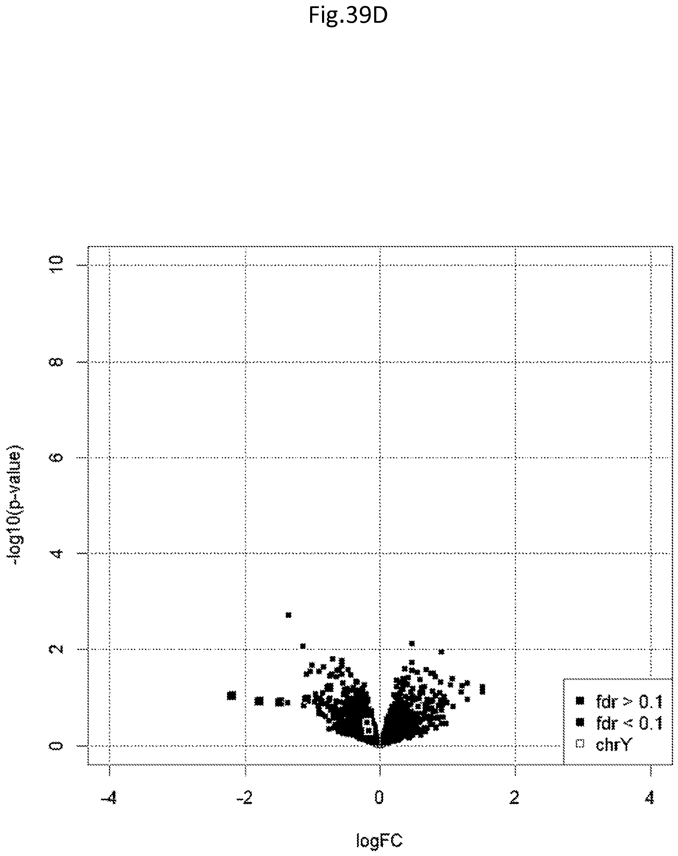

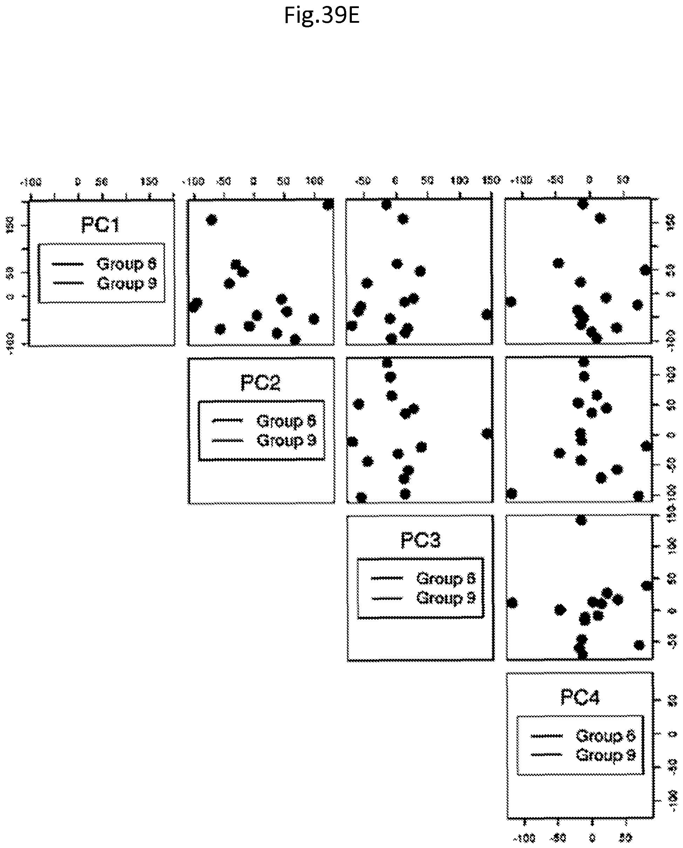

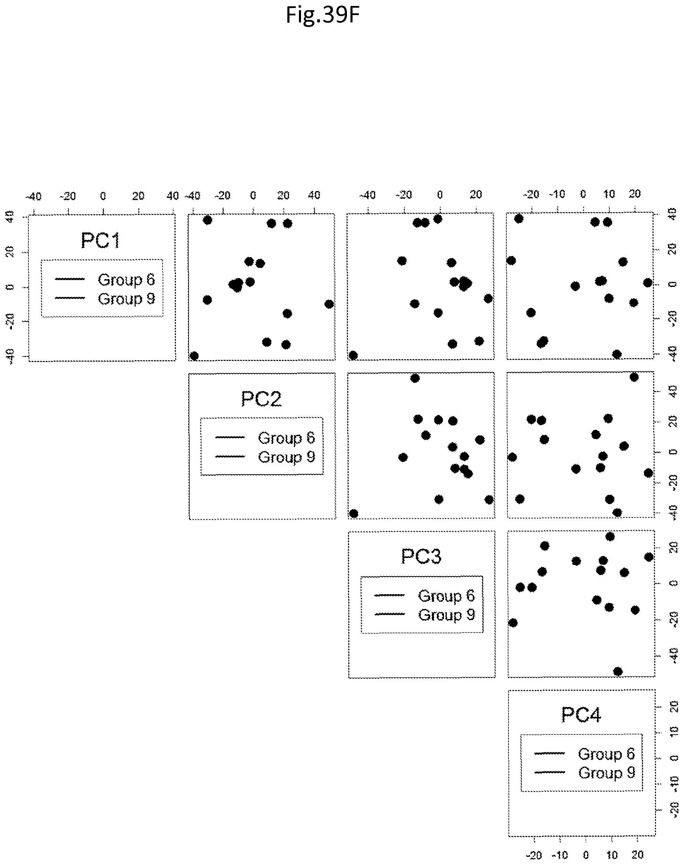

**Figure 39** represents charts of RNAseq, and Microarray results of BRAF-aggressive or non-aggressive PTC samples, analyzed for differential expression using EdgeR and significance was established with an FDR p-value <0.1. 162 genes are differentially expressed between Aggressive and Not Aggressive BRAF- samples.

## DETAILED DESCRIPTION

### I. Introduction

The present disclosure describes methods of identifying, classifying, or characterizing biological samples and related kits and compositions. The methods, and related kits and compositions, disclosed herein can be used for identifying abnormal cellular proliferation in a biological test sample. Methods of differentiating benign from suspicious (or malignant) tissue are provided, as well as methods of identifying definitive benign tissue. Sets of biomarkers useful for identifying benign or suspicious tissue are provided, as well as methods of obtaining such sets of biomarkers. For example, this disclosure provides novel classification panels that can be obtained from gene expression analysis of sample cohorts exhibiting different pathologies. This disclosure also provides methods of reclassifying an indeterminate biological sample (*e.g.*, surgical tissue, blood tissue, thyroid tissue, thyroid FNA sample, *etc.*) into a benign versus suspicious (or malignant) category. In some cases, this disclosure provides a "main classifier" obtained from expression analysis using panels of biomarkers, and that can be used to designate a sample as benign or suspicious (or malignant). This disclosure also provides a series of steps that can precede applying a main classifier to expression level data from a biological sample, such as a clinical sample. Such series of steps can include an initial cytology or histopathology study of the biological sample, followed by analysis of gene (or other biomarker) expression levels in the sample. In some embodiments, the cytology or histopathology study occurs before, concurrently with, or after the step of applying any of the classifiers described herein. The methods, kits, and compositions provided herein can also be used in predicting gender, predicting genetic mutations, and/or pre-screening the samples for the presence of a confounding condition prior to the application of the main classifier.

Expression levels for a sample can be compared to gene expression data for two or more different sets of biomarkers, the gene expression data for each set of biomarkers comprising one or more reference gene expression levels correlated with the presence of one or more tissue types, wherein the expression level is compared to gene expression data for the two or more sets of biomarkers in sequential fashion. Comparison of expression levels to gene expression data for sets of biomarkers can comprise the application of a classifier. For example, analysis of the gene expression levels can involve sequential application of different classifiers described herein to the gene expression data. Such sequential analysis can involve applying a classifier obtained from gene expression analysis of cohorts of diseased tissue, followed by applying a classifier obtained from analysis of a mixture of different biological samples, some of such samples containing diseased tissues and others containing benign tissue. The diseased tissue can be malignant or cancerous tissue (including tissue that has metastasized from another organ). The diseased tissue can be thyroid cancer or a non-thyroid cancer that has metastasized to the thyroid. The classifier can be obtained from gene expression analysis of samples hosting or containing foreign tissue (*e.g.*, a thyroid tissue sample containing parathyroid tissue). Griffith O. et al., J. clinical Oncology, 24, 2006, pp.5043-5051; Durand S. et al., J. clinical Endocrinology, 93, 2008, pp.1195-1202; WO2011143361 A2; WO2010056374 A2, Kebebew E. et al., Annals of surgery, 246,2007, pp.466-471, describe methods and products for evaluating thyroid cancer.

Classifiers used early in the sequential analysis can be used to either rule-in or rule-out a sample as benign or suspicious. Classifiers used in the sequential analysis can also be used to identify sample mix-ups; screen out samples that are inappropriate for the application of a main classifier; and/or to provide further diagnostic, theranostic, or prognostic information. In some embodiments, such sequential analysis ends with the application of a "main" classifier to data from samples that have not been ruled out by the preceding classifiers, wherein the main classifier is obtained from data analysis of gene expression levels in multiple types of tissue and wherein the main classifier is capable of designating the sample as benign or suspicious (or malignant).

Classifiers can also be used to pre-screen expression data derived from samples in order to determine whether it is appropriate to apply a main classifier to the samples. For example, a classifier can be applied to determine whether an individual sample fits a profile for the samples used to train the main classifier. A classifier can also be used to pre-screen samples to determine whether the sample contains a confounding condition. For example, a classifier can be used to pre-screen thyroid samples for the presence of non-thyroid cell types (*e.g.*, cancers that have metastasized from another tissue, *e.g.*, lymphomas). The use of pre-screening classifiers can reduce the percentage of false positives returned by the main classifier. Classifiers can also be used to screen expression data from samples in order to determine whether there has been a sample mix-up.

One example of a condition that can be identified or characterized using the subject methods is thyroid cancer. The thyroid has at least two kinds of cells that make hormones. Follicular cells make thyroid hormone, which affects heart rate, body temperature, and energy level. C cells make calcitonin, a hormone that helps control the level of calcium in the blood. Abnormal growth in the thyroid can result in the formation of nodules, which can be either benign or suspicious (or malignant). Thyroid cancer includes at least four different kinds of malignant tumors of the thyroid gland: papillary, follicular, medullary and anaplastic.

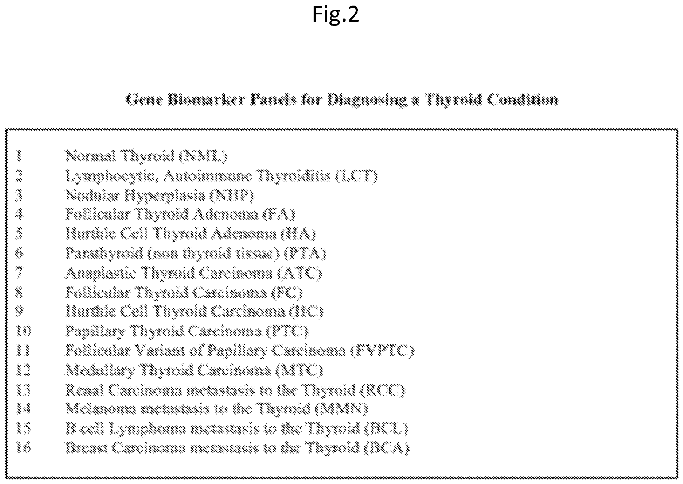

Expression profiling using panels of biomarkers can be used to characterize thyroid tissue as benign, suspicious, and/or malignant. Panels can be derived from analysis of gene expression levels of cohorts containing benign (non-cancerous) thyroid subtypes including follicular adenoma (FA), nodular hyperplasia (NHP), lymphocytic thyroiditis (LCT), and Hurthle cell adenoma (HA); malignant subtypes including follicular carcinoma (FC), papillary thyroid carcinoma (PTC), follicular variant of papillary carcinoma (FVPTC), medullary thyroid carcinoma (MTC), Hürthle cell carcinoma (HC), and anaplastic thyroid carcinoma (ATC). Such panels can also be derived from non-thyroid subtypes including renal carcinoma (RCC), breast carcinoma (BCA), melanoma (MMN), B cell lymphoma (BCL), and parathyroid (PTA). Biomarker panels associated with normal thyroid tissue (NML) can also be used in the methods and compositions provided herein. Exemplary panels of biomarkers are provided in Figure 2, and will be described further herein. Of note, each panel listed in Figure 2, relates to a signature, or pattern of biomarker expression (e.g., gene expression), that correlates with samples of that particular pathology or description.

The present disclosure also provides novel methods and compositions for identification of types of aberrant cellular proliferation through an iterative process (e.g., differential diagnosis) such as carcinomas including follicular carcinomas (FC), follicular variant of papillary thyroid carcinomas (FVPTC), Hurthle cell carcinomas (HC), Hurthle cell adenomas (HA); papillary thyroid carcinomas (PTC), medullary thyroid carcinomas (MTC), and anaplastic carcinomas (ATC); adenomas including follicular adenomas (FA); nodule hyperplasias (NHP); colloid nodules (CN); benign nodules (BN); follicular neoplasms (FN); lymphocytic thyroiditis (LCT), including lymphocytic autoimmune thyroiditis; parathyroid tissue; renal carcinoma metastasis to the thyroid; melanoma metastasis to the thyroid; B-cell lymphoma metastasis to the thyroid; breast carcinoma to the thyroid; benign (B) tumors, malignant (M) tumors, and normal (N) tissues. The present disclosure further provides novel gene expression markers and novel groups of genes and markers useful for the characterization, diagnosis, and/or treatment of cellular proliferation. Additionally, the present disclosure provides methods for providing enhanced diagnosis, differential diagnosis, monitoring, and treatment of cellular proliferation.

The present disclosure provides lists of specific biomarkers useful for classifying tissue (*e.g.*, thyroid tissue). However, the present disclosure is not meant to be limited solely to the specific biomarkers disclosed herein. Rather, it is understood that any biomarker, gene, group of genes or group of biomarkers identified through methods described herein is encompassed by the present disclosure.

All numbers expressing quantities of ingredients, reaction conditions, and so forth used in the specification are to be understood as being modified in all instances by the term "about." Accordingly, unless indicated to the contrary, the numerical parameters set forth herein are approximations that can vary depending upon the desired properties sought to be obtained.

In some cases, the method provides a number, or a range of numbers, of biomarkers (including gene expression products) that can be used to diagnose or otherwise characterize a biological sample. The number of biomarkers used can be between about 1 and about 500; for example about 1-500, 1-400, 1-300, 1-200, 1-100, 1-50, 1-25, 1-10, 10-500, 10-400, 10-300, 10-200, 10-100, 10-50, 10-25, 25-500, 25-400, 25-300, 25-200, 25-100, 25-50, 50-500, 50-400, 50-300, 50-200, 50-100, 100-500, 100-400, 100-300, 100-200, 200-500, 200-400, 200-300, 300-500, 300-400, 400-500, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, or any included range or integer. For example, at least about 1, 2, 3, 4, 5, 6, 7, 8, 9, 10, 15, 20, 25, 30, 33, 35, 38, 40, 43, 45, 48, 50, 53, 58, 63, 65, 68, 100, 120, 140, 142, 145, 147, 150, 152, 157, 160, 162, 167, 175, 180, 185, 190, 195, 200, 300, 400, 500 or more total biomarkers can be used. The number of biomarkers used can be less than or equal to about 1, 2, 3, 4, 5, 6, 7, 8, 9, 10, 15, 20, 25, 30, 33, 35, 38, 40, 43, 45, 48, 50, 53, 58, 63, 65, 68, 100, 120, 140, 142, 145, 147, 150, 152, 157, 160, 162, 167, 175, 180, 185, 190, 195, 200, 300, 400, 500, or more.

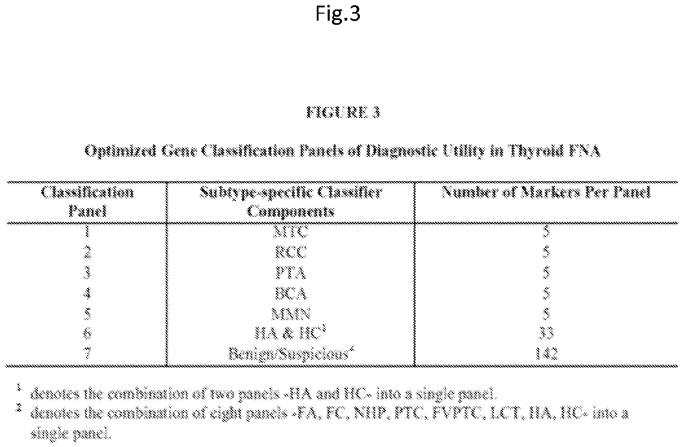

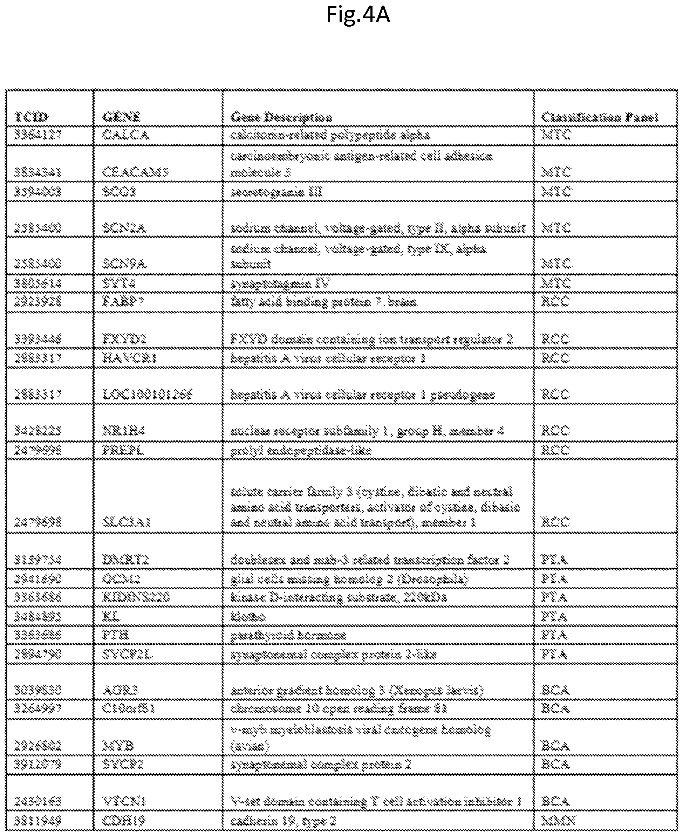

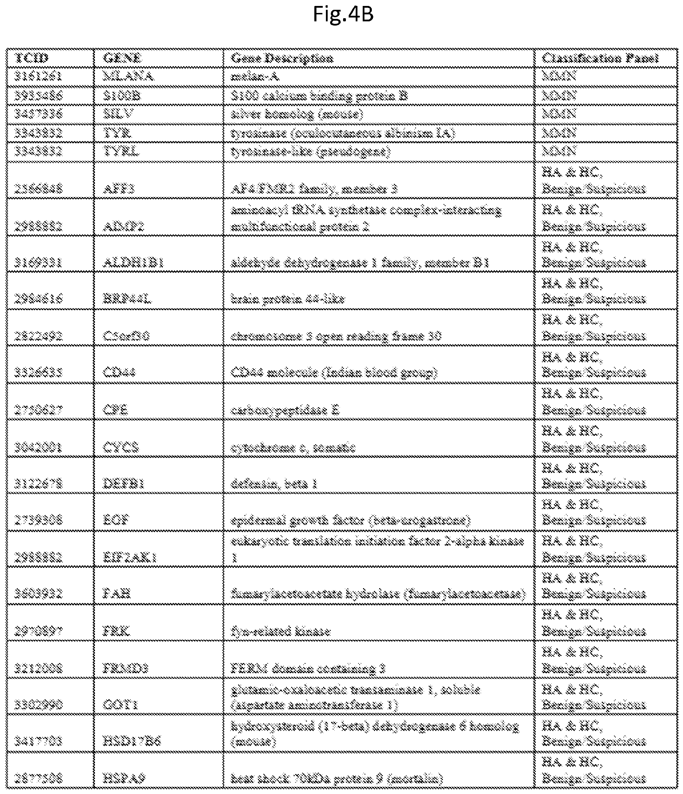

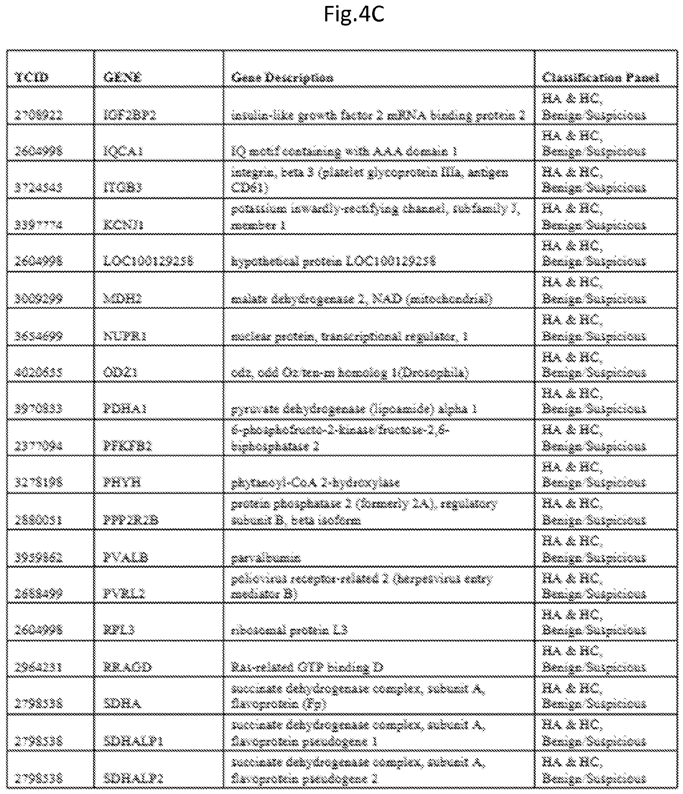

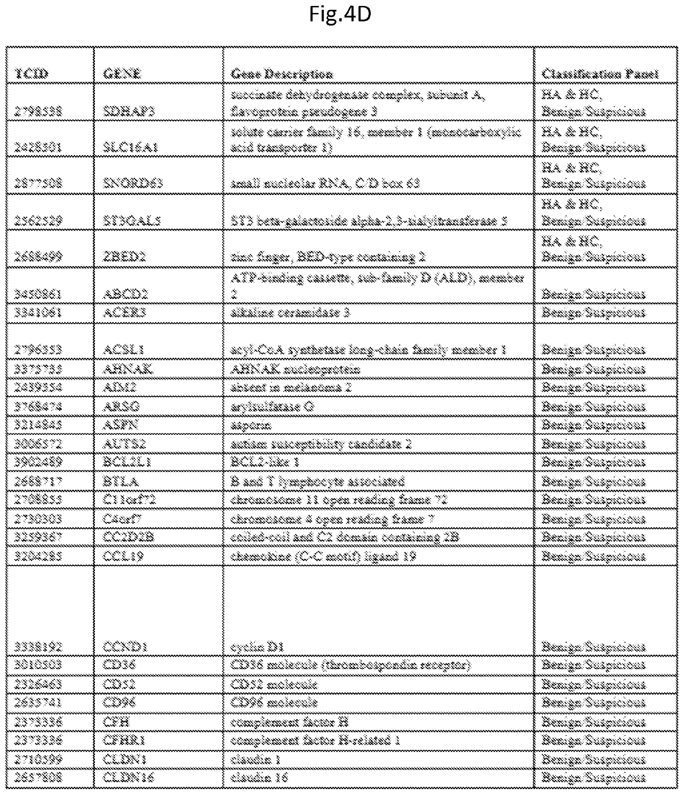

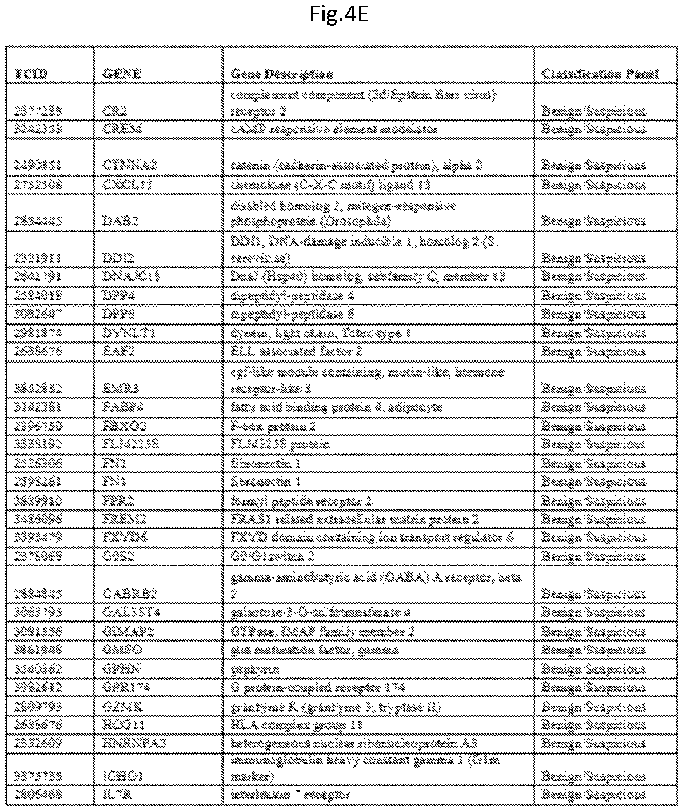

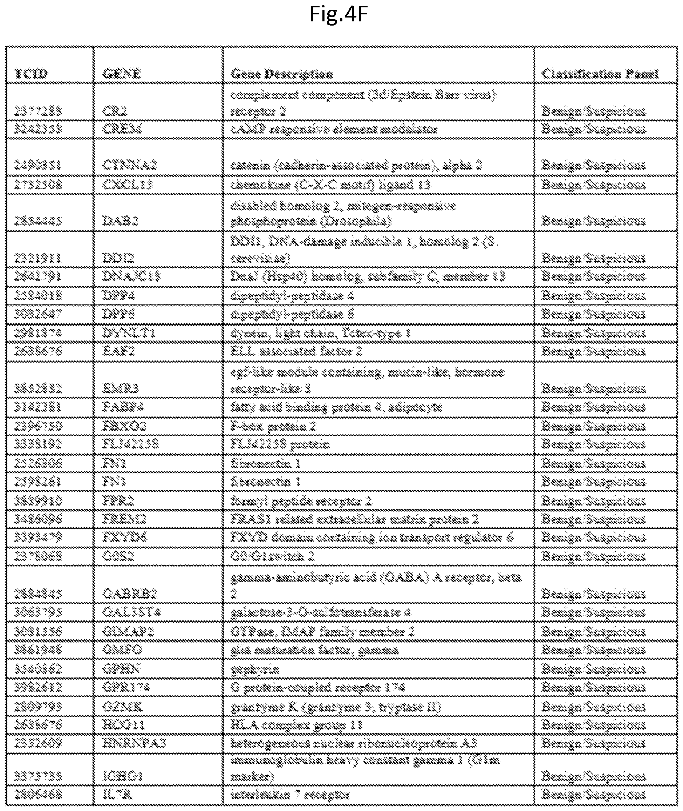

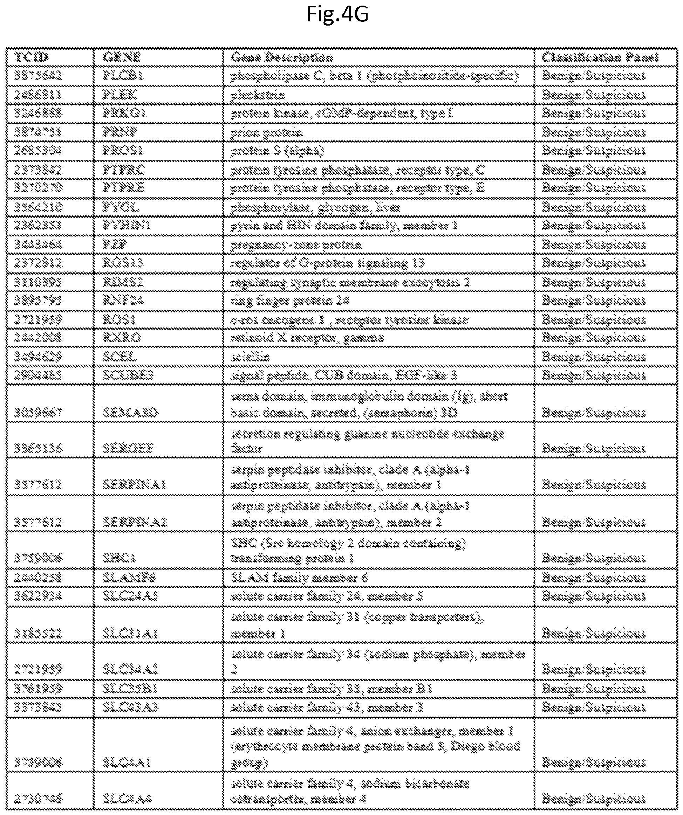

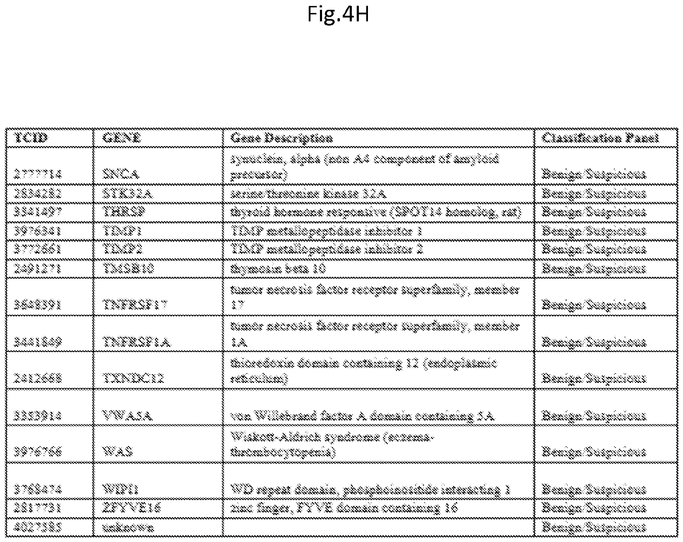

The present methods and compositions also relate to the use of "biomarker panels" for purposes of identification, classification, diagnosis, or to otherwise characterize a biological sample. The methods and compositions can also use groups of biomarker panels, herein described as "classification panels," examples of which can be found in Figure 3, Figure 4, Table 1, Table 2, Table 3, Table 4, Table 5, Table 9, Table 10, Table 11, Table 12, Table 14, Table 15, Table 18 and Table 19. Often the pattern of levels of gene expression of biomarkers in a panel (also known as a signature) is determined and then used to evaluate the signature of the same panel of biomarkers in a biological sample, such as by a measure of similarity between the sample signature and the reference signature. In some embodiments, the method involves measuring (or obtaining) the levels of two or more gene expression products that are within a biomarker panel and/or within a classification panel. The number of biomarkers in the panel can be between about 1 and about 500; for example about 1-500, 1-400, 1-300, 1-200, 1-100, 1-50, 1-25, 1-10, 10-500, 10-400, 10-300, 10-200, 10-100, 10-50, 10-25, 25-500, 25-400, 25-300, 25-200, 25-100, 25-50, 50-500, 50-400, 50-300, 50-200, 50-100, 100-500, 100-400, 100-300, 100-200, 200-500, 200-400, 200-300, 300-500, 300-400, 400-500, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, or any included range or integer. For example, the biomarker panel or a classification panel can contain at least about 1, 2, 3, 4, 5, 6, 7, 8, 9, 10, 15, 20, 25, 30, 33, 35, 38, 40, 43, 45, 48, 50, 53, 58, 63, 65, 68, 100, 120, 140, 142, 145, 147, 150, 152, 157, 160, 162, 167, 175, 180, 185, 190, 195, 200, 300, 400, 500, or more biomarkers. The biomarker panel or a classification panel can contain no more than about 1, 2, 3, 4, 5, 6, 7, 8, 9, 10, 15, 20, 25, 30, 33, 35, 38, 40, 43, 45, 48, 50, 53, 58, 63, 65, 68, 100, 120, 140, 142, 145, 147, 150, 152, 157, 160, 162, 167, 175, 180, 185, 190, 195, 200, 300, 400, or 500 biomarkers. The classification panel can contain between about 1 and about 25 different biomarker panels; for example, about 1-25, 1-20, 1-15, 1-10, 1-5, 5-25, 5-20, 5-15, 5-10, 10-25, 10-20, 10-15, 15-25, 15-20, 20-25, 1, 2, 3, 4, 5, 6, 7, 8, 9, 10, 11, 12, 13, 14, 15, 16, 17, 18, 19, 20, 21, 22, 23, 24, or 25 different biomarker panels. The classification panel can contain at least about 1, 2, 3, 4, 5, 6, 7, 8, 9, 10, 11, 12, 13, 14, 15, 16, 17, 18, 19, 20, 21, 22, 23, 24, or 25 different biomarker panels. The classification panel can contain no more than about 1, 2, 3, 4, 5, 6, 7, 8, 9, 10, 11, 12, 13, 14, 15, 16, 17, 18, 19, 20, 21, 22, 23, 24, or 25 different biomarker panels. The methods can comprise pre-screening samples for the presence of confounding conditions; for example, pre-screening thyroid tissue samples for the presence of lymphomas. The methods can comprise diagnosing a subject with a cancer (e.g., a thyroid cancer). The methods can comprise predicting whether a subject has a genetic mutation (e.g., BRAF V600E) based upon a cohort of gene expression products in a sample from the subject. The present disclosure provides methods of identifying, classifying, or diagnosing cancer comprising the steps of: obtaining an expression level for one or more gene expression products of a biological sample; and identifying the biological sample as benign wherein the gene expression level indicates a lack of cancer in the biological sample. Also provided are methods of identifying, classifying, or diagnosing cancer comprising the steps of: obtaining an expression level for one or more gene expression products of a biological sample; and identifying the biological sample as malignant or suspicious wherein the gene expression level is indicative of a cancer in the biological sample. For example, this can be done by correlating the patterns of gene expression levels, as defined in classification panels described herein, with the gene expression level in the sample, in order to identify (or rule out) the presence of thyroid cancer in the biological sample. Methods to identify thyroid cancer can also comprise one or more pre- and/or post-screening steps. Screening steps can comprise screening samples for the presence of a confounding condition, such as lymphoma; and/or screening a sample for the presence of a genetic mutation (e.g., BRAF V600E). The methods for identifying, characterizing, diagnosing, and/or screening samples can comprise covariate analysis to account for sample heterogeneity. The gene expression products can be associated with one or more of the biomarkers in Figure 3, Figure 4, Table 1, Table 2, Table 3, Table 4, Table 5, Table 9, Table 10, Table 11, Table 12, Table 14, Table 15, Table 18, Table 19, Table 23, Table 24, Table 25, Table 26 or Table 27.

The present disclosure provides methods of identifying, classifying, and/or characterizing samples (e.g., diagnosing cancer or other condition, predicting genetic mutations, pre-screening for a confounding condition, *etc.*), wherein both the specificity and/or sensitivity are between about 50% and about 100%; for example, about 50-100%, 50-99%, 50-95%, 50-90%, 50-80%, 50-70%, 50-60%, 60-100%, 60-99%, 60-95%, 60-90%, 60-80%, 60-70%, 70-100%, 70-99%, 70-95%, 70-90%, 70-80%, 80-100%, 80-99%, 80-95%, 80-90%, 90-100%, 90-99%, 90-95%, 95-100%, 95-99%, 99-100%, 50%, 55%, 60%, 65%, 70%, 75%, 80%, 85%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9%, or 100%. In some embodiments, the specificity or sensitivity is between about 40% and about 100%. The methods can comprise comparing gene expression product levels (*e.g.*, profile) from a biological sample with a biomarker panel and/or a classification panel; and characterizing the biological sample (*e.g.*, as cancerous, suspicious, or benign; as male or female; as mutant or wild-type; *etc.*) based on the comparison. The specificity of the methods disclosed herein can be at least about 45%, 50%, 55%, 60%, 65%, 70%, 75%, 80%, 85%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9%, or 100%. The sensitivity of the methods disclosed herein can be at least about 45%, 50%, 55%, 60%, 65%, 70%, 75%, 80%, 85%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9%, or 100%. In some cases, the specificity can be at least about 50% and the sensitivity of the can be at least about 50%. In some cases, the specificity can be at least about 70% and the sensitivity can be at least about 70%. In some cases, the specificity can be at least about 50%, and the sensitivity can be at least about 70%.

The present disclosure provides methods of identifying, classifying, or characterizing samples (*e.g.*, diagnosing cancer or other condition, predicting genetic mutations, prescreening for a confounding condition, *etc.*)*,* wherein the negative predictive value (NPV) can be greater than or equal to about 90%; for example, the NPV can be at least about 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, 99.1%, 99.2%, 99.3%, 99.4%, 99.5%, 99.6%, 99.7%, 99.8%, 99.9%, or 100%. The methods can further be characterized by having a specificity (or positive predictive value (PPV)) that can be at least about 30%; for example, the PPV can be at least about 30%, 35%, 40%, 45%, 50%, 55%, 60%, 65%, 70%, 75%, 80%, 85%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9%, or 100%. In some cases, the NPV can be at least 95%, and the specificity can be at least 50%. In some cases, the NPV can be at least 95% and the specificity can be at least 70%.

Marker panels (*e.g.*, classifiers, biomarker panels, classifier panels) can be chosen to accommodate adequate separation of conditions (*e.g.*, benign from non-benign or suspicious expression profiles; male from female expression profiles; mutant from wild-type profiles; mixed tissue from tissue specific profiles; *etc.*). Training of such multidimensional classifiers (*e.g.*, algorithms) can be performed on a plurality of biological samples. The plurality of biological samples can comprise between about 2 samples and about 4000 samples, or more; for example, about 2-4000, 2-2500, 2-1000, 2-500, 2-250, 2-100, 2-50, 2-10, 10-4000, 10-2500, 10-1000, 10-500, 10-250, 10-100, 10-50, 50-4000, 50-2500, 50-1000, 50-500, 50-250, 50-100, 100-4000, 100-2500, 100-1000, 100-500, 100-250, 250-4000, 250-2500, 250-1000, 250-500, 500-4000, 500-2500, 500-1000, 1000-4000, 1000-2500, 2500-4000, 2, 3, 4, 5, 6, 7, 8, 9, 10, 15, 20, 25, 30, 35, 40, 45, 50, 60, 70, 80, 90, 100, 125, 150, 175, 200, 225, 250, 300, 350, 400, 450, 500, 600, 700, 800, 900, 1000, 1250, 1500, 1750, 2000, 2250, 2500, 3000, 3500, 4000 such as at least 50, 100, 200, 300, 400, 500, 600, 700, 800, 900, 1000, 1500, 2000, 2500, 3000, 3500, or 4000, or more, biological samples. The biological samples can be any samples from which genetic material can be obtained. Exemplary sources of biological samples include fine needle aspiration, core needle biopsy, vacuum assisted biopsy, incisional biopsy, excisional biopsy, punch biopsy, shave biopsy or skin biopsy. In some cases, the biological samples comprise fine needle aspiration samples. In some cases, the biological samples comprise tissue samples (*e.g.*, from excisional biopsy, incisional biopsy, or other biopsy). The biological samples can comprise a mixture of two or more sources; for example, fine needle aspirates and tissue samples. The percent of the total sample population that is obtained by FNA's can be greater than 10, 20, 30, 40, 50, 60, 70, 80, 90, or 95%. The biological samples can be samples derived from any tissue type. In some aspects, the biological samples comprise thyroid tissue or cells.

One or more training/test sets can be used in developing an algorithm or classifier. The overall algorithm error rate can be shown as a function of gene number for classification sub-type (*e.g.*, benign vs. non-benign, male vs. female, mutant vs. wildtype, target vs. confounding cell types, *etc.*) Other performance metrics can be used, such as a performance metric that is a function of gene number for either subtypes or benign vs. malignant (B vs. M). Such performance metric can be obtained using CV, or other method known in the art. All results can be obtained using a support vector machine model which is trained and tested in a cross-validated mode on the samples.

There can be a specific (or range of) difference in gene expression between subtypes or sets of samples being compared to one another. In some examples, the gene expression of some similar subtypes can be merged to form a super-class that is then compared to another subtype, or another super-class, or the set of all other subtypes. The difference in gene expression level can be at least about 5%, 10%, 15%, 20%, 25%, 30%, 35%, 40%, 45%, 50%, 55%, 60%, 65%, 70%, 75%, 80%, 85%, 90%, 95%, 100% or more. The difference in gene expression level can be at least about 2, 3, 4, 5, 6, 7, 8, 9, 10 fold or more.

The present disclosure provides methods of identifying, classifying, or characterizing samples (*e.g.*, diagnosing cancer or other condition, predicting genetic mutations, pre-screening for confounding conditions, *etc.*), with an accuracy that can be between about 50% and about 100%; for example, about 50-100%, 50-99%, 50-95%, 50-90%, 50-80%, 50-70%, 50-60%, 60-100%, 60-99%, 60-95%, 60-90%, 60-80%, 60-70%, 70-100%, 70-99%, 70-95%, 70-90%, 70-80%, 80-100%, 80-99%, 80-95%, 80-90%, 90-100%, 90-99%, 90-95%, 95-100%, 95-99%, 99-100%, 50%, 55%, 60%, 65%, 70%, 75%, 80%, 82%, 84%, 86%, 88%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9%, or 100%. In some aspects, the methods can identify a biological sample as suspicious or malignant with an accuracy of at least about 50%, 60%, 70%, 75%, 80%, 85%, 90%, 95%, 99% or more. In some aspects, the biological sample can be identified as benign with an accuracy of greater than about 50%, 60%, 70%, 75%, 80%, 85%, 90%, 95%, 99% or more.

The present disclosure provides gene expression products corresponding to biomarkers selected from Figure 4. The methods and compositions provided herein can include gene expression products corresponding to any or all of the biomarkers selected from Figure 4, as well as any subset thereof, in any combination. For example, the methods can use gene expression products corresponding to at least 1, 2, 3, 4, 5, 6, 7, 8, 9, 10, 15, 20, 25, 30, 35, 40, 45 or 50, 100, 120, 140, 160 of the genetic markers provided in Figure 4. In some cases, certain biomarkers can be excluded or substituted with other biomarkers, for example with biomarkers that exhibit a similar expression level profile with respect to a particular tissue type or sub-type.

The present disclosure provides methods and compositions (e.g., gene expression products, biomarker panels, and classifier panels) for use in identifying lymphomas in samples of non-lymphoid origin (e.g., thyroid samples). Lymphomas are cancers that can originate in the lymph nodes, but can metastasize to other tissues (e.g., thyroid tissue). Lymphocytic thyroiditis is group of non-malignant disorders characterized by thyroidal inflammation due to infiltration of the thyroid by lymphocytes. The methods and compositions disclosed herein can be used to separate or classify lymphoma from lymphocytic thyroiditis (LCT) samples. The methods and compositions disclosed herein can be used to separate lymphoma-containing thyroid samples from other thyroid samples. The methods and compositions disclosed herein can be used to pre-screen thyroid samples for the presence of lymphomas prior to the application of a main thyroid classifier (e.g., prior to characterizing or diagnosing a thyroid sample as suspicious/malignant or benign). The methods and compositions disclosed herein can be used to reduce the rate of false positives when using the main thyroid classifier. The methods and compositions for use in identifying lymphomas in the sample can include gene expression products, biomarker panels, and/or classifier panels corresponding to any or all of the biomarkers from Table 1. The methods and compositions for use in identifying lymphomas in the sample can include gene expression products, biomarker panels, and/or classifier panels corresponding to between about 1 and about 200 biomarkers from Table 1; for example, about 1-200, 1-150, 1-100, 1-75, 1-50, 1-25, 25-200, 25-150, 25-100, 25-75, 25-50, 50-200, 50-150, 50-100, 50-75, 75-200, 75-150, 75-100, 100-200, 100-150, 150-200, 1, 2, 3, 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, 110, 120, 130, 140, 150, 160, 170, 180, 190, or 200 biomarkers from Table 1.

The present disclosure provides methods and compositions (e.g., gene expression products, biomarker panels, classifier panels, analytical methods, etc.) to predict a mutation status of a subject from a biological sample obtained from the subject. The mutation status can be a BRAF mutation; for example, the mutation status can be positive or negative for BRAF V600E. The biological sample can be a thyroid sample; for example, the biological sample can be a fine needle aspiration of thyroid tissue. The methods and compositions disclosed herein can be used to categorize biological samples as originating from a subject that is wild-type for the BRAF gene or from a subject that is heterozygous for the BRAF V600E point mutation. The methods and compositions disclosed herein can be used to determine, diagnose, or predict whether a papillary thyroid carcinoma sample comprises the BRAF V600E point mutation. The BRAF V600E point mutation status can be used, for example, to decide upon a course of treatment for papillary thyroid carcinoma. The methods and compositions to predict the mutation status of a subject can include gene expression products, biomarker panels and/or classifier panels corresponding to any or all of the biomarkers in Table 19, Table 23, Table 24, Table 25, Table 26 or Table 27. The gene expression products, biomarker panels, and/or classifier panels can correspond to between about 1 and about 477 biomarkers from Table 19; for example, about 1-477, 1-300, 1-150, 1-100, 1-50, 1-10, 10-477, 10-300, 10-150, 10-100, 10-50, 50-477, 50-300, 50-150, 50-100, 100-477, 100-300, 100-150, 150-477, 150-300, 300-477, 1, 2, 3, 4, 5, 6, 7, 8, 9, 10, 15, 20, 25, 30, 35, 40, 45, 50, 60, 70, 80, 90, 100, 110, 120, 130, 140, 150, 175, 200, 225, 250, 275, 300, 325, 350, 375, 400, 425, 450, or 477 biomarkers from Table 19, Table 23, Table 24, Table 25, Table 26 or Table 27.

Methods and compositions (e.g., gene expression products, biomarker panels, classifier panels, etc.) to predict a mutation status of a subject (e.g., BRAF V600E mutation status) can adjust for cellular content variation; for example, by using covariate analysis incorporating cell-type signal strength. For example, methods and compositions to predict mutation status in a thyroid sample can adjust for follicular cell signal strength, lymphocytic cell signal strength, and/or Hurthle cell signal strength. Any or all of the biomarkers in Table 3 (e.g., about 1, 2, 3, 4, 5, 6, 7, 8, 9, or 10 biomarkers from Table 3) can be used to adjust for, or estimate, Follicular cell signal strength. Any or all of the biomarkers in Table 4 (e.g., about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, or 41 biomarkers from Table 12), can be used to adjust for, or estimate, Hurthle cell signal strength. Any or all of the biomarkers in Table 5 (e.g., about 1, 2, 3, 4, 5, 6, 7, 8, 9, 10, 11, 12, 13, 14, 15, 16, 17, 18, 19, 20, 21, 22, or 23 biomarkers from Table 5), can be used to adjust for, or estimate, Lymphocytic cell signal strength. Methods and compositions to predict mutation status (e.g., BRAF V600E mutation status) that comprise covariate analysis can include gene expression products, biomarker panels, and/or classifier panels corresponding to any or all of the biomarkers in Table 2. Methods and compositions to predict mutation status, such as BRAF V600E mutation status, can comprise gene expression products, biomarker panels, and/or classifier panels that correspond to between about 1 and about 36 biomarkers from Table 2; for example, about 1-36, 1-24, 1-12, 1-6, 6-36, 6-24, 6-12, 12-36, 12-24, 24-36, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, or 36 biomarkers from Table 2.

The methods of the present disclosure can improve upon the accuracy of current methods of cancer diagnosis. The methods can provide improved accuracy of identifying benign, or definitively benign, samples (*e.g.*, thyroid samples). Improved accuracy can be obtained by using algorithms trained with specific sample cohorts, high numbers of samples, and/or samples from individuals located in diverse geographical regions. The sample cohort can be from at least 1, 2, 3, 4, 5, 6, 67, 8, 9, 10, 15, 20, 25, 30, 35, 40, 45, 50, 55, 60, 65, 70, 75, or 80 different geographical locations (*e.g.*, sites spread out across a nation, such as the United States, across a continent, or across the world). Geographical locations can include, but are not limited to, test centers, medical facilities, medical offices, post office addresses, cities, counties, states, nations, and continents. A classifier that is trained using sample cohorts from a first geographical region (*e.g.*, the United States) can be re-trained for use on sample cohorts from other geographical regions (*e.g.*, India, Asia, Europe, Africa, *etc.*).

The present disclosure provides methods of classifying cancer, wherein the methods comprise the steps of: obtaining a biological sample comprising gene expression products; determining the expression level for one or more gene expression products of the biological sample that are differentially expressed in different subtypes of a cancer; and identifying the biological sample as cancerous wherein the gene expression level is indicative of a subtype of cancer. In some cases, the subject methods distinguish follicular carcinoma from medullary carcinoma. In some cases, the subject methods are used to classify a thyroid tissue sample as comprising one or more benign or malignant tissue types (e.g. a cancer subtype), including but not limited to follicular adenoma (FA), nodular hyperplasia (NHP), lymphocytic thyroiditis (LCT), and Hurthle cell adenoma (HA), follicular carcinoma (FC), papillary thyroid carcinoma (PTC), follicular variant of papillary carcinoma (FVPTC), medullary thyroid carcinoma (MTC), Hürthle cell carcinoma (HC), and anaplastic thyroid carcinoma (ATC), renal carcinoma (RCC), breast carcinoma (BCA), melanoma (MMN), B cell lymphoma (BCL), and parathyroid (PTA). In some cases, the subject methods are used to classify a sample of thyroid tissue as comprising HC and/or HA tissue types. In some cases, the subject methods distinguish a benign thyroid disease from a malignant thyroid tumor/carcinoma.

In some cases, the biological sample is classified as cancerous or positive for a subtype of cancer with an accuracy of greater than about 75%, 80%, 85%, 86%, 87%, 88%, 89%, 90%, 91%, 92%, 93%, 94%, 95%, 96%, 97%, 98%, 99%, or 99.5%. The classification accuracy as used herein includes specificity, sensitivity, positive predictive value, negative predictive value, and/or false discovery rate.

Gene expression product markers of the present disclosure can provide increased accuracy of identifying, classifying, or characterizing samples (*e.g.*, diagnosing cancer or other condition, predicting genetic mutations, pre-screening for a confounding condition, *etc.*) through the use of multiple gene expression product markers in low quantity and quality, and statistical analysis using the algorithms of the present disclosure. The present disclosure provides, but is not limited to, methods of characterizing, classifying, or diagnosing gene expression profiles associated with thyroid cancer signatures, lymphoma signatures, and BRAF mutation signatures. The present disclosure also provides algorithms for characterizing and classifying biological samples (*e.g.*, thyroid tissue samples) and kits and compositions useful for the application of the methods.

Markers and genes can be identified to have differential expression between conditions (*e.g.*, in thyroid cancer samples compared to thyroid benign samples; in samples from males compared to samples from females; in samples comprising lymphomas compared to samples with benign lymphatic signatures; in samples with genetic mutations such as BRAF V600E compared to wild type BRAF; *etc.*). Illustrative examples having a benign pathology include follicular adenoma, Hurthle cell adenoma, lymphocytic thyroiditis, and nodular hyperplasia. Illustrative examples having a malignant pathology include follicular carcinoma, follicular variant of papillary thyroid carcinoma, medullary carcinoma, and papillary thyroid carcinoma.

Biological samples can be treated to extract nucleic acids such as DNA or RNA. The nucleic acid can be contacted with an array of probes under conditions to allow hybridization, or the nucleic acids can be sequenced by any method known in the art. The degree of hybridization can be assayed in a quantitative matter using a number of methods known in the art. In some cases, the degree of hybridization at a probe position can be related to the intensity of signal provided by the assay, which therefore is related to the amount of complementary nucleic acid sequence present in the sample. Software can be used to extract, normalize, summarize, and/or analyze array intensity data from probes across the human genome or transcriptome including expressed genes, exons, introns, and miRNAs. The intensity of a given probe in samples (*e.g.*, benign samples, malignant samples, *etc.*) can be compared against a reference set to determine whether differential expression is occuring in a sample. An increase or decrease in relative intensity at a marker position on an array corresponding to an expressed sequence can be indicative of an increase or decrease respectively of expression of the corresponding expressed sequence. An increase or decrease in relative intensity can also be indicative of a mutation in the expressed sequence.

The resulting intensity values for each sample can be analyzed using feature selection techniques including filter techniques, which can assess the relevance of features by looking at the intrinsic properties of the data; wrapper methods, which embed the model hypothesis within a feature subset search; and/or embedded techniques in which the search for an optimal set of features is built into a classifier algorithm.

Filter techniques useful in the methods of the present disclosure can include (1) parametric methods such as the use of two sample t-tests, ANOVA analyses, Bayesian frameworks, and Gamma distribution models; (2) model free methods such as the use of Wilcoxon rank sum tests, between-within class sum of squares tests, rank products methods, random permutation methods, and/or TNoM (Threshold Number of Misclasifications) which involves setting a threshold point for fold-change differences in expression between two datasets and then detecting the threshold point in each gene that minimizes the number of misclassifications; (3) and multivariate methods such as bivariate methods, correlation based feature selection methods (CFS), minimum redundancy maximum relavance methods (MRMR), Markov blanket filter methods, and/or uncorrelated shrunken centroid methods. Wrapper methods useful in the methods of the present disclosure can include sequential search methods, genetic algorithms, and/or estimation of distribution algorithms. Embedded methods useful in the methods of the present disclosure can include random forest algorithms, weight vector of support vector machine algorithms, and/or weights of logistic regression algorithms. Bioinformatics. 2007 Oct 1;23(19):2507-17 provides an overview of the relative merits of the filter techniques provided above for the analysis of intensity data.

Selected features can be classified using a classifier algorithm. Illustrative algorithms can include, but are not limited to, methods that reduce the number of variables such as principal component analysis algorithms, partial least squares methods, and/or independent component analysis algorithms. Illustrative algorithms can further include, but are not limited to, methods that handle large numbers of variables directly such as statistical methods and methods based on machine learning techniques. Statistical methods can include penalized logistic regression, prediction analysis of microarrays (PAM), methods based on shrunken centroids, support vector machine analysis, and regularized linear discriminant analysis. Machine learning techniques can include bagging procedures, boosting procedures, random forest algorithms, and/or combinations thereof. Cancer Inform. 2008; 6: 77-97 provides an overview of the classification techniques provided above for the analysis of microarray intensity data.

The markers and genes of the present disclosure can be utilized to identify, classify, and/or characterize cells or tissues (*e.g.*, as cancerous or benign, as from a male or female, as comprising a genetic mutation or wild-type, *etc.*). The present disclosure includes methods for identifying, classifying, and/or characterizing tissues or cells comprising determining the differential expression of one or more markers or genes in a biological sample (*e.g.*, a thyroid sample) of a subject wherein at least one of the markers or genes are listed in Figure 3, Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27.

The present disclosure also includes methods for identifying thyroid pathology subtypes comprising determining the differential expression of one or more markers or genes in a thyroid sample of a subject wherein the markers or genes are listed in Figure 4, Table 2, Table 9, Table 10, Table 14, Table 15, Table 18, Table 19, Table 23, Table 24, Table 25, Table 26 and Table 27.

In accordance with the foregoing, the differential expression of a gene, genes, markers, mRNA, miRNAs, or a combination thereof as disclosed herein can be determined using northern blotting and employing the sequences as identified herein to develop probes for this purpose. Such probes can be composed of DNA or RNA or synthetic nucleotides or a combination of these and can advantageously be comprised of a contiguous stretch of nucleotide residues matching, or complementary to, a sequence corresponding to a genetic marker identified in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27. Such probes can comprise a contiguous stretch of at least about 10-500 residues, or more; for example, about 10-500, 10-200, 10-150, 10-100, 10-75, 10-50, 10-25, 25-500, 25-200, 25-150, 25-100, 25-75, 25-50, 50-500, 50-200, 50-150, 50-100, 50-75, 75-500, 75-200, 75-150, 75-100, 100-500, 100-200, 100-150, 150-500, 150-200, 200-500, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 80, 85, 90, 95, 100, 110, 120, 130, 140, 150, 160, 170, 180, 190, 200, 225, 250, 275, 300, 325, 350, 375, 400, 425, 450, 475, or 500 nucleotides, or more, derived from one or more of the sequences corresponding to a genetic marker identified in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27. Thus, where a single probe binds multiple times to the transcriptome of a sample of cells that are in a first category (*e.g.*, cancerous, suspected of being cancerous, predisposed to become cancerous, male, mutant, *etc.*), whereas binding of the same probe to a similar amount of transcriptome derived from the genome of cells of the same organ or tissue in a second category (*e.g.*, benign, non-cancerous, female, wildtype, *etc.*) results in observably more or less binding, this is indicative of differential expression of a gene, multiple genes, markers, or miRNAs comprising, or corresponding to, the sequences corresponding to a genetic marker identified in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27 from which the probe sequenced is derived.

Altered or differential gene expression between cell types or categories can be determined by measuring the relative amounts of gene expression products. Gene expression products can be RNA. The amount of RNA transcription can be determined, for example, by producing corresponding cDNAs and then analyzing the resulting DNA using probes developed from the gene sequences as corresponding to one or more genetic markers identified in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27. The cDNA produced by use of reverse transcriptase can be amplified using polymerase chain reaction, or some other means, such as linear amplification, isothermal amplification, NASB, or rolling circle amplification, to determine the relative levels of resulting cDNA and, thereby, the relative levels of gene expression.

Altered or differential gene expression can also be determined by measuring gene expression products, such as proteins, by using agents that selectively bind to, and thereby detect, the presence of proteins encoded by the genes disclosed herein. Suitable agents can include antibodies. Antibodies can be bound to a fluorescent label or radiolabel. Antibodies can be generated against one of the polypeptides that is encoded by all or a fragment of one of the gene sequences corresponding to a genetic marker identified in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27. The relative levels of antibody binding to biological samples (*e.g.*, protein extracts of cells or tissues) can be used as a measure of the extent of expression, or differential expression, of the genes. Exemplary antibody related means of detecting protein levels include western blotting, Enzyme-Linked Immunosorbent Assays, protein chip arrays, or any other means known in the art. The genes and biomarkers disclosed herein can be differentially expressed due to increased copy number, decreased copy number, and/or altered transcription levels (*e.g.*, over- or under-transcription, such as where the over-expression is due to over- or under-production of a transcription factor that activates or represses the gene and leads to repeated binding of RNA polymerase), which can thereby generating altered levels of RNA transcripts. Following translation, altered levels of RNA transcripts can produce altered levels of polypeptides or proteins, such as polypeptides encoded by all or a part of a polynucleotide sequence corresponding to a genetic marker identified in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27. Protein level analysis can provide an additional means of ascertaining the expression of the genes identified according to the disclosure and can thereby be used in determining, or categorizing, biological samples (*e.g.*, to diagnose the presence of a cancerous state in a sample derived from a patient to be tested, or the predisposition to develop cancer at a subsequent time in the patient; to predict the mutation state of the patient; *etc.*).

In employing the methods of the disclosure, gene or marker expression indicative of a sample category or classification (*e.g.*, cancerous state vs. benign, male vs. female, mutant vs. wildtype, lymphoma vs. non-lymphoma, *etc.*) need not be characteristic of every cell in the sample. Thus, the methods disclosed herein are useful for detecting the presence of a condition or state (*e.g.*, a cancerous condition) within a tissue where less than all cells exhibit the complete pattern of differential expression. For example, a set of selected genes or markers, comprising sequences homologous under stringent conditions, or at least 90%, preferably 95%, identical to at least one of the sequences corresponding to a genetic marker identified in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27; or probe sequences complementary to all or a portion thereof, can be found, using appropriate probes (e.g., DNA or RNA probes) to be present in about, less than about, or more than about 20%, 30%, 40%, 50%, 60%, 70%, 80%, 90%, or more of cells derived from a biological sample (*e.g.,* of tumorous or malignant tissue). In some cases, a set of selected genes or markers correlated with a cancerous condition, and forming an expression pattern, can be absent from about, less than about, or more than about 20%, 30%, 40%, 50%, 60%, 70%, 80%, 90%, or more cells derived from corresponding non-cancerous, or otherwise normal, tissue. In one case, an expression pattern of a cancerous condition is detected in at least 70% of cells drawn from a cancerous tissue and absent from at least 70% of a corresponding normal, non-cancerous, tissue sample. In some cases, such expression pattern is found to be present in at least 80% of cells drawn from a cancerous tissue and absent from at least 80% of a corresponding normal, non-cancerous, tissue sample. In some cases, such expression pattern is found to be present in at least 90% of cells drawn from a cancerous tissue and absent from at least 90% of a corresponding normal, non-cancerous, tissue sample. In some cases, such expression pattern is found to be present in at least 100% of cells drawn from a cancerous tissue and absent from at least 100% of a corresponding normal, non-cancerous, tissue sample, although the latter case can represent a rare occurrence. It should also be noted that the expression pattern can be either completely present, partially present, or absent within affected cells, as well as unaffected cells. Therefore, in some cases, the expression pattern is present in variable amounts within affected cells; in some cases, the expression pattern is present in variable amounts within unaffected cells.

Molecular profiling can include detection, analysis, or quantification of one or more gene expression products (*e.g.,* one or more nucleic acids (*e.g.,* DNA or RNA), one or more proteins, or a combination thereof). The diseases or conditions to be diagnosed or characterized by the methods of the present disclosure can include, for example, conditions of abnormal growth, mutation state, and/or heterogeneity of cellular content in one or more tissues of a subject. The tissues analyzed can include, but are not limited to, skin, heart, lung, kidney, breast, pancreas, liver, muscle, smooth muscle, bladder, gall bladder, colon, intestine, brain, esophagus, or prostate. The tissues analyzed by the methods of the present disclosure can include thyroid tissues.

## II. Obtaining a Biological Sample

The methods of the present disclosure, but not being part of the invention, provide for obtaining a biological sample from a subject. As used herein, the term subject refers to any animal (e.g., a mammal), including but not limited to humans, non-human primates, rodents, dogs, cats, pigs, fish, and the like. The present methods and compositions can apply to biological samples from humans. The human can be a new-born, a baby, a child, an adolescent, a teenager, an adult, or a senior citizen. The human can be between about 1 month and 12 months old; for example, about 1, 2, 3, 4, 5, 6, 7, 8, 9, 10, 11, or 12 months old. The human can be between about 1 years old and about 110 years old; for example, about 1-110, 1-65, 1-35, 1-18, 1-11, 1-6, 1-2, 2-110, 2-65, 2-35, 2-18, 2-11, 2-6, 6-110, 6-65, 6-35, 6-18, 6-11, 11-110, 11-65, 11-35, 11-18, 18-110, 18-65, 18-35, 35-110, 35-65, 65-110, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50, 55, 60, 65, 70, 75, 80, 90, 100, 110 years of age.

The methods of obtaining provided herein include methods of biopsy including fine needle aspiration, core needle biopsy, vacuum assisted biopsy, incisional biopsy, excisional biopsy, punch biopsy, shave biopsy or skin biopsy. In some cases, the classifiers provided herein are applied to data only from biological samples obtained by FNA. In some cases, the classifiers provided herein are applied to data only from biological samples obtained by FNA or surgical biopsy. In some cases, the classifiers provided herein are applied to data only from biological samples obtained by surgical biopsy. In some cases, the classifiers themselves are obtained from analysis of data from samples obtained by a specific procedure. For example, a cohort of samples, wherein some are obtained by FNA, and others are obtained by surgical biopsy, can be the source of the samples that are analyzed for the classifiers used herein. In other cases, only data from samples obtained by FNA are used to obtain the classifiers herein. In other cases, only data from samples obtained by surgical procedures are used to obtain the classifiers herein.

Biological samples can be obtained from any of the tissues provided herein; including, but not limited to, skin, heart, lung, kidney, breast, pancreas, liver, muscle, smooth muscle, bladder, gall bladder, colon, intestine, brain, prostate, esophagus, or thyroid. Alternatively, the sample can be obtained from any other source; including, but not limited to, blood, sweat, hair follicle, buccal tissue, tears, menses, feces, or saliva. The biological sample can be obtained by a medical professional. The medical professional can refer the subject to a testing center or laboratory for submission of the biological sample. The subject can directly provide the biological sample. In some cases, a molecular profiling business can obtain the sample. In some cases, the molecular profiling business obtains data regarding the biological sample, such as biomarker expression level data, or analysis of such data.

A biological sample can be obtained by methods known in the art such as the biopsy methods provided herein, swabbing, scraping, phlebotomy, or any other suitable method. The biological sample can be obtained, stored, or transported using components of a kit of the present disclosure. In some cases, multiple biological samples, such as multiple thyroid samples, can be obtained for analysis, characterization, or diagnosis according to the methods of the present disclosure. In some cases, multiple biological samples, such as one or more samples from one tissue type (e.g., thyroid) and one or more samples from another tissue type (e.g., buccal) can be obtained for diagnosis or characterization by the methods of the present disclosure. In some cases, multiple samples, such as one or more samples from one tissue type (e.g., thyroid) and one or more samples from another tissue (e.g., buccal) can be obtained at the same or different times. In some cases, the samples obtained at different times are stored and/or analyzed by different methods. For example, a sample can be obtained and analyzed by cytological analysis (e.g., using routine staining). In some cases, a further sample can be obtained from a subject based on the results of a cytological analysis. The diagnosis of cancer or other condition can include an examination of a subject by a physician, nurse or other medical professional. The examination can be part of a routine examination, or the examination can be due to a specific complaint including, but not limited to, one of the following: pain, illness, anticipation of illness, presence of a suspicious lump or mass, a disease, or a condition. The subject may or may not be aware of the disease or condition. The medical professional can obtain a biological sample for testing. In some cases the medical professional can refer the subject to a testing center or laboratory for submission of the biological sample.

In some cases, the subject can be referred to a specialist such as an oncologist, surgeon, or endocrinologist for further diagnosis. The specialist can likewise obtain a biological sample for testing or refer the individual to a testing center or laboratory for submission of the biological sample. In any case, the biological sample can be obtained by a physician, nurse, or other medical professional such as a medical technician, endocrinologist, cytologist, phlebotomist, radiologist, or a pulmonologist.

A medical professional need not be involved in the initial diagnosis or sample acquisition. An individual can alternatively obtain a sample through the use of an over the counter kit. The kit can contain a means for obtaining the sample as described herein, a means for storing the sample for inspection, and instructions for proper use of the kit. In some cases, molecular profiling services are included in the price for purchase of the kit. In other cases, the molecular profiling services are billed separately.

A biological sample suitable for use by the molecular profiling business can be any material containing tissues, cells, nucleic acids, genes, gene fragments, expression products, gene expression products, and/or gene expression product fragments of an individual to be tested. Methods for determining sample suitability and/or adequacy are provided. The biological sample can include, but is not limited to, tissue, cells, and/or biological material from cells or derived from cells of an individual. The sample can be a heterogeneous or homogeneous population of cells or tissues. The biological sample can be obtained using any method known to the art that can provide a sample suitable for the analytical methods described herein.

A biological sample can be obtained by non-invasive methods, such methods including, but not limited to: scraping of the skin or cervix, swabbing of the cheek, saliva collection, urine collection, feces collection, collection of menses, tears, or semen. The biological sample can be obtained by an invasive procedure, such procedures including, but not limited to: biopsy, alveolar or pulmonary lavage, needle aspiration, or phlebotomy. The method of biopsy can further include incisional biopsy, excisional biopsy, punch biopsy, shave biopsy, or skin biopsy. The method of needle aspiration can further include fine needle aspiration, core needle biopsy, vacuum assisted biopsy, or large core biopsy. Multiple biological samples can be obtained by the methods herein to ensure a sufficient amount of biological material. Methods of obtaining suitable samples of thyroid are known in the art and are further described in the ATA Guidelines for thyroid nodule management (Cooper et al. Thyroid Vol. 16 No. 2 2006). Generic methods for obtaining biological samples are also known in the art and further described in for example Ramzy, Ibrahim Clinical Cytopathology and Aspiration Biopsy 2001. The biological sample can be a fine needle aspirate of a thyroid nodule or a suspected thyroid tumor. The fine needle aspirate sampling procedure can be guided by the use of an ultrasound, X-ray, or other imaging device.

A molecular profiling business can obtain a biological sample from a subject directly, from a medical professional, from a third party, and/or from a kit provided by the molecular profiling business or a third party. The biological sample can be obtained by the molecular profiling business after the subject, the medical professional, or the third party acquires and sends the biological sample to the molecular profiling business. The molecular profiling business can provide suitable containers and/or excipients for storage and transport of the biological sample to the molecular profiling business.

### III. Storing the sample

The methods of the present disclosure provide for storing a biological sample for a period of time, wherein the period of time can be seconds, minutes, hours, days, weeks, months, years or longer after the biological sample is obtained and before the biological sample is analyzed by one or more methods of the disclosure. The biological sample obtained from a subject can be subdivided prior to the step of storage or further analysis such that different portions of the biological sample are subject to different downstream methods or processes. The downstream methods or processes can include, but are not limited to, storage, cytological analysis, adequacy tests, nucleic acid extraction, molecular profiling and/or a combination thereof.

A portion of a biological sample can be stored while another portion of the biological sample is further manipulated. Such manipulations can include, but are not limited to, molecular profiling; cytological staining; nucleic acid (RNA or DNA) extraction, detection, or quantification; gene expression product (*e.g.*, RNA or protein) extraction, detection, or quantification; fixation (*e.g.*, formalin fixed paraffin embedded samples); and/or examination. The biological sample can be fixed prior to or during storage by any method known to the art, such methods including, but not limited to, the use of glutaraldehyde, formaldehyde, and/or methanol. In other cases, the sample is obtained and stored and subdivided after the step of storage for further analysis such that different portions of the sample are subject to different downstream methods or processes including but not limited to storage, cytological analysis, adequacy tests, nucleic acid extraction, molecular profiling or a combination thereof. In some cases, one or more biological samples are obtained and analyzed by cytological analysis, and the resulting sample material is further analyzed by one or more molecular profiling methods of the present disclosure. In such cases, the biological samples can be stored between the steps of cytological analysis and the steps of molecular profiling. The biological samples can be stored upon acquisition; for example, to facilitate transport or to wait for the results of other analyses. Biological samples can be stored while awaiting instructions from a physician or other medical professional.

A biological sample can be placed in a suitable medium, excipient, solution, and/or container for short term or long term storage. The storage can involve keeping the biological sample in a refrigerated or frozen environment. The biological sample can be quickly frozen prior to storage in a frozen environment. The biological sample can be contacted with a suitable cryopreservation medium or compound prior to, during, and/or after cooling or freezing the biological sample. The cryopreservation medium or compound can include, but is not limited to: glycerol, ethylene glycol, sucrose, and/or glucose. The suitable medium, excipient, or solution can include, but is not limited to: hanks salt solution; saline; cellular growth medium; an ammonium salt solution, such as ammonium sulphate or ammonium phosphate; and/or water. Suitable concentrations of ammonium salts can include solutions of between about 0.1 g/mL to 2.5 g/L, or higher; for example, about 0.1g/ml, 0.2g/ml, 0.3g/ml, 0.4g/ml, 0.5g/ml, 0.6 g/ml, 0.7g/ml, 0.8 g/ml, 0.9g/ml, 1.0 g/ml, 1.1 g/ml, 1.2 g/ml, 1.3g/ml, 1.4g/ml, 1.5g/ml, 1.6 g/ml, 1.7 g/ml, 1.8 g/ml, 1.9 g/ml, 2.0 g/ml, 2.2 g/ml, 2.3g/ml, 2.5 g/ml or higher. The medium, excipient, or solution can optionally be sterile.

A biological sample can be stored at room temperature; at reduced temperatures, such as cold temperatures (*e.g.*, between about 20°C and about 0°C); and/or freezing temperatures, including for example about 0°C, -1°C, -2°C, -3°C, -4°C, -5°C, -6°C, -7°C, -8°C, -9°C, -10°C, -12°C, -14°C, -15°C, -16°C, -20°C, -22°C, -25°C, -28°C, -30°C, - 35°C, -40°C, -45°C, -50°C, -60°C, -70°C, -80°C, -100°C, -120°C, -140°C, -180°C, - 190°C, or -200°C. The biological samples can be stored in a refrigerator, on ice or a frozen gel pack, in a freezer, in a cryogenic freezer, on dry ice, in liquid nitrogen, and/or in a vapor phase equilibrated with liquid nitrogen.

A medium, excipient, or solution for storing a biological sample can contain preservative agents to maintain the sample in an adequate state for subsequent diagnostics or manipulation, or to prevent coagulation. The preservatives can include, but are not limited to, citrate, ethylene diamine tetraacetic acid, sodium azide, and/or thimersol. The medium, excipient or solution can contain suitable buffers or salts such as Tris buffers, phosphate buffers, sodium salts (*e.g.*, NaCl), calcium salts, magnesium salts, and the like. In some cases, the sample can be stored in a commercial preparation suitable for storage of cells for subsequent cytological analysis, such preparations including, but not limited to Cytyc ThinPrep, SurePath, and/or Monoprep.

A sample container can be any container suitable for storage and or transport of a biological sample; such containers including, but not limited to: a cup, a cup with a lid, a tube, a sterile tube, a vacuum tube, a syringe, a bottle, a microscope slide, or any other suitable container. The container can optionally be sterile.

## IV. Transportation of the Sample

The methods of the present disclosure provide for transport of a biological sample. In some cases, the biological sample is transported from a clinic, hospital, doctor's office, or other location to a second location whereupon the sample can be stored and/or analyzed by, for example, cytological analysis or molecular profiling.

A biological sample can be transported in any medium or excipient, including any medium or excipient provided herein suitable for storing the biological sample such as a cryopreservation medium or a liquid based cytology preparation. In some cases, the biological sample can be transported frozen or refrigerated, such as at any of the suitable sample storage temperatures provided herein.

Upon receipt of a biological sample by a molecular profiling business, a representative or licensee thereof, a medical professional, researcher, or a third party laboratory or testing center (*e.g.*, a cytology laboratory), the biological sample can be assayed using a variety of analyses known to the art, such as cytological assays and genomic analysis. Such assays or tests can be indicative of cancer, a type of cancer, any other disease or condition, the presence of disease markers, the presence of genetic mutations, or the absence of cancer, diseases, conditions, or disease markers. The tests can take the form of cytological examination including microscopic examination as described below. The tests can involve the use of one or more cytological stains. The biological sample can be manipulated or prepared for the test prior to administration of the test by any suitable method known to the art for biological sample preparation. The specific assay can be chosen based on the likelihood of obtaining a definite diagnosis, the cost of the assay, the speed of the assay, or the suitability of the assay to the type of material provided.

## V. Test for adequacy

Subsequent to or during biological sample acquisition, including before or after a step of storing the sample, the biological material can be assessed for adequacy, for example, to assess the suitability of the sample for use in the methods and compositions of the present disclosure. The assessment can be performed by an individual who obtains the sample; a molecular profiling business; an individual using a kit; or a third party, such as a cytological lab, pathologist, endocrinologist, or a researcher. The sample can be determined to be adequate or inadequate for further analysis due to many factors, such factors including, but not limited to: insufficient cells; insufficient genetic material; insufficient protein, DNA, or RNA; inappropriate cells for the indicated test; inappropriate material for the indicated test; age of the sample; manner in which the sample was obtained; and/or manner in which the sample was stored or transported. Adequacy can be determined using a variety of methods known in the art such as a cell staining procedure, measurement of the number of cells or amount of tissue, measurement of total protein, measurement of nucleic acid, visual examination, microscopic examination, or temperature or pH determination. Sample adequacy can be determined from a result of performing a gene expression product level analysis experiment. Sample adequacy can be determined by measuring the content of a marker of sample adequacy. Such markers can include elements such as iodine, calcium, magnesium, phosphorous, carbon, nitrogen, sulfur, iron etc.; proteins such as, but not limited to, thyroglobulin; cellular mass; and cellular components such as protein, nucleic acid, lipid, or carbohydrate.

Iodine can be measured by a chemical method such as described in US Pat. No. 3645691 or other chemical methods known in the art for measuring iodine content. Chemical methods for iodine measurement include but are not limited to methods based on the Sandell and Kolthoff reaction. The reaction proceeds according to the following equation:

$$2\ Ce^{4+} + As^{3+} \rightarrow 2\ Ce^{3+} + As^{5} + I.$$

Iodine can have a catalytic effect upon the course of the reaction, e.g., the more iodine present in the preparation to be analyzed, the more rapidly the reaction proceeds. The speed of reaction is proportional to the iodine concentration. In some cases, this analytical method can carried out in the following manner: A predetermined amount of a solution of arsenous oxide $As_2O_3$ in concentrated sulfuric or nitric acid is added to the biological sample and the temperature of the mixture is adjusted to reaction temperature, i.e., usually to a temperature between 20° C. and 60° C. A predetermined amount of a cerium (IV) sulfate solution in sulfuric or nitric acid is added thereto. Thereupon, the mixture is allowed to react at the predetermined temperature for a definite period of time. The reaction time is selected in accordance with the order of magnitude of the amount of iodine to be determined and with the respective selected reaction temperature. The reaction time is usually between about 1 minute and about 40 minutes. Thereafter, the content of the test solution of cerium (IV) ions is determined photometrically. The lower the photometrically determined cerium (IV) ion concentration is, the higher is the speed of reaction and, consequently, the amount of catalytic agent, i.e., of iodine. In this manner the iodine of the sample can directly and quantitatively be determined.

Iodine content of a sample of thyroid tissue can also be measured by detecting a specific isotope of iodine such as for example $^{123}$I, [124]I, $^{125}$I, and [131]I. In still other cases, the marker can be another radioisotope such as an isotope of carbon, nitrogen, sulfur, oxygen, iron, phosphorous, or hydrogen. The radioisotope in some instances can be administered prior to sample collection. Methods of radioisotope administration suitable for adequacy testing are well known in the art and include injection into a vein or artery, or by ingestion. A suitable period of time between administration of the isotope and acquisition of thyroid nodule sample so as to effect absorption of a portion of the isotope into the thyroid tissue can include any period of time between about a minute and a few days or about one week including about 1 minute, 2 minutes, 5 minutes, 10 minutes, 15 minutes, ½ an hour, an hour, 8 hours, 12 hours, 24 hours, 48 hours, 72 hours, or about one, one and a half, or two weeks, and can readily be determined by one skilled in the art. Alternatively, samples can be measured for natural levels of isotopes such as radioisotopes of iodine, calcium, magnesium, carbon, nitrogen, sulfur, oxygen, iron, phosphorous, or hydrogen.

### (i) Cell and/or Tissue Content Adequacy Test

Methods for determining the amount of a tissue in a biological sample can include, but are not limited to, weighing the sample or measuring the volume of sample. Methods for determining the amount of cells in the biological sample can include, but are not limited to, counting cells, which can in some cases be performed after dis-aggregation of the biological sample (e.g., with an enzyme such as trypsin or collagenase or by physical means such as using a tissue homogenizer). Alternative methods for determining the amount of cells in the biological sample can include, but are not limited to, quantification of dyes that bind to cellular material or measurement of the volume of cell pellet obtained following centrifugation. Methods for determining that an adequate number of a specific type of cell is present in the biological sample can also include PCR, Q-PCR, RT-PCR, immuno-histochemical analysis, cytological analysis, microscopic, and or visual analysis. The relative levels of difference cell types (*e.g.*, Follicular cells, Hurthle cells, lymphocytic cells, *etc.*) in a sample of thyroid tissue can be determined by expression profiling of one or more marker disclosed in Table 3, Table 4, and/or Table 5.

### (ii) Nucleic Acid Content Adequacy Test

Biological samples can be analyzed by determining nucleic acid content after extraction from the biological sample using a variety of methods known to the art. Nucleic acids, such as RNA or mRNA, can be extracted from other nucleic acids prior to nucleic acid content analysis. Nucleic acid content can be extracted, purified, and measured by ultraviolet absorbance, including but not limited to absorbance at 260 nanometers using a spectrophotometer. Nucleic acid content or adequacy can be measured by fluorometer after contacting the sample with a stain. Nucleic acid content or adequacy can be measured after electrophoresis, or using an instrument such as an Agilent bioanalyzer. It is understood that the methods of the present disclosure are not limited to a specific method for measuring nucleic acid content and or integrity.

In some cases, the RNA quantity or yield from a biological sample is measured shortly after purification using a NanoDrop spectrophotometer in a range of nano- to micrograms. RNA quality can be measured using an Agilent 2100 Bioanalyzer instrument, wherein quality is characterized by a calculated RNA Integrity Number (RIN, 1-10). The NanoDrop is a cuvette-free spectrophotometer. It can use 1 microliter to measure from about 5 ng/$\mu$l to about 3,000 ng/$\mu$l of sample. Features of the NanoDrop include low volume of sample and no cuvette; large dynamic range 5 ng/$\mu$l to 3,000 ng/$\mu$l; and it allows quantitation of DNA, RNA and proteins. NanoDrop™ 2000c allows for the analysis of 0.5 $\mu$l - 2.0 $\mu$l samples, without the need for cuvettes or capillaries.

RNA quality in a biological sample can be measured by a calculated RNA Integrity Number (RIN). The RNA integrity number (RIN) is an algorithm for assigning integrity values to RNA measurements. The integrity of RNA can be a major concern for gene expression studies and traditionally has been evaluated using the 28S to 18S rRNA ratio, a method that can be inconsistent. The RIN algorithm is applied to electrophoretic RNA measurements and based on a combination of different features that contribute information about the RNA integrity to provide a more robust universal measure. RNA quality can be measured using an Agilent 2100 Bioanalyzer instrument. Protocols for measuring RNA quality are known and available commercially, for example, at Agilent website. Briefly, in the first step, researchers deposit total RNA sample into an RNA Nano LabChip. In the second step, the LabChip is inserted into the Agilent bioanalyzer and the analysis is run, generating a digital electropherogram. In the third step, the RIN algorithm then analyzes the entire electrophoretic trace of the RNA sample, including the presence or absence of degradation products, to determine sample integrity. Then, the algorithm assigns a 1 to 10 RIN score, where level 10 RNA is completely intact. Because interpretation of the electropherogram is automatic and not subject to individual interpretation, universal and unbiased comparison of samples can be enabled and repeatability of experiments can be improved. The RIN algorithm was developed using neural networks and adaptive learning in conjunction with a large database of eukaryote total RNA samples, which are obtained mainly from human, rat, and mouse tissues. Advantages of RIN can include obtaining a numerical assessment of the integrity of RNA; directly comparing RNA samples (*e.g.*, before and after archival, between different labs); and ensuring repeatability of experiments [*e.g.*, if RIN shows a given value and is suitable for microarray experiments, then the RIN of the same value can always be used for similar experiments given that the same organism/tissue/extraction method is used (Schroeder A, et al. BMC Molecular Biology 2006, 7:3 (2006)].

RNA quality can be measured on a scale of RIN 1 to 10, 10 being highest quality. In one aspect, the present disclosure provides a method of analyzing gene expression from a sample with an RNA RIN value equal or less than 6.0; for example, a sample containing RNA with an RIN number of about 1.0, 2.0, 3.0, 4.0, 5.0 or 6.0 can be analyzed for microarray gene expression using the subject methods and algorithms of the present disclosure. The sample can be a fine needle aspirate of thyroid tissue. The sample can comprise, or yield upon extraction, RNA with an RIN as low as 2.0.

Determination of gene expression in a given sample can be a complex, dynamic, and expensive process. RNA samples with RIN $\leq$5.0 are typically not used for multi-gene microarray analysis, and can be limited to single-gene RT-PCR and/or TaqMan assays. This dichotomy in the usefulness of RNA according to quality can limit the usefulness of samples and hamper research and/or diagnostic efforts. The present disclosure provides methods via which low quality RNA can be used to obtain meaningful multi-gene expression results from samples containing low concentrations of RNA.

In addition, samples having a low and/or un-measurable RNA concentration by NanoDrop normally deemed inadequate for multi-gene expression profiling, can be measured and analyzed using the subject methods and algorithms of the present disclosure. A sensitive apparatus that can be used to measure nucleic acid yield is the NanoDrop spectrophotometer. Like many quantitative instruments of its kind, the accuracy of a NanoDrop measurement can decrease significantly with very low RNA concentration. The minimum amount of RNA necessary for input into a microarray experiment also limits the usefulness of a given sample. In the present disclosure, a sample containing a very low amount of nucleic acid can be estimated using a combination of the measurements from both the NanoDrop and the Bioanalyzer instruments, thereby optimizing the sample for multi-gene expression assays and analysis.

**(iii) Protein Content Adequacy Test**

Protein content in a biological sample can be measured using a variety of methods known to the art, including, but not limited to: ultraviolet absorbance at 280 nanometers, cell staining as described herein, or protein staining with for example coomassie blue, or bichichonic acid. In some cases, protein is extracted from the biological sample prior to measurement of the sample. In some cases, multiple tests for adequacy of the sample can be performed in parallel, or one at a time. In some cases, the sample can be divided into aliquots for the purpose of performing multiple diagnostic tests prior to, during, or after assessing adequacy. In some cases, the adequacy test is performed on a small amount of the sample which may or may not be suitable for further diagnostic testing. In other cases, the entire sample is assessed for adequacy.

A biological sample can be tested for adequacy soon or immediately after collection. In some cases, when the sample adequacy test does not indicate a sufficient amount sample or sample of sufficient quality, additional samples can be taken.

## VI. Analysis of Sample

In one aspect, the present disclosure provides methods for performing microarray gene expression analysis with low quantity and quality of polynucleotide, such as DNA or RNA. The present disclosure describes methods of diagnosing, characterizing and/or monitoring a cancer by analyzing gene expression with low quantity and/or quality of RNA. The cancer can be a thyroid cancer. The present disclosure also describes methods of identifying, classifying, or characterizing samples by predicting genetic mutations (*e.g.*, BRAF V600E), and/or prescreening for the presence of a confounding condition (*e.g.*, lymphoma) by analyzing gene expression with low quantity and/or quality of RNA. Samples can be thyroid samples. Thyroid RNA can be obtained from fine needle aspirates (FNA). A gene expression profile can be obtained from samples with an RNA RIN value of less than or equal to about 10.0, 9.0, 8.0, 7.0, 6.0, 5.0, 4.0, 3.0, 2.0, 1.0 or less. The gene expression profile can be obtained from a sample with an RIN of equal or less than about 6 (*e.g.*, about 6.0, 5.0, 4.0, 3.0, 2.0, 1.0 or less). Provided by the present disclosure are methods by which low quality RNA can be used to obtain meaningful gene expression results from samples containing low concentrations of nucleic acid, such as thyroid FNA samples.

Another estimate of sample usefulness is RNA yield, typically measured in nanogram to microgram amounts for gene expression assays. An apparatus that can be used to measure nucleic acid yield in the laboratory is the NanoDrop spectrophotometer. Like many quantitative instruments of its kind, the accuracy of a NanoDrop measurement can decrease significantly with very low RNA concentration. The minimum amount of RNA necessary for input into a microarray experiment can also limits the usefulness of a given sample. In some aspects, the present disclosure solves the low RNA concentration problem by estimating sample input using a combination of the measurements from both the NanoDrop and the Bioanalyzer instruments. Since the quality of data obtained from a gene expression study can be dependent on RNA quantity, meaningful gene expression data can be generated from samples having a low or un-measurable RNA concentration as measured by NanoDrop.

The subject methods and algorithms enable: 1) gene expression analysis of samples containing low amount and/or low quality of nucleic acid; 2) a significant reduction of false positives and false negatives, 3) a determination of the underlying genetic, metabolic, or signaling pathways responsible for the resulting pathology, 4) the ability to assign a statistical probability to the accuracy of the diagnosis of genetic disorders, 5) the ability to resolve ambiguous results, 6) the ability to distinguish between sub-types of cancer, 7) the ability to pre-screen samples for the presence of a confounding condition (*e.g.*, lymphoma), which can be used to assess the suitability of the sample for the main classifier, and 8) the ability to predict whether a sample comprises a genetic mutation (*e.g.*, BRAF V600E). The subject methods and algorithms can comprise covariate analysis to account for varying cell-type signal strength in a sample.

## Cytological Analysis

Samples can be analyzed by cell staining combined with microscopic examination of the cells in the biological sample. Cell staining, or cytological examination, can be performed by a number of methods and suitable reagents known to the art including but not limited to: EA stains, hematoxylin stains, cytostain, papanicolaou stain, eosin, nissl stain, toluidine blue, silver stain, azocarmine stain, neutral red, or j anus green. In some cases the cells are fixed and/or permeablized with for example methanol, ethanol, glutaraldehyde or formaldehyde prior to or during the staining procedure. In some cases, the cells are not fixed. In some cases, more than one stain is used in combination. In other cases no stain is used at all. In some cases measurement of nucleic acid content is performed using a staining procedure, for example with ethidium bromide, hematoxylin, nissl stain or any nucleic acid stain known to the art.

In some cases of the present disclosure, cells can be smeared onto a slide by standard methods well known in the art for cytological examination. In other cases, liquid based cytology (LBC) methods can be utilized. In some cases, LBC methods provide for an improved means of cytology slide preparation, more homogenous samples, increased sensitivity and specificity, and improved efficiency of handling of samples. In liquid based cytology methods, biological samples are transferred from the subject to a container or vial containing a liquid cytology preparation solution such as for example Cytyc ThinPrep, SurePath, or Monoprep or any other liquid based cytology preparation solution known in the art. Additionally, the sample can be rinsed from the collection device with liquid cytology preparation solution into the container or vial to ensure substantially quantitative transfer of the sample. The solution containing the biological sample in liquid based cytology preparation solution can then be stored and/or processed by a machine or by one skilled in the art to produce a layer of cells on a glass slide. The sample can further be stained and examined under the microscope in the same way as a conventional cytological preparation.

In some cases of the present disclosure, samples can be analyzed by immuno-histochemical staining. Immuno-histochemical staining provides for the analysis of the presence, location, and distribution of specific molecules or antigens by use of antibodies in a biological sample (e.g. cells or tissues). Antigens can be small molecules, proteins, peptides, nucleic acids or any other molecule capable of being specifically recognized by an antibody. Samples can be analyzed by immuno-histochemical methods with or without a prior fixing and/or permeabilization step. In some cases, the antigen of interest can be detected by contacting the sample with an antibody specific for the antigen and then non-specific binding can be removed by one or more washes. The specifically bound antibodies can then be detected by an antibody detection reagent such as for example a labeled secondary antibody, or a labeled avidin/streptavidin. In some cases, the antigen specific antibody can be labeled directly instead. Suitable labels for immuno-histochemistry include but are not limited to fluorophores such as fluoroscein and rhodamine, enzymes such as alkaline phosphatase and horse radish peroxidase, and radionuclides such as $^{32}$P and $^{125}$I. Gene product markers that can be detected by immuno-histochemical staining include but are not limited to Her2/Neu, Ras, Rho, EGFR, VEGFR, UbcH10, RET/PTC1, cytokeratin 20, calcitonin, GAL-3, thyroid peroxidase, and thyroglobulin.

## VII. Assay Results

The results of routine cytological or other assays can indicate a sample as negative (cancer, disease or condition free), ambiguous or suspicious (suggestive of the presence of a cancer, disease or condition), diagnostic (positive diagnosis for a cancer, disease or condition), or non diagnostic (providing inadequate information concerning the presence or absence of cancer, disease, or condition). The diagnostic results can be further classified as malignant or benign. The diagnostic results can also provide a score indicating for example, the severity or grade of a cancer, or the likelihood of an accurate diagnosis, such as via a p-value, a corrected p-value, or a statistical confidence indicator. In some cases, the diagnostic results can be indicative of a particular type of a cancer, disease, or condition, such as for example follicular adenoma (FA), nodular hyperplasia (NHP), lymphocytic thyroiditis (LCT), Hurthle cell adenoma (HA), follicular carcinoma (FC), papillary thyroid carcinoma (PTC), follicular variant of papillary carcinoma (FVPTC), medullary thyroid carcinoma (MTC), Hürthle cell carcinoma (HC), anaplastic thyroid carcinoma (ATC), renal carcinoma (RCC), breast carcinoma (BCA), melanoma (MMN), B cell lymphoma (BCL), parathyroid (PTA), hyperplasia, papillary carcinoma, or any of the diseases or conditions provided herein. In some cases, the diagnostic results can be indicative of a particular stage of a cancer, disease, or condition. The diagnostic results can include information related to the prediction of genetic mutations, such as heterogeneity for the BRAF V600E mutation. The diagnostic results can inform a particular treatment or therapeutic intervention for the condition (e.g., type or stage of the specific cancer disease or condition) diagnosed. In some cases, the results of the assays performed can be entered into a database. In some cases, all or some steps other than molecular profiling are performed by a cytological laboratory or a medical professional.

## VIII. Molecular Profiling

Cytological assays mark the current diagnostic standard for many types of suspected tumors, including for example thyroid tumors or nodules. Samples that assay as negative, indeterminate, diagnostic, or non diagnostic can be subjected to subsequent assays to obtain more information. In the present disclosure, these subsequent assays can comprise the steps of molecular profiling of genomic DNA, RNA, mRNA expression product levels, miRNA levels, gene expression product levels and/or gene expression product alternative splicing. Molecular profiling can comprise the determination of the number (*e.g.*, copy number) and/or type of genomic DNA in a biological sample. In some cases, the number and/or type can further be compared to a control sample or a sample considered normal. In some case, genomic DNA can be analyzed for copy number variation, such as an increase (amplification) or decrease in copy number, or variants, such as insertions, deletions, truncations and the like. Molecular profiling can be performed on the same sample, a portion of the same sample, or a new sample can be acquired using any of the methods described herein. A molecular profiling company can request an additional sample by directly contacting the individual or through an intermediary such as a physician, third party testing center or laboratory, or a medical professional. In some cases, samples are assayed using methods and compositions of the disclosure in combination with some or all cytological staining or other diagnostic methods. In other cases, samples are directly assayed using the methods and compositions of the disclosure without the previous use of routine cytological staining or other diagnostic methods. In some cases the results of molecular profiling alone or in combination with cytology or other assays can enable those skilled in the art to characterize a tissue sample, diagnose a subject, or suggest treatment for a subject. In some cases, molecular profiling can be used alone or in combination with cytology to monitor tumors or suspected tumors over time for malignant changes. In some cases, molecular profiling can be used to predict whether a sample comprises a genetic mutation; for example, whether a sample is heterologous or wild-type with respect to the BRAF V600E mutation. In some cases, molecular profiling can be used to determine whether the samples are suitable for analysis with a main classifier; for example, whether a sample comprises cells indicative of a confounding condition such as lymphoma.

The molecular profiling methods of the present disclosure provide for extracting and analyzing protein or nucleic acid (RNA or DNA) from one or more biological samples from a subject. In some cases, nucleic acid is extracted from the entire sample obtained. In other cases, nucleic acid is extracted from a portion of the sample obtained. In some cases, the portion of the sample not subjected to nucleic acid extraction can be analyzed by cytological examination or immuno-histochemistry. Methods for RNA or DNA extraction from biological samples are well known in the art and include for example the use of a commercial kit, such as the Qiagen DNeasy Blood and Tissue Kit, or the Qiagen EZ 1 RNA Universal Tissue Kit.

**(i)Tissue-type fingerprinting**

In many cases, biological samples such as those provided by the methods of the present disclosure can contain several cell types or tissues, including but not limited to thyroid follicular cells, thyroid medullary cells, blood cells (RBCs, WBCs, platelets), smooth muscle cells, ducts, duct cells, basement membrane, lumen, lobules, fatty tissue, skin cells, epithelial cells, and infiltrating macrophages and lymphocytes. In the case of thyroid samples, diagnostic classification of the biological samples can involve for example primarily follicular cells (for cancers derived from the follicular cell such as papillary carcinoma, follicular carcinoma, and anaplastic thyroid carcinoma) and medullary cells (for medullary cancer). The diagnosis of indeterminate biological samples from thyroid biopsies in some cases concerns the distinction of follicular adenoma vs. follicular carcinoma. The molecular profiling signal of a follicular cell for example can thus be diluted out and possibly confounded by other cell types present in the sample. Similarly diagnosis of biological samples from other tissues or organs often involves diagnosing one or more cell types among the many that can be present in the sample.

The methods of the present disclosure provide for an upfront method of determining the cellular make-up of a particular biological sample so that the resulting molecular profiling signatures can be calibrated against the dilution effect due to the presence of other cell and/or tissue types. In one aspect, this upfront method is an algorithm that uses a combination of known cell and/or tissue specific gene expression patterns as an upfront mini-classifier for each component of the sample. This algorithm can utilize this molecular fingerprint to pre-classify the samples according to their composition and then apply a correction/normalization factor (e.g., covariate analysis). This data can in some cases then feed in to a final classification algorithm which may incorporate that information to aid in the final diagnosis.

**(ii) Genomic Analysis**

Genomic sequence analysis, or genotyping, can be performed on a biological sample. Genotyping can take the form of mutational analysis such as single nucleotide polymorphism (SNP) analysis, insertion deletion polymorphism (InDel) analysis, variable number of tandem repeat (VNTR) analysis, copy number variation (CNV) analysis or partial or whole genome sequencing. Methods for performing genomic analyses are known to the art and can include high throughput sequencing such as but not limited to those methods described in US Patent Nos. 7,335,762; 7,323,305; 7,264,929; 7,244,559; 7,211,390; 7,361,488; 7,300,788; and 7,280,922. Methods for performing genomic analyses can also include microarray methods as described hereinafter. In some cases, genomic analysis can be performed in combination with any of the other methods herein. For example, a sample can be obtained, tested for adequacy, and divided into aliquots. One or more aliquots can then be used for cytological analysis of the present disclosure, one or more can be used for RNA expression profiling methods of the present disclosure, and one or more can be used for genomic analysis. It is further understood that the present disclosure anticipates that one skilled in the art can perform other analyses on the biological sample that are not explicitly provided herein.

**(iii) Expression Product Profiling**

Gene expression profiling can comprise the measurement of the activity (or the expression) of one or more genes. Gene expression profiling can comprise the measurement of the activity or expression of a plurality of genes at once, to create a global picture of cellular function. Gene expression profiling can comprise measuring the activity or expression of between about 1 and about 20,000 or more genes; for example, about 1-20000, 1-10000, 1-5000, 1-1000, 1-500, 1-250, 1-100, 1-50, 1-10, 10-20000, 10-10000, 10-5000, 10-1000, 10-500, 10-250, 10-100, 10-50, 50-20000, 50-10000, 50-5000, 50-1000, 50-500, 50-250, 50-100, 100-20000, 100-10000, 100-5000, 100-1000, 100-500, 100-250, 250-20000, 250-10000, 250-5000, 250-1000, 250-500, 500-20000, 500-10000, 500-5000, 500-1000, 1000-20000, 1000-10000, 1000-5000, 5000-20000, 5000-10000, 10000-20000, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 110, 120, 130, 140, 150, 160, 170, 180, 190, 200, 210, 220, 230, 240, 250, 275, 300, 325, 350, 375, 400, 425, 450, 475, 500, 550, 600, 650, 700, 750, 800, 850, 900, 950, 1000, 1100, 1200, 1300, 1400, 1500, 1600, 1700, 1800, 1900, 2000, 2200, 2400, 2600, 2800, 3000, 3200, 3400, 3600, 3800, 4000, 4200, 4400, 4600, 4800, 5000, 5500, 6000, 6500, 7000, 7500, 8000, 8500, 9000, 9500, 10000, 11000, 12000, 13000, 14000, 15000, 16000, 17000, 18000, 19000, 20000 or more genes. Gene expression profiles can be used, for example, to distinguish between cells that are actively dividing, or to show how the cells may be predicted react to a particular treatment. Many experiments of this sort measure an entire genome simultaneously, that is, every gene present in a particular cell. Microarray technology can be used to measure the relative activity of previously identified target genes and other expressed sequences. Sequence based techniques, like serial analysis of gene expression (SAGE, Super-SAGE) are also used for gene expression profiling. SuperSAGE is especially accurate and can measure any active gene, not just a predefined set. In an RNA, mRNA or gene expression profiling microarray, the expression levels of thousands of genes can be simultaneously monitored to study the effects of certain treatments, diseases, and developmental stages on gene expression. For example, microarray-based gene expression profiling can be used to characterize gene signatures of a genetic disorder disclosed herein, or different cancer types, subtypes of a cancer, and/or cancer stages.

RNA (including mRNA, miRNA, siRNA, and cRNA) can be measured by one or more of the following: microarray, SAGE, blotting, RT-PCR, quantitative PCR, sequencing, RNA sequencing, DNA sequencing (e.g., sequencing of cDNA obtained from RNA); Next-Gen sequencing, nanopore sequencing, pyrosequencing, or Nanostring sequencing.

Expression profiling experiments can involve measuring the relative amount of gene expression products, such as mRNA, expressed in two or more experimental conditions. This is because altered levels of a specific sequence of a gene expression product can suggest a changed need for the protein coded for by the gene expression product, perhaps indicating a homeostatic response or a pathological condition. For example, if breast cancer cells express higher levels of mRNA associated with a particular transmembrane receptor than normal cells do, it may be that this receptor plays a role in breast cancer. One aspect of the present disclosure encompasses gene expression profiling as part of a process of identification or characterization of a biological sample, such as a diagnostic test for genetic disorders and cancers (e.g., thyroid cancer or lymphoma), and /or a test to predict the mutation state of one or more genes (e.g., BRAF V600E point mutation state) of the subject providing the biological sample. The tests disclosed herein can be used alone or in combination.

In some cases, RNA samples with RIN ≤5.0 are typically not used for multi-gene microarray analysis, and may instead be used only for single-gene RT-PCR and/or TaqMan assays. Microarray, RT-PCR and TaqMan assays are standard molecular techniques well known in the relevant art. TaqMan probe-based assays are widely used in real-time PCR including gene expression assays, DNA quantification and SNP genotyping.

In one case, gene expression products related to cancer that are known to the art are profiled. Such gene expression products have been described and include but are not limited to the gene expression products detailed in US patent Nos. 7,358,061; 7,319,011; 5,965,360; 6,436,642; and US patent applications 2003/0186248, 2005/0042222, 2003/0190602, 2005/0048533, 2005/0266443, 2006/0035244, 2006/083744, 2006/0088851, 2006/0105360, 2006/0127907, 2007/0020657, 2007/0037186, 2007/0065833, 2007/0161004, 2007/0238119, and 2008/0044824.

It is further anticipated that other gene expression products related to cancer may become known, and that the methods and compositions described herein can include such newly identified gene expression products.

In some cases of the present disclosure gene expression products are analyzed alternatively or additionally for characteristics other than expression level. For example, gene products can be analyzed for alternative splicing. Alternative splicing, also referred to as alternative exon usage, is the RNA splicing variation mechanism wherein the exons of a primary gene transcript, the pre-mRNA, are separated and reconnected (e.g., spliced) so as to produce alternative mRNA molecules from the same gene. In some cases, these linear combinations then undergo the process of translation where a specific and unique sequence of amino acids is specified by each of the alternative mRNA molecules from the same gene resulting in protein isoforms. Alternative splicing can include incorporating different exons or different sets of exons, retaining certain introns, or utilizing alternate splice donor and acceptor sites.

In some cases, markers or sets of markers can be identified that exhibit alternative splicing that is diagnostic for benign, malignant or normal samples. Additionally, alternative splicing markers can further provide an identifier for a specific type of thyroid cancer (e.g. papillary, follicular, medullary, or anaplastic). Alternative splicing markers diagnostic for malignancy known to the art include those listed in US Pat. No. 6,436,642.

In some cases, expression of gene expression products that do not encode for proteins such as miRNAs, and siRNAs can be assayed by the methods of the present disclosure. Differential expression of these gene expression products can be indicative of benign, malignant or normal samples. Differential expression of these gene expression products can further be indicative of the subtype of the benign sample (e.g. FA, NHP, LCT, BN, CN, HA) or malignant sample (e.g. FC, PTC, FVPTC, ATC, MTC). In some cases, differential expression of miRNAs, siRNAs, alternative splice RNA isoforms, mRNAs or any combination thereof can be assayed by the methods of the present disclosure.

**(1) In Vitro methods of determining expression product levels**

The general methods for determining gene expression product levels are known to the art and can include but are not limited to one or more of the following: additional cytological assays, assays for specific proteins or enzyme activities, assays for specific expression products including protein or RNA or specific RNA splice variants, *in situ* hybridization, whole or partial genome expression analysis, microarray hybridization assays, SAGE, enzyme linked immuno-absorbance assays, mass-spectrometry, immuno-histochemistry, blotting, sequencing, RNA sequencing, DNA sequencing (e.g., sequencing of cDNA obtained from RNA); Next-Gen sequencing, nanopore sequencing, pyrosequencing, or Nanostring sequencing. Gene expression product levels can be normalized to an internal standard such as total mRNA or the expression level of a particular gene including but not limited to glyceraldehyde 3 phosphate dehydrogenase, or tublin.

The gene expression product of the subject methods can be a protein, and the amount of protein in a particular biological sample can be analyzed using a classifier derived from protein data obtained from cohorts of samples. The amount of protein can be determined by one or more of the following: ELISA, mass spectrometry, blotting, immunohistochemistry, protein chip arrays, or any other protein quantitation technique.

Gene expression product markers and alternative splicing markers can be analyzed by microarray analysis using, for example, Affymetrix arrays, cDNA microarrays, oligonucleotide microarrays, spotted microarrays, or other microarray products from Biorad, Agilent, or Eppendorf. Microarrays can provide particular advantages because they can contain a large number of genes or alternative splice variants that can be assayed in a single experiment. In some cases, the microarray device can contain the entire human genome or transcriptome or a substantial fraction thereof allowing a comprehensive evaluation of gene expression patterns, genomic sequence, or alternative splicing. Markers can be found using standard molecular biology and microarray analysis techniques as described in Sambrook Molecular Cloning a Laboratory Manual 200 1 and Baldi, P., and Hatfield, W.G., DNA Microarrays and Gene Expression 2002.

Microarray analysis generally begins with extracting and purifying nucleic acid from a biological sample (e.g., a biopsy or fine needle aspirate) using methods known to the art. For expression and alternative splicing analysis it can be advantageous to extract and/or purify RNA from DNA. It can further be advantageous to extract and/or purify mRNA from other forms of RNA such as tRNA and rRNA.

Purified nucleic acid can further be labeled with a fluorescent label, radionuclide, or chemical label such as biotin, digoxigenin, or digoxin for example by reverse transcription, PCR, ligation, chemical reaction or other techniques. The labeling can be direct or indirect which can further require a coupling stage. The coupling stage can occur before hybridization, for example, using aminoallyl-UTP and NHS amino-reactive dyes (like cyanine dyes) or after, for example, using biotin and labelled streptavidin. In one example, modified nucleotides (e.g. at a 1 aaUTP: 4 TTP ratio) are added enzymatically at a lower rate compared to normal nucleotides, typically resulting in 1 every 60 bases (measured with a spectrophotometer). The aaDNA can then be purified with, for example, a column or a diafiltration device. The aminoallyl group is an amine group on a long linker attached to the nucleobase, which reacts with a reactive label (e.g. a fluorescent dye).

The labeled samples can then be mixed with a hybridization solution which can contain SDS, SSC, dextran sulfate, a blocking agent (such as COT1 DNA, salmon sperm DNA, calf thymum DNA, PolyA or PolyT), Denhardt's solution, formamine, or a combination thereof.

A hybridization probe can be a fragment of DNA or RNA of variable length, which is used to detect in DNA or RNA samples the presence of nucleotide sequences that are complementary to the sequence in the probe. The probe thereby hybridizes to single-stranded nucleic acid (DNA or RNA) whose base sequence allows probe-target base pairing due to complementarity between the probe and target. The labeled probe can be first denatured (by heating or under alkaline conditions) into single DNA strands and then hybridized to the target DNA.

To detect hybridization of the probe to its target sequence, the probe can be tagged (or labeled) with a molecular marker; commonly used markers including $^{32}$P or Digoxigenin, which is non-radioactive antibody-based marker. DNA sequences or RNA transcripts that have moderate to high sequence complementarity (*e.g.*, at least about 70%, 80%, 90%, 95%, 96%, 97%, 98%, 99%, or more complementarity) to the probe can then be detected by visualizing the hybridized probe via autoradiography or other imaging techniques. Detection of sequences with moderate or high complementarity can depend on how stringent the hybridization conditions are applied - high stringency, such as high hybridization temperature and low salt in hybridization buffers, can permit only hybridization between nucleic acid sequences that are highly similar, whereas low stringency, such as lower temperature and high salt, allows hybridization when the sequences are less similar. Hybridization probes used in DNA microarrays can comprise DNA covalently attached to an inert surface, such as coated glass slides or gene chips, and to which a mobile cDNA target is hybridized.

A mix comprising target nucleic acid to be hybridized to probes on an array can be denatured by heat or chemical means and added to a port in a microarray. The holes or ports can then be sealed and the microarray hybridized, for example, in a hybridization oven, where the microarray can be mixed by rotation, or in a mixer. After an overnight hybridization, non specific binding can be washed off (*e.g.*, with SDS and SSC). The microarray can then be dried and scanned in a machine comprising an illumination source (*e.g.*, laser) that excites the dye and a detector that measures emission by the dye. The image can be overlaid with a template grid and the intensities of the features (e.g., a feature comprising several pixels) can be quantified.

Various kits can be used for the amplification of nucleic acid and probe generation of the subject methods. Examples of kit that can be used in the present disclosure include but are not limited to Nugen WT-Ovation FFPE kit, cDNA amplification kit with Nugen Exon Module and Frag/Label module. The NuGEN WT-Ovation™ FFPE System V2 is a whole transcriptome amplification system that enables conducting global gene expression analysis on the vast archives of small and degraded RNA derived from FFPE samples. The system is comprised of reagents and a protocol required for amplification of as little as 50 ng of total FFPE RNA. The protocol can be used for qPCR, sample archiving, fragmentation, and labeling. The amplified cDNA can be fragmented and labeled in less than two hours for GeneChip® 3' expression array analysis using NuGEN's FL-Ovation™ cDNA Biotin Module V2. For analysis using Affymetrix Gene-Chip® Exon and Gene ST arrays, the amplified cDNA can be used with the WT-Ovation Exon Module, then fragmented and labeled using the FL-Ovation™ cDNA Biotin Module V2. For analysis on Agilent arrays, the amplified cDNA can be fragmented and labeled using NuGEN's FL-Ovation™ cDNA Fluorescent Module. More information on Nugen WT-Ovation FFPE kit can be obtained at www.nugeninc.com/nugen/index.cfm/products/amplification-systems/wt-ovation-ffpe/.

The Ambion WT-expression kit can be used in the subject methods. Ambion WT-expression kit allows amplification of total RNA directly without a separate ribosomal RNA (rRNA) depletion step. With the Ambion® WT Expression Kit, samples as small as 50 ng of total RNA can be analyzed on Affymetrix® GeneChip® Human, Mouse, and Rat Exon and Gene 1.0 ST Arrays. In addition to the lower input RNA requirement and high concordance between the Affymetrix® method and TaqMan® real-time PCR data, the Ambion® WT Expression Kit provides a significant increase in sensitivity. For example, a greater number of probe sets detected above background can be obtained at the exon level with the Ambion® WT Expression Kit as a result of an increased signal-to-noise ratio. Ambion WT-expression kit can be used in combination with additional Affymetrix labeling kit.

The AmpTec Trinucleotide Nano mRNA Amplification kit (6299-A15) can be used in the subject methods. The ExpressArt® TR*inucleotide* mRNA amplification Nano kit is suitable for a wide range, from 1 ng to 700 ng of input total RNA. According to the amount of input total RNA and the required yields of aRNA, it can be used for 1-round (input >300 ng total RNA) or 2-rounds (minimal input amount 1 ng total RNA), with aRNA yields in the range of >10 μg. AmpTec's proprietary TR*inucleotide* priming technology results in preferential amplification of mRNAs (independent of the universal eukaryotic 3'-poly(A)-sequence), combined with selection against rRNAs. More information on AmpTec Trinucleotide Nano mRNA Amplification kit can be obtained at www.amp-tec.com/products.htm. This kit can be used in combination with cDNA conversion kit and Affymetrix labeling kit.

Raw data from a microarray can then be normalized, for example, by subtracting the background intensity and then dividing the intensities making either the total intensity of the features on each channel equal or the intensities of a reference gene and then the t-value for all the intensities can be calculated. More sophisticated methods, include z-ratio, loess and lowess regression and RMA (robust multichip analysis), such as for Affymetrix chips.

(2) **In** Vivo methods of determining gene expression product levels

It is further anticipated that the methods and compositions of the present disclosure can be used to determine gene expression product levels in an individual without first obtaining a sample. For example, gene expression product levels can be determined *in vivo,* that is in the individual. Methods for determining gene expression product levels *in vivo* are known to the art and include imaging techniques such as CAT, MRI; NMR; PET; and optical, fluorescence, or biophotonic imaging of protein or RNA levels using antibodies or molecular beacons. Such methods are described in US 2008/0044824, US 2008/0131892. Additional methods for *in vivo* molecular profiling are contemplated to be within the scope of the present disclosure.

Molecular profiling can include the step of binding the sample or a portion of the sample to one or more probes of the present disclosure. Suitable probes bind to components of the sample *(e.g.,* gene expression products, *e.g.,* polynucleotides, DNA, RNA, polypeptides, and/or proteins) that are to be measured, such probes including, but not limited to antibodies or antibody fragments, aptamers, nucleic acids, and oligonucleotides. The binding of the sample, or sample components to the probes of the present disclosure represents a transformation of matter from sample to sample bound to one or more probes. In one case, the method of identifying, characterizing, or diagnosing biological samples (e.g., as cancerous or benign, as male or female, as mutant or wild-type) based on molecular profiling further comprises the steps of detecting gene expression products (e.g., mRNA or protein) levels in the sample; and classifying the test sample by inputting one or more differential gene expression product levels to a trained algorithm of the present disclosure; validating the sample classification using the selection and classification algorithms of the present disclosure; and identifying the sample as belonging to a tested category (e.g., as positive for a genetic disorder, a type of cancer, or any other test disclosed herein).

**(i) Comparison of sample to normal**

Results of molecular profiling performed on a sample from a subject (e.g., a test sample or a biological sample) can be compared to a biological sample that is known or suspected to be normal. A normal sample can be a sample that does not comprise or is expected to not comprise one or more cancers, diseases, or conditions under evaluation, or may test negative in the molecular profiling assay for the one or more cancers, diseases, or conditions under evaluation. A normal sample can be that which is, or is expected to be, free of any cancer, disease, or condition, or a sample that may test negative for any cancer disease or condition in the molecular profiling assay. The normal sample can be from a different subject from the subject being tested, or from the same subject. In some cases, the normal sample is a sample obtained from a buccal swab of a subject such as the subject being tested for example. The normal sample can be assayed at the same time, or at a different time from the test sample.

The results of an assay on the test sample can be compared to the results of the same assay on a normal sample. In some cases the results of the assay on the normal sample are from a database, or a reference. In some cases, the results of the assay on the normal sample are a known or generally accepted value or range of values by those skilled in the art. In some cases the comparison is qualitative. In other cases the comparison is quantitative. In some cases, qualitative or quantitative comparisons can involve but are not limited to one or more of the following: comparing fluorescence values, spot intensities, absorbance values, chemiluminescent signals, histograms, critical threshold values, statistical significance values, gene product expression levels, gene product expression level changes, alternative exon usage, changes in alternative exon usage, protein levels, DNA polymorphisms, copy number variations, indications of the presence or absence of one or more DNA markers or regions, or nucleic acid sequences.

**(ii) Evaluation of results**

The molecular profiling results can be evaluated using methods known to the art for correlating gene expression product levels or alternative exon usage with specific phenotypes such as malignancy, the type of malignancy (*e.g.*, follicular carcinoma), benignancy, normalcy (*e.g.*, disease or condition free), male, female, heterozygous, homozygous, mutant, or wild-type. A specified statistical confidence level can be determined in order to provide a diagnostic confidence level. For example, it can be determined that a confidence level of greater than 90% can be a useful predictor of malignancy, type of malignancy, benignancy, normalcy, male, female, heterozygous, homozygous, mutant, or wild-type. In other cases, more or less stringent confidence levels can be chosen. For example, a confidence level of about or at least about 50%, 60%, 70%, 75%, 80%, 85%, 90%, 95%, 97.5%, 99%, 99.5%, or 99.9% can be chosen as a useful phenotypic predictor. The confidence level provided can in some cases be related to the quality of the sample, the quality of the data, the quality of the analysis, the specific methods used, and/or the number of gene expression products analyzed. The specified confidence level for providing a diagnosis can be chosen on the basis of the expected number of false positives or false negatives and/or cost. Methods for choosing parameters for achieving a specified confidence level or for identifying markers with diagnostic power include but are not limited to Receiver Operating Characteristic (ROC) curve analysis, binormal ROC, principal component analysis, partial least squares analysis, singular value decomposition, least absolute shrinkage and selection operator analysis, least angle regression, and the threshold gradient directed regularization method.

**(iii) Data analysis**

Raw gene expression level and alternative splicing data can, in some cases, be improved through the application of algorithms designed to normalize and or improve the reliability of the data. The data analysis can require a computer or other device, machine or apparatus for application of the various algorithms described herein due to the large number of individual data points that are processed. A "machine learning algorithm" can refer to a computational-based prediction methodology, also known to persons skilled in the art as a "classifier", employed for characterizing a gene expression profile. The signals corresponding to certain expression levels, which can be obtained by, *e.g.*, microarray-based hybridization assays, can be subjected to the algorithm in order to classify the expression profile. Supervised learning can involve "training" a classifier to recognize the distinctions among classes and then "testing" the accuracy of the classifier on an independent test set. For new, unknown samples, the classifier can be used to predict the class in which the samples belong.

In some cases, the robust multi-array Average (RMA) method can be used to normalize raw data. The RMA method begins by computing background-corrected intensities for each matched cell on a number of microarrays. The background corrected values can be restricted to positive values as described by Irizarry et al. Biostatistics 2003 April 4 (2): 249-64. After background correction, the base-2 logarithm of each background corrected matched-cell intensity can then obtained. The background corrected, log-transformed, matched intensity on each microarray can then normalized using the quantile normalization method in which, for each input array and each probe expression value, the array percentile probe value is replaced with the average of all array percentile points. This normalization method is more completely described by Bolstad et al. Bioinformatics 2003. Following quantile normalization, the normalized data can then be fit to a linear model to obtain an expression measure for each probe on each microarray. Tukey's median polish algorithm (Tukey, J.W., Exploratory Data Analysis. 1977) can then be used to determine the log-scale expression level for the normalized probe set data.

Data can further be filtered to remove data that can be considered suspect. In some cases, data deriving from microarray probes that have fewer than about 4, 5, 6, 7 or 8 guanosine + cytosine nucleotides can be considered to be unreliable due to their aberrant hybridization propensity or secondary structure issues. Similarly, data deriving from microarray probes that have more than about 12, 13, 14, 15, 16, 17, 18, 19, 20, 21, or 22 guanosine + cytosine nucleotides can be considered unreliable due to their aberrant hybridization propensity or secondary structure issues.

In some cases, unreliable probe sets can be selected for exclusion from data analysis by ranking probe-set reliability against a series of reference datasets. For example, RefSeq or Ensembl (EMBL) can be considered very high quality reference datasets. Data from probe sets matching RefSeq or Ensembl sequences can, in some cases, be specifically included in microarray analysis experiments due to their expected high reliability. Similarly data from probe-sets matching less reliable reference datasets can be excluded from further analysis, or considered on a case by case basis for inclusion. In some cases, the Ensembl high throughput cDNA (HTC) and/or mRNA reference datasets can be used to determine the probe-set reliability separately or together. In other cases, probe-set reliability can be ranked. For example, probes and/or probe-sets that match perfectly to all reference datasets such as for example RefSeq, HTC, and mRNA, can be ranked as most reliable (1). Furthermore, probes and/or probe-sets that match two out of three reference datasets can be ranked as next most reliable (2), probes and/or probe-sets that match one out of three reference datasets can be ranked next (3) and probes and/or probe sets that match no reference datasets can be ranked last (4). Probes and or probe-sets can then be included or excluded from analysis based on their ranking. For example, one can choose to include data from category 1, 2, 3, and 4 probe-sets; category 1, 2, and 3 probe-sets; category 1 and 2 probe-sets; or category 1 probe-sets for further analysis. In another example, probe-sets can be ranked by the number of base pair mismatches to reference dataset entries. It is understood that there are many methods understood in the art for assessing the reliability of a given probe and/or probe-set for molecular profiling and the methods of the present disclosure encompass any of these methods and combinations thereof.

Data from probe-sets can be excluded from analysis if they are not expressed or expressed at an undetectable level (e.g., not above background). A probe-set can be judged to be expressed above background if for any group:

Integral from T0 to Infinity of the standard normal distribution < Significance (0.01)

Where:

T0 = Sqr(GroupSize) (T - P) / Sqr(Pvar),
GroupSize = Number of CEL files in the group,
T = Average of probe scores in probe-set,
P = Average of Background probes averages of GC content, and
Pvar = Sum of Background probe variances / (Number of probes in probe-set)^2,

This can allow including probe-sets in which the average of probe-sets in a group is greater than the average expression of background probes of similar GC content as the probe-set probes as the center of background for the probe-set and enables one to derive the probe-set dispersion from the background probe-set variance.

Probe-sets that exhibit no, or low, variance can be excluded from further analysis. Low-variance probe-sets can be excluded from the analysis via a Chi-Square test. A probe-set can be considered to be low-variance if its transformed variance is to the left of the 99 percent confidence interval of the Chi-Squared distribution with (N-1) degrees of freedom.

$$\text{(N-1)} * \text{Probe-set Variance} / \text{(Gene Probe-set Variance)} \sim \text{Chi-Sq(N-1)}$$

where N is the number of input CEL files, (N-1) is the degrees of freedom for the Chi-Squared distribution, and the 'probe-set variance for the gene' is the average of probe-set variances across the gene.

Probe-sets for a given gene or transcript cluster can be excluded from further analysis if they contain less than a minimum number of probes that pass through the previously described filter steps for GC content, reliability, variance and the like. For example, probe-sets for a given gene or transcript cluster can be excluded from further analysis if they contain less than about 1, 2, 3, 4, 5, 6, 7, 8, 9, 10, 11, 12, 13, 14, 15, or less than about 20 probes.

Methods of data analysis of gene expression levels or of alternative splicing can further include the use of a feature selection algorithm as provided herein. In some cases, feature selection is provided by use of the LIMMA software package (Smyth, G. K. (2005). Limma: linear models for microarray data. In: Bioinformatics and Computational Biology Solutions using Rand Bioconductor, R. Gentleman, V. Carey, S. Dudoit, R. Irizarry, W. Huber (eds.), Springer, New York, pages 397-420).

Methods of data analysis of gene expression levels and or of alternative splicing can further include the use of a pre-classifier algorithm. For example, an algorithm can use a cell-specific molecular fingerprint to pre-classify the samples according to their composition and then apply a correction/normalization factor. This data/information can then be fed in to a final classification algorithm which may incorporate that information to aid in the final diagnosis. In another example, an algorithm can use a confounding condition expression profile, such as a lymphoma signature, prior to application of a main classifier for another condition (e.g., thyroid cancer).

Methods of data analysis of gene expression levels and/or of alternative splicing can further include the use of a classifier algorithm as provided herein. A diagonal linear discriminant analysis, k-nearest neighbor algorithm, support vector machine (SVM) algorithm, linear support vector machine, random forest algorithm, or a probabilistic model-based method or a combination thereof is provided for classification of differential gene expression data (e.g., microarray data). Identified markers that distinguish samples (e.g., benign vs. malignant, normal vs. malignant, male vs. female, mutant vs. wildtype) or distinguish subtypes (e.g. PTC vs. FVPTC) can be selected based on statistical significance of the difference in expression levels between classes of interest. In some cases, the statistical significance is adjusted by applying a Benjamini Hochberg or another correction for false discovery rate (FDR).

In some cases, the classifier algorithm can be supplemented with a meta-analysis approach such as that described by Fishel and Kaufman et al. 2007 Bioinformatics 23(13): 1599-606. In some cases, the classifier algorithm can be supplemented with a meta-analysis approach such as a repeatability analysis. In some cases, the repeatability analysis selects markers that appear in at least one predictive expression product marker set.

Methods for deriving and applying posterior probabilities to the analysis of microarray data have been described for example in Smyth, G.K. 2004 Stat. Appl. Genet. Mol. Biol. 3: Article 3. In some cases, the posterior probabilities can be used to rank the markers provided by the classifier algorithm. In some cases, markers can be ranked according to their posterior probabilities and those that pass a chosen threshold can be chosen as markers whose differential expression is indicative of, or diagnostic for, samples that are in a category under investigation (e.g., benign, malignant, normal, ATC, PTC, MTC, FC, FN, FA, FVPTC, RCC, BCA, MMN, BCL, PTA, CN, HA, HC, LCT, NHP, male, female, BRAF wildtype, BRAF V600E, etc.). Illustrative threshold values include prior probabilities of about 0.7, 0.75, 0.8, 0.85, 0.9, 0.925, 0.95, 0.975, 0.98, 0.985, 0.99, 0.995 or higher.

A statistical evaluation of the results of the molecular profiling can provide a quantitative value or values indicative of one or more of the following: the likelihood of diagnostic accuracy; the likelihood of cancer, disease or condition; the likelihood of a particular cancer, disease or condition (e.g., tissue type or cancer subtype); the likelihood of a particular mutation state; and the likelihood of the success of a particular therapeutic intervention. Thus a physician, who is not likely to be trained in genetics or molecular biology, need not understand the raw data. Rather, the data can be presented directly to the physician in its most useful form to guide patient care. The results of the molecular profiling can be statistically evaluated using a number of methods known to the art including, but not limited to: the students T test, the two sided T test, pearson rank sum analysis, hidden markov model analysis, analysis of q-q plots, principal component analysis, one way ANOVA, two way ANOVA, LIMMA and the like.

The use of molecular profiling, alone or in combination with cytological analysis, can provide a classification, identification, or diagnosis that is between about 85% accurate and about 99% or about 100% accurate. In some cases, the molecular profiling process and/or cytology provide a classification, identification, diagnosis of malignant, benign, or normal that is about, or at least about 85%, 86%, 87%, 88%, 90%, 91%, 92%, 93%, 94%, 95%, 96%, 97%, 97.5%, 98%, 98.5%, 99%, 99.5%, 99.75%, 99.8%, 99.85%, or 99.9% accurate. In some cases, the molecular profiling process and/or cytology provide a classification, identification, or diagnosis of the presence of a particular tissue type (e.g. NML, FA, NHP, LCT, HA, FC, PTC, FVPTC, MTC, HC, ATC, RCC, BCA, MMN, BCL, and/or PTA) that is about, or at least about 85%, 86%, 87%, 88%, 90%, 91%, 92%, 93%, 94%, 95%, 96%, 97%, 97.5%, 98%, 98.5%, 99%, 99.5%, 99.75%, 99.8%, 99.85%, or 99.9% accurate.

In some cases, accuracy can be determined by tracking the subject over time to determine the accuracy of the original diagnosis. In other cases, accuracy can be established in a deterministic manner or using statistical methods. For example, receiver operator characteristic (ROC) analysis can be used to determine the optimal assay parameters to achieve a specific level of accuracy, specificity, positive predictive value, negative predictive value, and/or false discovery rate. Methods for using ROC analysis in cancer diagnosis are known in the art and have been described for example in US Patent Application No. 2006/019615.

Gene expression products and compositions of nucleotides encoding for such products that are determined to exhibit the greatest difference in expression level or the greatest difference in alternative splicing between categories (e.g., benign and normal, benign and malignant, malignant and normal, male and female, lymphoma and LCT, mutant and wildtype, etc.) can be chosen for use as molecular profiling reagents of the present disclosure. Such gene expression products can be particularly useful by providing a wider dynamic range, greater signal to noise, improved diagnostic power, lower likelihood of false positives or false negative, or a greater statistical confidence level than other methods known or used in the art.

The use of molecular profiling alone, or in combination with cytological analysis, can reduce the number of samples scored as non-diagnostic by about, or at least about 100%, 99%, 95%, 90%, 80%, 75%, 70%, 65%, or about 60% when compared to the use of standard cytological techniques known to the art. In some cases, the methods of the present disclosure can reduce the number of samples scored as intermediate or suspicious by about, or at least about100%, 99%, 98%, 97%, 95%, 90%, 85%, 80%, 75%, 70%, 65%, or about 60%, when compared to the standard cytological methods used in the art.

Molecular profile results can be presented as a report on a computer screen or as a paper record. In some cases, the report can include, but is not limited to, such information as one or more of the following: the number of genes differentially expressed, the suitability of the original sample, the number of genes showing differential alternative splicing, a diagnosis, a statistical confidence for the diagnosis, the likelihood of cancer or malignancy, and indicated therapies.

**(iv) Categorization of samples based on molecular profiling results**

The results of the molecular profiling can be classified into one of the following: benign (free of a malignant cancer, disease, or condition), malignant (positive diagnosis for a cancer, disease, or condition), or non diagnostic (providing inadequate information concerning the presence or absence of a cancer, disease, or condition; or as unsuitable for the selected test due to a confounding condition). The results of molecular profiling can also be to categorize a sample according to a mutation state (e.g., BRAF V600E state). In some cases, the results of the molecular profiling can be classified into benign versus suspicious (suspected to be positive for a cancer, disease, or condition) categories. In some cases, a diagnostic result can further classify the type of cancer, disease or condition, such as by identifying the presence or absence of one or more types of tissues, including but not limited to NML, FA, NHP, LCT, HA, FC, PTC, FVPTC, MTC, HC, ATC, RCC, BCA, MMN, BCL, and PTA. In other cases, a diagnostic result can indicate a certain molecular pathway is involved in the cancer disease or condition, or a certain grade or stage of a particular cancer disease or condition. In still other cases a diagnostic result can inform an appropriate therapeutic intervention, such as a specific drug regimen like a kinase inhibitor such as Gleevec or any drug known to the art, or a surgical intervention like a thyroidectomy or a hemithyroidectomy.

Biological samples can be classified using a trained algorithm. Trained algorithms of the present disclosure include algorithms that have been developed using two or more reference sets of known categorization (e.g., malignant, benign, and normal samples including but not limited to samples with one or more histopathologies listed in Figure 2; mutant and wild-type samples, *etc.).* The algorithms can be further trained using one or more of the classification panels in Figure 3, Table 1, Table 2, Table 9, Table 10, Table 11, Table 12, Table 14, Table 15, Table 23, Table 24, Table 25, Table 26 and Table 27 and using any combination of panels.

Training can comprise comparison of gene expression product levels in a first set of one or more tissue types to gene expression product levels in a second set of one or more tissue types, where the first set of tissue types includes at least one tissue type that is not in the second set. In some cases, either the entire algorithm or portions of the algorithm can be trained using comparisons of expression levels of biomarker panels within a classification panel against all other biomarker panels (or all other biomarker signatures) used in the algorithm. The first set of tissue types and/or the second set of tissue types can include 1, 2, 3, 4, 5, 6, 7, 8, 9, 10, 11, 12, 13, 14, or 15 of the types selected from NML, FA, NHP, LCT, HA, FC, PTC, FVPTC, MTC, HC, ATC, RCC, BCA, MMN, BCL, and PTA, in any combination, and from any source, including surgical and/or FNA samples.

Algorithms suitable for categorization of samples include but are not limited to k-nearest neighbor algorithms, support vector algorithms, naive Bayesian algorithms, neural network algorithms, hidden Markov model algorithms, genetic algorithms, or any combination thereof.

In some cases, trained algorithms of the present disclosure can incorporate data other than gene expression or alternative splicing data such as, but not limited to, DNA polymorphism data, sequencing data, scoring or diagnosis by cytologists or pathologists of the present disclosure, information provided by the pre-classifier algorithm of the present disclosure, or information about the medical history of the subject.

When classifying a biological sample (e.g., for diagnosis of cancer, as male or female, as mutant or wild-type, *etc.),* there are typically two possible outcomes from a binary classifier. When a binary classifier is compared with actual true values (e.g., known values from the biological sample), there are typically four possible outcomes. If the outcome from a prediction is $p$ (where "p" is a positive classifier output, such as a malignancy, or presence of a particular disease tissue as described herein) and the actual value is also $p$, then it is called a *true positive* (TP); however if the actual value is $n$ then it is the to be a *false positive* (FP). Conversely, a *true negative* (e.g., definitive benign) has occurred when both the prediction outcome and the actual value are $n$ (where "n" is a negative classifier output, such as benign, or absence of a particular disease tissue as described herein), and *false negative* is when the prediction outcome is $n$ while the actual value is $p$. For example, consider a diagnostic test that seeks to determine whether a person has a certain disease. A false positive in this case occurs when the person tests positive, but actually does not have the disease. A false negative, on the other hand, occurs when the person tests negative, suggesting they are healthy, when they actually do have the disease. In some cases, a Receiver Operator Characteristic (ROC) curve assuming real-world prevalence of subtypes can be generated by re-sampling errors achieved on available samples in relevant proportions.

The positive predictive value (PPV), or precision rate, or post-test probability of a classification or diagnosis (e.g., a disease diagnosis) can be the proportion of patients with positive test results who are correctly diagnosed. The PPV value can be a measure of a diagnostic method as it reflects the probability that a positive test reflects the underlying condition being tested for; however, its value can depend on the prevalence of the condition tested (e.g., disease), which can vary. In one example, FP (false positive); TN (true negative); TP (true positive); FN (false negative).

$$\text{False positive rate } (\alpha) = FP / (FP + TN) - \text{specificity}$$

$$\text{False negative rate } (\beta) = FN / (TP + FN) \text{ -- sensitivity}$$

$$|\text{Power} = \text{sensitivity} = 1 - \beta$$

$$|\text{Likelihood-ratio positive} = \text{sensitivity} / (1 - \text{specificity})$$

$$\text{Likelihood-ratio negative} = (1 - \text{sensitivity}) / \text{specificity}$$

The negative predictive value can be defined as the proportion of patients with negative test results who are correctly diagnosed. PPV and NPV measurements can be derived using appropriate disease subtype prevalence estimates. An estimate of the pooled malignant disease prevalence can be calculated from the pool of indeterminates, which roughly classify into B vs M by surgery. For subtype specific estimates, in some cases, disease prevalence can sometimes be incalculable because there are not any available samples. In these cases, the subtype disease prevalence can be substituted by the pooled disease prevalence estimate.

The level of expression products or alternative exon usage can indicate of one or the following: NML, FA, NHP, LCT, HA, FC, PTC, FVPTC, MTC, HC, ATC, RCC, BCA, MMN, BCL, and PTA. The level of expression products or alternative exon usage can be indicative of one of the following: follicular cell carcinoma, anaplastic carcinoma, medullary carcinoma, or papillary carcinoma. In some cases, the level of gene expression products or alternative exon usage in indicative of Hurthle cell carcinoma or Hurthle cell adenoma. In some cases, the one or more genes selected using the methods of the present disclosure for diagnosing cancer contain representative sequences corresponding to a set of metabolic or signaling pathways indicative of cancer.

The results of the expression analysis of the subject methods can provide a statistical confidence level that a given diagnosis or categorization is correct. The statistical confidence level can be at least about, or more than about 85%, 90%, 91%, 92%, 93%, 94%, 95%, 96%, 97%, 98%, 99% 99.5%, or more.

In another aspect, the present disclosure provides a composition for diagnosing cancer comprising oligonucleotides comprising a portion of one or more of the genes listed in Figure 4, Table 18, Table 23, Table 24, Table 25, Table 26, or Table 27, or their complement(s), and a substrate upon which the oligonucleotides are covalently attached. The composition of the present disclosure is suitable for use in diagnosing cancer at a specified confidence level using a trained algorithm. In one example, the composition of the present disclosure is used to diagnose thyroid cancer.

For example, in the specific case of thyroid cancer, molecular profiling of the present disclosure can further provide a diagnosis for the specific type of thyroid cancer (e.g., papillary, follicular, medullary, or anaplastic), or other tissue type selected from NML, FA, NHP, LCT, HA, FC, PTC, FVPTC, MTC, HC, ATC, RCC, BCA, MMN, BCL, and PTA. The methods of the disclosure can also provide a diagnosis of the presence or absence of Hurthle cell carcinoma or Hurthle cell adenoma. The results of the molecular profiling can further allow one skilled in the art, such as a scientist or medical professional, to suggest or prescribe a specific therapeutic intervention. Molecular profiling of biological samples can also be used to monitor the efficacy of a particular treatment after the initial diagnosis. It is further understood that in some cases, molecular profiling can be used in place of, rather than in addition to, established methods of cancer diagnosis.

In another aspect, the present disclosure provides compositions for identifying lymphomas in a biological sample comprising polynucleotides that correspond to all or a fragment of one or more biomarkers found in Table 1. The polynucleotides can be attached to a substrate; for example, the polynucleotides can be attached to a glass slide or a microarray chip. The compositions for identifying lymphomas in the biological sample can be used to pre-screen samples prior to the application of a main classifier. In one example, the biological sample can be pre-screened for the presence of lymphoma prior to the application of a diagnostic classifier to identify thyroid cancers. In this example, the presence of a lymphoma signature in the biological sample can indicate that the thyroid cancer classifier should not be used on the sample.

In another aspect, the present disclosure provides compositions for predicting whether a subject is heterozygous, homozygous, or wild-type for a genetic mutation (e.g., a BRAF V600E mutation) comprising polynucleotides corresponding to all or a fragment of one or more genes found in Table 1, Table 2, Table 9, Table 10, Table 23, Table 24, Table 25, Table 26 or Table 27. Compositions are also provided that can be used to adjust for cell content variation in biological samples comprising polynucleotides corresponding to all or a fragment of one or more genes found in Table 1 or Table 23. The polynucleotides can be attached to a substrate, such as a glass slide or microarray chip. The compositions, and associated methods, for predicting genetic mutations can be used alone or in combination with one or more of the compositions and methods disclosed herein. For example, the compositions and methods for predicting whether a biological sample comprises the BRAF V600E genetic mutation can be used in addition to a main thyroid cancer classifier.

### (v) Monitoring of Subjects or Therapeutic Interventions via Molecular Profiling

Subjects can be monitored using methods and compositions of the present disclosure. For example, a subject can be diagnosed with cancer or a genetic disorder. This initial diagnosis can optionally involve the use of molecular profiling. The subject can be prescribed a therapeutic intervention such as a thyroidectomy for a subject suspected of having thyroid cancer. The results of the therapeutic intervention can be monitored on an ongoing basis by molecular profiling to detect the efficacy of the therapeutic intervention. In another example, a subject can be diagnosed with a benign tumor or a precancerous lesion or nodule, and the tumor, nodule, or lesion can be monitored on an ongoing basis by molecular profiling to detect any changes in the state of the tumor or lesion.

Molecular profiling can also be used to ascertain the potential efficacy of a specific therapeutic intervention prior to administering to a subject. For example, a subject can be diagnosed with cancer. Molecular profiling can indicate the upregulation of a gene expression product known to be involved in cancer malignancy, such as for example the RAS oncogene. A tumor sample can be obtained and cultured *in vitro* using methods known to the art. The application of various inhibitors of the aberrantly activated or dysregulated pathway, or drugs known to inhibit the activity of the pathway can then be tested against the tumor cell line for growth inhibition. Molecular profiling can also be used to monitor the effect of these inhibitors on for example down-stream targets of the implicated pathway.

### (vi) Molecular Profiling as a Research Tool

Molecular profiling can be used as a research tool to identify new markers for diagnosis of suspected tumors; to monitor the effect of drugs or candidate drugs on biological samples such as tumor cells, cell lines, tissues, or organisms; or to uncover new pathways for oncogenesis and/or tumor suppression.

### (vii) Biomarker groupings based on molecular profiling

The current disclosure provides groupings or panels of biomarkers that can be used to characterize, rule in, rule out, identify, and/or diagnose pathology within the thyroid. Such biomarker panels are obtained from correlations between patterns of gene (or biomarker) expression levels and specific types of samples (*e.g.*, malignant subtypes, benign subtypes, normal tissue, or samples with foreign tissue). The panels of biomarkers can also be used to characterize, rule in, rule out, identify, and/or diagnose benign conditions of the thyroid. In some cases, the number of panels of biomarkers is greater than 1, 2, 3, 4, 5, 6, 7, 8, 9,10, 11, 12, 13, 14, 15, 16, 17, 18, 19, 20, 25, 30, 40, 50, 60, 70, 80, 90, or 100 panels of biomarkers. The number of panels of biomarkers can be greater than 12 panels, (*e.g.*, 16 panels of biomarkers). Examples of sixteen panels of biomarkers include, but are not limited to the following (they are also provided in Figure 2):

1    Normal Thyroid (NML)

2    Lymphocytic, Autoimmune Thyroiditis (LCT)

3    Nodular Hyperplasia (NHP)

4    Follicular Thyroid Adenoma (FA)

5    Hurthle Cell Thyroid Adenoma (HC)

6    Parathyroid (non thyroid tissue)

7    Anaplastic Thyroid Carcinoma (ATC)

8    Follicular Thyroid Carcinoma (FC)

9      Hurthle Cell Thyroid Carcinoma (HC)

10     Papillary Thyroid Carcinoma (PTC)

11     Follicular Variant of Papillary Carcinoma (FVPTC)

12     Medullary Thyroid Carcinoma (MTC)

13     Renal Carcinoma metastasis to the Thyroid (RCC)

14     Melanoma metastasis to the Thyroid (MMN)

15     B cell Lymphoma metastasis to the Thyroid (BCL)

16     Breast Carcinoma metastasis to the Thyroid (BCA)

Each panel includes a set of biomarkers (*e.g.*, gene expression products or alternatively spliced exons associated with the particular cell type) that can be used to characterize, rule in, rule out, and/or diagnose a given pathology (or lack thereof) within the thyroid. Biomarkers can be associated with more than one cell type. Panels 1-6 describe benign pathology, while panels 7-16 describe malignant pathology. These multiple panels can be combined (each in different proportion) to create optimized panels that are useful in a two-class classification system (*e.g.*, benign versus malignant). Alternatively, biomarker panels can be used alone or in any combination as a reference or classifier in the classification, identification, or diagnosis of a thyroid tissue sample as comprising one or more tissues selected from NML, FA, NHP, LCT, HA, FC, PTC, FVPTC, MTC, HC, ATC, RCC, BCA, MMN, BCL, and PTA. Combinations of biomarker panels can contain at least about 1, 2, 3, 4, 5, 6, 7, 8, 9, 10, 11, 12, 13, 14, 15, 16, or more biomarker panels. In some cases, where two are more panels are used in the classification, identification, or diagnosis, the comparison is sequential. Sequential comparison can comprise 1, 2, 3, 4, 5, 6, 7, 8, 9, 10, or more sets comprising 2, 3, 4, 5, 6, 7, 8, 9, 10, or more biomarker panels that are compared simultaneously as a step in the sequential comparison, each set comprising at least one different biomarker panel than compared at other steps in the sequence (and can optionally be completely nonoverlapping).

The biological nature of the thyroid and each pathology found within it suggest there can be some redundancy between the plurality of biomarkers in one panel versus the plurality of biomarkers in another panel. For each pathology subtype, each diagnostic panel can be heterogeneous and semi-redundant, or not redundant, with the biomarkers in another panel. In general, heterogeneity and redundancy can reflect the biology of the tissues samples in a given thyroid sample (*e.g.*, surgical or FNA sample) and the differences in gene expression that differentiates each pathology subtype from one another.

In one aspect, the diagnostic value of the present disclosure lies in the comparison of i) one or more markers in one panel, versus ii) one or more markers in each additional panel.

The pattern of gene expression demonstrated by a particular biomarker panel reflects the "signature" of each panel. For example, the panel of Lymphocytic Autoimmune Thyroiditis (LCT) can have certain sets of biomarkers that display a particular pattern or signature. Within such signature, specific biomarkers can be upregulated, others can be not differentially expressed, and still others can be down regulated. The signatures of particular panels of biomarkers can themselves be grouped in order to diagnose or otherwise characterize a thyroid condition; such groupings can be referred to as "classification panels". Each classification panel can comprise at least 1, 2, 3, 4, 5, 6, 7, 8, 9, 10, 15, or more than 20 biomarker panels.

Classification panels can contain specified biomarkers (TCIDs) and use information saved during algorithm training to rule in, or rule out a given sample as "benign," "suspicious," or as comprising or not comprising one or more tissue types (e.g. NML, FA, NHP, LCT, HA, FC, PTC, FVPTC, MTC, HC, ATC, RCC, BCA, MMN, BCL, and PTA). Each classification panel can use simple decision rules to filter incoming samples, effectively removing any flagged samples from subsequent evaluation if the decision rules are met (*e.g.,* a sample can be characterized regarding the identity or status of one or more tissue types contained therein). The biomarker panels and classification panels provided herein can be useful for classifying, characterizing, identifying, and/or diagnosing thyroid cancer or other thyroid condition (including diagnosing the thyroid as normal). The biomarker panels and classification panels provided herein can also be useful for classifying, characterizing, identifying, and/or diagnosing samples according to gender, mutation state, cell-type composition, and/or the presence of confounding conditions. However, biomarker panels and classification panels similar to the present panels can be obtained using similar methods and can be used for other diseases or disorders, such as other diseases or disorder described herein.

Figure 3 provides an example of a set of classification panels that can be used to diagnose a thyroid condition.

For example, as shown in Figure 3, one classification panel can contain a single biomarker panel such as the MTC biomarker panel (e.g., classification panel #1); another classification panel can contain a single biomarker panel such as the RCC biomarker panel (e.g., classification panel #2); yet another classification panel can contain a single biomarker panel such as the PTA biomarker panel (e.g., classification panel #3); yet another classification panel can contain a single biomarker panel such as the BCA biomarker panel (e.g., classification panel #4); yet another classification panel can contain a single biomarker panel such as the MMN biomarker panel (e.g., classification panel #5); yet another classification panel can contain a two biomarker panels such as the HA and HC biomarker panels (e.g., classification panel 6); and yet another classification panel can contain a combination of the FA, FC, NHP, PTC, FVPTC, HA, HC, and LCT panels (e.g., classification panel #7, which is also an example of a "main" classifier). One or more such classifiers can be used simultaneously or in sequence, and in any combination, to classify, characterize, identify, or diagnose a thyroid sample. In some cases, a sample is identified as containing or not containing tissue having an HA or HC tissue type.

Other potential classification panels that can be useful for characterizing, identifying, and/or diagnosing thyroid cancers can include: 1) biomarkers of metastasis to the thyroid from non-thyroid organs (e.g., one of or any combination of two or more of the following: RCC, MTC, MMN, BCL, and BCA panels); 2) biomarkers correlated with thyroid tissue that originated from non-thyroid organs (e.g., any one of or any combination of two or more of the following: RCC, MTC, MMN, BCL, BCA, and PTA panels); 3) biomarkers with significant changes in alternative gene splicing, 4) KEGG Pathways, 5) gene ontology; 6) biomarker panels associated with thyroid cancer (e.g., one of or groups of two or more of the following panels: FC, PTC, FVPTC, MTC, HC, and ATC); 7) biomarker panels associated with benign thyroid conditions (e.g., one of or groups of two or more of the following: FA, NHP, LCT, or HA); 8) biomarker panels associated with benign thyroid conditions or normal thyroid tissue (e.g., one of or groups of two or more of the following: FA, NHP, LCT, HA or NML); 9) biomarkers related to signaling pathways such as adherens pathway, focal adhesion pathway, and tight junction pathway, or other pathway described in International Application No. PCT/US2009/006162, filed 11/17/2009. In addition, biomarkers that indicate metastasis to the thyroid from a non-thyroid organ can be used in the subject methods and compositions. Metastatic cancers that metastasize to thyroid that can be used for a classifier to diagnose a thyroid condition include but are not limited to: metastatic parathyroid cancer, metastatic melanoma, metastatic renal carcinoma, metastatic breast carcinoma, and metastatic B cell lymphoma.

Classification panels that can be used for characterizing, identifying, and/or diagnosing thyroid cancers can also include panels to identify sample mix-ups, panels to provide further information about the genetic underpinnings of a cancer, and/or panels to pre-screen samples prior to the application of the thyroid cancer classifier panels. In another example, a classifier panel to predict whether a biological sample is heterozygous or wild type for the BRAF V600E point mutation can be used to further classify a malignant diagnosis. In some cases, a classifier panel to predict the presence of a driver mutation (e.g., BRAF mutation) can be used to further classify cancer subtype. Driver mutations can be causally implicated in oncogenesis or tumor survival. Such mutations can be positively selected during carcinogenesis and can show a recurrent pattern within or across tumor types. There are DNA driver mutations that putatively drive aggressive forms of cancer, such as BRAF, KRAS, etc. However not all subjects with these mutations develop an aggressive disease in thyroid as having an extrathyroid invasion, lymph node or distant metastasis and accelerated progression to death. Similarly, many subjects that lack these DNA driver mutations may have aggressive thyroid cancer. The methods or classification panels described herein can be useful in identifying the presence of the one or more driver mutations. The BRAF mutations may be driver mutations that can cause a more aggressive tumor. In some cases, the biological samples may be further classified as having an aggressive prognosis or not having an aggressive prognosis. The subject can be treated based upon the classification. In another example, a classifier panel that can detect or diagnose the presence of lymphoma can be used prior to a thyroid cancer classifier; the used of the lymphoma classifier can reduce the rate of false positives for a thyroid cancer classifier.

In some cases, the method provides a number, or a range of numbers, of biomarkers (including gene expression products) that are used to diagnose or otherwise characterize a biological sample. As described herein, such biomarkers can be identified using the methods provided herein, particularly the methods of correlating gene expression signatures with specific types of tissue, such as the types listed in Figure 2. The sets of biomarkers indicated in Figure 4, Table 1, Table 2, Table 3, Table 4, Table 5, Table 9, Table 10, Table 11, Table 12, Table 14, Table 15, Table 18 , Table 19 , Table 23, Table 24, Table 25, Table 26 and Table 27 can be obtained using the methods described herein. The biomarkers can also be used, in turn, to classify tissue. In some cases, all of the biomarkers in, Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27 are used to diagnose or otherwise characterize thyroid tissue. In some cases, a subset of the biomarkers in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27 are used to diagnose or otherwise characterize thyroid tissue. In some cases, all, or a subset, of the biomarkers in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27, along with additional biomarkers, are used to diagnose or otherwise characterize thyroid tissue. In some cases, at least 1, 2, 3, 4, 5, 6, 7, 8, 9, 10, 15, 20, 25, 30, 33, 35, 38, 40, 43, 45, 48, 50, 53, 58, 63, 65, 68, 100, 120, 140, 142, 145, 147, 150, 152, 157, 160, 162, 167, 175, 180, 185, 190, 195, 200, or 300 total biomarkers are used to diagnose or otherwise characterize thyroid tissue. In other cases, at most 2, 3, 4, 5, 6, 7, 8, 9, 10, 15, 20, 25, 30, 33, 35, 38, 40, 43, 45, 48, 50, 53, 58, 63, 65, 68, 100, 120, 140, 142, 145, 147, 150, 152, 157, 160, 162, 167, 175, 180, 185, 190, 195, 200, or 300 total biomarkers are used to diagnose or otherwise characterize thyroid tissue. In still other cases, at least 1, 2, 3, 4, 5, 6, 7, 8, 9, 10, 15, 20, 25, 30, 33, 35, 38, 40, 43, 45, 48, 50, 53, 58, 63, 65, 68, 100, 120, 140, 142, 145, 147, 150, 152, 157, 160, 162, 167, 175, 180, 185, 190, or more of the biomarkers identified in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27 are used to diagnose or otherwise characterize thyroid tissue.

Exemplary biomarkers and an example of their associated classification panel (and/or biomarker panel) are listed in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27. The methods and compositions provided herein can use any or all of the biomarkers listed in Figure 3, Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27. In some cases, the biomarkers listed in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27 are used as part of the corresponding classification panel indicated in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27.

In other cases, the biomarkers in Figure 3, Figure 4, Table 1, Table 2, Table 3, Table 4, Table 5, Table 9, Table 10, Table 11, Table 12, Table 14, Table 15, Table 18, Table 19, Table 23, Table 24, Table 25, Table 26 and Table 27 can be used for a different classification panel than the ones indicated in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27.

Optimized classification panels can be assigned specific numbers of biomarkers per classification panel. For example, an optimized classification panel can be assigned between about 1 and about 500; for example about 1-500, 1-400, 1-300, 1-200, 1-100, 1-50, 1-25, 1-10, 10-500, 10-400, 10-300, 10-200, 10-100, 10-50, 10-25, 25-500, 25-400, 25-300, 25-200, 25-100, 25-50, 50-500, 50-400, 50-300, 50-200, 50-100, 100-500, 100-400, 100-300, 100-200, 200-500, 200-400, 200-300, 300-500, 300-400, 400-500, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, or any included range or integer. biomarkers. For example, as shown in Figure 3, a classification panel can contain 5, 33, or 142 biomarkers. Methods and compositions of the disclosure can use biomarkers selected from 1, 2, 3, 4, 5, 6, 7, 8, 9, 10, 11, 12, 13, 14, 15 or 16 or more biomarker panels and each of these biomarker panels can have more than 1, 2, 3, 4, 5, 6, 7, 8, 9, 10, 15, 20, 25, 30, 33, 35, 38, 40, 43, 45, 48, 50, 53, 58, 63, 65, 68, 100, 120, 140, 142, 145, 147, 150, 152, 157, 160, 162, 167, 175, 180, 185, 190, 195, 200, 300, 400, 500, or more biomarkers, in any combination. In some cases, the set of markers combined give a specificity or sensitivity of greater than 60%, 70%, 75%, 80%, 85%, 86%, 87%, 88%, 89%, 90%, 91%, 92%, 93%, 94%, 95%, 96%, 97%, 98%, 99%, or 99.5%, or a positive predictive value or negative predictive value of at least 90%, 95%, 95.5%, 96%, 96.5%, 97%, 97.5%, 98%, 98.5%, 99%, 99.5% or more.

Analysis of the gene expression levels can involve sequential application of different classifiers described herein to the gene expression data. Such sequential analysis can involve applying a classifier obtained from gene expression analysis of cohorts of diseased thyroid tissue, followed by applying a classifier obtained from analysis of a mixture of different samples of thyroid tissue, with some of the samples containing diseased thyroid tissues and others containing benign thyroid tissue. The diseased tissue can be malignant or cancerous tissue (including tissue that has metastasized from a non-thyroid organ). The diseased tissue can be thyroid cancer or a non-thyroid cancer that has metastasized to the thyroid. The classifier can be obtained from analysis of gene expression patterns in benign tissue, normal tissue, and/or non-thyroid tissue (*e.g.*, parathyroid tissue). The diseased tissue can be HA and/or HC tissue.

The classification process can begin when each classification panel receives, as input, biomarker expression levels (*e.g.*, summarized microarray intensity values, qPCR, or sequencing data) derived from a biological sample. The biomarkers and expression levels specified in a classification panel can then be evaluated. If the data from a given sample matches the rules specified within the classification panel (or otherwise correlate with the signature of the classification panel), its data output can flag the sample and prevent it from further evaluation and scoring by the main (downstream) classifier. When a classification panel flags a sample, the system can be configured to automatically return a "suspicious" call for that sample. When a classification panel does not flag a sample, the evaluation can continue downstream to the next classification panel and it can be flagged or not flagged. In some situations, the classification panels are applied in a specific order; in other cases, the order of the applications can be any order. In some cases, classification panels 1-5 from Figure 3 in the optimized list of thyroid gene signature panels are executed in any particular order, but then are followed by classification panel 6, which then precedes application of the main classifier (*e.g.*, classification panel 7). In some cases, a classification panel to identify a confounding condition can be used to pre-screen samples prior to application of the main classifier. For example, a classification panel comprising any or all of the markers in Table 1 can be used to identify the presence of a lymphoma in the biological sample (*e.g.*, a thyroid sample). Pre-screening samples using the lymphoma classifier panel can reduce the number of false positives returned by the main classifier.

One or more classification panels can be used to further characterize the biological sample. For example, if the sample is positive for a cancer (*e.g.*, a thyroid cancer), a classification panel comprising any or all of the biomarkers in Table 19 or Table 23 can be used to predict whether the biological sample is heterozygous, homozygous, or wild-type for a BRAF V600E point mutation. The classification panel to predict the BRAF V600E point mutation can additionally or alternatively comprise any or all of the markers from Table 10 and can optionally involve covariate analysis to account for cellular heterogeneity. For biological samples of the thyroid (*e.g.*, fine needle aspirations or tissue samples of the thyroid), covariate analysis can comprise evaluation of Follicular cell signal strength (*e.g.*, using any or all of the markers in Table 3), Hurthle cell signal strength (*e.g.*, using any or all of the markers in Table 4), and/or lymphocytic cell signal strength (*e.g.*, using any or all of the markers in Table 5) in any combination.

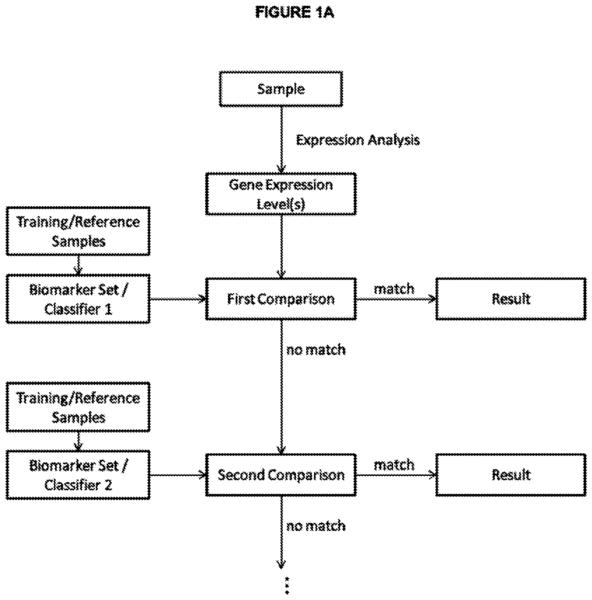

An example illustration of a classification process in accordance with the methods of the disclosure is provided in Figure 1A. The process begins with determining, such as by gene expression analysis, expression level(s) for one or more gene expression products from a sample (*e.g.*, a thyroid tissue sample) from a subject. Separately, one or more sets of reference or training samples can be analyzed to determine gene expression data for at least two different sets of biomarkers, the gene expression data for each biomarker set comprising one or more gene expression levels correlated with the presence of one or more tissue types. The gene expression data for a first set of biomarkers can be used to train a first classifier; gene expression data for a second set can be used to train a second classifier; and so on for 2, 3, 4, 5, 6, 7, 8, 9, 10, 11, 12, 13, 14, 15, 16, or more sets of biomarkers and optionally corresponding classifiers. The sets of reference or training samples used in the analysis of each of the sets of biomarkers can be overlapping or nonoverlapping. In some cases, the reference or training samples comprise HA and/or HC tissue. In the next step of the example classification process, a first comparison is made between the gene expression level(s) of the sample and the first set of biomarkers or first classifier. If the result of this first comparison is a match, the classification process ends with a result, such as designating the sample as suspicious, cancerous, or containing a particular tissue type (e.g. HA or HC). If the result of the comparison is not a match, the gene expression level(s) of the sample are compared in a second round of comparison to a second set of biomarkers or second classifier. If the result of this second comparison is a match, the classification process ends with a result, such as designating the sample as suspicious, cancerous, or containing a particular tissue type (e.g. HA or HC). If the result of the comparison is not a match, the process continues in a similar stepwise process of comparisons until a match is found, or until all sets of biomarkers or classifiers included in the classification process are used as a basis of comparison. If no match is found between the gene expression level(s) of the sample and any set of biomarkers or classifiers utilized in the classification process, the sample can be designated as "benign." In some examples, the final comparison in the classification process is between the gene expression level(s) of the sample and a main classifier, as described herein.

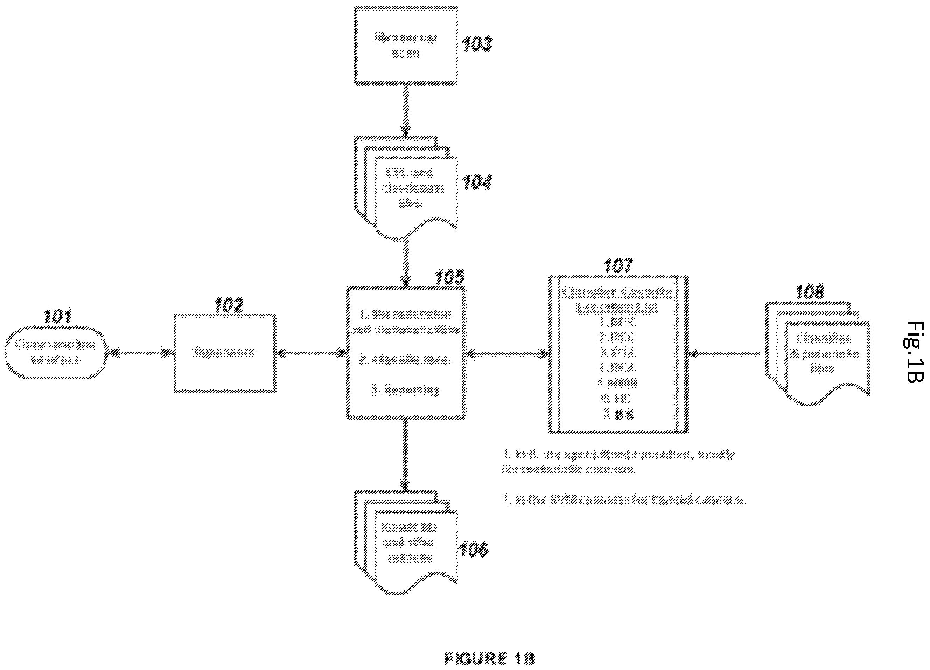

A further example of a classification process in accordance with the methods of the disclosure is illustrated in Figure 1B. Gene expression analysis is performed by microarray hybridization. Scanning of the microarray **103** produces gene expression data **104** in the form of CEL files (the data) and checksum files (for verification of data integrity). Separately, gene expression data for training samples are analyzed to produce classifier and parameter files **108** comprising gene expression data correlated with the presence of one or more tissue types. Classifier cassettes are compiled into an ordered execution list **107.** Analysis of sample data using the classifier cassettes is initiated with input of commands using a command line interface **101,** the execution of which commands are coordinated by a supervisor **102.** The classification analysis in this example process is further detailed at **105** and **107.** Gene expression data **104** is normalized and summarized, and subsequently analyzed with each classifier cassette in sequence for the cassettes in the execution list **105.** In this example, gene expression data is classified using classification cassettes comprising biomarker expression data correlated with medullary thyroid carcinoma (MTC), followed in sequence by comparison using classifier cassettes for renal carcinoma metastasis to the thyroid (RCC), parathyroid (PTA), breast carcinoma metastasis to the thyroid (BCA), melanoma metastasis to the thyroid (MMN), Hurthle cell carcinoma and/or Hurthle cell adenoma (HC), and concluding with a main classifier to distinguish benign from suspicious tissue samples (BS). The result of sequentially analyzing the gene expression data with each classifier cassette is then reported in a result file and any other report information or output **106.**

The classification process can use a main classifier (*e.g.*, classification panel 7) to designate a sample as "benign" or "suspicious," or as containing or not containing one or more tissues of a particular type (*e.g.*, HA or HC). Gene expression data obtained from the sample can undergoe a series of "filtering" steps, where the data is sequentially run through different classification panels or biomarker panels. For example, the sample can be analyzed with the MMN biomarker panel followed by the MTC biomarker panel. In some cases, the sequence of classification panels is classification panels 1 through 5 in any order, followed by classification panel 6, followed by the main classifier (as shown in Figure 3). In some cases, one classification panel is used followed by the main classifier. In some cases, 1, 2, 3, 4, 5, 6, 7, 8, 9, or 10 classifier panels are used followed by the main classifier. In some cases, classifier 6 (HA and HC combined) is used directly before the main classifier. In some cases, one or more of the classifiers 1 through 5 are applied, in any combination, followed by classifier 7. In some cases, one or more of the classifiers 1 through 5 are applied, in any combination or sequence, followed by application of classifier 6, followed by application of classifier 7. In some cases, one or more of the classifiers 1 through 6 are applied, in any combination or sequence, followed by application of classifier 7 (or other main classifier).

The biomarkers within each panel can be interchangeable (modular). The plurality of biomarkers in all panels can be substituted, increased, reduced, or improved to accommodate the definition of new pathologic subtypes (*e.g.*, new case reports of metastasis to the thyroid from other organs). The current disclosure describes a plurality of biomarkers that define each of sixteen heterogeneous, semi-redundant, and distinct pathologies found in the thyroid. Such biomarkers can allow separation between malignant and benign representatives of the sixteen heterogeneous thyroid pathologies. In some cases, all sixteen panels are required to arrive at an accurate diagnosis, and any given panel alone does not have sufficient power to make a true characterization, classification, identification, or diagnostic determination. In other cases, only a subset of the panels is required to arrive at an accurate characterization, classification, identification, or diagnostic determination, such as less than 2, 3, 4, 5, 6, 7, 8, 9, 10, 11, 12, 13, 14, 15, or 16 of the biomarker panels. In some cases, the biomarkers in each panel are interchanged with a suitable combination of biomarkers, such that the plurality of biomarkers in each panel still defines a given pathology subtype within the context of examining the plurality of biomarkers that define all other pathology subtypes.

Classifiers used early in a sequential analysis can be used to either rule-in or rule-out a sample as benign or suspicious, or as containing or not containing one or more tissues of a particular type (*e.g.* HA or HC). Classifiers used in the sequential analysis can also be used to identify sample mix-ups, and/or to pre-screen samples for confounding conditions (*e.g.*, conditions that are not represented in training cohorts used to develop the classification panels), and/or to further characterize a classified sample (*e.g.*, by predicting genetic mutations). Sequential analysis can end with the application of a "main" classifier to data from samples that have not been ruled out by the preceding classifiers, wherein the main classifier is obtained from data analysis of gene expression levels in multiple types of tissue and wherein the main classifier is capable of designating the sample as benign or suspicious (or malignant), or as containing or not containing one or more tissues of a particular type (e.g. HA or HC). Sequential analysis can continue after the application of the main classifier; for example, to further characterize a suspicious (or malignant) biological sample.

Provided herein are thyroid biomarker panels. Two or more biomarker panels associated with tissue types selected from NML, FA, NHP, LCT, HA, FC, PTC, FVPTC, MTC, HC, ATC, RCC, BCA, MMN, BCL, and PTA tissue types can be used to distinguish i) benign FNA thyroid samples from malignant (or suspicious) FNA thyroid samples, ii) the presence of from the absence of one or more of NML, FA, NHP, LCT, HA, FC, PTC, FVPTC, MTC, HC, ATC, RCC, BCA, MMN, BCL, and PTA tissue types in a sample, and/or iii) the presence of HA and/or HC tissue from the absence of HA and/or HC tissue in a sample. The benign versus malignant characterization can be more accurate after examination and analysis of the differential gene expression that defines each pathology subtype in the context of all other subtypes. The current disclosure describes a plurality of markers that can be useful in accurate classification of thyroid FNA.

Classification optimization and simultaneous and/or sequential examination of the initial sixteen biomarker panels described in Figure 2 can be used to select a set of 2, 3, 4, 5, 6, 7, 8, 9, 10 or more (*e.g.*, seven classification panels in Figure 3), which optimization can include a specified order of sequential comparison using such classification panels. Each modular series of subtype panels can be mutually exclusive and sufficient to arrive at accurate thyroid FNA classification.

Examples of biomarkers that can be used to classify, identify, diagnose, or otherwise characterize biological samples (*e.g.*, thyroid samples, *e.g.*, thyroid tissue and/or fine needle aspirations) are shown in Figure 3, Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27. It can be not necessary for biomarkers to reach statistical significance the benign versus malignant comparison in order to be useful in a panel for accurate classification. In some cases, the benign versus malignant (or benign versus suspicious) comparison is not statistically significant. In some cases, the benign versus malignant (or benign versus suspicious) comparison is statistically significant. In some cases, a comparison or correlation of a specific subtype is not statistically significant. In some cases, a comparison or correlation of a specific subtype is statistically significant.

The sixteen panels described in Figure 2 represent distinct pathologies found in the thyroid (whether of thyroid origin or not). However, subtype prevalence in a given population can vary. For example, NHP and PTC can be far more common than rare subtypes such as FC or ATC. The relative frequency of biomarkers in each subtype panel can be subsequently adjusted to give the molecular test sufficient sensitivity and specificity.

The biomarker groupings provided herein are examples of biomarker groupings that can be used to characterize biological samples (*e.g.*, for thyroid conditions, genetic mutations, lymphomas, *etc.).* However, biomarker groupings can be used for other diseases or disorders as well, e.g., any disease or disorder described herein.

**(viii) Characterization of Tissue Components: General method for Target Tissue Content**

Specific components, or target tissue, of any heterogenous sample may be characterized. Generally this may be performed using a two step process. First, a large list of published markers in scientific literature the literature may be used to, examine differential gene expression within a highly curated sample cohort. Markers that change the least between different types of tissue subtypes within the sample may be selected as "seed" markers. Generally, only those markers showing stable, unsaturated expression, across all tissue subtypes are retained for further evaluation. Expression level changes may be evaluated using a LIMMA approach as described herein.

The next step in the process uses the seed list as a "fishing pole" to identify novel markers with correlated expression (negative or positive) which are also insensitive to the tissue subtypes of the samples. This may be evaluated using Pearson correlation coefficient. These correlation searches may be done to identify other potential markers not known from the literature, but which show consistent expression across thyroid subtypes and correlate well with known markers.

In some cases, one or more markers with strong undifferentiated signal, may be determined. The average normalized expression level of these markers may be used to generate a statistic for a particular tissue or target tissue type, such as blood or follicular cells as described herein. This statistic may also be used to extrapolate the relative strength of the particular target tissue in any set of samples containing the tissue. This method may be applied to any other heterogeneous cell mixture situations.

A statistic derived in this manner may be used to extrapolate the relative strength of gene signals arising from a particular target tissue. This statistic may be used in developing empirical cut-offs for the statistic and can be used as either (a) quality control mechanism to remove samples with insufficient content of a specific target tissue, or (b) modify estimates of post-test risk of malignancy using the information about the specific tissue content of the sample and effectively establish classifier decision cut-off boundaries as a function of specific tissue content.

In addition, a statistic can be used to adjust expression levels for genes whose expression correlates with the amount of specific tissue or cells in the mixture using a linear modeling approach. This may aid in searching for genes that are differentially expressed across a variable of interest. Using a standard linear modeling approach, the statistic may be added as a covariate to the equation as in:

$$Y \sim Phenotype + Stat$$

where Y is expression intensity of a given marker. Standard approaches such as LIMMA may then be used to identify genes differentially expressed by phenotype after adjusting for differences in the content of the specific target tissue. In addition, intensity profiles for new samples may be adjusted for the observed level of a statistic to restore true expression profiles characterizing the expression intensities of a given sample at a given target value of the statistic representing a pure sample state.

This can be done using models for tech factor removal as previously described in patent application 12/964,666. In some cases, expression levels for a marker of interest may be previously modeled as *Y~Phenotype + Stat* using training data, and the coefficients of this model are treated as known and fixed. In the real data sets generated by samples, thousands of markers may show significant dependence on the *Stat* variable. The coefficient for the dependence on statistic may be represented as $\beta$. Further, the samples may have a "target" statistic value of $F_t$ in the 'pure' non-contaminated state. For an incoming test sample with follicular stat value of F, the predicted intensity value for this marker at the target follicular stat level may be $Y_{adj} = Y + (F_t - F) * \beta$. In another embodiment of the disclosure these adjusted values of intensities can be used as the input to the classifier in place of the observed intensity values.

**(ix) Characterization of Tissue Components: Blood Content**

Thyroid FNAs can be heterogeneous samples comprising unknowable proportions of varied cell mixtures. In some cases, FNA samples may contain contaminants such as whole blood which may accompany a sample during biopsy. Expression of various markers can be used to inform about the level of contamination in a given sample. This information may be used as either a quality control metric to reject samples with high contamination, or adjust cut-off values for the classifier. This quality control metric may be referred to as a "blood statistic." In some cases, selection of markers may be derived from known markers in the literature. In some cases, selection of markers may be derived from data from previously characterized samples or experimental data.

Generally, a blood statistic may be applicable or useful in the analysis of any hetergenous sample in which whole blood is a suspected contaminant. In some cases, heterogenous samples are not limited to thyroid cancer, but any cell mixtures, or heterogenous tumors and the like.

**(x) Characterization of Tissue Components: Follicular Content**

Given the general heterogeneity of thyroid FNA samples, the follicular content of the sample may also be determined. In some cases, not all cell types present in an FNA are informative toward benign vs. malignant classification. Depending on the nature of the nodule and the precise site of aspiration, sufficient thyroid follicular cells (as opposed to stromal cells, lymphocytic cells, colloid, or fibrotic tissue) may not have been sampled, thereby yielding an incomplete/inaccurate picture on the nature of the nodule. In some cases, selection of markers may be derived from known markers in the literature. In some cases, selection of markers may be derived from data from previously characterized samples or experimental data.

**(xi)** *In silico* Mixture Modeling

Generally, reproducibility of analysis of samples is an important feature of the disclosure. The present disclosure also provides application for in silico mixture modeling to improve reproducibility, whereby observed signals from mixed samples may be used to reconstruct the proportions of pure components. This is validated using in vitro results of mixing with known proportions.

Multiple studies have investigated the effects of mixing independent RNA sources on the resulting microarray signal (Affymetrix White Paper "Human Gene 1.0 ST Array performance", 2007; Robinson & Speed, 2007; Chudova, 2010). In some cases, the signal for the mixed RNA can be approximated by a linear combination of signals of the unmixed RNA sources, unless the gene signals fall into the background range or saturate at the higher intensity levels. Data generated within an *in vitro* mixing study may be used to confirm the selection of an analytical model for approximating mixture signals *in silico.* This model choice may be specific to the markers (transcript clusters) used by a given molecular test.

Two alternative analytical models for the expression intensities measured for the mixed samples may be used and compared to the actual observed intensity signals for in-vitro mixtures of pure RNA sources.

In the first model, $M_0$ may be a null model corresponding to linear mixing of sources in a raw (not log-transformed) intensity space. This is a model previously utilized, as known in the art. This model may be applicable at the higher intensity range or for genes with high log fold changes between pure samples [in this case, benign and malignant conditions], where multiplicative noise dominates in at least one of the mixture components.

In the second model, $M_1$ may be the alternative model assuming linear mixing of sources in the log-transformed space.

These two models may be compared in their ability to predict intensity profiles observed for actual mixtures of benign and malignant tissue. In some cases the malignant tissue may be FNAs with normal adjacent thyroid tissue. The comparison of models may be made based on the likelihood of observed log-transformed and normalized signals for the markers of interest under two alternative models.

In some cases, model specification may be determined using the following equation. $Y_A^T$ may be defined as the quantile normalized and log-transformed summarized latent intensity vector for unmixed sample A; $Y_B^T$ may be the quantile normalized and log-transformed summarized latent intensity vector for unmixed sample B. $\alpha$ may be dfined as the mixing proportion of unmixed sample A in the mix Y. The signal distribution for the mixed sample Y under the null and alternative models can be expressed as follows:

$$M_0: P(Y|\alpha, Y_A^T, Y_B^T) = \prod_{g=1}^{G} N(\log_2 (\alpha * 2^{Y_{Ag}^T} + (1 - \alpha) * 2^{Y_{Bg}^T}), \sigma^2)$$

$$M_1: P(Y|\alpha, Y_A^T, Y_B^T) = \prod_{g=1}^{G} N(\alpha * Y_{Ag}^T + (1 - \alpha) * Y_{Bg}^T, \sigma^2)$$

where G is the total number of markers in the Afirma-T molecular classifier, $\sigma^2$ is the variance of log-transformed intensity values for technical replicates (same under both models) and $N(\mu, \sigma^{2'})$ is a normal distributions with mean $\mu$ and variance $\sigma^{2'}$. Analysis of prior technical replicates run on the Afirma-T chips shows that the standard deviation of intensity values can be estimated as $\sigma$ = 0.15, which will be treated as a fixed value for both alternative models.

While some mixing proportions may be pre-specified, the actual proportions in the resulting mixture may depend on the quantitation accuracy of the total RNA in the unmixed sources and the accuracy of pipetting. The mixing proportion $\alpha$ may be treated as a random variable centered around the mixing proportion specified in the design. The mixing proportion may be given the same Beta prior, under both $M_0$ and $M_1$ models. The two parameters of the prior mixture j are set to ensure the mean matches the mixing proportion specified by design for mixture j:

$$P(\alpha_j) = Beta\left(A_j * B, (1 - A_j) * B\right)$$

In this case, j is the number of experimental mixture ($j$ = 1, ...,7) and B is the strength of the prior (taken to be B = 20).

To perform model comparison, marginal likelihood of the observed intensities for relevant markers in experimental mixtures may be computed under both models. Marginal likelihood may be computed given observed signals for unmixed components $Y_A^{obs}$ and $Y_B^{obs}$ by integrating out mixture proportion $\alpha$: $P\left(Y \middle| Y_A^{obs}, Y_B^{Obs}, A\right)$ for each of the experimental mixtures passing QC requirements.

The model resulting in higher marginal likelihood for experimental mixes may be further evaluated for agreement with linear classifier scores for the *in vitro* mixes. Specifically, the predictive distributions of linear classifier scores given observed signals for unmixed RNA and each of the experimental mixtures may be generated. In some cases, the model may be accepted as approximating linear classifier scores with sufficient precision, if the observed scores fall within 0.28 of the mean of the predictive distribution. In some cases, the model may be accepted if the observed scores fall within 0.1, 0.2, 0.21, 0.22, 0.23, 0.24, 0.25, 0.26, 0.27, 0.28, 0.29, 0.30, 0.31, 0.32, 0.33, 0.34, 0.35, 0.36, 0.37, 0.38, 0.39, or 0.4 of the mean of the predictive distribution. This value may be determined from pilot data.

Evaluation of mean squared error between model predictions and observed data for markers of interest may be done as a part of additional exploratory analysis for refining the analytical model.

### (xii) Classification Error Rates

Top biomarkers (*e.g.*, thyroid biomarkers) can be subdivided into bins *(e.g.,* 50 TCIDs per bin) to demonstrate the minimum number of genes required to achieve an overall classification error rate of less than 4%. The original TCIDs used for classification correspond to the Affymetrix Human Exon 1.0ST microarray chip and each can map to more than one gene or no genes at all (Affymetrix annotation file: HuEx-1_0-st-v2.na29.hg18.transcript.csv). When no genes map to a TCID the biomarker is denoted as TCID-######.

### IX. Compositions

### (i) Gene Expression Products and splice variants of the present disclosure

Molecular profiling can also include, but is not limited to, assays of the present disclosure including assays for one or more of the following: proteins, protein expression products, DNA, DNA polymorphisms, RNA, RNA expression products, RNA expression product levels, or RNA expression product splice variants of the genes or markers provided in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27. In some cases, the methods of the present disclosure provide for improved cancer diagnostics by molecular profiling of at least about 1, 2, 3, 4, 5, 6, 7, 8, 9, 10, 15, 20, 25, 30, 35, 40, 45, 50, 60, 70, 80, 90, 100, 120, 140, 160, 180, 200, 240, 280, 300, 350, 400, 450, 500, 600, 700, 800, 1000, 1500, 2000, 2500, 3000, 3500, 4000, 5000 or more DNA polymorphisms, expression product markers, and/or alternative splice variant markers.

Molecular profiling can involve microarray hybridization that is performed to determine gene expression product levels for one or more genes selected from Figure 4,

Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27. In some cases, gene expression product levels of one or more genes from one group are compared to gene expression product levels of one or more genes in another group or groups. As an example only and without limitation, the expression level of gene TPO can be compared to the expression level of gene GAPDH. In another case, gene expression levels are determined for one or more genes involved in one or more of the following metabolic or signaling pathways: thyroid hormone production and/or release, protein kinase signaling pathways, lipid kinase signaling pathways, and cyclins. In some cases, the methods of the present disclosure provide for analysis of gene expression product levels and or alternative exon usage of at least one gene of 1, 2, 3, 4, 5, 6, 7, 9, 10, 11, 12, 13, 14, or 15 or more different metabolic or signaling pathways.

**(ii) Compositions of the present disclosure**

Compositions of the present disclosure are also provided which composition comprises one or more of the following: polynucleotides (e.g., DNA or RNA) corresponding to the genes or a portion of the genes provided in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27, and nucleotides (e.g., DNA or RNA) corresponding to the complement of the genes or a portion of the complement of the genes provided in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27. This disclosure provides for collections of probes, such as sets of probes that can bind to between about 1 and about 500 of the biomarkers identified in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27; for example about 1-500, 1-400, 1-300, 1-200, 1-100, 1-50, 1-25, 1-10, 10-500, 10-400, 10-300, 10-200, 10-100, 10-50, 10-25, 25-500, 25-400, 25-300, 25-200, 25-100, 25-50, 50-500, 50-400, 50-300, 50-200, 50-100, 100-500, 100-400, 100-300, 100-200, 200-500, 200-400, 200-300, 300-500, 300-400, 400-500, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500 of the biomarkers identified in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27.

The nucleotides (including probes) of the present disclosure can be at least about 10, 15, 20, 25, 30, 35, 40, 45, 50, 55, 60, 65, 70, 75, 100, 150, 200, 250, 300, 350, or about 400 or 500 nucleotides in length. The nucleotides (including probes) of the present disclosure can be between about 10-500 residues, or more; for example, about 10-500, 10-200, 10-150, 10-100, 10-75, 10-50, 10-25, 25-500, 25-200, 25-150, 25-100, 25-75, 25-50, 50-500, 50-200, 50-150, 50-100, 50-75, 75-500, 75-200, 75-150, 75-100, 100-500, 100-200, 100-150, 150-500, 150-200, 200-500, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 80, 85, 90, 95, 100, 110, 120, 130, 140, 150, 160, 170, 180, 190, 200, 225, 250, 275, 300, 325, 350, 375, 400, 425, 450, 475, or 500 nucleotides, or more. The nucleotides can be natural or man-made derivatives of ribonucleic acid or deoxyribonucleic acid including, but not limited to, peptide nucleic acids, pyranosyl RNA, nucleosides, methylated nucleic acid, pegylated nucleic acid, cyclic nucleotides, and chemically modified nucleotides. The nucleotides of the present disclosure can be chemically modified to include a detectable label. The biological sample, or gene expression products derived from the biological sample (e.g., DNA, RNA, protein, *etc.*) can be chemically modified to include a label.

A further composition of the present disclosure comprises oligonucleotides for detecting and/or measuring gene expression products corresponding to the markers or genes provided in Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27 and/or their complement. A further composition of the present disclosure comprises oligonucleotides for detecting and/or measuring the gene expression products of polymorphic alleles of the genes and their complement. Such polymorphic alleles include but are not limited to splice site variants, single nucleotide polymorphisms, variable number repeat polymorphisms, insertions, deletions, and homologues. In some cases, the variant alleles are between about 99.9% and about 70% identical to the genes listed in Figure 4, including about, less than about, or more than about 99.75%, 99.5%, 99.25%, 99%, 97.5%, 95%, 92.5%, 90%, 85%, 80%, 75%, and about 70% identical. In some cases, the variant alleles differ by between about 1 nucleotide and about 500 nucleotides from the genes provided in Figure 4, including about, less than about, or more than about 1, 2, 3, 5, 7, 10, 15, 20, 25, 30, 35, 50, 75, 100, 150, 200, 250, 300, and about 400 nucleotides.

In some cases, the composition of the present disclosure can be selected from the top differentially expressed gene products between categories (e.g., benign and malignant samples; normal and benign or malignant samples; presence and absence of one or more particular tissue types, such as HA and/or HC; male and female; mutant and wild-type), or the top differentially spliced gene products between (*e.g.*, benign and malignant samples; normal and benign or malignant samples; presence and absence of one or more particular tissue types, such as HA and/or HC; male and female; mutant and wild-type). In some cases the top differentially expressed gene products can be selected from Figure 4, Table 1, Table 2, Table 3, Table 4, Table 5, Table 9, Table 10, Table 11, Table 12, Table 14, Table 15, Table 18 , Table 19, Table 23, Table 24, Table 25, Table 26 and Table 27.

**(iii) Diseases and Disorders**

In some cases, the subject methods and algorithm are used to diagnose, characterize, detect, exclude and/or monitor thyroid cancer. Thyroid cancer includes any type of thyroid cancer, including but not limited to, any malignancy of the thyroid gland, *e.g.*, papillary thyroid cancer, follicular thyroid cancer, medullary thyroid cancer and/or anaplastic thyroid cancer. In some cases, the thyroid cancer is differentiated. In some cases, the thyroid cancer is undifferentiated. In some cases, the instant methods are used to diagnose, characterize, detect, exclude and/or monitor one or more of the following types of thyroid cancer: papillary thyroid carcinoma (PTC), follicular variant of papillary thyroid carcinoma (FVPTC), follicular carcinoma (FC), Hurthle cell carcinoma (HC) or medullary thyroid carcinoma (MTC).

Other types of cancer that can be diagnosed, characterized and/or monitored using the algorithms and methods of the present disclosure include but are not limited to adrenal cortical cancer, anal cancer, aplastic anemia, bile duct cancer, bladder cancer, bone cancer, bone metastasis, central nervous system (CNS) cancers, peripheral nervous system (PNS) cancers, breast cancer, Castleman's disease, cervical cancer, childhood Non-Hodgkin's lymphoma, lymphoma, colon and rectum cancer, endometrial cancer, esophagus cancer, Ewing's family of tumors (e.g. Ewing's sarcoma), eye cancer, gallbladder cancer, gastrointestinal carcinoid tumors, gastrointestinal stromal tumors, gestational trophoblastic disease, hairy cell leukemia, Hodgkin's disease, Kaposi's sarcoma, kidney cancer, laryngeal and hypopharyngeal cancer, acute lymphocytic leukemia, acute myeloid leukemia, children's leukemia, chronic lymphocytic leukemia, chronic myeloid leukemia, liver cancer, lung cancer, lung carcinoid tumors, Non-Hodgkin's lymphoma, male breast cancer, malignant mesothelioma, multiple myeloma, myelodysplastic syndrome, myeloproliferative disorders, nasal cavity and paranasal cancer, nasopharyngeal cancer, neuroblastoma, oral cavity and oropharyngeal cancer, osteosarcoma, ovarian cancer, pancreatic cancer, penile cancer, pituitary tumor, prostate cancer, retinoblastoma, rhabdomyosarcoma, salivary gland cancer, sarcoma (adult soft tissue cancer), melanoma skin cancer, non-melanoma skin cancer, stomach cancer, testicular cancer, thymus cancer, uterine cancer (e.g. uterine sarcoma), vaginal cancer, vulvar cancer, and Waldenstrom's macroglobulinemia.

Expression profiling using panels of biomarkers can be used to characterize thyroid tissue as benign, suspicious, and/or malignant. Panels can be derived from analysis of gene expression levels of cohorts containing benign (non-cancerous) thyroid subtypes including follicular adenoma (FA), nodular hyperplasia (NHP), lymphocytic thyroiditis (LCT), and Hurthle cell adenoma (HA); malignant subtypes including follicular carcinoma (FC), papillary thyroid carcinoma (PTC), follicular variant of papillary carcinoma (FVPTC), medullary thyroid carcinoma (MTC), Hürthle cell carcinoma (HC), and anaplastic thyroid carcinoma (ATC). Such panels can also be derived from non-thyroid subtypes including renal carcinoma (RCC), breast carcinoma (BCA), melanoma (MMN), B cell lymphoma (BCL), and parathyroid (PTA). Biomarker panels associated with normal thyroid tissue (NML) can also be used in the methods and compositions provided herein. Exemplary panels of biomarkers are provided in Figure 2, and will be described further herein. Of note, each panel listed in Figure 2, relates to a signature, or pattern of biomarker expression (e.g., gene expression), that correlates with samples of that particular pathology or description.

The present disclosure also provides novel methods and compositions for identification of types of aberrant cellular proliferation through an iterative process (e.g., differential diagnosis) such as carcinomas including follicular carcinomas (FC), follicular variant of papillary thyroid carcinomas (FVPTC), Hurthle cell carcinomas (HC), Hurthle cell adenomas (HA); papillary thyroid carcinomas (PTC), medullary thyroid carcinomas (MTC), and anaplastic carcinomas (ATC); adenomas including follicular adenomas (FA); nodule hyperplasias (NHP); colloid nodules (CN); benign nodules (BN); follicular neoplasms (FN); lymphocytic thyroiditis (LCT), including lymphocytic autoimmune thyroiditis; parathyroid tissue; renal carcinoma metastasis to the thyroid; melanoma metastasis to the thyroid; B-cell lymphoma metastasis to the thyroid; breast carcinoma to the thyroid; benign (B) tumors, malignant (M) tumors, and normal (N) tissues. The present disclosure further provides novel gene expression markers and novel groups of genes and markers useful for the characterization, diagnosis, and/or treatment of cellular proliferation.

In some cases, the diseases or conditions classified, characterized, or diagnosed by the methods of the present disclosure include benign and malignant hyperproliferative disorders including but not limited to cancers, hyperplasias, or neoplasias. In some cases, the hyperproliferative disorders classified, characterized, or diagnosed by the methods of the present disclosure include but are not limited to breast cancer such as a ductal carcinoma in duct tissue in a mammary gland, medullary carcinomas, colloid carcinomas, tubular carcinomas, and inflammatory breast cancer; ovarian cancer, including epithelial ovarian tumors such as adenocarcinoma in the ovary and an adenocarcinoma that has migrated from the ovary into the abdominal cavity; uterine cancer; cervical cancer such as adenocarcinoma in the cervix epithelial including squamous cell carcinoma and adenocarcinomas; prostate cancer, such as a prostate cancer selected from the following: an adenocarcinoma or an adenocarinoma that has migrated to the bone; pancreatic cancer such as epitheliod carcinoma in the pancreatic duct tissue and an adenocarcinoma in a pancreatic duct; bladder cancer such as a transitional cell carcinoma in urinary bladder, urothelial carcinoma (transitional cell carcinomas), tumors in the urothelial cells that line the bladder, squamous cell carcinomas, adenocarcinomas, and small cell cancers; leukemia such as acute myeloid leukemia (AML), acute lymphocytic leukemia, chronic lymphocytic leukemia, chronic myeloid leukemia, hairy cell leukemia, myelodysplasia, myeloproliferative disorders, acute myelogenous leukemia (AML), chronic myelogenous leukemia (CML), mastocytosis, chronic lymphocytic leukemia (CLL), multiple myeloma (MM), and myelodysplastic syndrome (MDS); bone cancer; lung cancer such as non-small cell lung cancer (NSCLC), which is divided into squamous cell carcinomas, adenocarcinomas, and large cell undifferentiated carcinomas, and small cell lung cancer; skin cancer such as basal cell carcinoma, melanoma, squamous cell carcinoma and actinic keratosis, which is a skin condition that sometimes develops into squamous cell carcinoma; eye retinoblastoma; cutaneous or intraocular (eye) melanoma; primary liver cancer (cancer that begins in the liver); kidney cancer; AIDS-related lymphoma such as diffuse large B-cell lymphoma, B-cell immunoblastic lymphoma and small non-cleaved cell lymphoma; Kaposi's Sarcoma; viral-induced cancers including hepatitis B virus (HBV), hepatitis C virus (HCV), and hepatocellular carcinoma; human lymphotropic virus-type 1 (HTLV-1) and adult T-cell leukemia/lymphoma; and human papilloma virus (HPV) and cervical cancer; central nervous system cancers (CNS) such as primary brain tumor, which includes gliomas (astrocytoma, anaplastic astrocytoma, or glioblastoma multiforme), Oligodendroglioma, Ependymoma, Meningioma, Lymphoma, Schwannoma, and Medulloblastoma; peripheral nervous system (PNS) cancers such as acoustic neuromas and malignant peripheral nerve sheath tumor (MPNST) including neurofibromas and schwannomas, malignant fibrous cytoma, malignant fibrous histiocytoma, malignant meningioma, malignant mesothelioma, and malignant mixed Müllerian tumor; oral cavity and oropharyngeal cancer such as, hypopharyngeal cancer, laryngeal cancer, nasopharyngeal cancer, and oropharyngeal cancer; stomach cancer such as lymphomas, gastric stromal tumors, and carcinoid tumors; testicular cancer such as germ cell tumors (GCTs), which include seminomas and nonseminomas, and gonadal stromal tumors, which include Leydig cell tumors and Sertoli cell tumors; thymus cancer such as to thymomas, thymic carcinomas, Hodgkin disease, non-Hodgkin lymphomas carcinoids or carcinoid tumors; rectal cancer; and colon cancer. In some cases, the diseases or conditions classified, characterized, or diagnosed by the methods of the present disclosure include but are not limited to thyroid disorders such as for example benign thyroid disorders including but not limited to follicular adenomas, Hurthle cell adenomas, lymphocytic throiditis, and thyroid hyperplasia. In some cases, the diseases or conditions classified, characterized, or diagnosed by the methods of the present disclosure include but are not limited to malignant thyroid disorders such as for example follicular carcinomas, follicular variant of papillary thyroid carcinomas, medullary carcinomas, and papillary carcinomas. In some cases, the methods of the present disclosure provide for a classification, characterization, or diagnosis of a tissue as diseased or normal. In other cases, the methods of the present disclosure provide for a classification, characterization, or diagnosis of normal, benign, or malignant. In some cases, the methods of the present disclosure provide for a classification, characterization, or diagnosis of benign/normal, or malignant. In some cases, the methods of the present disclosure provide for a classification, characterization, or diagnosis of one or more of the specific diseases or conditions provided herein.

In one aspect, the present disclosure provides algorithms and methods that can be used for classification, characterization, or diagnosis and monitoring of a genetic disorder. A genetic disorder is an illness caused by abnormalities in genes or chromosomes. While some diseases, such as cancer, are due in part to genetic disorders, they can also be caused by environmental factors. In some cases, the algorithms and the methods disclosed herein are used for classification, characterization, or diagnosis and monitoring of a cancer such as thyroid cancer.

Genetic disorders can be typically grouped into two categories: single gene disorders and multifactorial and polygenic (complex) disorders. A single gene disorder is the result of a single mutated gene. There are estimated to be over 4000 human diseases caused by single gene defects. Single gene disorders can be passed on to subsequent generations in several ways. There are several types of inheriting a single gene disorder including but not limited to autosomal dominant, autosomal recessive, X-linked dominant, X-linked recessive, Y-linked and mitochondrial inheritance. Only one mutated copy of the gene can be necessary for a person to be affected by an autosomal dominant disorder. Examples of autosomal dominant type of disorder include, but are not limited to, Huntington's disease, Neurofibromatosis 1, Marfan Syndrome, Hereditary nonpolyposis colorectal cancer, and Hereditary multiple exostoses. In autosomal recessive disorder, two copies of the gene can be mutated for a person to be affected by an autosomal recessive disorder. Examples of this type of disorder include, but are not limited to, cystic fibrosis, sickle-cell disease (also partial sickle-cell disease), Tay-Sachs disease, Niemann-Pick disease, spinal muscular atrophy, and dry earwax. X-linked dominant disorders are caused by mutations in genes on the X chromosome. Only a few disorders have this inheritance pattern, with a prime example being X-linked hypophosphatemic rickets. Males and females are both affected in these disorders, with males typically being more severely affected than females. Some X-linked dominant conditions such as Rett syndrome, Incontinentia Pigmenti type 2 and Aicardi Syndrome can be fatal in males either in utero or shortly after birth, and are therefore predominantly seen in females. X-linked recessive disorders can also be caused by mutations in genes on the X chromosome. Examples of this type of disorder include, but are not limited to, Hemophilia A, Duchenne muscular dystrophy, red-green color blindness, muscular dystrophy and Androgenetic alopecia. Y-linked disorders can be caused by mutations on the Y chromosome. Examples include but are not limited to Male Infertility and hypertrichosis pinnae. Mitochondrial inheritance, also known as maternal inheritance, applies to genes in mitochondrial DNA. An example of this type of disorder is Leber's Hereditary Optic Neuropathy.

Genetic disorders can also be complex, multifactorial or polygenic. Polygenic genetic disorders can be associated with the effects of multiple genes in combination with lifestyle and environmental factors. Although complex disorders often cluster in families, they can lack a clear-cut pattern of inheritance. This can make it difficult to determine a person's risk of inheriting or passing on these disorders. Complex disorders can also be difficult to study and treat; in some cases, because the specific factors that cause most of these disorders have not yet been identified. Multifactorial, or polygenic, disorders that can be diagnosed, characterized and/or monitored using the algorithms and methods of the present disclosure include but are not limited to heart disease, diabetes, asthma, autism, autoimmune diseases such as multiple sclerosis, cancers, ciliopathies, cleft palate, hypertension, inflammatory bowel disease, mental retardation and obesity.

Other genetic disorders that can be diagnosed, characterized and/or monitored using the algorithms and methods of the present disclosure include but are not limited to 1p36 deletion syndrome, 21-hydroxylase deficiency, 22q11.2 deletion syndrome, 47,XYY syndrome, 48, XXXX, 49, XXXXX, aceruloplasminemia, achondrogenesis, type II, achondroplasia, acute intermittent porphyria, adenylosuccinate lyase deficiency, Adrenoleukodystrophy, ALA deficiency porphyria, ALA dehydratase deficiency, Alexander disease, alkaptonuria, alpha-1 antitrypsin deficiency, Alstrom syndrome, Alzheimer's disease (type 1, 2, 3, and 4), Amelogenesis Imperfecta, amyotrophic lateral sclerosis, Amyotrophic lateral sclerosis type 2, Amyotrophic lateral sclerosis type 4, amyotrophic lateral sclerosis type 4, androgen insensitivity syndrome, Anemia, Angelman syndrome, Apert syndrome, ataxia-telangiectasia, Beare-Stevenson cutis gyrata syndrome, Benjamin syndrome, beta thalassemia, biotinidase deficiency, Birt-Hogg-Dube syndrome, bladder cancer, Bloom syndrome, Bone diseases, breast cancer, CADASIL, Camptomelic dysplasia, Canavan disease, Cancer, Celiac Disease, CGD Chronic Granulomatous Disorder, Charcot-Marie-Tooth disease, Charcot-Marie-Tooth disease Type 1, Charcot-Marie-Tooth disease Type 4, Charcot-Marie-Tooth disease, type 2, Charcot-Marie-Tooth disease, type 4, Cockayne syndrome, Coffin-Lowry syndrome, collagenopathy, types II and XI, Colorectal Cancer, Congenital absence of the vas deferens, congenital bilateral absence of vas deferens, congenital diabetes, congenital erythropoietic porphyria, Congenital heart disease, congenital hypothyroidism, Connective tissue disease, Cowden syndrome, Cri du chat, Crohn's disease, fibrostenosing, Crouzon syndrome, Crouzonodermoskeletal syndrome, cystic fibrosis, De Grouchy Syndrome, Degenerative nerve diseases, Dent's disease, developmental disabilities, DiGeorge syndrome, Distal spinal muscular atrophy type V, Down syndrome, Dwarfism, Ehlers-Danlos syndrome, Ehlers-Danlos syndrome arthrochalasia type, Ehlers-Danlos syndrome classical type, Ehlers-Danlos syndrome dermatosparaxis type, Ehlers-Danlos syndrome kyphoscoliosis type, vascular type, erythropoietic protoporphyria, Fabry's disease, Facial injuries and disorders, factor V Leiden thrombophilia, familial adenomatous polyposis, familial dysautonomia, fanconi anemia, FG syndrome, fragile X syndrome, Friedreich ataxia, Friedreich's ataxia, G6PD deficiency, galactosemia, Gaucher's disease (type 1, 2, and 3), Genetic brain disorders, Glycine encephalopathy, Haemochromatosis type 2, Haemochromatosis type 4, Harlequin Ichthyosis, Head and brain malformations, Hearing disorders and deafness, Hearing problems in children, hemochromatosis (neonatal, type 2 and type 3), hemophilia, hepatoerythropoietic porphyria, hereditary coproporphyria, Hereditary Multiple Exostoses, hereditary neuropathy with liability to pressure palsies, hereditary nonpolyposis colorectal cancer, homocystinuria, Huntington's disease, Hutchinson Gilford Progeria Syndrome, hyperoxaluria, primary, hyperphenylalaninemia, hypochondrogenesis, hypochondroplasia, idic15, incontinentia pigmenti, Infantile Gaucher disease, infantile-onset ascending hereditary spastic paralysis, Infertility, Jackson-Weiss syndrome, Joubert syndrome, Juvenile Primary Lateral Sclerosis, Kennedy disease, Klinefelter syndrome, Kniest dysplasia, Krabbe disease, Learning disability, Lesch-Nyhan syndrome, Leukodystrophies, Li-Fraumeni syndrome, lipoprotein lipase deficiency, familial, Male genital disorders, Marfan syndrome, McCune-Albright syndrome, McLeod syndrome, Mediterranean fever, familial, MEDNIK, Menkes disease, Menkes syndrome, Metabolic disorders, methemoglobinemia beta-globin type, Methemoglobinemia congenital methaemoglobinaemia, methylmalonic acidemia, Micro syndrome, Microcephaly, Movement disorders, Mowat-Wilson syndrome, Mucopolysaccharidosis (MPS I), Muenke syndrome, Muscular dystrophy, Muscular dystrophy, Duchenne and Becker type, muscular dystrophy, Duchenne and Becker types, myotonic dystrophy, Myotonic dystrophy type 1 and type 2, Neonatal hemochromatosis, neurofibromatosis, neurofibromatosis 1, neurofibromatosis 2, Neurofibromatosis type I, neurofibromatosis type II, Neurologic diseases, Neuromuscular disorders, Niemann-Pick disease, Nonketotic hyperglycinemia, nonsyndromic deafness, Nonsyndromic deafness autosomal recessive, Noonan syndrome, osteogenesis imperfecta (type I and type III), otospondylomegaepiphyseal dysplasia, pantothenate kinase-associated neurodegeneration, Patau Syndrome (Trisomy 13), Pendred syndrome, Peutz-Jeghers syndrome, Pfeiffer syndrome, phenylketonuria, porphyria, porphyria cutanea tarda, Prader-Willi syndrome, primary pulmonary hypertension, prion disease, Progeria, propionic acidemia, protein C deficiency, protein S deficiency, pseudo-Gaucher disease, pseudoxanthoma elasticum, Retinal disorders, retinoblastoma, retinoblastoma FA - Friedreich ataxia, Rett syndrome, Rubinstein-Taybi syndrome, SADDAN, Sandhoff disease, sensory and autonomic neuropathy type III, sickle cell anemia, skeletal muscle regeneration, Skin pigmentation disorders, Smith Lemli Opitz Syndrome, Speech and communication disorders, spinal muscular atrophy, spinal-bulbar muscular atrophy, spinocerebellar ataxia, spondyloepimetaphyseal dysplasia, Strudwick type, spondyloepiphyseal dysplasia congenita, Stickler syndrome, Stickler syndrome COL2A1, Tay-Sachs disease, tetrahydrobiopterin deficiency, thanatophoric dysplasia, thiamine-responsive megaloblastic anemia with diabetes mellitus and sensorineural deafness, Thyroid disease, Tourette's Syndrome, Treacher Collins syndrome, triple X syndrome, tuberous sclerosis, Turner syndrome, Usher syndrome, variegate porphyria, von Hippel-Lindau disease, Waardenburg syndrome, Weissenbacher-Zweymüller syndrome, Wilson disease, Wolf-Hirschhorn syndrome, Xeroderma Pigmentosum, X-linked severe combined immunodeficiency, X-linked sideroblastic anemia, and X-linked spinal-bulbar muscle atrophy.

**X. Mutation Detection Using RNA-SEQ Data**

The compositions and methods of this disclosure also provide for general data analysis tools that may be employed to increase sensitivity and/or selectivity of one or more assays or tests using RNA-SEQ data. These methods may be applicable to a variety of applications, including but not limited to sample analysis of a disease such as cancer. Cancers may include but are not limited to lymphoma or thyroid cancer as described herein.

Generally, one or more algorithms, such as mutation callers, are available for next-generation DNA sequence (DNA-Seq) datasets to detect mutations. However, such algorithms for next-generation RNA sequence (RNA-Seq) data are limited and are generally restricted by difficulties and biases such as: 1) low coverage (total number of reads) for some genes contrasted by high-depth coverage for others, and/or 2) technical variation due to library preparation, alignment artifacts and/or other sequencing artifacts. The present disclosure provides for improved methods for identifying and removing mutation calls resulting from technical variation. In the methods described herein, likely somatic variants may be identified, whereby biological population variation may be removed during pre-processing steps.

In some cases, this disclosure provides for the use of existing algorithms or tools such as GATK or samtools as known in the art, to be used to detect mutations in aligned RNA-Seq reads across multiple samples or on a per-sample basis. In some cases, examining the resulting output and filtering out mutation calls that are also observed in the germline DNA of the population being studied may then be used to enrich for rare and somatic variants. These mutations may occur due to natural biological variability and may be removed by cross-correlating genomic coordinates of found mutations in affected samples with that observed across a reference sample. In some cases the reference sample may comprise at least 1, 10, 100 or 1000 genomes. In some cases, the reference sample may comprise at most 1, 10, 100, or 1000 genomes. In some cases, a reference may be used as provided by the "1000 genome project" as known in the art. In some cases false positive mutation calls that are caused by technical variability may be identified and filtered. Generally, library preparation and alignment methods may contribute to technical variability.

Generally, technical artifacts or technical variability across samples may be identified by analyzing and comparing 2 or more distinct sample cohorts. In one example a thyroid sample cohort is analyzed by comparing genomic coordinates or base positions in affected samples with that of mutation calls generated using a similar library prep method, a similar alignment and a similar mutation calling procedure of a second non-thyroid sample cohort. The second sample cohort is selected such that it is not expected to carry the same somatic mutation profile as the first cohort of interest.

For example, the following method may be used to detect mutations. In the first step, a BED file may be created with genomic coordinates of the coding sequence. Next, a mutation caller may be used to identify mutations across all samples within the regions of interest constructed in the previous step (i.e. samtools in a single sample mode, or GATK simultaneously across multiple samples). Mutations may then be compare to the normal biological variation observed across > 1000 individuals in the 1000 genome project such that separate variants detected are compared to non detected in normal germline DNA.

In some cases, variants not identified as mutations in the normal germline DNA are retained and further compared to the mutation calls of another references. Generally, an suitable reference may be used, such that the reference aids in identifying false positive mutations. In one example a reference may be generated using samples from a specific tissue. In some cases, a reference may be generated across at least 40 non-thyroid samples, such as pancreas, brain, etc. Comparison to one or more references may remove technical library preparation and alignment artifacts and exclude those sites as likely false positives. In some cases, this method may not be dependent on relative expression levels for any given transcript in non-thyroid & thyroid (e.g., genes exclusively not expressed in non-thyroid may not be filtered out and may remain among the pool of candidate mutation calls).

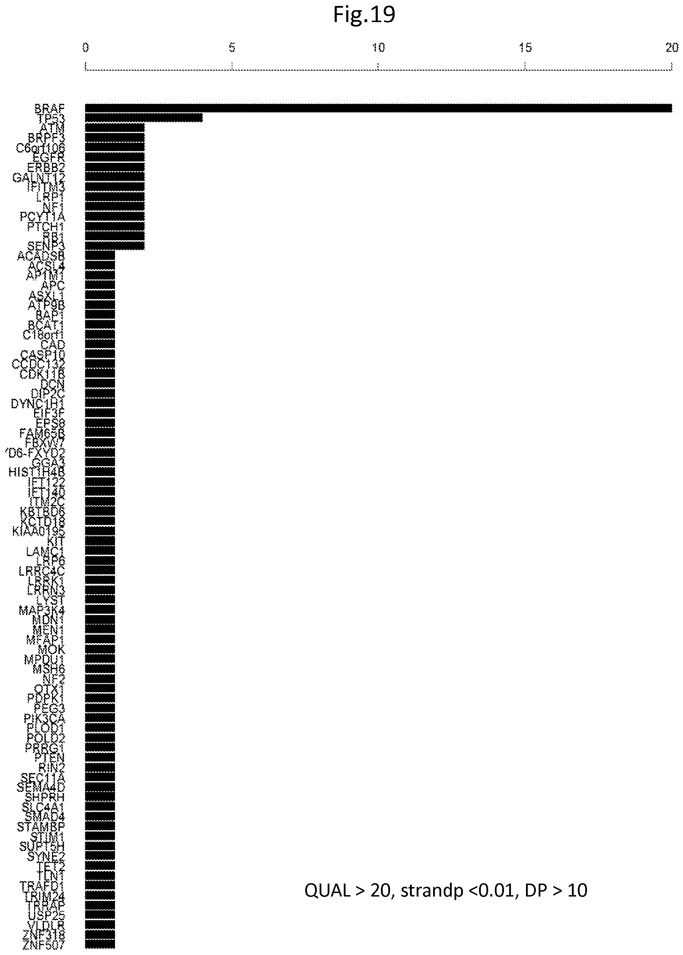

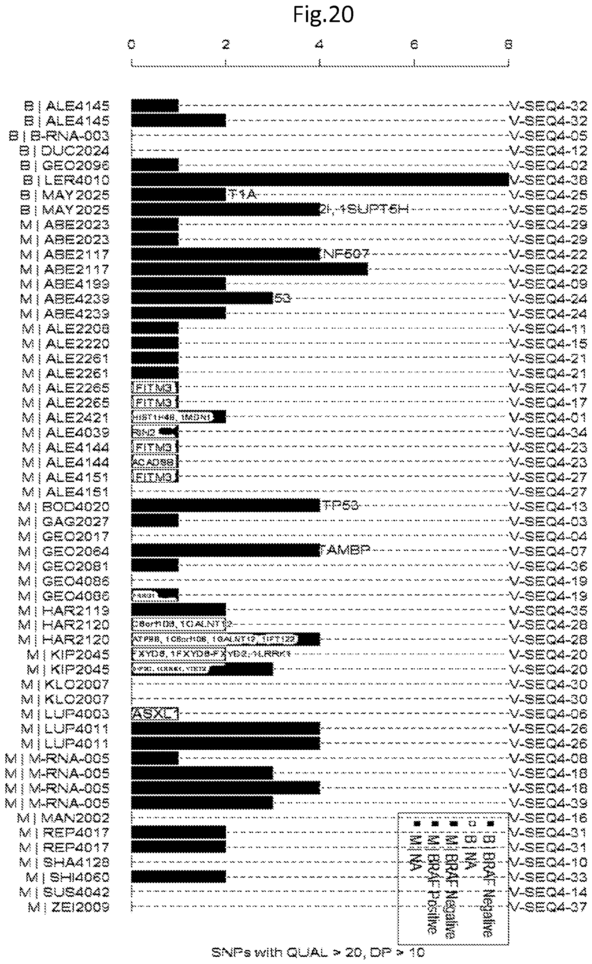

Data may then be aggregated across multiple samples and additional filtering performed based on quality, strand bias, variant allele frequency and predicted variant effects etc . In some cases this may be performed in an R layer of the post-processing steps. Aggregated and filtered data may be used to generate a profile or mutation profile reflective of called mutations as shown in Figures 19-23.

In some cases, mutation calls may be used to generate a profile using the COSMIC database of known sites of somatic variation in a disease such as cancer. In some cases, more filtering may be performed, based on quality, variant effects, strand bias etc.

**XI. 3'-5' -Amplification Bias Normalization**

The present disclosure also provides for compositions and methods for normalizing microarray data susceptible to 3' amplification bias. These methods may be applicable to a variety of applications, including but not limited to sample analysis of cancers such as lymphoma or thyroid as described herein

Generally, nucleic acid amplification may introduce a 3' bias on the relative abundance of resulting amplicons. Generally, nucleic acids, such as expressed mRNA transcripts are isolated from a sample from a subject and further amplified. In some cases, mRNA transcripts may be amplified using a combination of RT-PCR and PCR or other methods as described herein to produce amplified products. Amplification may be aided with one or probes. In some cases, amplified products may be analyzed on a microarray. In some cases microarray probe intensity signals may vary systematically as a function of the distance of the probe from the 3' end of the transcript. In some cases, this may occur because of a lack of priming sites beyond the 3' terminal end of a nucleic acid template. In contrast, priming sites that are farther away from the 3' terminal end of a given template may benefit from the processivity of the polymerase, which may give rise to multiple amplicons that overlap the same region. In some cases, despite the use of a combination of random hexamers and poly-dT primers during amplification, t 3' bias may be observed when probeset intensity signals are mapped to their coordinates on a transcript. Some transcripts may appear more sensitive than others to 3' terminal end priming and amplification bias. In some cases, use of differing ratios of random hexamer/poly-dT primers between experiments may bias data analysis between one or more experiments. For example, calculated gene expression signals between one or more experiments may be affected by 3' terminal amplification bias. This experimental variability may limit use of data reproducibility in a clinical diagnostic setting.

The present disclosure provides compositions and methods capable of calculating the extent of the 3' bias for all transcripts using data from identical biological samples run in at least two distinct microarray experiments, and, applying a normalization procedure to correct for the 3' bias. Normalization may be performed with quantile normalization, prior to transcript summarization, and subsequent differential gene expression analysis. In some cases, the compositions and methods provide a normalization procedure that further provides an output latent variable that may be used to characterize the effective 3' distance for a given probe mapped to an individual mRNA transcript. This 3' variable may then be calculated for all probes within all transcripts in the array. In some cases, a relative or effective distance value may be calculated. In some cases, the pattern of response of probes within each transcript to the effective 3' distance may be identified and used to train an algorithm. The trained algorithm may be used to normalize or adjust data generated using probes for which the effective 3' distance has been determined.

In some cases, the training information may then be used as input for another step in analysis, whereby calculated 3' variables may be used to normalize incoming microarray data from future (or past) experiments by standardizing the response to this newly estimated factor. This method may be used to normalize probe intensities and may be used to remove systematic deviations caused by the 3' bias, as empirically characterized per transcript with the training set. In some cases, 3' variables may be a collection of data-derived correction factors that may be applied to compensate for the technical variability associated with nucleic acid amplification.

The composition and methods of the disclosure for establishing effective distances to the 3' end of a transcript may be performed in a variety of ways. This may include but is not limited to the following:

**A. Method 1: Use of genome annotations**

In one method, genome annotations, RefSeq data, and array probe positions along the genomic coordinates may be used to assemble full length transcripts, estimate positions of probes along the length of the transcript and calculate transcriptomic distance to the 3' end of the transcript. The transcriptomic distance to 3' end may be used as the effective distance. In some instances, this may be applicable if the response of the amplification procedure is primarily driven by the location of poly-A sites.

**B. Method 2: Use of existing polyA site annotations**

In another method, existing poly-A site annotations (polyA-DB, available publicly through the genome browser), and array probe positions along the genomic coordinates may be used to assemble full length transcripts, estimate positions of probes along the length of the transcript and calculate transcriptomic distance to the nearest downstream poly-A site. The distance to poly-A site may be used as as the effective distance; in the absence of poly-A site within the transcript, a specific code may be used for the effective distance. In some cases poly-A site usage may be tissue specific and existing annotations may be based primarily on motif searches.

**C. Method 3: Use of polyA sites based on RNA-seq data**

In another method, existing databases for poly-A site location based on RNA sequencing data (HELICOS data set), and array probe positions along the genomic coordinates may be used to assemble full length transcripts, estimate positions of probes along the length of the transcript and calculate median weighted distance to the downstream poly-A sites within the transcript, weighted by the read counts associated with each poly-A site. The median weighted distance may be used as the effective distance. In some cases of tissue-specific expression, measurements may be done in one tissue (i.e., human liver). In some cases a single tissue may not provide coverage for poly-A sites in genes not expressed in human liver. In some cases, since poly-A site usage is tissue specific, even in case of expressed mRNA sequences, the location may be different in the tissue of interest.

**D. Method 4: Use of variability between two reagent batches**

In another method, paired intensity profiles obtained for tissues of interest on a specific microarray platform under two reagent batches showing variability with respect to probe positions may be used to assemble full length transcripts, estimate positions of probes along the length of the transcript and calculate relative transcriptomic distances to the 3'-most probe within the transcript. A per-transcript alignment of probes may then be performed within the transcript to provide the effective distance variable.

An initial approximation may then be estimated and compared to the shape of the dependence of probe-wise median residuals on the relative position of probes. One of the annotation methods, as described herein, may be used to compute an "initial approximation" for the effective distance variable. Then a series of computations may be performed including but not limited to: a computation for pair-wise residual matrix per probe per sample ; a computation for median residuals per probe across samples; and a computation for median profile for the dependence of median residuals on the initial approximation of the effective distance variable. The transcripts may then be aligned. Alignments of each transcript may be refined to the effective distance variable using intensity profiles obtained under two different reagent lots for the same biological samples. Then another series of computations may be performed including but not limited to: a computation of pairwise residual matrix per probe per sample and a computation of median residuals per probe. In some cases, fixed relative distances from one or more probes to the 3'-most probe may then be estimated using one of the methods described herein. In some cases, alignment of the 3'-most probe within the transcript at varying positions along the effective distance variable may be assumed so as to minimize an objective criterion that characterizes deviation of the observed median residual profile per transcript to the median profile estimated from all probes obtained in the previous step, given a particular alignment. An objective criterion may include but is not limited to mean squared error. After this step, the best alignment to adjust the effective distance for all probes within a transcript may be used.

**E. Normalization**

After these steps, in some cases, data may be normalized given the calculated distances. Given assignment of probes to the effective distance variable, a normalization procedure can be applied to microarray data generated using a variety of amplification reagents to minimize the variability attributable to the variable amplification along the length of the transcript.

One such procedure may be based on the quantile normalization approach. For example, a set of microarrays from a specific reagent batch may be designated as the "normalization seed set" from which a normalization target distribution may be derived. In a generic application of quantile normalization, such distribution may be derived for the entire set of probes on the array. To implement removal of the amplification bias, the probes may be binned into sufficiently large groups of probes representing uniform behavior with respect to the effective distance variable. Such grouping may be achieved by binning the effective distance variable into bins of equal size containing sufficient number of probes per bin (~10K) or instead devising the bins of variable sizes that minimize the variability of the seed set profile within the bins. After such grouping of probes is established, the quantile normalization can be applied within each bin to normalize to the median intensity of probes within this bin among the normalization seed set as reflected in Figures 23-27.

Standard summarization methods may then be applied to the normalize probe intensities and be followed by any gene expression analysis methods on the summarized intensities.

**XII. Specific Methods**

Parties can utilize the molecular profiling results, for example, to selectively indicate drugs or therapeutic interventions to patients likely to benefit the most from the drugs or interventions, or to identify individuals who may not benefit or can be harmed by the unnecessary use of drugs or other therapeutic interventions.

**(i) Methods utilizing a computer**

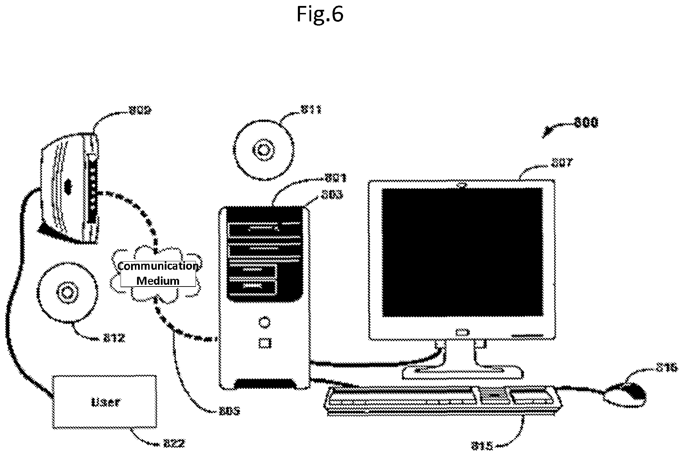

A molecular profiling business can utilize one or more computers in the methods of the present disclosure such as a computer **800** as illustrated in Figure 6. The computer **800** can be used for managing customer and sample information such as sample or customer tracking, database management, analyzing molecular profiling data, analyzing cytological data, storing data, billing, marketing, reporting results, or storing results. The computer can include a monitor **807** or other graphical interface for displaying data, results, billing information, marketing information (e.g. demographics), customer information, or sample information. The computer can also include means for data or information input **815, 816**. The computer can include a processing unit **801** and fixed **803** or removable **811** media or a combination thereof.

The computer can be accessed by a user in physical proximity to the computer, for example via a keyboard and/or mouse, or by a user **822** that does not necessarily have access to the physical computer through a communication medium **805** such as a modem, an internet connection, a telephone connection, or a wired or wireless communication signal carrier wave. In some cases, the computer can be connected to a server **809** or other communication device for relaying information from a user to the computer or from the computer to a user. In some cases, the user can store data or information obtained from the computer through a communication medium **805** on media, such as removable media **812**. It is envisioned that data relating to the present disclosure can be transmitted over such networks or connections for reception and/or review by a party. The receiving party can be but is not limited to an individual, a health care provider or a health care manager. In one case, a computer-readable medium includes a medium suitable for transmission of a result of an analysis of a biological sample, such as a gene expression profile or other bio-signature. The medium can include a result regarding a gene expression profile or other bio-signature of a subject, wherein such a result is derived using the methods described herein.

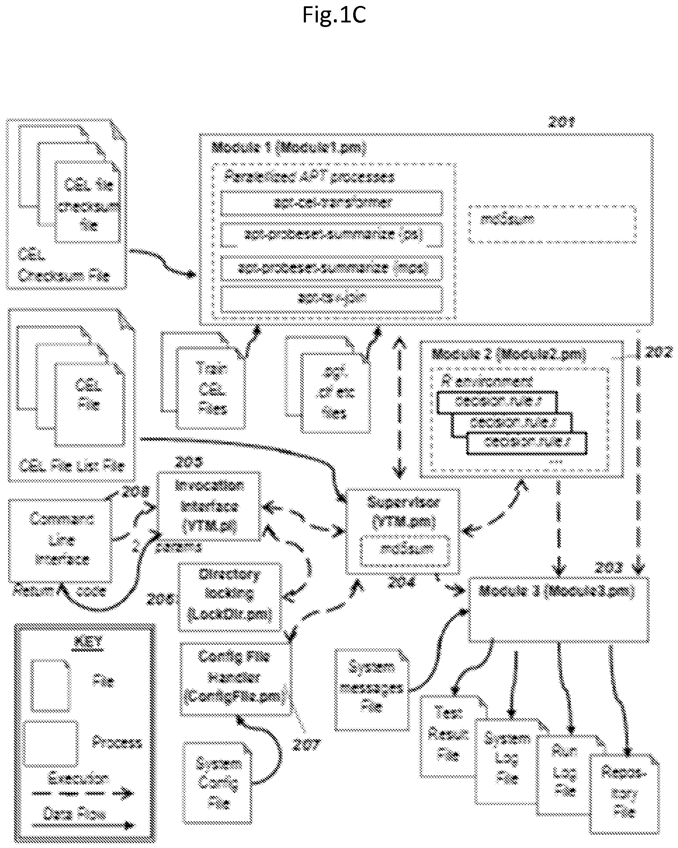

An example architecture of a system for conducting analysis according to the methods of the disclosure is provided in Figure 1C. This system comprises a number of components for processing, generating, storing, and outputting various files and information. In this example, the process is initiated using a command line interface **208,** commands from which are transmitted via an invocation interface **205** to a supervisor **204.** The supervisor **204** coordinates the functions of the system to carry out the analysis and comparison steps of the process. The first step in the analysis, illustrated at Module 1 **201,** includes a quality control check for the data to be analyzed by comparing the gene expression data file ("CEL" file) for a thyroid tissue sample to a corresponding checksum file. If data integrity is confirmed, Module 1 **201** progresses to normalization and summarization of the gene expression data, such as by utilizing the Affymetrix Power Tools (APT) suite of programs according to methods known in the art. The system can further comprise files needed for APT processes (e.g. .pgf files, .elf files, and others). Module 1 **201** is also applied to gene expression data for training sample sets ("Train CEL Files"), which are grouped to produce classifiers comprising sets of biomarkers, with gene expression data for each set of biomarkers comprising one or more reference gene expression levels correlated with the presence of one or more tissue types. Gene expression data from Module 1 **201** is next processed by Module 2 **202**, which uses the statistical software environment "R" to compare classifiers to gene expression data for the thyroid tissue sample. Each classifier is used to establish a rule for scoring the sample gene expression data as a match or non-match. Each classifier in a set of classifiers for comparison is applied to the gene expression data one after the other. The result of the comparisons performed by Module 2 **202** are processed by Module 3 **203** to report the result by generating a "test result file," which can contain for each CEL file analyzed the name of the CEL file, a test result (e.g. benign, suspicious, or a specific tissue type), and/or a comment (e.g. classifiers used, matches found, errors encountered, or other details about the comparison process). In some cases, a result of "suspicious" is reported if a sample is scored as a match to any of the classifiers at any point in a sequence of comparisons. In some cases, a result of "benign" is reported if no match between the sample gene expression data and any of the classifiers is found. Module 3 **203** also generates system log, run log, and repository files that catalogue what happened at each step of the data handling and analysis, the output from all stages of the analysis (*e.g.*, data integrity check and any error messages), and a table of results from each step, respectively. The log and repository files can be used for diagnosing errors in the comparison process, such as if a data analysis process fails to run through to completion and generation of a result. Module 3 **203** can reference a system messages file that contains a list of error messages. The system of this example architecture can also comprise a directory locking component **205** to prevent multiple analyses of the same CEL file at the same time, and a config file handler **207** to contain information regarding file location (e.g., executable files and CEL files) to help manage execution of the work flow of the system processes.

**(ii) Control Systems**

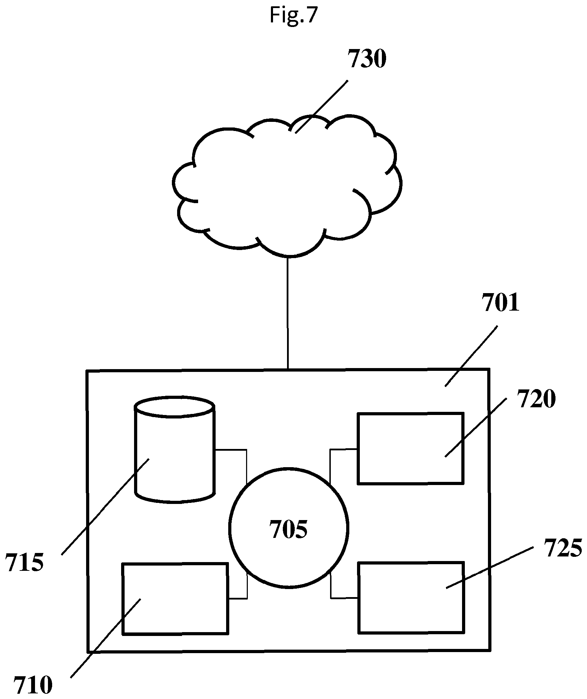

The present disclosure provides computer control systems that are programmed to implement methods of the disclosure. **FIG. 7** shows a computer system 701 that is programmed or otherwise configured to managing customer and sample information such as sample or customer tracking, database management, analyzing molecular profiling data, analyzing cytological data, storing data, billing, marketing, reporting results, storing results or other methods of the disclosures. The computer system 701 can regulate various aspects of methods of the present disclosure, such as, for example, managing customer and sample information such as sample or customer tracking, database management, analyzing molecular profiling data, analyzing cytological data, storing data, billing, marketing, reporting results, or storing results. In preferred embodiments, the computer system 701 can be useful for analyzing molecular profiling data as described in elsewhere in this application.

The computer system 701 includes a central processing unit (CPU, also "processor" and "computer processor" herein) 705, which can be a single core or multi core processor, or a plurality of processors for parallel processing. The computer system 1001 also includes memory or memory location 710 (e.g., random-access memory, read-only memory, flash memory), electronic storage unit 715 (e.g., hard disk), communication interface 720 (e.g., network adapter) for communicating with one or more other systems, and peripheral devices 725, such as cache, other memory, data storage and/or electronic display adapters. The memory 710, storage unit 715, interface 720 and peripheral devices 725 are in communication with the CPU 705 through a communication bus (solid lines), such as a motherboard. The storage unit 715 can be a data storage unit (or data repository) for storing data. The computer system 1001 can be operatively coupled to a computer network ("network") 730 with the aid of the communication interface 720. The network 730 can be the Internet, an internet and/or extranet, or an intranet and/or extranet that is in communication with the Internet. The network 730 in some cases is a telecommunication and/or data network. The network 730 can include one or more computer servers, which can enable distributed computing, such as cloud computing. The network 730, in some cases with the aid of the computer system 701, can implement a peer-to-peer network, which may enable devices coupled to the computer system 701 to behave as a client or a server.

The CPU 705 can execute a sequence of machine-readable instructions, which can be embodied in a program or software. The instructions may be stored in a memory location, such as the memory 710. Examples of operations performed by the CPU 705 can include fetch, decode, execute, and writeback.

The storage unit 715 can store files, such as drivers, libraries and saved programs. The storage unit 715 can store user data, e.g., user preferences and user programs. The computer system 701 in some cases can include one or more additional data storage units that are external to the computer system 701, such as located on a remote server that is in communication with the computer system 701 through an intranet or the Internet.

The computer system 701 can communicate with one or more remote computer systems through the network 730. For instance, the computer system 701 can communicate with a remote computer system of a user (e.g., patient or healthcare provider). Examples of remote computer systems include personal computers (e.g., portable PC), slate or tablet PC's (e.g., Apple® iPad, Samsung® Galaxy Tab), telephones, Smart phones (e.g., Apple® iPhone, Android-enabled device, Blackberry®), or personal digital assistants. The user can access the computer system 701 via the network 1030.

Methods as described herein can be implemented by way of machine (e.g., computer processor) executable code stored on an electronic storage location of the computer system 701, such as, for example, on the memory 710 or electronic storage unit 715. The machine executable or machine readable code can be provided in the form of software. During use, the code can be executed by the processor 705. In some cases, the code can be retrieved from the storage unit 715 and stored on the memory 710 for ready access by the processor 705. In some situations, the electronic storage unit 715 can be precluded, and machine-executable instructions are stored on memory 710.

The code can be pre-compiled and configured for use with a machine have a processer adapted to execute the code, or can be compiled during runtime. The code can be supplied in a programming language that can be selected to enable the code to execute in a pre-compiled or as-compiled fashion.

Aspects of the systems and methods provided herein, such as the computer system 701, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 701 can include or be in communication with an electronic display that comprises a user interface (UI) for providing, for example, results for a molecular profiling analysis. Examples of UI's include, without limitation, a graphical user interface (GUI) and web-based user interface.

Methods and systems of the present disclosure can be implemented by way of one or more algorithms. An algorithm can be implemented by way of software upon execution by one or more computer processors. Non-limiting examples for the algorithm are described elsewhere in the specification of this application.

## (iii) Process Flow

Biological samples (e.g., thyroid cells), for example, can be obtained by an endocrinologist perhaps via fine needle aspiration. Samples can be subjected to routine cytological staining procedures. The routine cytological staining can provides, for example, four different possible preliminary diagnoses: non-diagnostic, benign, ambiguous or suspicious, or malignant. The molecular profiling business can then analyze gene expression product levels as described herein. The analysis of gene expression product levels, molecular profiling, can lead to a definitive diagnosis of malignant or benign. In some cases, only a subset of samples are analyzed by molecular profiling such as those that provide ambiguous and non-diagnostic results during routine cytological examination.

In some cases, the molecular profiling results confirm the routine cytological test results. In other cases, the molecular profiling results differ. In such cases where the results differ, samples can be further tested, data can be reexamined, or the molecular profiling results or cytological assay results can be taken as the correct classification, characterization, or diagnosis. Classification, characterization, or diagnosis as benign can also include diseases or conditions that, while not malignant cancer, can indicate further monitoring or treatment (*e.g.*, HA). Similarly, classification, characterization, or diagnosis as malignant can further include classification, characterization, or diagnosis of the specific type of cancer (*e.g.*, HC) or a specific metabolic or signaling pathway involved in the disease or condition. A classification, characterization, or diagnosis can indicate a treatment or therapeutic intervention such as radioactive iodine ablation, surgery, thyroidectomy, administering one or more therapeutic agents; or further monitoring.

Administering one or more therapeutic agent can comprise administering one or more chemotherapeutic agents. In general, a "chemotherapeutic agent" refers to any agent useful in the treatment of a neoplastic condition. "Chemotherapy" means the administration of one or more chemotherapeutic drugs and/or other agents to a cancer patient by various methods, including intravenous, oral, intramuscular, intraperitoneal, intravesical, subcutaneous, transdermal, buccal, or inhalation or in the form of a suppository. In some cases, the chemotherapeutic is selected from the group consisting of mitotic inhibitors, alkylating agents, anti-metabolites, intercalating antibiotics, growth factor inhibitors, cell cycle inhibitors, enzymes, topoisomerase inhibitors, biological response modifiers, anti-hormones, angiogenesis inhibitors, and anti-androgens. Non-limiting examples are chemotherapeutic agents, cytotoxic agents, and non-peptide small molecules such as Gleevec (Imatinib Mesylate), Velcade (bortezomib), Casodex (bicalutamide), Iressa (gefitinib), and Adriamycin as well as a host of chemotherapeutic agents. Non-limiting examples of chemotherapeutic agents include alkylating agents such as thiotepa and cyclosphosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, Casodex™, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfomithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK.R™; razoxane; sizofiran; spirogermanium; tenuazonic acid; triaziquone;

2,2',2"-trichlorotriethyla-mine; urethan; vindesine; dacarbazine; mannomustine; mitobronitol; mitolactol; pipobroman; gacytosine; arabinoside ("Ara-C"); cyclophosphamide; thiotepa; taxanes, e.g. paclitaxel (TAXOL™, Bristol-Myers Squibb Oncology, Princeton, N.J.) and docetaxel (TAXOTERE™, Rhone-Poulenc Rorer, Antony, France); retinoic acid; esperamicins; capecitabine; and pharmaceutically acceptable salts, acids or derivatives of any of the above. Also included as suitable chemotherapeutic cell conditioners are anti-hormonal agents that act to regulate or inhibit hormone action on tumors such as anti-estrogens including for example tamoxifen (Nolvadex™), raloxifene, aromatase inhibiting 4(5)-imidazoles, 4-hydroxytamoxifen, trioxifene, keoxifene, LY 117018, onapristone, and toremifene (Fareston); and anti-androgens such as flutamide, nilutamide, bicalutamide, leuprolide, and goserelin; chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; camptothecin-11 (CPT-11); topoisomerase inhibitor RFS 2000; difluoromethylomithine (DMFO). Where desired, the compounds or pharmaceutical composition of the present disclosure can be used in combination with commonly prescribed anti-cancer drugs such as Herceptin®, Avastin®, Erbitux®, Rituxan®, Taxol®, Arimidex®, Taxotere®, and Velcade®.

## XIII. Kits

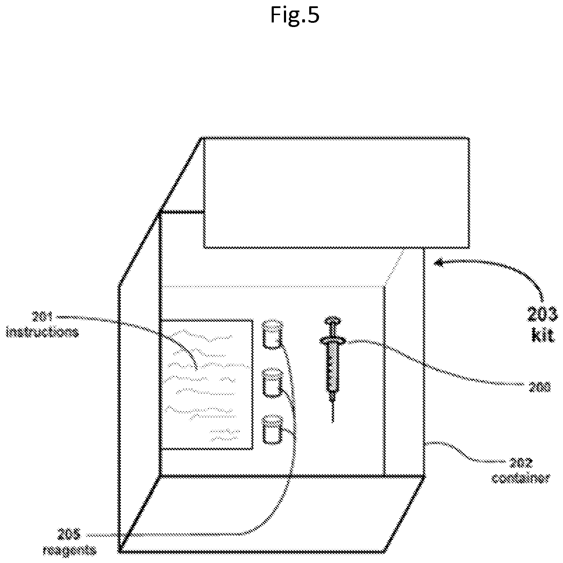

The molecular profiling business can provide a kit for obtaining a suitable sample. The kit can comprise a container, a means for obtaining a sample, reagents for storing the sample, and/or instructions for use of the kit. Figure 5 depicts an exemplary kit 203, comprising a container 202, a means 200 for obtaining a sample, reagents 205 for storing the sample, and instructions 201 for use of the kit. The kit can further comprise reagents and materials for performing the molecular profiling analysis. In some cases, the reagents and materials include a computer program for analyzing the data generated by the molecular profiling methods. In still other cases, the kit contains a means by which the biological sample is stored and transported to a testing facility such as a molecular profiling business or a third party testing center.

The molecular profiling business can also provide a kit for performing molecular profiling. The kit can comprise a means for extracting protein or nucleic acids, including any or all necessary buffers and reagents; and, a means for analyzing levels of protein or nucleic acids including controls, and reagents. The kit can further comprise software for analysis of the data provided using the methods and compositions of the present disclosure.

## Examples

### Example 1: Lymphoma Signature Markers

In this example, a list of gene markers is provided representing lymphoma signature biomarkers (as previously described in US. Appl. 13/708,439).

**Table 1. Lymphoma signature markers.**

| Table 1: Lymphoma Markers | | | | |
|---|---|---|---|---|
| TCID | Gene Symbol | Description | FDR p-value | Effect Size (log scale) |
| 2734784 | AFF1 | AF4/FMR2 family, member 1 | 7.93E-11 | -1.17 |
| 3994231 | AFF2 | AF4/FMR2 family, member 2 | 4.38E-13 | 1.48 |
| 2566848 | AFF3 | AF4/FMR2 family, member 3 | 1.94E-13 | 1.94 |
| 3443206 | AICDA | activation-induced cytidine deaminase | 7.79E-18 | 2.09 |
| 2439554 | AIM2 | absent in melanoma 2 | 1.93E-13 | 3.76 |
| 3714068 | ALDH3A2 | aldehyde dehydrogenase 3 family, member A2 | 9.91E-12 | -1.47 |

(continued)

| | Table 1: Lymphoma Markers | | | |
|---|---|---|---|---|
| **TCID** | **Gene Symbol** | **Description** | **FDR p-value** | **Effect Size (log scale)** |
| 3391149 | ALG9 | asparagine-linked glycosylation 9, alpha-1,2-mannosyltransferase homolog (S. cerevisiae) | 5.18E-15 | -3.65 |
| 3356115 | APLP2 | amyloid beta (A4) precursor-like protein 2 | 8.35E-20 | -2.38 |
| 3927226 | APP | amyloid beta (A4) precursor protein | 1.70E-43 | -2.77 |
| 3587457 | ARHGAP11A | Rho GTPase activating protein 11A | 6.88E-16 | 2.48 |
| 3587457 | ARHGAP11B | Rho GTPase activating protein 11B | 6.88E-16 | 2.48 |
| 2449559 | ASPM | asp (abnormal spindle) homolog, microcephaly associated (Drosophila) | 7.22E-18 | 2.60 |
| 2366422 | ATPIBI | ATPase, Na+/K+ transporting, beta 1 polypeptide | 4.07E-16 | -2.53 |
| 2737596 | BANK1 | B-cell scaffold protein with ankyrin repeats 1 | 2.00E-12 | 2.67 |
| 3736290 | BIRC5 | baculoviral IAP repeat-containing 5 | 3.86E-15 | 1.66 |
| 3608298 | BLM | Bloom syndrome, RecQ helicase-like | 7.50E-21 | 2.27 |
| 2798915 | BRD9 | bromodomain containing 9 | 1.53E-16 | 1.76 |
| 3765580 | BRIP1 | BRCA1 interacting protein C-terminal helicase 1 | 3.42E-16 | 2.17 |
| 3915479 | BTG3 | BTG family, member 3 | 9.84E-12 | -3.70 |
| 2570616 | BUB1 | budding uninhibited by benzimidazoles 1 homolog (yeast) | 1.55E-16 | 2.12 |
| 3589697 | BUB1B | budding uninhibited by benzimidazoles 1 homolog beta (yeast) | 1.54E-15 | 2.39 |
| 3543979 | C14orf45 | chromosome 14 open reading frame 45 | 9.09E-12 | -1.89 |
| 2949971 | C6orf10 | chromosome 6 open reading frame 10 | 1.23E-13 | 1.60 |
| 2382117 | CAPN2 | calpain 2, (m/II) large subunit | 7.01E-17 | -1.43 |
| 3590014 | CASC5 | cancer susceptibility candidate 5 | 5.75E-19 | 2.36 |
| 2784113 | CCNA2 | cyclin A2 | 1.97E-15 | 2.75 |
| 3595979 | CCNB2 | cyclin B2 | 6.37E-13 | 2.92 |

(continued)

| TCID | Gene Symbol | Description | FDR p-value | Effect Size (log scale) |
|------|-------------|-------------|-------------|-------------------------|
| | | **Table 1: Lymphoma Markers** | | |
| 3655109 | CD19 | CD 19 molecule | 1.10E-16 | 1.30 |
| 3830353 | CD22 | CD22 molecule | 1.57E-16 | 1.36 |
| 3248289 | CDC2 | cell division cycle 2, G1 to S and G2 to M | 4.38E-13 | 1.92 |
| 3936913 | CDC45L | CDC45 cell division cycle 45-like (S. cerevisiae) | 8.74E-13 | 1.52 |
| 3720896 | CDC6 | cell division cycle 6 homolog (S. cerevisiae) | 2.92E-11 | 2.18 |
| 3090697 | CDCA2 | cell division cycle associated 2 | 5.18E-15 | 1.63 |
| 2516023 | CDCA7 | cell division cycle associated 7 | 1.08E-16 | 2.16 |
| 3666409 | CDH1 | cadherin 1, type 1, E-cadherin (epithelial) | 1.97E-15 | -2.97 |
| 2780172 | CENPE | centromere protein E, 312kDa | 1.03E-22 | 2.75 |
| 2379863 | CENPF | centromere protein F, 350/400ka (mitosin) | 2.10E-14 | 2.64 |
| 2813442 | CENPH | centromere protein H | 1.29E-12 | 1.63 |
| 3258444 | CEP55 | centrosomal protein 55kDa | 1.86E-13 | 2.39 |
| 3354799 | CHEK1 | CHK1 checkpoint homolog (S. pombe) | 2.02E-13 | 2.51 |
| 2571457 | CKAP2L | cytoskeleton associated protein 2-like | 1.18E-14 | 1.88 |
| 3404436 | CLEC2D | C-type lectin domain family 2, member D | 2.36E-11 | 2.94 |
| 2406420 | CLSPN | claspin homolog (Xenopus laevis) | 1.76E-16 | 2.26 |
| 3391149 | CRYAB | crystallin, alpha B | 5.18E-15 | -3.65 |
| 2830946 | CTNNA1 | catenin (cadherin-associated protein), alpha 1, 102kDa | 2.73E-12 | -1.35 |
| 3331487 | CTNND1 | catenin (cadherin-associated protein), delta 1 | 5.75E-19 | -1.81 |
| 3915479 | CXADR | coxsackie virus and adenovirus receptor | 9.84E-12 | -3.70 |
| 3915479 | CXADRP2 | coxsackie virus and adenovirus receptor pseudogene 2 | 9.84E-12 | -3.70 |

(continued)

| TCID | Gene Symbol | Description | FDR p-value | Effect Size (log scale) |
|---|---|---|---|---|
| | | **Table 1: Lymphoma Markers** | | |
| 2417528 | DEPDC1 | DEP domain containing 1 | 4.08E-12 | 2.31 |
| 3565663 | DLGAP5 | discs, large (Drosophila) homolog-associated protein 5 | 6.95E-16 | 3.06 |
| 3269939 | DOCK1 | dedicator of cytokinesis 1 | 2.15E-13 | -1.98 |
| 3150715 | DSCC1 | defective in sister chromatid cohesion 1 homolog (S. cerevisiae) | 6.65E-14 | 2.02 |
| 2893794 | DSP | Desmoplakin | 5.05E-11 | -2.56 |
| 3365776 | E2F8 | E2F transcription factor 8 | 5.87E-22 | 1.94 |
| 2883878 | EBF1 | early B-cell factor 1 | 3.53E-12 | 1.99 |
| 3343202 | EED | embryonic ectoderm development | 1.67E-15 | 1.17 |
| 3621623 | ELL3 | elongation factor RNA polymerase II-like 3 | 4.09E-11 | 1.52 |
| 2480961 | EPCAM | epithelial cell adhesion molecule | 1.67E-15 | -3.74 |
| 2388219 | EXO1 | exonuclease 1 | 1.88E-17 | 2.05 |
| 3078348 | EZH2 | enhancer of zeste homolog 2 (Drosophila) | 8.91E-20 | 2.74 |
| 3331903 | FAM111B | family with sequence similarity 111, member B | 2.08E-11 | 2.51 |
| 4052881 | FAM72A | family with sequence similarity 72, member A | 2.78E-21 | 3.26 |
| 4052881 | FAM72B | family with sequence similarity 72, member B | 2.78E-21 | 3.26 |
| 4052881 | FAM72C | family with sequence similarity 72, member C | 2.78E-21 | 3.26 |
| 4052881 | FAM72D | family with sequence similarity 72, member D | 2.78E-21 | 3.26 |
| 3704980 | FANCA | Fanconi anemia, complementation group A | 4.48E-20 | 1.17 |
| 2610241 | FANCD2 | Fanconi anemia, complementation group D2 | 5.90E-21 | 1.46 |
| 3607537 | FANCI | Fanconi anemia, complementation group I | 1.47E-17 | 2.24 |
| 3257031 | FAS | Fas (TNF receptor superfamily, member 6) | 1.52E-16 | 2.42 |

(continued)

| TCID | Gene Symbol | Description | FDR p-value | Effect Size (log scale) |
|------|-------------|-------------|-------------|--------------------------|
| | | **Table 1: Lymphoma Markers** | | |
| 2980241 | FBXOS | F-box protein 5 | 9.91E-12 | 1.48 |
| 2439101 | FCRL1 | Fc receptor-like 1 | 4.50E-13 | 1.82 |
| 2439052 | FCRL2 | Fc receptor-like 2 | 3.19E-41 | 2.22 |
| 2439001 | FCRL3 | Fc receptor-like 3 | 8.17E-37 | 2.89 |
| 2438892 | FCRL5 | Fc receptor-like 5 | 3.91E-36 | 2.39 |
| 2363852 | FCRLA | Fc receptor-like A | 5.82E-12 | 1.90 |
| 3391149 | FDXACB1 | ferredoxin-fold anticodon binding domain containing 1 | 5.18E-15 | -3.65 |
| 2923661 | GJA1 | gap junction protein, alpha 1, 43kDa | 4.25E-12 | -2.71 |
| 3210808 | GNAQ | guanine nucleotide binding protein (G protein), q polypeptide | 7.23E-16 | -1.36 |
| 2417272 | GNG12 | guanine nucleotide binding protein (G protein), gamma 12 | 1.54E-15 | -2.63 |
| 3456805 | GTSF1 | gametocyte specific factor 1 | 1.09E-13 | 3.28 |
| 3445123 | HEBP1 | heme binding protein 1 | 1.45E-11 | -1.97 |
| 3258910 | HELLS | helicase, lymphoid-specific | 3.87E-17 | 2.56 |
| 2604254 | HJURP | Holliday junction recognition protein | 2.35E-15 | 1.81 |
| 2838656 | HMMR | hyaluronan-mediated motility receptor (RHAMM) | 2.10E-16 | 2.73 |
| 2897453 | ID4 | inhibitor of DNA binding 4, dominant negative helix-loop-helix protein | 2.33E-13 | -2.57 |
| 3610958 | IGF1R | insulin-like growth factor 1 receptor | 6.65E-14 | -2.01 |
| 3755862 | IKZF3 | IKAROS family zinc finger 3 (Aiolos) | 3.73E-11 | 2.92 |
| 2452948 | IL 10 | interleukin 10 | 2.37E-12 | 1.18 |
| 3988538 | IL13RA1 | interleukin 13 receptor, alpha 1 | 6.18E-13 | -1.64 |
| 3689880 | ISY1 | ISY1 splicing factor homolog (S. cerevisiae) | 1.24E-13 | 2.09 |

(continued)

| TCID | Gene Symbol | Description | FDR p-value | Effect Size (log scale) |
|---|---|---|---|---|
| **Table 1: Lymphoma Markers** | | | | |
| 2748198 | KIAA0922 | KIAA0922 | 1.70E-11 | 2.02 |
| 3258168 | KIF11 | kinesin family member 11 | 4.30E-19 | 3.03 |
| 3599811 | KIF23 | kinesin family member 23 | 9.99E-18 | 2.57 |
| 2334098 | KIF2C | kinesin family member 2C | 2.19E-17 | 1.18 |
| 3980560 | KIF4A | kinesin family member 4A | 4.89E-16 | 2.08 |
| 3980560 | KIF4B | kinesin family member 4B | 4.89E-16 | 2.08 |
| 3435362 | KNTC1 | kinetochore associated 1 | 5.74E-14 | 1.84 |
| 2720251 | LCORL | ligand dependent nuclear receptor corepressor-like | 1.08E-16 | 2.66 |
| 3777470 | LOC100128219 | hypothetical protein LOC100128219 | 4.81E-11 | -2.11 |
| 3756193 | LOC100131821 | hypothetical protein LOC100131821 | 4.64E-13 | 3.07 |
| 2364677 | LOC100131938 | hypothetical LOC100131938 | 5.37E-14 | -2.41 |
| 3599811 | LOC145694 | hypothetical protein LOC145694 | 9.99E-18 | 2.57 |
| 2709486 | LOC730139 | hypothetical protein LOC730139 | 2.02E-12 | 1.66 |
| 3661718 | LPCAT2 | lysophosphatidylcholine acyltransferase 2 | 2.52E-12 | -2.69 |
| 3408505 | LRMP | lymphoid-restricted membrane protein | 1.95E-12 | 3.30 |
| 3113180 | MAL2 | mal, T-cell differentiation protein 2 | 8.22E-14 | -3.47 |
| 3861413 | MAP4K1 | mitogen-activated protein kinase kinase kinase kinase 1 | 2.77E-11 | 1.53 |
| 3235789 | MCM10 | minichromosome maintenance complex component 10 | 7.44E-18 | 1.52 |
| 2577896 | MCM6 | minichromosome maintenance complex component 6 | 7.16E-11 | 1.66 |
| 2420642 | MCOLN2 | mucolipin 2 | 1.44E-28 | 3.26 |
| 3168508 | MELK | maternal embryonic leucine zipper kinase | 1.76E-16 | 2.52 |

(continued)

| TCID | Gene Symbol | Description | FDR p-value | Effect Size (log scale) |
|---|---|---|---|---|
| | | Table 1: Lymphoma Markers | | |
| 3312490 | MKI67 | antigen identified by monoclonal antibody Ki-67 | 6.32E-18 | 2.98 |
| 2734784 | MLL | myeloid/lymphoid or mixed-lineage leukemia (trithorax homolog, Drosophila) | 7.93E-11 | -1.17 |
| 2748163 | MND1 | meiotic nuclear divisions 1 homolog (S. cerevisiae) | 1.69E-12 | 3.02 |
| 3541073 | MPP5 | membrane protein, palmitoylated 5 (MAGUK p55 subfamily member 5) | 5.43E-11 | -1.29 |
| 3332403 | MS4A1 | membrane-spanning 4-domains, subfamily A, member 1 | 1.22E-11 | 2.84 |
| 2926802 | MYB | v-myb myeloblastosis viral oncogene homolog (avian) | 9.80E-11 | 1.79 |
| 2720251 | NCAPG | non-SMC condensin I complex, subunit G | 1.08E-16 | 2.66 |
| 2494484 | NCAPH | non-SMC condensin I complex, subunit H | 2.84E-17 | 1.82 |
| 2590736 | NCKAP1 | NCK-associated protein 1 | 1.60E-11 | -2.43 |
| 3776139 | NDC80 | NDC80 homolog, kinetochore complex component (S. cerevisiae) | 7.27E-14 | 2.52 |
| 2454444 | NEK2 | NIMA (never in mitosis gene a)-related kinase 2 | 5.74E-14 | 2.39 |
| 4019465 | NKRF | NFKB repressing factor | 2.30E-14 | 1.20 |
| 3842456 | NLRP4 | NLR family, pyrin domain containing 4 | 4.48E-12 | 1.26 |
| 3404436 | NPM1 | nucleophosmin (nucleolar phosphoprotein B23, numatrin) | 2.36E-11 | 2.94 |
| 2571457 | NT5DC4 | 5'-nucleotidase domain containing 4 | 1.18E-14 | 1.88 |
| 2364438 | NUF2 | NUF2, NDC80 kinetochore complex component, homolog (S. cerevisiae) | 6.32E-18 | 2.91 |
| 3741547 | P2RX5 | purinergic receptor P2X, ligand-gated ion channel, 5 | 5.24E-13 | 1.66 |
| 3589697 | PAK6 | p21 protein (Cdc42/Rac)-activated kinase 6 | 1.54E-15 | 2.39 |
| 3284596 | PARD3 | par-3 partitioning defective 3 homolog (C. elegans) | 3.42E-11 | -1.88 |
| 2638988 | PARP15 | poly (ADP-ribose) polymerase family, member 15 | 1.86E-18 | 2.83 |
| 3129149 | PBK | PDZ binding kinase | 3.72E-13 | 2.42 |

(continued)

| TCID | Gene Symbol | Description | FDR p-value | Effect Size (log scale) |
|---|---|---|---|---|
| 2364677 | PBX1 | pre-B-cell leukemia homeobox 1 | 5.37E-14 | -2.41 |
| 3921599 | PCP4 | Purkinje cell protein 4 | 3.03E-13 | -4.08 |
| 3452970 | PFKM | phosphofructokinase, muscle | 2.94E-14 | 1.07 |
| 3108226 | PGCP | plasma glutamate carboxypeptidase | 1.94E-12 | -2.14 |
| 2742985 | PLK4 | polo-like kinase 4 (Drosophila) | 1.74E-18 | 1.99 |
| 2699564 | PLOD2 | procollagen-lysine, 2-oxoglutarate 5-dioxygenase 2 | 4.66E-16 | -3.39 |
| 3987996 | PLS3 | plastin 3 (T isoform) | 1.70E-13 | -3.23 |
| 3607537 | POLG | polymerase (DNA directed), gamma | 1.47E-17 | 2.24 |
| 3130211 | PPP2CB | protein phosphatase 2 (formerly 2A), catalytic subunit, beta isoform | 6.81E-11 | -1.46 |
| 3639031 | PRC1 | protein regulator of cytokinesis 1 | 3.02E-11 | 1.90 |
| 2548500 | PRKD3 | protein kinase D3 | 1.74E-14 | 1.69 |
| 3777470 | PTPRM | protein tyrosine phosphatase, receptor type, M | 4.81E-11 | -2.11 |
| 3689880 | RAB43 | RAB43, member RAS oncogene family | 1.24E-13 | 2.09 |
| 3590086 | RAD51 | RAD51 homolog (RecA homolog, E. coli) (S. cerevisiae) | 1.67E-15 | 1.67 |
| 3401804 | RAD51AP1 | RAD51 associated protein 1 | 5.50E-11 | 2.00 |
| 2369339 | RALGPS2 | Ral GEF with PH domain and SH3 binding motif 2 | 1.25E-13 | 2.08 |
| 2476671 | RASGRP3 | RAS guanyl releasing protein 3 (calcium and DAG-regulated) | 2.31E-13 | 2.26 |
| 3485074 | RFC3 | replication factor C (activator 1) 3, 38kDa | 1.01E-14 | 1.78 |
| 2709486 | RFC4 | replication factor C (activator 1) 4, 37kDa | 2.02E-12 | 1.66 |
| 2372812 | RGS 13 | regulator of G-protein signaling 13 | 9.59E-19 | 5.19 |
| 3391149 | RPL37AP8 | ribosomal protein L37a pseudogene 8 | 5.18E-15 | -3.65 |

Table 1: Lymphoma Markers

(continued)

| TCID | Gene Symbol | Description | FDR p-value | Effect Size (log scale) |
|------|-------------|-------------|-------------|-------------------------|
| | | **Table 1: Lymphoma Markers** | | |
| 2469252 | RRM2 | ribonucleotide reductase M2 | 2.73E-12 | 3.58 |
| 4045676 | S100A1 | S100 calcium binding protein A1 | 1.22E-11 | -1.92 |
| 4045676 | S100A13 | S100 calcium binding protein A13 | 1.22E-11 | -1.92 |
| 3108146 | SDC2 | syndecan 2 | 1.11E-14 | -3.08 |
| 3452970 | SENP1 | SUMO1/sentrin specific peptidase 1 | 2.94E-14 | 1.07 |
| 3621623 | SERINC4 | serine incorporator 4 | 4.09E-11 | 1.52 |
| 3577683 | SERPINA9 | serpin peptidase inhibitor, clade A (alpha-1 antiproteinase, antitrypsin), member 9 | 1.17E-12 | 1.60 |
| 2665572 | SGOL1 | shugoshin-like 1 (S. pombe) | 7.41E-20 | 2.85 |
| 2914693 | SH3BGRL2 | SH3 domain binding glutamic acid-rich protein like 2 | 1.37E-23 | -3.77 |
| 3689880 | SHCBP1 | SHC SH2-domain binding protein 1 | 1.24E-13 | 2.09 |
| 3182781 | SMC2 | structural maintenance of chromosomes 2 | 8.44E-11 | 1.47 |
| 2427007 | SORT1 | sortilin 1 | 3.10E-17 | -2.06 |
| 2531233 | SP140 | SP140 nuclear body protein | 1.76E-12 | 3.27 |
| 2531233 | SP140L | SP140 nuclear body protein-like | 1.76E-12 | 3.27 |
| 2585933 | SPC25 | SPC25, NDC80 kinetochore complex component, homolog (S. cerevisiae) | 2.24E-13 | 3.29 |
| 3257031 | STAMBPL1 | STAM binding protein-like 1 | 1.52E-16 | 2.42 |
| 2411228 | STIL | SCL/TAL1 interrupting locus | 4.36E-12 | 1.22 |
| 2902178 | TCF19 | transcription factor 19 | 1.96E-11 | 1.07 |
| 3264621 | TCF7L2 | transcription factor 7-like 2 (T-cell specific, HMG-box) | 1.17E-12 | -1.44 |
| 3615579 | TJP 1 | tight junction protein 1 (zona occludens 1) | 3.75E-13 | -2.43 |
| 2766192 | TLR10 | toll-like receptor 10 | 1.64E-16 | 3.65 |

(continued)

| Table 1: Lymphoma Markers | | | | |
|---|---|---|---|---|
| TCID | Gene Symbol | Description | FDR p-value | Effect Size (log scale) |
| 3331487 | TMX2 | thioredoxin-related transmembrane protein 2 | 5.75E-19 | -1.81 |
| 3756193 | TOP2A | topoisomerase (DNA) II alpha 170kDa | 4.64E-13 | 3.07 |
| 3881443 | TPX2 | TPX2, microtubule-associated, homolog (Xenopus laevis) | 1.52E-12 | 2.36 |
| 2378662 | TRAF5 | TNF receptor-associated factor 5 | 3.17E-11 | 2.04 |
| 2798915 | TRIP 13 | thyroid hormone receptor interactor 13 | 1.53E-16 | 1.76 |
| 2914777 | TTK | TTK protein kinase | 1.52E-12 | 1.90 |
| 2451200 | UBE2T | ubiquitin-conjugating enzyme E2T (putative) | 3.78E-11 | 1.80 |
| 3340697 | UVRAG | UV radiation resistance associated gene | 1.97E-15 | 1.48 |
| 3985523 | WBP5 | WW domain binding protein 5 | 6.27E-11 | -2.21 |
| 3591704 | WDR76 | WD repeat domain 76 | 1.59E-15 | 2.19 |
| 3704980 | ZNF276 | zinc finger protein 276 | 4.48E-20 | 1.17 |

## Example 2: Biomarkers used for a BRAF mRNA signature classifier

In this example, 4 lists of gene markers are provided (as previously described in US. Appl. 13/708,439). Table 2 provides BRAF signature biomarkers. Tables 3,4 and 5, provide for markers relating to follicular cell signal strength, lymphocytic cell signal strength, and Hurthle cell signal strength which may be used in classification of cancer. **Table 2. BRAF signature biomarkers. PTC hetmut vs. PTC wild type, with covariates.**
The results from a LIMMA analysis (after adjusting for additional confounding covariates) are filtered based on FDR p-value ($\leq$ 0.05). Listed below are the 36 genes that passed the filter.

### Table 2: BRAF Markers, with covariates

| TCID | Gene Symbol | Description | Effect size (log scale) with covariates | FDR-adjusted p-value with covariate s |
|---|---|---|---|---|
| 3628498 | CA12 | carbonic anhydrase XII | -1.14 | 1.29E-02 |
| 3396770 | CDON | Cdon homolog (mouse) | -1.13 | 1.31E-02 |
| 3595315 | CGNL1 | cingulin-like 1 | -1.07 | 1.55E-02 |
| 3863640 | CXCL 17 | chemokine (C-X-C motif) ligand 17 | 1.36 | 2.69E-02 |
| 2858592 | DEPDC1 B | DEP domain containing 1B | 1.31 | 1.85E-03 |
| 3113280 | DEPDC6 | DEP domain containing 6 | -1.07 | 1.63E-02 |

(continued)

| TCID | Gene Symbol | Description | Effect size (log scale) with covariates | FDR-adjusted p-value with covariate s |
|---|---|---|---|---|
| 2358360 | ECM1 | extracellular matrix protein 1 | -1.76 | 2.28E-02 |
| 3331903 | FAM111 B | family with sequence similarity 111, member B | 1.23 | 2.60E-02 |
| 4019784 | FAM70A | family with sequence similarity 70, member A | -1.06 | 3.27E-02 |
| 3507282 | FLT1 | fins-related tyrosine kinase 1 (vascular endothelial growth factor/vascular permeability factor receptor) | -1.06 | 1.31E-02 |
| 3151086 | HAS2 | hyaluronan synthase 2 | -2.02 | 2.09E-02 |
| 3727583 | HLF | hepatic leukemia factor | -1.58 | 9.85E-04 |
| 3049292 | IGFBP3 | insulin-like growth factor binding protein 3 | -1.40 | 8.62E-03 |
| 2809245 | ITGA2 | integrin, alpha 2 (CD49B, alpha 2 subunit of VLA-2 receptor) | 1.27 | 2.79E-02 |
| 2608469 | ITPR1 | inositol 1,4,5-triphosphate receptor, type 1 | -1.08 | 7.28E-03 |
| 2648991 | KCNAB 1 | potassium voltage-gated channel, shaker-related subfamily, beta member 1 | -1.01 | 2.10E-02 |
| 3868783 | KLK7 | kallikrein-related peptidase 7 | 1.41 | 8.84E-03 |
| 2872848 | LOX | lysyl oxidase | 1.32 | 2.57E-02 |
| 2586038 | LRP2 | low density lipoprotein-related protein 2 | -1.15 | 4.03E-02 |
| 3040518 | MACC1 | metastasis associated in colon cancer 1 | 1.21 | 7.28E-03 |
| 2539607 | MBOAT 2 | membrane bound O-acyltransferase domain containing 2 | 1.03 | 2.87E-02 |
| 3692999 | MTIG | metallothionein 1G | -1.95 | 3.55E-02 |
| 2437118 | MUC1 | mucin 1, cell surface associated | 1.09 | 7.28E-03 |
| 3527514 | NP | nucleoside phosphorylase | 1.09 | 1.00E-02 |
| 2792127 | NPYIR | neuropeptide Y receptor Y 1 | 1.11 | 4.93E-02 |
| 2816681 | PDE8B | phosphodiesterase 8B | -1.24 | 7.28E-03 |
| 4000560 | PIR | pirin (iron-binding nuclear protein) | -1.11 | 4.30E-02 |
| 2967276 | POPDC3 | popeye domain containing 3 | -1.28 | 3.47E-02 |
| 3246888 | PRKG 1 | protein kinase, cGMP-dependent, type I | -1.07 | 2.25E-02 |
| 2580802 | RND3 | Rho family GTPase 3 | 1.17 | 1.00E-02 |
| 3467949 | SLC5A8 | solute carrier family 5 (iodide transporter), member 8 | -1.04 | 3.45E-02 |
| 2378256 | SYT14 | synaptotagmin XIV | 1.08 | 7.28E-03 |
| 2414958 | TACSTD 2 | tumor-associated calcium signal transducer 2 | 1.05 | 7.28E-03 |
| 3110608 | TM7SF4 | transmembrane 7 superfamily member 4 | 2.51 | 1.85E-03 |
| 3351200 | TMPRSS 4 | transmembrane protease, serine 4 | 1.14 | 2.69E-02 |

(continued)

| TCID | Gene Symbol | Description | Effect size (log scale) with covariates | FDR-adjusted p-value with covariate s |
|---|---|---|---|---|
| 2466554 | TPO | thyroid peroxidase | -1.75 | 2.69E-02 |

**Table 3. Markers of Follicular cell signal strength.**

| Follicular Cell Markers | |
|---|---|
| **TCID** | **Gene Symbol** |
| 3415320 | KRT7 |
| 3666409 | CDH1 |
| 3113180 | MAL2 |
| 3107548 | RBM35A |
| 4045676 | S100A13 |
| 2480961 | TACSTD1 |
| 3615579 | TJP 1 |
| 3987996 | PLS3 |
| 2699564 | PLOD2 |
| 2700585 | PFN2 |

**Table 4. Markers of Hurthle cell signal strength.**

| Hurthle Cell Markers | |
|---|---|
| **TCID** | **Gene Symbol** |
| 2566848 | AFF3 |
| 2988882 | AIMP2 |
| 3169331 | ALDH1B1 |
| 2984616 | BRP44L |
| 2822492 | C5orf30 |
| 3326635 | CD44 |
| 2750627 | CPE |
| 3042001 | CYCS |
| 3122678 | DEFB1 |
| 2739308 | EGF |
| 2988882 | EIF2AK 1 |
| 3603932 | FAH |
| 2970897 | FRK |
| 3212008 | FRMD3 |
| 3302990 | GOT1 |
| 3417703 | HSD 17B6 |
| 2877508 | HSPA9 |

(continued)

| Hurthle Cell Markers | |
|---|---|
| **TCID** | **Gene Symbol** |
| 2708922 | IGF2BP2 |
| 2604998 | IQCA1 |
| 3724545 | ITGB3 |
| 3397774 | KCNJ1 |
| 2604998 | LOC100129258 |
| 3009299 | MDH2 |
| 3654699 | NUPR1 |
| 4020655 | ODZ1 |
| 3970833 | PDHA1 |
| 2377094 | PFKFB2 |
| 3278198 | PHYH |
| 2880051 | PPP2R2B |
| 3959862 | PVALB |
| 2688499 | PVRL2 |
| 2604998 | RPL3 |
| 2964231 | RRAGD |
| 2798538 | SDHA |
| 2798538 | SDHALP1 |
| 2798538 | SDHALP2 |
| 2798538 | SDHAP3 |
| 2428501 | SLC16A1 |
| 2877508 | SNORD63 |
| 2562529 | ST3GAL5 |
| 2688499 | ZBED2 |

**Table 5. Markers of Lymphocytic cell signal strength.**

| LCT markers | |
|---|---|
| **TCID** | **Gene Symbol** |
| 3648391 | TNFRSF17 |
| 3982612 | GPR174 |
| 3404030 | KLRG1 |
| 2732508 | CXCL13 |
| 2809810 | GZMA |
| 3046520 | TARP |
| 3046520 | TRGC2 |
| 2377283 | CR2 |

(continued)

| LCT markers | |
| --- | --- |
| TCID | Gene Symbol |
| 3450861 | ABCD2 |
| 3444086 | KLRC4 |
| 3444086 | KLRK1 |
| 2440258 | SLAMF6 |
| 2427619 | KCNA3 |
| 3982560 | P2RY10 |
| 2635349 | TRAT1 |
| 2809793 | GZMK |
| 2373842 | PTPRC |
| 2363202 | SLAMF7 |
| 3204285 | CCL 19 |
| 3031556 | GIMAP2 |
| 2806468 | IL7R |
| 3443464 | PZP |
| 2362351 | PYHIN1 |

**Example 3: Biomarkers used for an alternative BRAF mRNA signature classifier**

V600E is the most common somatic point mutation in papillary thyroid carcinomas (PTC), detectable in approximately 70% of all PTCs. The BRAF mutational status is characterized in a cohort of prospectively collected thyroid FNAs (n=206), for which definitive post-surgical histopathology diagnosis as PTC was available. In order to identify a BRAF-specific mRNA signature, the samples can also be examined at the gene level using the Affymetrix Exon 1.0 ST microarray.

Two LIMMA analyses are performed comparing gene expression profiles between all available BRAF V600E mutation positive (BRAF+) and BRAF negative (BRAF-) thyroid samples. A linear SVM classifier is trained using these data in order to predict BRAF DNA mutation status.

A previous mRNA/gene level classifier has been developed and is trained exclusively on thyroid PTC samples (as previously described in US. Appl. 13/708,439). A sample list of biomarkers used for a classifier is shown in the Example 2. In this example, an alternative list of biomarkers for a BRAF mRNA signature a classifier is used to detect throid cancer. In this example, the classifier works to aid in identifying multiple malignant subtypes such as FVPTC, PTC-TCV, Hurthle-PTC, as well as benign subtypes BFN, LCT, HCA, NHP, etc.

A standard LIMMA comparison is performed using a differential gene expression model and unlike in other analyses (as previously described in US. Appl. 13/708,439) this did not adjust for covariates of follicular cell signal strength, lymphocytic cell signal strength, or Hurthle cell signal strength. The model is run according to the equation below. This model is used to train a linear SVM classifier in order to predict BRAF DNA mutation status of unknown samples.

$$Y_g = \alpha.BRAF + \varepsilon$$

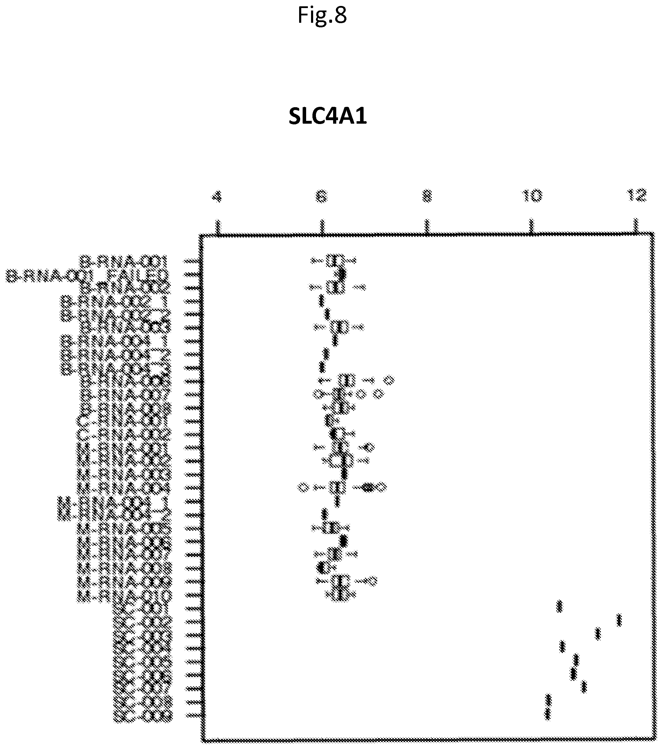

FNA biopsies may contain highly variable (heterogeneous) cellular content and a diverse number of distinct cellular types mixed together in unknowable proportions. Thyroid FNA sample pose difficulty in interpreting gene expression profiles across many samples. In order to distill a highly accurate BRAF mRNA signature, the gene expression data is analyzed using LIMMA comparisons of BRAF het mut vs. BRAF wild type. The gene list output of each analysis is filtered by LIMMA FDR p-value ≤0. 1. Preferred markers used in the classifier (Figure 8) are shown in Table 9, while a comprehensive list of differentially expressed markers is shown in Table 10).

**Table 6. FNA cytology results of sample cohort used in training (n=206).**

| DNA Mutation Status | Benign | Indeterminate | Malignant | NA |
|---|---|---|---|---|
| BRAF het mut | 0 | 0 | 25 | 3 |
| BRAF wild type | 74 | 66 | 27 | 11 |
| **Totals (n=59)** | **74** | **66** | **52** | **14** |

**Table 7. Post-surgical histopathology results of sample cohort used in training (n=206).**

| DNA Mutation Status | Benign | Malignant | NA |
|---|---|---|---|
| BRAF heterozygous mutant | 2 | 26 | 0 |
| BRAF wild type | 128 | 49 | 1 |

**Table 8. Histopathology subtypes of sample cohort used in training (n=206).**

| **Benign Histopathology Subtype** | |
|---|---|
| BLN | 1 |
| BN | 18 |
| CN | 6 |
| CYN | 5 |
| FA | 16 |
| FT-UMP | 3 |
| HA | 5 |
| LCT | 18 |
| NHP | 52 |
| NA | 4 |
| WDT-UMP | 2 |
| **Total Benign** | **130** |

| **Malignant Histopathology Subtype** | |
|---|---|
| ATC | 2 |
| FC | 3 |
| FVPTC | 8 |
| MET PTC | 1 |
| mFVPTC | 2 |
| MLN | 2 |
| mPTC | 2 |
| MTC | 1 |
| OM | 1 |
| NA | 2 |
| PTC | 50 |
| PTC-TCV | 1 |

(continued)

| Malignant Histopathology Subtype | |
|---|---|
| Total Malignant | 75 |
| | |
| Histopathology Unknown | |
| NA | 1 |
| Grand Total | 206 |

**Table 9. Preferred genes in BRAF+ vs. BRAF- classifier spanning multiple thyroid subtypes**

| TCID | Gene Symbol | Rank |
|---|---|---|
| 2828441 | PDLIM4 | 1 |
| 2809245 | ITGA2 | 2 |
| 3863640 | CXCL17 | 3 |
| 2414958 | TACSTD2 | 4 |
| 3417249 | ERBB3 | 5 |
| 3868828 | KLK10 | 6 |
| 3351200 | TMPRSS4 | 7 |
| 3110608 | TM7SF4 | 8 |
| 2884845 | GABRB2 | 9 |
| 2783596 | PDE5A | 10 |
| 2827645 | SLC27A6 | 11 |
| 2430163 | VTCN1 | 12 |
| 3154002 | KCNQ3 | 13 |
| 2497082 | IL1RL1 | 14 |
| 2608469 | ITPR1 | 15 |
| 3638204 | MFGE8 | 16 |
| 3040518 | 7A5, MACC1 | 17 |
| 2685304 | PROS 1 | 18 |
| 3497195 | CLDN10 | 19 |
| 3757108 | KRT19 | 20 |
| 2562435 | SFTPB | 21 |
| 2635906 | PHLDB2 | 22 |
| 2805078 | CDH6 | 23 |
| 3335894 | CST6 | 24 |
| 2738664 | SGMS2 | 25 |
| 2708855 | LIPH | 26 |
| 3326461 | EHF | 27 |
| 3832280 | C19orf33 | 28 |
| 3581221 | AHNAK2 | 29 |

(continued)

| TCID | Gene Symbol | Rank |
|------|-------------|------|
| 3726154 | ITGA3 | 30 |

**Classification using mutant BRAF mRNA expression signature markers.**

BRAF+ vs. BRAF- classification performance is estimated during cross-validation using the leave-one-out method. The feature selection used LIMMA and top differentially expressed markers are ranked based on lowest FDR p-value. The classifier used is linear SVM. Error rates are estimated during training using 30-fold cross validation.

**Table 10. BRAF signature biomarkers. All BRAF+ vs. All BRAF-.**

The results from a LIMMA analysis (without adjusting for additional confounding covariates) are filtered based on FDR p-value ($\leq 0.1$). Listed below are the 1192 genes that passed the filter ranked by FDR p-value. Genes with a positive Log FC value are overexpressed in BRAF+ samples

| Transcript cluster ID | Gene symbol | LogFC | P.Value | FDR adj.P.Val |
|-----------------------|-------------|-------|---------|---------------|
| 2828441 | PDLIM4 | 1.99 | 1.43E-47 | 4.57E-44 |
| 2809245 | ITGA2 | 2.99 | 5.76E-46 | 1.84E-42 |
| 3863640 | CXCL17 | 2.31 | 1.22E-45 | 3.89E-42 |
| 2414958 | TACSTD2 | 1.88 | 1.49E-42 | 4.75E-39 |
| 3417249 | ERBB3 | 1.75 | 8.57E-42 | 2.73E-38 |
| 3868828 | KLK10 | 2.12 | 1.72E-41 | 5.49E-38 |
| 3351200 | TMPRSS4 | 1.80 | 5.76E-40 | 1.83E-36 |
| 3110608 | TM7SF4 | 3.53 | 4.02E-39 | 1.28E-35 |
| 2884845 | GABRB2 | 4.12 | 2.03E-38 | 6.47E-35 |
| 2783596 | PDE5A | 2.82 | 3.57E-37 | 1.13E-33 |
| 2827645 | SLC27A6 | 3.47 | 1.01E-35 | 3.22E-32 |
| 2430163 | VTCN1 | 1.70 | 1.46E-35 | 4.65E-32 |
| 3154002 | KCNQ3 | 1.20 | 1.75E-35 | 5.55E-32 |
| 2497082 | IL1RL1 | 1.92 | 3.40E-35 | 1.08E-31 |
| 2608469 | ITPR1 | -1.70 | 1.08E-34 | 3.44E-31 |
| 3638204 | MFGE8 | 2.43 | 2.21E-34 | 7.01E-31 |
| 3040518 | 7A5, MACC1 | 2.43 | 2.57E-34 | 8.15E-31 |

(continued)

| Transcript cluster ID | Gene symbol | LogFC | P.Value | FDR adj.P.Val |
|---|---|---|---|---|
| 2685304 | PROS1 | 3.14 | 7.10E-34 | 2.25E-30 |
| 3497195 | CLDN10 | 2.33 | 5.35E-33 | 1.70E-29 |
| 3757108 | KRT19 | 2.65 | 6.30E-33 | 2.00E-29 |
| 2562435 | SFTPB | 2.75 | 8.13E-33 | 2.58E-29 |
| 2635906 | PHLDB2 | 1.68 | 6.81E-32 | 2.16E-28 |
| 2805078 | CDH6 | 2.73 | 8.58E-32 | 2.72E-28 |
| 3335894 | CST6 | 3.43 | 8.81E-32 | 2.79E-28 |
| 2738664 | SGMS2 | 1.82 | 1.07E-31 | 3.39E-28 |
| 2708855 | LIPH | 3.18 | 1.73E-31 | 5.46E-28 |
| 3326461 | EHF | 1.69 | 2.32E-31 | 7.34E-28 |
| 3832280 | C19orf33 | 1.57 | 2.57E-31 | 8.12E-28 |
| 3581221 | AHNAK2 | 1.99 | 3.23E-31 | 1.02E-27 |
| 3726154 | ITGA3 | 1.86 | 4.50E-31 | 1.42E-27 |
| 2721959 | SLC34A2 | 4.16 | 9.72E-31 | 3.07E-27 |
| 2807359 | OSMR | 1.95 | 3.98E-30 | 1.26E-26 |
| 3868783 | KLK7 | 1.77 | 7.81E-30 | 2.47E-26 |
| 2700365 | TM4SF1 | 2.84 | 6.57E-29 | 2.07E-25 |
| 4018454 | AMOT | 1.25 | 9.72E-29 | 3.07E-25 |
| 2333318 | PTPRF | 1.09 | 9.84E-29 | 3.10E-25 |
| 2966193 | C6orf168 | 1.20 | 1.84E-28 | 5.81E-25 |
| 3679959 | EMP2 | 2.02 | 2.41E-28 | 7.59E-25 |
| 3678462 | PPL | 1.07 | 2.52E-28 | 7.95E-25 |
| 3759587 | LOC100129115,PLCD3 | 1.01 | 3.87E-28 | 1.22E-24 |
| 3263743 | DUSP5 | 1.12 | 4.58E-28 | 1.44E-24 |
| 3837431 | EHD2 | 1.33 | 1.22E-27 | 3.84E-24 |

(continued)

| Transcript cluster ID | Gene symbol | LogFC | P.Value | FDR adj.P.Val |
|---|---|---|---|---|
| 2468811 | DDEF2 | 1.44 | 1.30E-27 | 4.09E-24 |
| 2657808 | CLDN16 | 3.83 | 2.06E-27 | 6.50E-24 |
| 2647315 | TM4SF4 | 1.89 | 2.52E-27 | 7.93E-24 |
| 2819044 | RASA1 | 0.68 | 2.88E-27 | 9.07E-24 |
| 2991860 | ITGB8 | 1.75 | 3.47E-27 | 1.09E-23 |
| 2902103 | C6orf205 | 1.39 | 4.84E-27 | 1.52E-23 |
| 3415744 | IGFBP6 | 2.90 | 1.04E-26 | 3.28E-23 |
| 3973891 | CXorf27,SYTL5 | 1.69 | 1.09E-26 | 3.44E-23 |
| 2437118 | MUC1 | 1.53 | 1.74E-26 | 5.47E-23 |
| 3621728 | FRMD5,hCG_1789710 | 1.03 | 1.96E-26 | 6.16E-23 |
| 2373336 | CFH | 2.51 | 2.36E-26 | 7.39E-23 |
| 2871896 | CDO1 | 1.45 | 2.50E-26 | 7.83E-23 |
| 3044072 | NOD1 | 1.19 | 3.06E-26 | 9.60E-23 |
| 3645555 | TNFRSF12A | 1.67 | 5.01E-26 | 1.57E-22 |
| 3494629 | SCEL | 2.41 | 6.57E-26 | 2.06E-22 |
| 2600689 | EPHA4 | 2.11 | 1.21E-25 | 3.78E-22 |
| 3046197 | ELMO1 | -1.88 | 1.42E-25 | 4.43E-22 |
| 3020343 | MET | 2.96 | 2.06E-25 | 6.44E-22 |
| 2720584 | SLIT2 | 1.78 | 2.09E-25 | 6.54E-22 |
| 3385951 | NOX4 | 0.88 | 2.13E-25 | 6.66E-22 |
| 3984945 | ARMCX3 | 1.24 | 2.40E-25 | 7.51E-22 |
| 2734421 | ARHGAP24 | -1.46 | 2.50E-25 | 7.81E-22 |
| 3522398 | DOCK9 | 1.70 | 2.93E-25 | 9.16E-22 |
| 3907234 | SDC4 | 2.36 | 2.96E-25 | 9.24E-22 |
| 2816298 | IQGAP2 | -1.73 | 4.54E-25 | 1.42E-21 |

(continued)

| Transcript cluster ID | Gene symbol | LogFC | P.Value | FDR adj.P.Val |
|---|---|---|---|---|
| 2872848 | LOX | 2.01 | 5.43E-25 | 1.69E-21 |
| 2370123 | XPR1 | 1.11 | 1.04E-24 | 3.25E-21 |
| 3765689 | LOC100129112,MED13 | 0.64 | 1.08E-24 | 3.37E-21 |
| 2558612 | TGFA | 1.18 | 1.92E-24 | 5.99E-21 |
| 3666366 | CDH3 | 1.53 | 1.93E-24 | 6.02E-21 |
| 3994710 | MAMLD1 | 0.84 | 2.01E-24 | 6.26E-21 |
| 2326774 | SFN | 1.13 | 2.55E-24 | 7.96E-21 |
| 2598261 | FN1 | 3.32 | 3.13E-24 | 9.74E-21 |
| 3279058 | ACBD7 | 1.65 | 3.40E-24 | 1.06E-20 |
| 3922793 | LOC100132338,PDE9A | 0.87 | 3.59E-24 | 1.12E-20 |
| 3451375 | PRICKLE1 | 2.00 | 4.07E-24 | 1.27E-20 |
| 2948790 | CDSN | 0.83 | 1.88E-23 | 5.85E-20 |
| 3345427 | ENDOD1 | 1.08 | 1.95E-23 | 6.08E-20 |
| 3352438 | POU2F3 | 0.66 | 2.82E-23 | 8.76E-20 |
| 3044129 | C7orf24 | 1.19 | 2.93E-23 | 9.10E-20 |
| 3329343 | MDK | 1.49 | 3.60E-23 | 1.12E-19 |
| 2560625 | TMEM166 | 0.74 | 3.90E-23 | 1.21E-19 |
| 2400177 | CAMK2N1 | 3.38 | 4.23E-23 | 1.31E-19 |
| 2738244 | FLJ20184 | 1.81 | 4.59E-23 | 1.43E-19 |
| 2525533 | LOC648149,MAP2 | 1.52 | 4.65E-23 | 1.44E-19 |
| 3187686 | GSN | 1.16 | 5.42E-23 | 1.68E-19 |
| 3824596 | B3GNT3 | 0.85 | 6.58E-23 | 2.04E-19 |
| 2451870 | ETNK2 | 1.43 | 1.31E-22 | 4.08E-19 |
| 3183757 | RAD23B | 0.57 | 1.56E-22 | 4.83E-19 |
| 2397025 | DHRS3 | 1.54 | 1.97E-22 | 6.10E-19 |

(continued)

| Transcript cluster ID | Gene symbol | LogFC | P.Value | FDR adj.P.Val |
|---|---|---|---|---|
| 4012178 | CITED1 | 3.06 | 2.36E-22 | 7.32E-19 |
| 2858023 | PLK2 | 1.46 | 3.05E-22 | 9.44E-19 |
| 3802924 | DSC3 | 1.31 | 3.57E-22 | 1.10E-18 |
| 2973376 | PTPRK | 1.13 | 3.83E-22 | 1.19E-18 |
| 2648535 | SGEF | 1.00 | 5.31E-22 | 1.64E-18 |
| 3416895 | METTL7B | 1.85 | 6.05E-22 | 1.87E-18 |
| 2371139 | LAMC2 | 1.61 | 6.29E-22 | 1.94E-18 |
| 3343452 | PRSS23 | 2.02 | 6.74E-22 | 2.08E-18 |
| 2924492 | HEY2 | 1.63 | 9.46E-22 | 2.92E-18 |
| 3484117 | C13orf33 | 0.94 | 9.68E-22 | 2.99E-18 |
| 2792127 | NPYIR | 1.41 | 1.18E-21 | 3.64E-18 |
| 2442008 | RXRG | 2.43 | 1.27E-21 | 3.93E-18 |
| 3125116 | DLC1 | 0.90 | 1.29E-21 | 3.99E-18 |
| 2710599 | CLDN1 | 3.02 | 1.45E-21 | 4.48E-18 |
| 3890333 | TFAP2C | 0.77 | 2.22E-21 | 6.85E-18 |
| 2452478 | LEMD1 | 1.75 | 2.23E-21 | 6.87E-18 |
| 3393720 | MPZL2 | 2.30 | 2.25E-21 | 6.94E-18 |
| 2438458 | CRABP2 | 2.10 | 2.81E-21 | 8.65E-18 |
| 2583465 | ITGB6 | 1.68 | 3.23E-21 | 9.94E-18 |
| 2781736 | CFI | 2.46 | 3.80E-21 | 1.17E-17 |
| 2451931 | GOLT1A | 1.03 | 7.25E-21 | 2.23E-17 |
| 3321150 | ARNTL | 1.37 | 9.00E-21 | 2.77E-17 |
| 2742109 | FGF2 | 1.37 | 9.37E-21 | 2.88E-17 |
| 3417809 | NAB2 | 0.79 | 1.26E-20 | 3.87E-17 |
| 3784344 | MAPRE2 | -1.05 | 1.32E-20 | 4.07E-17 |

(continued)

| Transcript cluster ID | Gene symbol | LogFC | P.Value | FDR adj.P.Val |
|---|---|---|---|---|
| 2822215 | PAM | 1.40 | 3.00E-20 | 9.22E-17 |
| 2453065 | C1orf116 | 0.88 | 3.06E-20 | 9.39E-17 |
| 3590164 | SPINT1 | 1.09 | 3.34E-20 | 1.03E-16 |
| 2751936 | GALNT7 | 1.13 | 3.66E-20 | 1.12E-16 |
| 3289235 | SGMS1 | 0.83 | 4.00E-20 | 1.23E-16 |
| 3336486 | C11orf80,RCE1 | 0.70 | 4.21E-20 | 1.29E-16 |
| 3694657 | CDH11 | 1.77 | 4.97E-20 | 1.52E-16 |
| 2979871 | C6orf98,SYNE1 | -0.99 | 7.22E-20 | 2.21E-16 |
| 3907190 | SLPI | 2.46 | 7.73E-20 | 2.37E-16 |
| 3628832 | DAPK2 | 1.64 | 7.85E-20 | 2.40E-16 |
| 2759582 | AFAP1 | 0.56 | 8.92E-20 | 2.73E-16 |
| 2344393 | PRKACB | -1.36 | 1.07E-19 | 3.27E-16 |
| 2567167 | LONRF2 | 1.75 | 1.11E-19 | 3.40E-16 |
| 3751002 | RAB34 | 1.30 | 1.19E-19 | 3.63E-16 |
| 2649113 | TIPARP | 1.00 | 1.27E-19 | 3.89E-16 |
| 3368940 | ABTB2 | 0.61 | 1.38E-19 | 4.22E-16 |
| 3683377 | GPRC5B | 1.62 | 1.54E-19 | 4.72E-16 |
| 3126191 | PSD3 | 1.79 | 1.87E-19 | 5.71E-16 |
| 3925639 | NRIP1 | 1.07 | 2.09E-19 | 6.39E-16 |
| 2582562 | ACVR1 | 0.88 | 4.78E-19 | 1.46E-15 |
| 3464860 | DUSP6 | 1.56 | 5.71E-19 | 1.74E-15 |
| 2903782 | ITPR3 | 0.88 | 6.48E-19 | 1.98E-15 |
| 3095313 | C8orf4 | 1.92 | 7.49E-19 | 2.28E-15 |
| 3441885 | SCNN1A | 1.69 | 8.01E-19 | 2.44E-15 |
| 2453793 | LAMB3 | 0.87 | 8.88E-19 | 2.71E-15 |

(continued)

| Transcript cluster ID | Gene symbol | LogFC | P.Value | FDR adj.P.Val |
|---|---|---|---|---|
| 3088213 | SH2D4A | 1.37 | 1.08E-18 | 3.29E-15 |
| 3445908 | EPS8 | 1.99 | 1.32E-18 | 4.03E-15 |
| 2980449 | PIP3-E | -1.59 | 1.67E-18 | 5.08E-15 |
| 3744463 | MYH10 | 1.67 | 2.16E-18 | 6.57E-15 |
| 3757917 | PTRF | 0.94 | 2.46E-18 | 7.47E-15 |
| 3143643 | C8orf57,LOC100128271 | 1.91 | 4.00E-18 | 1.22E-14 |
| 2742224 | SPRY1 | 1.46 | 4.85E-18 | 1.48E-14 |
| 3190190 | LCN2 | 2.10 | 5.61E-18 | 1.70E-14 |
| 3238962 | KIAA1217 | 1.77 | 7.21E-18 | 2.19E-14 |
| 2611779 | TMEM43 | 0.62 | 8.15E-18 | 2.47E-14 |
| 3087167 | TUSC3 | 2.79 | 9.74E-18 | 2.96E-14 |
| 3408831 | SSPN | 1.21 | 1.06E-17 | 3.22E-14 |
| 3850676 | KANK2 | 0.60 | 1.13E-17 | 3.42E-14 |
| 3040967 | RAPGEF5 | 1.09 | 1.20E-17 | 3.65E-14 |
| 3867458 | PLEKHA4 | 0.62 | 1.24E-17 | 3.77E-14 |
| 2659039 | MUC20,SDHA,SDHALP1,SDHALP2 | 0.66 | 1.32E-17 | 4.00E-14 |
| 2571217 | ZC3H8 | -0.63 | 1.64E-17 | 4.97E-14 |
| 3666146 | SLC7A6,TRPV6 | -0.89 | 1.94E-17 | 5.87E-14 |
| 3476012 | MPHOSPH9 | -0.84 | 2.66E-17 | 8.06E-14 |
| 3007960 | CLDN4 | 1.77 | 2.93E-17 | 8.87E-14 |
| 2962026 | LCA5 | 1.50 | 3.02E-17 | 9.15E-14 |
| 2761829 | FGFBP1 | 1.07 | 3.20E-17 | 9.68E-14 |
| 2356818 | BCL9 | 0.73 | 3.28E-17 | 9.93E-14 |
| 3493543 | KLF5 | 0.98 | 3.85E-17 | 1.16E-13 |
| 2401581 | GALE | 0.81 | 4.81E-17 | 1.45E-13 |

| Transcript cluster ID | Gene symbol | LogFC | P.Value | FDR adj.P.Val |
|---|---|---|---|---|

(continued)

| Transcript cluster ID | Gene symbol | LogFC | P.Value | FDR adj.P.Val |
|---|---|---|---|---|
| 3222170 | TNC | 1.75 | 5.85E-17 | 1.77E-13 |
| 2327817 | PTPRU | 0.60 | 6.72E-17 | 2.03E-13 |
| 3717870 | TMEM98 | 1.94 | 7.54E-17 | 2.28E-13 |
| 2400518 | ECE1 | 1.08 | 7.64E-17 | 2.31E-13 |
| 3091475 | SCARA3 | 1.13 | 8.56E-17 | 2.58E-13 |
| 3173880 | TJP2 | 0.92 | 9.00E-17 | 2.71E-13 |
| 2598828 | IGFBP5 | 2.01 | 9.04E-17 | 2.73E-13 |
| 3976341 | TIMP1 | 1.84 | 9.46E-17 | 2.85E-13 |
| 3250278 | HK1 | -0.60 | 1.08E-16 | 3.24E-13 |
| 3988596 | ZCCHC12 | 2.42 | 1.16E-16 | 3.50E-13 |
| 3126368 | PSD3 | 1.87 | 1.47E-16 | 4.44E-13 |
| 2618940 | CTNNB1 | 0.75 | 1.60E-16 | 4.81E-13 |
| 2924330 | TPD52L1 | 1.87 | 1.60E-16 | 4.83E-13 |
| 2936857 | LOC730031,MLLT4 | 1.19 | 1.78E-16 | 5.34E-13 |
| 2746591 | EDNRA | 1.51 | 1.81E-16 | 5.45E-13 |
| 2381249 | C1orf115 | 1.16 | 2.02E-16 | 6.08E-13 |
| 3762198 | COL1A1 | 0.96 | 2.50E-16 | 7.53E-13 |
| 2994981 | PRR15 | 1.27 | 2.55E-16 | 7.67E-13 |
| 4045643 | S100A16 | 1.57 | 2.65E-16 | 7.96E-13 |
| 3338192 | CCND1 | 1.42 | 2.96E-16 | 8.88E-13 |
| 2435218 | TDRKH | 0.77 | 3.54E-16 | 1.06E-12 |
| 2706791 | ZMAT3 | 0.81 | 4.02E-16 | 1.21E-12 |
| 3389976 | SLC35F2 | 1.13 | 4.48E-16 | 1.34E-12 |
| 2991233 | AHR | 1.07 | 4.75E-16 | 1.43E-12 |
| 3997825 | MXRA5 | 1.32 | 5.02E-16 | 1.50E-12 |

(continued)

| Transcript cluster ID | Gene symbol | LogFC | P.Value | FDR adj.P.Val |
|---|---|---|---|---|
| 2526806 | NA | 2.43 | 5.11E-16 | 1.53E-12 |
| 2587790 | GPR155 | -1.11 | 5.13E-16 | 1.54E-12 |
| 3986514 | PRPS1 | -0.74 | 5.81E-16 | 1.74E-12 |
| 3978943 | KLF8 | 0.60 | 6.01E-16 | 1.80E-12 |
| 2678116 | FAM116A | -0.75 | 6.33E-16 | 1.90E-12 |
| 3143660 | MMP16 | 1.74 | 6.68E-16 | 2.00E-12 |
| 2686023 | DCBLD2 | 1.58 | 7.73E-16 | 2.31E-12 |
| 3611625 | ALDH1A3 | 1.46 | 8.33E-16 | 2.49E-12 |
| 2976360 | PERP | 1.91 | 8.97E-16 | 2.68E-12 |
| 2955999 | GPR110 | 1.46 | 9.42E-16 | 2.81E-12 |
| 2827057 | GRAMD3 | 1.25 | 1.13E-15 | 3.39E-12 |
| 3518086 | TBC1D4 | -0.81 | 1.16E-15 | 3.48E-12 |
| 3107342 | PPM2C | 0.60 | 1.34E-15 | 3.99E-12 |
| 3898355 | FLRT3 | 2.54 | 1.52E-15 | 4.54E-12 |
| 2897899 | SOX4 | 0.50 | 1.69E-15 | 5.03E-12 |
| 2539607 | MBOAT2 | 1.37 | 1.77E-15 | 5.29E-12 |
| 3136178 | PLAG1 | 1.47 | 1.84E-15 | 5.49E-12 |
| 2955932 | GPR110 | 1.80 | 1.90E-15 | 5.65E-12 |
| 3815116 | PALM | 0.54 | 2.12E-15 | 6.32E-12 |
| 2455418 | AP3S1,PTPN14 | 1.01 | 2.29E-15 | 6.82E-12 |
| 3768535 | FAM20A | 1.24 | 2.40E-15 | 7.14E-12 |
| 2808748 | PARP8 | -0.72 | 2.40E-15 | 7.15E-12 |
| 3322251 | NUCB2 | -0.78 | 2.77E-15 | 8.25E-12 |
| 3267382 | INPP5F | 0.83 | 2.83E-15 | 8.42E-12 |
| 2607020 | MTERFD2 | -0.56 | 2.83E-15 | 8.42E-12 |

(continued)

| Transcript cluster ID | Gene symbol | LogFC | P.Value | FDR adj.P.Val |
|---|---|---|---|---|
| 2617188 | ITGA9 | 1.39 | 3.10E-15 | 9.21E-12 |
| 2380590 | TGFB2 | 1.41 | 3.44E-15 | 1.02E-11 |
| 3554452 | KIAA0284 | 0.69 | 3.88E-15 | 1.15E-11 |
| 3489138 | CYSLTR2 | 1.72 | 4.55E-15 | 1.35E-11 |
| 2902958 | C4A,C4B | 1.49 | 5.17E-15 | 1.54E-11 |
| 4015548 | XKRX | 0.95 | 5.52E-15 | 1.64E-11 |
| 3154263 | SLA | -1.46 | 6.41E-15 | 1.90E-11 |
| 3848644 | CTXN1 | 0.73 | 7.42E-15 | 2.20E-11 |
| 3969396 | LOC170082 | -0.81 | 8.41E-15 | 2.49E-11 |
| 2450798 | LAD1 | 0.55 | 8.49E-15 | 2.52E-11 |
| 3571944 | LTBP2 | 0.71 | 8.67E-15 | 2.57E-11 |
| 3067478 | NRCAM | 1.75 | 8.69E-15 | 2.57E-11 |
| 3466206 | TMCC3 | 1.09 | 1.08E-14 | 3.19E-11 |
| 3778252 | ANKRD12 | -0.62 | 1.15E-14 | 3.39E-11 |
| 2580802 | RND3 | 1.64 | 1.15E-14 | 3.39E-11 |
| 2669184 | LRRFIP2 | -0.54 | 1.20E-14 | 3.54E-11 |
| 2522094 | LOC26010 | 1.03 | 1.23E-14 | 3.63E-11 |
| 3110395 | RIMS2 | 1.12 | 1.27E-14 | 3.75E-11 |
| 3323052 | NAV2 | 1.06 | 1.30E-14 | 3.84E-11 |
| 2424102 | CNN3 | 1.48 | 1.37E-14 | 4.03E-11 |
| 3456081 | RARG | 0.56 | 1.38E-14 | 4.09E-11 |
| 2831209 | LOC153095,PAIP2 | -0.56 | 1.46E-14 | 4.31E-11 |
| 3044597 | PDE1C | 1.03 | 1.81E-14 | 5.33E-11 |
| 3454331 | LIMA1 | 0.78 | 2.00E-14 | 5.90E-11 |
| 2562529 | ST3GAL5 | 0.98 | 2.49E-14 | 7.34E-11 |

(continued)

| Transcript cluster ID | Gene symbol | LogFC | P.Value | FDR adj.P.Val |
|---|---|---|---|---|
| 2423829 | ARHGAP29 | 1.53 | 2.51E-14 | 7.39E-11 |
| 3445768 | ERP27 | 1.32 | 2.52E-14 | 7.41E-11 |
| 2876608 | CXCL14 | 2.07 | 2.56E-14 | 7.55E-11 |
| 2389718 | C1orf71 | -0.61 | 2.83E-14 | 8.32E-11 |
| 3216931 | C9orf156 | -0.51 | 2.84E-14 | 8.37E-11 |
| 2622121 | DAG1 | 0.68 | 2.94E-14 | 8.64E-11 |
| 2958325 | DST | 1.32 | 3.02E-14 | 8.89E-11 |
| 2350489 | KIAA1324 | -1.48 | 3.06E-14 | 9.01E-11 |
| 2511820 | PKP4 | 1.05 | 3.73E-14 | 1.10E-10 |
| 3763390 | TMEM100 | 1.36 | 3.93E-14 | 1.15E-10 |
| 2834282 | STK32A | 1.48 | 4.42E-14 | 1.30E-10 |
| 2334932 | CYP4B1 | 0.80 | 4.55E-14 | 1.34E-10 |
| 3416921 | RDH5 | 0.61 | 4.60E-14 | 1.35E-10 |
| 2768654 | OCIAD2 | 0.98 | 4.62E-14 | 1.36E-10 |
| 3417583 | RBMS2 | 1.55 | 5.11E-14 | 1.50E-10 |
| 2633390 | COL8A1 | 0.77 | 5.53E-14 | 1.62E-10 |
| 3002640 | EGFR | 1.06 | 5.58E-14 | 1.63E-10 |
| 3815097 | FSTL3 | 0.54 | 6.34E-14 | 1.86E-10 |
| 2880292 | DPYSL3 | 1.22 | 6.40E-14 | 1.87E-10 |
| 2992963 | CCDC126 | -0.66 | 6.81E-14 | 1.99E-10 |
| 3174816 | ANXA1 | 0.86 | 7.15E-14 | 2.09E-10 |
| 2878943 | PCDH1 | 0.71 | 7.21E-14 | 2.11E-10 |
| 3267314 | BAG3 | 0.67 | 7.28E-14 | 2.13E-10 |
| 3751830 | BLMH | -0.52 | 7.88E-14 | 2.30E-10 |
| 2732844 | ANXA3 | 1.34 | 9.37E-14 | 2.74E-10 |

(continued)

| Transcript cluster ID | Gene symbol | LogFC | P.Value | FDR adj.P.Val |
|---|---|---|---|---|
| 3316344 | CD151 | 0.87 | 1.01E-13 | 2.96E-10 |
| 2328273 | KIAA0746,SERINC2 | 0.99 | 1.06E-13 | 3.08E-10 |
| 3056292 | CLDN3 | 1.00 | 1.18E-13 | 3.46E-10 |
| 2396750 | FBXO2 | 0.96 | 1.24E-13 | 3.62E-10 |
| 3690747 | CBLN1 | 0.96 | 1.24E-13 | 3.62E-10 |
| 3452478 | AMIGO2 | 1.28 | 1.39E-13 | 4.04E-10 |
| 2582701 | CCDC148 | 1.33 | 1.43E-13 | 4.18E-10 |
| 2893794 | DSP | 1.53 | 1.56E-13 | 4.54E-10 |
| 2915828 | NT5E | 1.65 | 1.60E-13 | 4.67E-10 |
| 2984884 | LOC100131869,RNASET2 | -0.99 | 1.70E-13 | 4.95E-10 |
| 3127385 | PHYHIP | 0.61 | 1.81E-13 | 5.27E-10 |
| 3944210 | RASD2 | 0.86 | 2.00E-13 | 5.81E-10 |
| 2664209 | SH3BP5 | -0.81 | 2.09E-13 | 6.09E-10 |
| 2480168 | PRKCE | -0.51 | 2.16E-13 | 6.29E-10 |
| 3811339 | BCL2 | -1.01 | 2.18E-13 | 6.33E-10 |
| 2378256 | SYT14 | 1.87 | 2.29E-13 | 6.66E-10 |
| 2650393 | PPM1L | -1.02 | 2.39E-13 | 6.93E-10 |
| 2608725 | BHLHB2 | 0.92 | 3.18E-13 | 9.25E-10 |
| 3367673 | MPPED2 | -2.03 | 3.23E-13 | 9.38E-10 |
| 3866958 | CARD8 | -0.92 | 3.49E-13 | 1.01E-09 |
| 4018327 | TRPC5 | 1.70 | 3.53E-13 | 1.03E-09 |
| 3987607 | CCDC121,ZCCHC16 | 1.63 | 3.65E-13 | 1.06E-09 |
| 2448971 | UCHL5 | -0.78 | 3.79E-13 | 1.10E-09 |
| 3511189 | MTRF1 | -0.63 | 3.90E-13 | 1.13E-09 |
| 3217361 | ANKS6 | 0.55 | 4.11E-13 | 1.19E-09 |

(continued)

| Transcript cluster ID | Gene symbol | LogFC | P.Value | FDR adj.P.Val |
|---|---|---|---|---|
| 3152558 | FAM84B | 1.02 | 4.14E-13 | 1.20E-09 |
| 3423622 | SYT1 | 0.92 | 4.74E-13 | 1.37E-09 |
| 2626802 | PTPRG | 1.25 | 5.50E-13 | 1.59E-09 |
| 3855218 | COMP | 0.65 | 5.57E-13 | 1.61E-09 |
| 2731986 | LOC100129583,STBD1 | 0.68 | 5.73E-13 | 1.66E-09 |
| 3320944 | TEAD1 | 1.38 | 6.03E-13 | 1.74E-09 |
| 3867965 | RRAS | 0.70 | 6.15E-13 | 1.78E-09 |
| 2711205 | ATP13A4 | 1.60 | 6.16E-13 | 1.78E-09 |
| 3461164 | MDM1 | -0.49 | 6.75E-13 | 1.95E-09 |
| 3610958 | IGF1R | 1.03 | 6.88E-13 | 1.99E-09 |
| 2890239 | MGAT4B | 0.61 | 7.24E-13 | 2.09E-09 |
| 2530713 | CCL20 | 1.26 | 7.87E-13 | 2.27E-09 |
| 2346625 | ABHD7 | 1.02 | 7.93E-13 | 2.29E-09 |
| 3556990 | JUB | 1.16 | 8.44E-13 | 2.43E-09 |
| 3577612 | SERPINA1,SERPINA2 | 1.37 | 1.04E-12 | 2.98E-09 |
| 2413484 | YIPF1 | -0.72 | 1.07E-12 | 3.09E-09 |
| 3988987 | NDUFA1 | -0.31 | 1.10E-12 | 3.17E-09 |
| 3465248 | LUM | 1.66 | 1.17E-12 | 3.37E-09 |
| 3020273 | CAV2 | 1.51 | 1.27E-12 | 3.67E-09 |
| 3020302 | CAV1 | 1.82 | 1.31E-12 | 3.76E-09 |
| 2491386 | TCF7L1 | -0.62 | 1.32E-12 | 3.80E-09 |
| 2323899 | UBXD3 | 0.86 | 1.49E-12 | 4.29E-09 |
| 2960955 | SLC17A5 | 1.10 | 1.50E-12 | 4.30E-09 |
| 3181600 | GALNT12 | 0.97 | 1.73E-12 | 4.97E-09 |
| 2325358 | GRHL3 | 0.41 | 1.83E-12 | 5.25E-09 |

(continued)

| Transcript cluster ID | Gene symbol | LogFC | P.Value | FDR adj.P.Val |
|---|---|---|---|---|
| 3600283 | THSD4 | 0.67 | 1.85E-12 | 5.30E-09 |
| 3338552 | CTTN | 0.91 | 2.25E-12 | 6.45E-09 |
| 2331213 | KIAA0754,MACF1 | 0.50 | 2.42E-12 | 6.93E-09 |
| 2341083 | GADD45A | 0.80 | 2.50E-12 | 7.17E-09 |
| 2830861 | EGR1 | 1.56 | 2.53E-12 | 7.24E-09 |
| 2603987 | NGEF | 0.41 | 2.56E-12 | 7.34E-09 |
| 3096214 | VDAC3 | -0.41 | 2.60E-12 | 7.45E-09 |
| 2825629 | TNFAIP8 | -0.93 | 3.71E-12 | 1.06E-08 |
| 3836614 | IGFL2,LOC100128529,LOC401923 | 0.93 | 3.87E-12 | 1.11E-08 |
| 2671728 | CDCP1 | 1.02 | 3.93E-12 | 1.13E-08 |
| 3247818 | BICC1,FAM133B | 0.72 | 4.15E-12 | 1.19E-08 |
| 3129731 | DUSP4 | 0.59 | 4.24E-12 | 1.21E-08 |
| 3376529 | HRASLS3 | 1.06 | 4.35E-12 | 1.24E-08 |
| 3577870 | DICER1 | -0.53 | 4.47E-12 | 1.28E-08 |
| 3116535 | PHF20L1 | -0.54 | 4.50E-12 | 1.29E-08 |
| 2377229 | CD55 | 0.80 | 4.79E-12 | 1.37E-08 |
| 3861326 | LOC541469 | 0.54 | 4.81E-12 | 1.37E-08 |
| 3710870 | RICH2 | 0.73 | 4.87E-12 | 1.39E-08 |
| 3217242 | GABBR2 | 1.54 | 4.87E-12 | 1.39E-08 |
| 2336891 | DIO1 | -2.43 | 5.37E-12 | 1.53E-08 |
| 3430620 | WSCD2 | -0.63 | 5.40E-12 | 1.54E-08 |
| 2612625 | OXNAD1 | -0.69 | 5.65E-12 | 1.61E-08 |
| 4024373 | CDR1 | 2.00 | 5.66E-12 | 1.61E-08 |
| 3646156 | VASN | 0.65 | 5.69E-12 | 1.62E-08 |
| 2371065 | LAMC1 | 1.01 | 5.86E-12 | 1.67E-08 |

(continued)

| Transcript cluster ID | Gene symbol | LogFC | P.Value | FDR adj.P.Val |
|---|---|---|---|---|
| 2577482 | TMEM163 | 1.52 | 5.89E-12 | 1.68E-08 |
| 4001223 | RAI2 | 0.55 | 6.02E-12 | 1.71E-08 |
| 3888835 | PARD6B | 0.87 | 6.08E-12 | 1.73E-08 |
| 4008427 | NUDT10,NUDT11 | 1.27 | 6.37E-12 | 1.81E-08 |
| 3187834 | DAB2IP | 0.60 | 6.73E-12 | 1.91E-08 |
| 3099566 | FAM110B | 1.19 | 7.56E-12 | 2.15E-08 |
| 3043264 | JAZF1 | -0.86 | 7.84E-12 | 2.23E-08 |
| 3550392 | PAPOLA | -0.46 | 7.90E-12 | 2.24E-08 |
| 2458338 | ENAH | 1.15 | 8.03E-12 | 2.28E-08 |
| 2931391 | ARL4A,MTHFD1L | 0.54 | 8.50E-12 | 2.41E-08 |
| 3129065 | CLU | 1.37 | 8.95E-12 | 2.54E-08 |
| 3198974 | MPDZ | 1.05 | 1.02E-11 | 2.88E-08 |
| 3132616 | ZMAT4 | -1.44 | 1.15E-11 | 3.27E-08 |
| 2782694 | ARSJ | 0.79 | 1.19E-11 | 3.39E-08 |
| 3510066 | POSTN | 1.52 | 1.22E-11 | 3.45E-08 |
| 3848039 | C3 | 1.63 | 1.25E-11 | 3.55E-08 |
| 2417362 | DIRAS3 | 1.21 | 1.30E-11 | 3.68E-08 |
| 2558150 | AAK1,SNORA36C | -0.59 | 1.32E-11 | 3.73E-08 |
| 3320865 | PARVA | 1.22 | 1.32E-11 | 3.73E-08 |
| 3124388 | C8orf13 | -1.10 | 1.37E-11 | 3.86E-08 |
| 3377669 | LOC100128383,LTBP3 | 0.48 | 1.46E-11 | 4.13E-08 |
| 3743551 | CLDN7 | 1.63 | 1.48E-11 | 4.19E-08 |
| 3597914 | SNX22 | 0.76 | 1.49E-11 | 4.21E-08 |
| 2875193 | P4HA2 | 1.28 | 1.49E-11 | 4.22E-08 |
| 2426385 | VAV3 | -1.06 | 1.50E-11 | 4.25E-08 |

(continued)

| Transcript cluster ID | Gene symbol | LogFC | P.Value | FDR adj.P.Val |
|---|---|---|---|---|
| 3159946 | SMARCA2 | -0.45 | 1.61E-11 | 4.55E-08 |
| 3410384 | C12orf35 | -1.15 | 1.64E-11 | 4.64E-08 |
| 2545953 | FNDC4 | 0.67 | 1.75E-11 | 4.92E-08 |
| 3214845 | ASPN | 1.24 | 2.21E-11 | 6.24E-08 |
| 2361279 | LMNA | 0.63 | 2.40E-11 | 6.77E-08 |
| 3720402 | ERBB2 | 0.76 | 2.58E-11 | 7.27E-08 |
| 3622934 | MYEF2 | 0.97 | 2.69E-11 | 7.56E-08 |
| 3939707 | CABIN1 | -0.34 | 2.69E-11 | 7.56E-08 |
| 2645906 | PLS1 | 0.88 | 2.74E-11 | 7.72E-08 |
| 3653123 | PRKCB1 | -1.59 | 2.79E-11 | 7.83E-08 |
| 2560076 | RTKN | 0.44 | 2.90E-11 | 8.14E-08 |
| 3662696 | CX3CL1 | 0.44 | 2.93E-11 | 8.23E-08 |
| 2325410 | NPAL3 | 0.60 | 2.95E-11 | 8.30E-08 |
| 3709417 | ALOX15B | 0.70 | 3.17E-11 | 8.90E-08 |
| 2466554 | TPO | -2.35 | 3.31E-11 | 9.28E-08 |
| 2677356 | WNT5A | 0.93 | 3.44E-11 | 9.67E-08 |
| 3484895 | KL | 0.68 | 4.23E-11 | 1.19E-07 |
| 3726298 | TMEM92 | 0.57 | 4.31E-11 | 1.21E-07 |
| 3662387 | HERPUD1 | -0.54 | 4.36E-11 | 1.22E-07 |
| 3941643 | CCDC117 | -0.46 | 4.95E-11 | 1.39E-07 |
| 3766960 | SMURF2 | 0.50 | 5.29E-11 | 1.48E-07 |
| 3731826 | PRKCA | -0.85 | 5.32E-11 | 1.49E-07 |
| 3117384 | KHDRBS3 | 0.54 | 5.57E-11 | 1.56E-07 |
| 3666033 | NFATC3 | -0.67 | 5.68E-11 | 1.59E-07 |
| 3593575 | SLC27A2 | 1.15 | 6.13E-11 | 1.71E-07 |

(continued)

| Transcript cluster ID | Gene symbol | LogFC | P.Value | FDR adj.P.Val |
|---|---|---|---|---|
| 3458033 | ATP5B,SNORD59A,SNORD59B | -0.36 | 6.52E-11 | 1.82E-07 |
| 3464417 | MGAT4C | 1.78 | 7.95E-11 | 2.22E-07 |
| 2637112 | GAP43 | 0.97 | 8.06E-11 | 2.25E-07 |
| 2711225 | ATP13A4 | 1.67 | 8.16E-11 | 2.28E-07 |
| 2730746 | SLC4A4 | -1.56 | 8.91E-11 | 2.49E-07 |
| 3911217 | PMEPA1 | 0.51 | 9.30E-11 | 2.60E-07 |
| 3031573 | GIMAP5 | -1.50 | 1.02E-10 | 2.85E-07 |
| 3854982 | ISYNA1 | 0.46 | 1.05E-10 | 2.92E-07 |
| 3255220 | GHITM | -0.42 | 1.09E-10 | 3.03E-07 |
| 4047493 | PCDH18 | 0.62 | 1.10E-10 | 3.07E-07 |
| 3212008 | FRMD3 | 1.29 | 1.13E-10 | 3.15E-07 |
| 3690154 | NETO2 | -0.99 | 1.16E-10 | 3.22E-07 |
| 3031517 | GIMAP7 | -1.59 | 1.20E-10 | 3.33E-07 |
| 3408505 | LRMP | -1.66 | 1.20E-10 | 3.34E-07 |
| 3228007 | SETX | -0.43 | 1.21E-10 | 3.38E-07 |
| 3299504 | ACTA2 | 0.79 | 1.22E-10 | 3.38E-07 |
| 3361971 | ST5 | 0.61 | 1.22E-10 | 3.40E-07 |
| 3450234 | PKP2 | 0.81 | 1.27E-10 | 3.54E-07 |
| 3751042 | TLCD1 | 0.88 | 1.37E-10 | 3.81E-07 |
| 3009838 | CCDC146,POLR2J4 | -0.93 | 1.41E-10 | 3.91E-07 |
| 4024420 | LDOC1 | 1.04 | 1.43E-10 | 3.98E-07 |
| 3832643 | ACTN4 | 0.43 | 1.66E-10 | 4.61E-07 |
| 3905145 | TGM2 | 1.12 | 1.67E-10 | 4.63E-07 |
| 4020655 | ODZ1 | 1.54 | 1.68E-10 | 4.66E-07 |
| 2578790 | LRP1B | -1.32 | 1.70E-10 | 4.71E-07 |

(continued)

| Transcript cluster ID | Gene symbol | LogFC | P.Value | FDR adj.P.Val |
|---|---|---|---|---|
| 3102372 | SULF1 | 1.38 | 1.73E-10 | 4.79E-07 |
| 3282974 | SVIL | 0.62 | 1.78E-10 | 4.93E-07 |
| 3933536 | TFF3 | -1.44 | 1.79E-10 | 4.96E-07 |
| 3942350 | SEC14L2 | 0.51 | 1.83E-10 | 5.07E-07 |
| 2902844 | CFB | 1.52 | 1.90E-10 | 5.25E-07 |
| 2607923 | CNTN4 | 1.02 | 1.93E-10 | 5.34E-07 |
| 3494137 | LMO7 | 1.04 | 1.94E-10 | 5.36E-07 |
| 3735151 | ITGB4 | 0.54 | 2.11E-10 | 5.82E-07 |
| 3987996 | PLS3 | 1.63 | 2.16E-10 | 5.98E-07 |
| 3781429 | RBBP8 | 0.70 | 2.32E-10 | 6.41E-07 |
| 4021777 | IGSF1 | 1.82 | 2.38E-10 | 6.56E-07 |
| 3830065 | HPN | 0.70 | 2.45E-10 | 6.75E-07 |
| 2790368 | SFRP2 | 1.13 | 2.47E-10 | 6.81E-07 |
| 3356175 | ST14 | 0.77 | 2.51E-10 | 6.92E-07 |
| 3420316 | HMGA2 | 0.89 | 2.57E-10 | 7.08E-07 |
| 3807965 | MRO | -0.95 | 2.60E-10 | 7.16E-07 |
| 2687255 | CBLB | -0.62 | 2.69E-10 | 7.42E-07 |
| 2520429 | MYO1B | 1.20 | 2.69E-10 | 7.42E-07 |
| 3404030 | KLRG1 | -1.44 | 2.81E-10 | 7.74E-07 |
| 2523045 | FZD7 | 0.96 | 2.85E-10 | 7.84E-07 |
| 3489350 | CDADC1 | -0.62 | 3.06E-10 | 8.41E-07 |
| 3561039 | NFKBIA | -0.60 | 3.13E-10 | 8.61E-07 |
| 3199207 | NFIB | 1.21 | 3.26E-10 | 8.98E-07 |
| 2408041 | HPCAL4 | 0.96 | 3.30E-10 | 9.08E-07 |
| 3028977 | GSTK1 | -0.50 | 3.62E-10 | 9.94E-07 |

(continued)

| Transcript cluster ID | Gene symbol | LogFC | P.Value | FDR adj.P.Val |
|---|---|---|---|---|
| 3758510 | ETV4 | 0.46 | 3.90E-10 | 1.07E-06 |
| 3373893 | SLC43A1 | -0.61 | 4.05E-10 | 1.11E-06 |
| 3321055 | TEAD1 | 1.59 | 4.06E-10 | 1.12E-06 |
| 2688759 | ATG3 | -0.39 | 4.32E-10 | 1.18E-06 |
| 2503257 | INHBB | 0.55 | 4.57E-10 | 1.25E-06 |
| 3797295 | L3MBTL4 | -0.74 | 4.58E-10 | 1.26E-06 |
| 3499453 | TPP2 | -0.39 | 4.58E-10 | 1.26E-06 |
| 3467949 | SLC5A8 | -1.79 | 4.59E-10 | 1.26E-06 |
| 2688605 | GCET2 | -1.00 | 4.63E-10 | 1.27E-06 |
| 3649714 | C16orf45 | 0.71 | 4.63E-10 | 1.27E-06 |
| 3284596 | PARD3 | 0.99 | 4.72E-10 | 1.29E-06 |
| 2654306 | TTC14 | -0.62 | 4.90E-10 | 1.34E-06 |
| 2450416 | DDX59 | -0.48 | 5.02E-10 | 1.37E-06 |
| 2882555 | C5orf3 | -0.49 | 5.07E-10 | 1.39E-06 |
| 2954022 | TRERF1 | -1.05 | 5.36E-10 | 1.46E-06 |
| 2653114 | NAALADL2 | 0.59 | 6.27E-10 | 1.71E-06 |
| 2830946 | CTNNA1 | 0.59 | 6.31E-10 | 1.72E-06 |
| 2440354 | CD48 | -1.66 | 6.34E-10 | 1.73E-06 |
| 3867264 | CA11 | 0.67 | 6.39E-10 | 1.74E-06 |
| 3031466 | GIMAP8,LOC285972 | -1.04 | 6.40E-10 | 1.75E-06 |
| 2948425 | PPP1R10 | -0.31 | 6.80E-10 | 1.85E-06 |
| 2573570 | TFCP2L1 | -0.81 | 7.70E-10 | 2.10E-06 |
| 3945314 | KDELR3 | 0.92 | 8.30E-10 | 2.26E-06 |
| 3739867 | LOC100130876,NXN | 0.36 | 8.64E-10 | 2.35E-06 |
| 3804195 | SLC39A6 | 0.51 | 8.78E-10 | 2.39E-06 |

(continued)

| Transcript cluster ID | Gene symbol | LogFC | P.Value | FDR adj.P.Val |
|---|---|---|---|---|
| 3471374 | PPPICC | -0.41 | 8.92E-10 | 2.43E-06 |
| 3371225 | CHST1 | 1.03 | 9.11E-10 | 2.48E-06 |
| 3380080 | ORAOV1 | -0.39 | 9.40E-10 | 2.56E-06 |
| 2954355 | CUL7 | 0.28 | 9.77E-10 | 2.66E-06 |
| 3430959 | ACACB | -0.48 | 9.85E-10 | 2.68E-06 |
| 3301218 | PDLIM1 | 0.86 | 9.93E-10 | 2.70E-06 |
| 3941010 | SRRD | -0.69 | 1.01E-09 | 2.76E-06 |
| 3345222 | AMOTL1 | 0.46 | 1.09E-09 | 2.96E-06 |
| 3430462 | BTBD11 | -1.01 | 1.09E-09 | 2.97E-06 |
| 3692999 | MT1G | -2.43 | 1.10E-09 | 2.99E-06 |
| 3403092 | PTPN6 | -1.02 | 1.11E-09 | 3.01E-06 |
| 3998766 | KALI | 1.05 | 1.15E-09 | 3.11E-06 |
| 3631397 | UACA | 1.15 | 1.19E-09 | 3.22E-06 |
| 3181728 | TGFBR1 | 0.75 | 1.20E-09 | 3.25E-06 |
| 3558043 | TGM1 | 0.41 | 1.43E-09 | 3.88E-06 |
| 3576284 | RPS6KA5 | -0.99 | 1.47E-09 | 3.97E-06 |
| 3537164 | PELI2 | -0.67 | 1.49E-09 | 4.03E-06 |
| 2879166 | FGF1 | 0.51 | 1.55E-09 | 4.19E-06 |
| 3124180 | PINX1 | -0.48 | 1.59E-09 | 4.29E-06 |
| 2707764 | DCUN1D1 | -0.39 | 1.61E-09 | 4.36E-06 |
| 3982462 | PGK1 | -0.47 | 1.65E-09 | 4.45E-06 |
| 2460817 | SIPA1L2 | 0.63 | 1.67E-09 | 4.51E-06 |
| 2662087 | SRGAP3 | 0.32 | 1.68E-09 | 4.52E-06 |
| 4054204 | APOD | 1.35 | 1.74E-09 | 4.70E-06 |
| 2847710 | FASTKD3 | -0.49 | 1.79E-09 | 4.83E-06 |

(continued)

| Transcript cluster ID | Gene symbol | LogFC | P.Value | FDR adj.P.Val |
|---|---|---|---|---|
| 3190558 | SPTAN1 | 0.41 | 1.81E-09 | 4.87E-06 |
| 3013255 | PEG10 | 0.97 | 1.84E-09 | 4.97E-06 |
| 2427469 | SLC16A4 | 1.64 | 1.85E-09 | 5.00E-06 |
| 2727587 | KIT | -1.60 | 1.88E-09 | 5.07E-06 |
| 2384401 | RHOU | 0.72 | 1.92E-09 | 5.18E-06 |
| 2454485 | LPGAT1 | -0.49 | 1.94E-09 | 5.21E-06 |
| 3074640 | LUZP6,MTPN | -0.40 | 2.04E-09 | 5.49E-06 |
| 3757078 | KRT15 | 0.50 | 2.07E-09 | 5.57E-06 |
| 3840142 | ZNF480 | -0.56 | 2.10E-09 | 5.65E-06 |
| 3134922 | PCMTD1,PXDNL | -0.54 | 2.16E-09 | 5.81E-06 |
| 2356300 | PIAS3 | 0.36 | 2.16E-09 | 5.81E-06 |
| 4024685 | SLITRK4 | 0.94 | 2.22E-09 | 5.97E-06 |
| 2353717 | PTGFRN | 0.51 | 2.27E-09 | 6.09E-06 |
| 3459120 | LRIG3 | 1.27 | 2.34E-09 | 6.28E-06 |
| 3221571 | RNF183 | 0.55 | 2.40E-09 | 6.43E-06 |
| 2658785 | FAM43A | 0.59 | 2.44E-09 | 6.55E-06 |
| 2369950 | FLJ23867,QSOX1 | 0.59 | 2.53E-09 | 6.79E-06 |
| 2521574 | PLCL1 | -0.74 | 2.68E-09 | 7.17E-06 |
| 3876084 | C20orf103 | 0.95 | 2.86E-09 | 7.68E-06 |
| 3788097 | MAPK4 | -0.88 | 2.87E-09 | 7.69E-06 |
| 3679564 | USP7 | -0.40 | 2.87E-09 | 7.69E-06 |
| 2440476 | F11R,hCG_20857,RP11-544M22.4 | 0.55 | 2.93E-09 | 7.85E-06 |
| 2584018 | DPP4 | 1.63 | 3.17E-09 | 8.47E-06 |
| 3917155 | USP16 | -0.40 | 3.42E-09 | 9.15E-06 |
| 2688813 | CCDC80 | 1.49 | 3.68E-09 | 9.82E-06 |

(continued)

| Transcript cluster ID | Gene symbol | LogFC | P.Value | FDR adj.P.Val |
|---|---|---|---|---|
| 3210737 | GNA14 | -1.30 | 3.69E-09 | 9.85E-06 |
| 3777470 | LOC100128219,PTPRM | 1.04 | 3.81E-09 | 1.02E-05 |
| 3446137 | LMO3 | 1.73 | 3.81E-09 | 1.02E-05 |
| 3910724 | CBLN4 | 0.82 | 3.82E-09 | 1.02E-05 |
| 3162529 | C9orf150 | 0.72 | 3.85E-09 | 1.03E-05 |
| 2459924 | ABCB10 | -0.53 | 3.86E-09 | 1.03E-05 |
| 3111561 | PKHD1L1 | -2.06 | 3.91E-09 | 1.04E-05 |
| 3042001 | CYCS | -0.49 | 4.03E-09 | 1.07E-05 |
| 2519577 | COL3A1 | 0.61 | 4.06E-09 | 1.08E-05 |
| 2910477 | FBXO9 | -0.53 | 4.08E-09 | 1.09E-05 |
| 3329537 | C11orf49 | 0.54 | 4.12E-09 | 1.10E-05 |
| 2974635 | VNN2 | -2.14 | 4.18E-09 | 1.11E-05 |
| 2578028 | CXCR4 | -1.33 | 4.21E-09 | 1.12E-05 |
| 2907671 | PTK7 | 0.43 | 4.47E-09 | 1.19E-05 |
| 3937743 | SERPIND1 | -0.59 | 4.58E-09 | 1.22E-05 |
| 3146661 | ANKRD46 | -0.41 | 4.81E-09 | 1.28E-05 |
| 3672455 | COX4I1 | -0.25 | 4.88E-09 | 1.29E-05 |
| 2819779 | GPR98 | -0.84 | 4.90E-09 | 1.30E-05 |
| 3615579 | TJP1 | 1.16 | 5.08E-09 | 1.35E-05 |
| 2796553 | ACSL1 | -0.99 | 5.13E-09 | 1.36E-05 |
| 3167220 | UBE2R2 | -0.37 | 5.34E-09 | 1.42E-05 |
| 2567447 | TBC1D8 | -0.50 | 5.36E-09 | 1.42E-05 |
| 2437871 | SSR2 | -0.43 | 5.38E-09 | 1.43E-05 |
| 3808600 | MBD2,SNORA37 | -0.35 | 5.43E-09 | 1.44E-05 |
| 3587495 | C15orf45,SCG5 | 1.43 | 5.78E-09 | 1.53E-05 |

(continued)

| Transcript cluster ID | Gene symbol | LogFC | P.Value | FDR adj.P.Val |
|---|---|---|---|---|
| 3707759 | MIS12 | -0.54 | 5.89E-09 | 1.56E-05 |
| 3442137 | LPAR5 | 0.55 | 5.90E-09 | 1.56E-05 |
| 2519480 | GULP1 | 1.37 | 5.98E-09 | 1.58E-05 |
| 2692136 | HSPBAP1 | -0.51 | 6.16E-09 | 1.63E-05 |
| 3031533 | GIMAP4 | -1.35 | 6.21E-09 | 1.64E-05 |
| 3643938 | TMEM204 | -0.87 | 6.25E-09 | 1.65E-05 |
| 2443450 | SELL | -1.87 | 6.37E-09 | 1.68E-05 |
| 2781138 | LEF1 | -1.41 | 6.38E-09 | 1.68E-05 |
| 2820925 | RHOBTB3 | 1.10 | 6.43E-09 | 1.70E-05 |
| 3127352 | LGI3 | -0.58 | 6.51E-09 | 1.72E-05 |
| 3406589 | MGST1 | 1.15 | 6.72E-09 | 1.77E-05 |
| 2329041 | KIAA1522 | 0.42 | 6.73E-09 | 1.77E-05 |
| 3201345 | LOC554202 | 0.78 | 6.81E-09 | 1.79E-05 |
| 2726072 | ATP10D | -0.44 | 6.82E-09 | 1.80E-05 |
| 3571904 | NPC2,TMEM90A | 0.56 | 6.86E-09 | 1.81E-05 |
| 3569754 | ZFP36L1 | 0.55 | 6.95E-09 | 1.83E-05 |
| 2452440 | KLHDC8A | 0.61 | 7.00E-09 | 1.84E-05 |
| 3839206 | MYH14 | 0.36 | 7.15E-09 | 1.88E-05 |
| 2775735 | SCD5 | 0.60 | 7.32E-09 | 1.92E-05 |
| 2791197 | PDGFC | 0.91 | 7.36E-09 | 1.94E-05 |
| 3992408 | FHL1 | -1.21 | 7.82E-09 | 2.05E-05 |
| 2519229 | ITGAV | 0.94 | 7.98E-09 | 2.10E-05 |
| 2590736 | NCKAP1 | 1.19 | 8.08E-09 | 2.12E-05 |
| 2321911 | DDI2 | -0.48 | 8.15E-09 | 2.14E-05 |
| 3597977 | TRIP4 | -0.40 | 8.23E-09 | 2.16E-05 |

(continued)

| Transcript cluster ID | Gene symbol | LogFC | P.Value | FDR adj.P.Val |
|---|---|---|---|---|
| 2520069 | MGC13057 | -1.11 | 8.29E-09 | 2.17E-05 |
| 3101893 | CSPP1 | -0.48 | 8.34E-09 | 2.19E-05 |
| 3160895 | JAK2 | -0.83 | 8.58E-09 | 2.25E-05 |
| 3031556 | GIMAP2 | -1.55 | 8.72E-09 | 2.28E-05 |
| 3125915 | MTUS1 | 0.63 | 9.07E-09 | 2.37E-05 |
| 2590582 | PDE1A | 1.38 | 9.20E-09 | 2.41E-05 |
| 3473480 | FBXO21 | 0.54 | 9.21E-09 | 2.41E-05 |
| 3883921 | MYL9 | 0.85 | 9.30E-09 | 2.43E-05 |
| 3442054 | CHD4,SCARNA11 | 0.32 | 9.81E-09 | 2.56E-05 |
| 3046444 | SFRP4 | 1.12 | 1.01E-08 | 2.63E-05 |
| 2417272 | GNG12 | 1.18 | 1.03E-08 | 2.69E-05 |
| 3450899 | SLC2A13 | 0.68 | 1.09E-08 | 2.86E-05 |
| 3833141 | SELV | -0.79 | 1.11E-08 | 2.90E-05 |
| 3222128 | TNFSF15 | 0.81 | 1.18E-08 | 3.07E-05 |
| 2452977 | FAIM3 | -1.71 | 1.23E-08 | 3.21E-05 |
| 2695453 | CPNE4 | 0.79 | 1.28E-08 | 3.33E-05 |
| 2536531 | FARP2 | -0.34 | 1.28E-08 | 3.34E-05 |
| 2331558 | BMP8A | -1.96 | 1.33E-08 | 3.46E-05 |
| 2450501 | KIF21B | -1.06 | 1.41E-08 | 3.68E-05 |
| 2319423 | PIK3CD | -0.60 | 1.43E-08 | 3.71E-05 |
| 2387126 | RYR2 | -0.64 | 1.43E-08 | 3.73E-05 |
| 3143330 | FAM82B,NTAN1 | -0.49 | 1.48E-08 | 3.84E-05 |
| 2390180 | OR2AJ1,OR2W3,TRIM58 | -0.98 | 1.53E-08 | 3.99E-05 |
| 2440258 | SLAMF6 | -1.60 | 1.54E-08 | 4.00E-05 |
| 3275922 | LOC100130920,PRKCQ | -0.75 | 1.55E-08 | 4.04E-05 |

(continued)

| Transcript cluster ID | Gene symbol | LogFC | P.Value | FDR adj.P.Val |
|---|---|---|---|---|
| 2699145 | SLC9A9,ST13 | -1.06 | 1.57E-08 | 4.09E-05 |
| 2372967 | CDC73 | -0.32 | 1.58E-08 | 4.10E-05 |
| 3232944 | AKR1CL2 | -0.90 | 1.60E-08 | 4.15E-05 |
| 3331903 | FAM111B | 1.19 | 1.65E-08 | 4.28E-05 |
| 3376976 | RASGRP2 | -0.95 | 1.66E-08 | 4.31E-05 |
| 3863435 | POU2F2 | -0.49 | 1.67E-08 | 4.34E-05 |
| 3567187 | DHRS7 | -0.42 | 1.75E-08 | 4.53E-05 |
| 3463571 | PPP1R12A | -0.40 | 1.76E-08 | 4.55E-05 |
| 2364381 | RGS4 | 0.78 | 1.78E-08 | 4.62E-05 |
| 3787187 | KATNAL2 | -0.67 | 1.84E-08 | 4.75E-05 |
| 3136888 | TOX | -1.25 | 1.95E-08 | 5.05E-05 |
| 3457696 | PAN2 | -0.36 | 1.97E-08 | 5.11E-05 |
| 3816509 | GADD45B | -0.49 | 2.01E-08 | 5.19E-05 |
| 2327677 | EPB41 | -1.39 | 2.03E-08 | 5.26E-05 |
| 2476671 | RASGRP3 | -1.08 | 2.08E-08 | 5.38E-05 |
| 3118818 | LOC100131062,PTP4A3 | 0.51 | 2.09E-08 | 5.41E-05 |
| 3531736 | NPAS3 | 0.42 | 2.12E-08 | 5.48E-05 |
| 3830246 | LSR | 0.35 | 2.14E-08 | 5.51E-05 |
| 2343231 | NEXN | -0.98 | 2.16E-08 | 5.58E-05 |
| 2453370 | PLXNA2 | 0.45 | 2.17E-08 | 5.58E-05 |
| 3818547 | VAV1 | -1.05 | 2.17E-08 | 5.60E-05 |
| 2995254 | C7orf41 | -0.57 | 2.18E-08 | 5.63E-05 |
| 3282117 | ANKRD26 | -0.39 | 2.24E-08 | 5.76E-05 |
| 3276337 | ITIH5 | 0.63 | 2.24E-08 | 5.76E-05 |
| 3443804 | KLRB1 | -1.63 | 2.30E-08 | 5.92E-05 |

(continued)

| Transcript cluster ID | Gene symbol | LogFC | P.Value | FDR adj.P.Val |
|---|---|---|---|---|
| 3252036 | PLAU | 1.08 | 2.30E-08 | 5.93E-05 |
| 3133233 | PLAT | 0.43 | 2.33E-08 | 5.99E-05 |
| 2817464 | CMYA5 | 0.72 | 2.36E-08 | 6.06E-05 |
| 3505781 | PARP4 | 0.45 | 2.36E-08 | 6.06E-05 |
| 3883819 | DLGAP4 | 0.32 | 2.46E-08 | 6.32E-05 |
| 3476130 | SBNO1 | -0.32 | 2.47E-08 | 6.35E-05 |
| 3743371 | ASGR1 | -0.43 | 2.48E-08 | 6.36E-05 |
| 2358949 | CGN | 0.44 | 2.48E-08 | 6.37E-05 |
| 3623771 | TRPM7 | -0.42 | 2.51E-08 | 6.44E-05 |
| 2538480 | TSSC1 | -0.45 | 2.70E-08 | 6.91E-05 |
| 3159330 | DOCK8 | -1.23 | 2.72E-08 | 6.97E-05 |
| 3535515 | FRMD6 | 0.60 | 2.90E-08 | 7.41E-05 |
| 4005627 | CXorf38 | -0.47 | 2.91E-08 | 7.46E-05 |
| 3610804 | IGF1R | 0.63 | 2.98E-08 | 7.62E-05 |
| 2524301 | NRP2 | 1.19 | 3.10E-08 | 7.93E-05 |
| 2901970 | DDR1 | 0.56 | 3.13E-08 | 8.00E-05 |
| 3755323 | PCGF2 | 0.65 | 3.33E-08 | 8.50E-05 |
| 2768145 | COMMD8 | -0.73 | 3.39E-08 | 8.66E-05 |
| 3659156 | PHKB | -0.42 | 3.42E-08 | 8.73E-05 |
| 3340697 | UVRAG | -0.54 | 3.52E-08 | 8.99E-05 |
| 3852691 | DDX39 | -0.65 | 3.64E-08 | 9.29E-05 |
| 3383130 | KCTD14 | 0.80 | 3.77E-08 | 9.62E-05 |
| 3945133 | POLR2F | -0.28 | 3.80E-08 | 9.69E-05 |
| 3059667 | SEMA3D | -2.25 | 3.82E-08 | 9.73E-05 |
| 3366903 | MUC15 | 1.80 | 3.90E-08 | 9.95E-05 |

(continued)

| Transcript cluster ID | Gene symbol | LogFC | P.Value | FDR adj.P.Val |
|---|---|---|---|---|
| 2776998 | KLHL8 | -0.53 | 3.97E-08 | 1.01E-04 |
| 2713664 | IQCG | -0.59 | 4.10E-08 | 1.04E-04 |
| 3709244 | CHD3 | -0.42 | 4.11E-08 | 1.05E-04 |
| 3571542 | PNMA1 | 0.39 | 4.31E-08 | 1.10E-04 |
| 2362351 | PYHIN1 | -1.05 | 4.36E-08 | 1.11E-04 |
| 3986346 | CLDN2 | 0.50 | 4.41E-08 | 1.12E-04 |
| 3625271 | RAB27A | 0.61 | 4.43E-08 | 1.13E-04 |
| 2531233 | SP140 | -1.37 | 4.48E-08 | 1.14E-04 |
| 2498274 | C2orf40 | 1.72 | 4.51E-08 | 1.15E-04 |
| 3989826 | SH2D1A | -1.30 | 4.53E-08 | 1.15E-04 |
| 2657831 | IL1RAP | 0.94 | 4.57E-08 | 1.16E-04 |
| 3240987 | MAP3K8 | -0.76 | 4.62E-08 | 1.17E-04 |
| 3471769 | LOC728543,TMEM116 | -0.64 | 5.31E-08 | 1.35E-04 |
| 3331926 | FAM111A | 0.38 | 5.47E-08 | 1.39E-04 |
| 2732068 | SHROOM3 | 0.37 | 5.56E-08 | 1.41E-04 |
| 3061805 | SGCE | 1.06 | 5.79E-08 | 1.47E-04 |
| 2815965 | HMGCR | -0.56 | 5.95E-08 | 1.51E-04 |
| 2773434 | CXCL2 | 1.80 | 5.98E-08 | 1.51E-04 |
| 2999755 | AEBP1 | 0.48 | 5.99E-08 | 1.52E-04 |
| 3927446 | ADAMTS1 | 0.80 | 6.04E-08 | 1.53E-04 |
| 3750785 | SPAG5 | -0.62 | 6.11E-08 | 1.54E-04 |
| 2879105 | SPRY4 | 0.82 | 6.66E-08 | 1.68E-04 |
| 3396770 | CDON | -1.10 | 7.16E-08 | 1.81E-04 |
| 2967650 | RTN4IP1 | -0.47 | 7.25E-08 | 1.83E-04 |
| 3719210 | MGC4172 | 0.60 | 7.34E-08 | 1.85E-04 |

| Transcript cluster ID | Gene symbol | LogFC | P.Value | FDR adj.P.Val |
|---|---|---|---|---|
| 3771773 | JMJD6 | -0.51 | 7.54E-08 | 1.90E-04 |
| 2778440 | LTNCSC | 0.65 | 7.63E-08 | 1.92E-04 |
| 3489418 | SETDB2 | -0.64 | 7.85E-08 | 1.98E-04 |
| 3662150 | MT1M | -1.69 | 8.00E-08 | 2.01E-04 |
| 3662201 | MT1F,MT1H,MT1P2 | -1.79 | 8.05E-08 | 2.03E-04 |
| 2680046 | ADAMTS9 | 1.02 | 8.17E-08 | 2.06E-04 |
| 2327338 | RNF216L,XKR8 | -0.42 | 8.87E-08 | 2.23E-04 |
| 2866225 | MEF2C | -0.89 | 8.93E-08 | 2.24E-04 |
| 3159483 | KANK1 | 0.37 | 9.01E-08 | 2.27E-04 |
| 2709132 | ETV5 | 1.09 | 9.43E-08 | 2.37E-04 |
| 3959953 | TMPRSS6 | 0.32 | 9.43E-08 | 2.37E-04 |
| 2691668 | HCLS1 | -1.27 | 9.65E-08 | 2.42E-04 |
| 3226138 | AK1 | 0.95 | 9.72E-08 | 2.44E-04 |
| 2385258 | C1orf124 | -0.39 | 1.00E-07 | 2.51E-04 |
| 3389450 | COP1 | -1.43 | 1.02E-07 | 2.57E-04 |
| 3313690 | TCERG1L | 0.74 | 1.03E-07 | 2.59E-04 |
| 2973232 | C6orf174,KIAA0408 | 0.71 | 1.09E-07 | 2.73E-04 |
| 3976766 | WAS | -0.94 | 1.10E-07 | 2.74E-04 |
| 3087659 | SLC7A2 | 1.42 | 1.11E-07 | 2.77E-04 |
| 3113180 | MAL2 | 1.33 | 1.14E-07 | 2.84E-04 |
| 2451593 | CHI3L1 | 1.41 | 1.14E-07 | 2.84E-04 |
| 3589458 | THBS1 | 1.07 | 1.14E-07 | 2.85E-04 |
| 3472000 | C12orf51 | -0.48 | 1.15E-07 | 2.88E-04 |
| 3605395 | ADAMTSL3 | 0.48 | 1.16E-07 | 2.90E-04 |
| 3545466 | AHSA1 | -0.34 | 1.18E-07 | 2.94E-04 |

(continued)

| Transcript cluster ID | Gene symbol | LogFC | P.Value | FDR adj.P.Val |
|---|---|---|---|---|
| 3294142 | PLA2G12B | 0.55 | 1.23E-07 | 3.08E-04 |
| 3332663 | CD6 | -0.77 | 1.23E-07 | 3.08E-04 |
| 3649811 | NDE1 | -0.74 | 1.26E-07 | 3.15E-04 |
| 3203311 | APTX | -0.29 | 1.27E-07 | 3.17E-04 |
| 3046556 | TARP,TRG@,TRGV11,TRGV9 | -0.89 | 1.27E-07 | 3.18E-04 |
| 2339511 | ATG4C | -0.61 | 1.29E-07 | 3.21E-04 |
| 3241316 | ZEB1 | -0.79 | 1.31E-07 | 3.27E-04 |
| 3074912 | DGKI,LOC100134677,NAG20 | -0.95 | 1.32E-07 | 3.29E-04 |
| 2372858 | RGS2 | -1.70 | 1.39E-07 | 3.47E-04 |
| 2866590 | LYSMD3 | -0.47 | 1.40E-07 | 3.48E-04 |
| 3401704 | CCND2 | 0.74 | 1.44E-07 | 3.57E-04 |
| 2635741 | CD96 | -1.21 | 1.45E-07 | 3.61E-04 |
| 3972929 | FTL,GK,GK3P | -0.82 | 1.46E-07 | 3.63E-04 |
| 3441941 | VAMP1 | -0.51 | 1.47E-07 | 3.66E-04 |
| 3223425 | CDK5RAP2 | 0.45 | 1.49E-07 | 3.69E-04 |
| 2657250 | LPP | -0.52 | 1.51E-07 | 3.74E-04 |
| 4027585 | MPP1 | -1.17 | 1.54E-07 | 3.82E-04 |
| 3741585 | ITGAE | -0.43 | 1.57E-07 | 3.88E-04 |
| 3043895 | SCRN1 | 0.55 | 1.65E-07 | 4.10E-04 |
| 2358646 | BNIPL | 0.46 | 1.74E-07 | 4.30E-04 |
| 2724671 | RHOH | -1.36 | 1.74E-07 | 4.31E-04 |
| 3779362 | IMPA2 | -0.61 | 1.74E-07 | 4.31E-04 |
| 3204648 | CD72 | -1.27 | 1.89E-07 | 4.67E-04 |
| 3578152 | TCL1A | -1.44 | 1.90E-07 | 4.71E-04 |
| 3008164 | LAT2 | -0.58 | 1.91E-07 | 4.71E-04 |

(continued)

| Transcript cluster ID | Gene symbol | LogFC | P.Value | FDR adj.P.Val |
|---|---|---|---|---|
| 2797202 | SORBS2 | -1.19 | 2.00E-07 | 4.95E-04 |
| 2950823 | IHPK3 | -0.38 | 2.02E-07 | 4.99E-04 |
| 2439554 | AIM2 | -1.42 | 2.15E-07 | 5.32E-04 |
| 2841699 | CPEB4 | -0.41 | 2.18E-07 | 5.38E-04 |
| 3770743 | GRB2 | -0.61 | 2.28E-07 | 5.64E-04 |
| 2891556 | FOXQ1 | 0.43 | 2.29E-07 | 5.66E-04 |
| 2987843 | SDK1 | 0.37 | 2.42E-07 | 5.96E-04 |
| 3329983 | OR4B1,PTPRJ | -0.58 | 2.43E-07 | 6.00E-04 |
| 2740507 | UGT8 | -0.63 | 2.44E-07 | 6.00E-04 |
| 3982612 | GPR174 | -1.79 | 2.52E-07 | 6.21E-04 |
| 2777714 | SNCA | -1.58 | 2.61E-07 | 6.42E-04 |
| 3655587 | QPRT,SPN | -0.42 | 2.65E-07 | 6.53E-04 |
| 3384704 | DLG2 | -1.12 | 2.69E-07 | 6.61E-04 |
| 3211579 | TLE1 | -0.38 | 2.81E-07 | 6.92E-04 |
| 3485740 | RP11-16L6.1 | -1.28 | 2.94E-07 | 7.24E-04 |
| 2809793 | GZMK | -1.71 | 3.02E-07 | 7.41E-04 |
| 3505937 | CENPJ | -0.74 | 3.02E-07 | 7.42E-04 |
| 2906824 | FOXP4 | 0.41 | 3.08E-07 | 7.56E-04 |
| 3107548 | RBM35A | 1.32 | 3.08E-07 | 7.57E-04 |
| 2373842 | PTPRC | -1.33 | 3.09E-07 | 7.58E-04 |
| 3948047 | PARVG | -0.84 | 3.19E-07 | 7.83E-04 |
| 3464983 | ATP2B1 | -0.60 | 3.21E-07 | 7.86E-04 |
| 2324634 | CDC42,LOC643751 | -0.51 | 3.23E-07 | 7.92E-04 |
| 3032647 | DPP6 | -0.87 | 3.27E-07 | 8.00E-04 |
| 3727583 | HLF | -1.11 | 3.29E-07 | 8.06E-04 |

(continued)

| Transcript cluster ID | Gene symbol | LogFC | P.Value | FDR adj.P.Val |
|---|---|---|---|---|
| 3333622 | POLR2G | -0.39 | 3.37E-07 | 8.24E-04 |
| 4000704 | AP1S2 | -0.95 | 3.40E-07 | 8.31E-04 |
| 2832459 | PCDHB14 | 0.97 | 3.43E-07 | 8.38E-04 |
| 3509473 | DCLK1 | 0.66 | 3.44E-07 | 8.41E-04 |
| 3683845 | DCUN1D3,EXOD1 | 0.42 | 3.56E-07 | 8.69E-04 |
| 2347732 | TMEM56 | -1.35 | 3.65E-07 | 8.91E-04 |
| 3405748 | EMP1 | 0.94 | 3.65E-07 | 8.91E-04 |
| 3451814 | NELL2 | 1.28 | 3.76E-07 | 9.19E-04 |
| 3527597 | ANG,RNASE4 | 0.50 | 3.83E-07 | 9.34E-04 |
| 3211938 | RASEF | 1.18 | 3.91E-07 | 9.53E-04 |
| 3323443 | PRMT3 | -0.48 | 3.94E-07 | 9.60E-04 |
| 3453405 | FKBP11 | -0.56 | 3.98E-07 | 9.69E-04 |
| 3959350 | APOL3 | -0.60 | 4.23E-07 | 1.03E-03 |
| 3820443 | ICAM1 | 0.83 | 4.28E-07 | 1.04E-03 |
| 2625793 | SLMAP | -0.35 | 4.28E-07 | 1.04E-03 |
| 3766533 | CD79B | -0.99 | 4.36E-07 | 1.06E-03 |
| 4015838 | ARMCX6 | 0.34 | 4.37E-07 | 1.06E-03 |
| 3389353 | CASP1,INCA | -1.28 | 4.38E-07 | 1.06E-03 |
| 3347658 | ATM,NPAT | -0.64 | 4.46E-07 | 1.08E-03 |
| 3085990 | BLK | -0.59 | 4.48E-07 | 1.09E-03 |
| 2403707 | TMEM200B | -0.41 | 4.50E-07 | 1.09E-03 |
| Transcript cluster ID | Gene symbol | LogFC | P.Value | FDR adj.P.Val |
| 3106310 | DECR1 | -0.34 | 4.56E-07 | 1.11E-03 |
| 3837504 | SEPW1 | 0.49 | 4.64E-07 | 1.12E-03 |
| 3834257 | CEACAM21 | -0.64 | 4.65E-07 | 1.13E-03 |

(continued)

| Transcript cluster ID | Gene symbol | LogFC | P.Value | FDR adj.P.Val |
|---|---|---|---|---|
| 4054405 | GJA4 | 0.59 | 4.74E-07 | 1.15E-03 |
| 3894601 | FKBP1A,FKBP1C | -0.52 | 4.75E-07 | 1.15E-03 |
| 3910785 | AURKA,AURKAPS1 | -0.85 | 4.76E-07 | 1.15E-03 |
| 3332403 | MS4A1 | -1.43 | 4.82E-07 | 1.17E-03 |
| 3183604 | ZNF462 | 0.86 | 4.96E-07 | 1.20E-03 |
| 3512294 | TSC22D1 | 0.41 | 5.02E-07 | 1.22E-03 |
| 4015397 | TSPAN6 | 1.22 | 5.06E-07 | 1.22E-03 |
| 3505319 | SACS | -0.72 | 5.07E-07 | 1.23E-03 |
| 3807595 | MYO5B | 0.78 | 5.23E-07 | 1.26E-03 |
| 3979101 | FAAH2 | 0.69 | 5.38E-07 | 1.30E-03 |
| 3767339 | GNA13 | -0.57 | 5.42E-07 | 1.31E-03 |
| 3580947 | C14orf2 | -0.32 | 5.46E-07 | 1.32E-03 |
| 2395890 | CLSTN1 | 0.30 | 5.54E-07 | 1.34E-03 |
| 3349293 | NCAM1 | -0.98 | 5.56E-07 | 1.34E-03 |
| 3744680 | PIK3R5 | -0.75 | 5.73E-07 | 1.38E-03 |
| 3216195 | HSD17B3 | -0.36 | 5.89E-07 | 1.42E-03 |
| 2331974 | ZNF684 | -0.41 | 5.91E-07 | 1.42E-03 |
| 2959039 | KHDRBS2 | -0.98 | 5.93E-07 | 1.43E-03 |
| 3687752 | SEPT1 | -1.08 | 5.94E-07 | 1.43E-03 |
| 3015395 | PVRIG | -0.98 | 6.03E-07 | 1.45E-03 |
| 3779579 | TUBB6 | 0.65 | 6.04E-07 | 1.45E-03 |
| 4013549 | ITM2A | -0.96 | 6.16E-07 | 1.48E-03 |
| 3422855 | GLIPR1 | -1.08 | 6.16E-07 | 1.48E-03 |
| 3096171 | POLB hCG_2033311,LOC641768,LOC645979,L | -0.39 | 6.18E-07 | 1.48E-03 |
| 2586744 | OC728937,METTL8,RPS26,RPS26L | -0.49 | 6.26E-07 | 1.50E-03 |

(continued)

| Transcript cluster ID | Gene symbol | LogFC | P.Value | FDR adj.P.Val |
|---|---|---|---|---|
| 3119339 | CDC42,LY6E | 0.83 | 6.32E-07 | 1.52E-03 |
| 3697183 | ABBA-1 | 0.38 | 6.42E-07 | 1.54E-03 |
| 2830698 | FAM53C | -0.39 | 6.58E-07 | 1.58E-03 |
| 2752560 | SPCS3 | -0.50 | 6.69E-07 | 1.60E-03 |
| 3216276 | SLC35D2 | -0.44 | 6.71E-07 | 1.61E-03 |
| 2565592 | SEMA4C | 0.32 | 6.72E-07 | 1.61E-03 |
| 3812385 | CD226 | -1.33 | 6.91E-07 | 1.65E-03 |
| 2648991 | KCNAB1 | -1.03 | 6.92E-07 | 1.65E-03 |
| 3856720 | LOC342994,LOC388523,ZNF676,ZNF99 | 1.56 | 7.17E-07 | 1.71E-03 |
| 2436401 | JTB,RAB13 | -0.49 | 7.22E-07 | 1.73E-03 |
| 2458773 | PARP1 | -0.57 | 7.26E-07 | 1.73E-03 |
| 2556752 | SPRED2 | 0.55 | 7.36E-07 | 1.76E-03 |
| 3011492 | ADAM22 | -0.73 | 7.37E-07 | 1.76E-03 |
| 3011675 | ZNF804B | -0.82 | 7.45E-07 | 1.78E-03 |
| 2439001 | FCRL3 | -0.76 | 7.63E-07 | 1.82E-03 |
| 3176209 | TLE4 | -0.88 | 7.70E-07 | 1.84E-03 |
| 2592532 | SDPR | -0.69 | 7.89E-07 | 1.88E-03 |
| 2685944 | CPOX | -0.36 | 8.24E-07 | 1.96E-03 |
| 2832297 | PCDHB2 | 0.94 | 8.29E-07 | 1.97E-03 |
| 2462329 | ERO1LB | -0.81 | 8.38E-07 | 1.99E-03 |
| 3486728 | SLC25A15 | -0.92 | 8.39E-07 | 1.99E-03 |
| 2493992 | KCNIP3 | -0.55 | 8.43E-07 | 2.00E-03 |
| 2495187 | ZAP70 | -0.44 | 8.51E-07 | 2.02E-03 |
| 2511603 | GALNT5 | 0.79 | 8.57E-07 | 2.03E-03 |
| 2548699 | CYP1B1 | 1.38 | 8.61E-07 | 2.04E-03 |

(continued)

| Transcript cluster ID | Gene symbol | LogFC | P.Value | FDR adj.P.Val |
|---|---|---|---|---|
| 3393257 | BACE1 | 0.42 | 8.93E-07 | 2.12E-03 |
| 3808854 | TCF4 | -0.61 | 9.41E-07 | 2.23E-03 |
| 2708066 | KLHL6 | -1.33 | 9.47E-07 | 2.24E-03 |
| 3288518 | C10orf72 | 1.02 | 9.53E-07 | 2.26E-03 |
| 3321512 | PDE3B | -1.15 | 9.55E-07 | 2.26E-03 |
| 3707335 | GP1BA | -0.54 | 9.56E-07 | 2.26E-03 |
| 2740067 | ANK2 | -0.78 | 9.60E-07 | 2.27E-03 |
| 2688717 | BTLA | -1.60 | 9.65E-07 | 2.28E-03 |
| 3271687 | PPP2R2D | -0.28 | 9.67E-07 | 2.29E-03 |
| 2523689 | ABI2 | 0.53 | 9.81E-07 | 2.32E-03 |
| 3202528 | LINGO2 | -0.68 | 9.85E-07 | 2.33E-03 |
| 2611211 | MKRN2 | -0.25 | 9.95E-07 | 2.35E-03 |
| 3157147 | LYNX1 | 0.32 | 9.96E-07 | 2.35E-03 |
| 2796951 | PDLIM3 | -0.63 | 1.00E-06 | 2.37E-03 |
| 3057370 | HIP1 | 0.50 | 1.01E-06 | 2.39E-03 |
| 3392332 | CADM1,LOC100132764 | 0.98 | 1.02E-06 | 2.40E-03 |
| 3927480 | ADAMTS5 | -0.83 | 1.02E-06 | 2.41E-03 |
| 3743393 | DLG4 | 0.41 | 1.02E-06 | 2.41E-03 |
| 4000155 | GPM6B | -0.37 | 1.04E-06 | 2.45E-03 |
| 3599709 | GLCE | 0.52 | 1.06E-06 | 2.49E-03 |
| 2820394 | NR2F1 | 0.32 | 1.06E-06 | 2.50E-03 |
| 3740664 | C17orf91 | -0.43 | 1.08E-06 | 2.54E-03 |
| 4004878 | RPGR | -0.38 | 1.08E-06 | 2.54E-03 |
| 3533499 | CTAGE5 | -0.27 | 1.12E-06 | 2.63E-03 |
| 3986261 | RNF128 | 1.22 | 1.13E-06 | 2.65E-03 |

(continued)

| Transcript cluster ID | Gene symbol | LogFC | P.Value | FDR adj.P.Val |
|---|---|---|---|---|
| 2891241 | DUSP22 | -0.38 | 1.13E-06 | 2.66E-03 |
| 3060450 | MGC26647 | -1.33 | 1.17E-06 | 2.74E-03 |
| 2439101 | FCRL1 | -0.80 | 1.17E-06 | 2.75E-03 |
| 3004665 | ZNF138 | -0.37 | 1.18E-06 | 2.78E-03 |
| 3384718 | DLG2,SOCS6 | -0.84 | 1.25E-06 | 2.94E-03 |
| 3954879 | VPREB3 | -1.52 | 1.29E-06 | 3.02E-03 |
| 3360401 | HBB | -1.01 | 1.30E-06 | 3.04E-03 |
| 3013565 | DYNC1I1 | -0.41 | 1.31E-06 | 3.06E-03 |
| 2905169 | CDKN1A | 0.69 | 1.32E-06 | 3.08E-03 |
| 3920003 | CHAF1B | 0.51 | 1.32E-06 | 3.09E-03 |
| 2339139 | INADL | 0.55 | 1.33E-06 | 3.10E-03 |
| 3982560 | P2RY10 | -1.29 | 1.33E-06 | 3.11E-03 |
| 2602653 | PID1 | -0.73 | 1.34E-06 | 3.13E-03 |
| 3982410 | COX7B | -0.37 | 1.36E-06 | 3.18E-03 |
| 2823880 | CAMK4 | -1.32 | 1.36E-06 | 3.19E-03 |
| 3756046 | NR1D1,THRA | 0.47 | 1.41E-06 | 3.28E-03 |
| 2737596 | BANK1 | -1.02 | 1.44E-06 | 3.35E-03 |
| 3538893 | PRKCH | -0.75 | 1.44E-06 | 3.36E-03 |
| 3634852 | LOC145899,RASGRF1 | 0.48 | 1.47E-06 | 3.42E-03 |
| 3025545 | CALD1 | 0.81 | 1.48E-06 | 3.44E-03 |
| 2378710 | C1orf97 | -0.43 | 1.49E-06 | 3.48E-03 |
| 3598662 | MAP2K1 | -0.37 | 1.50E-06 | 3.50E-03 |
| 2427619 | KCNA3 | -1.34 | 1.51E-06 | 3.52E-03 |
| 3960061 | RAC2 | -1.10 | 1.54E-06 | 3.59E-03 |
| 2386828 | EDARADD,ENO1,ENO1P | -0.50 | 1.55E-06 | 3.60E-03 |

(continued)

| Transcript cluster ID | Gene symbol | LogFC | P.Value | FDR adj.P.Val |
|---|---|---|---|---|
| 2748605 | LRAT | 0.70 | 1.58E-06 | 3.66E-03 |
| 3256689 | PTEN | -0.32 | 1.58E-06 | 3.68E-03 |
| 2319340 | LOC642740,SLC25A33 | -0.87 | 1.60E-06 | 3.71E-03 |
| 3529951 | KIAA1305 | 0.62 | 1.61E-06 | 3.73E-03 |
| 2880051 | PPP2R2B | -0.43 | 1.67E-06 | 3.86E-03 |
| 2515183 | C2orf37 | -0.40 | 1.69E-06 | 3.92E-03 |
| 2922840 | KPNA5 | -0.45 | 1.69E-06 | 3.92E-03 |
| 3497270 | DNAJC3,LOC144871 | -0.45 | 1.69E-06 | 3.92E-03 |
| 3216969 | XPA | -0.36 | 1.70E-06 | 3.95E-03 |
| 3960174 | LGALS2 | -1.38 | 1.71E-06 | 3.95E-03 |
| 2364677 | LOC100131938,PBX1 | 0.95 | 1.73E-06 | 4.00E-03 |
| 3788220 | ME2 | -0.51 | 1.74E-06 | 4.02E-03 |
| 3217167 | CORO2A | 0.36 | 1.76E-06 | 4.07E-03 |
| 3033209 | INSIG1 | -0.46 | 1.76E-06 | 4.08E-03 |
| 3846076 | TLE2 | 0.33 | 1.78E-06 | 4.11E-03 |
| 3655109 | CD19 | -0.47 | 1.84E-06 | 4.24E-03 |
| 3323413 | HTATIP2 hCG_1984468,LOC388524,RPSA,RPSAP1 | -0.50 | 1.89E-06 | 4.37E-03 |
| 3827218 | 5 | 0.91 | 1.92E-06 | 4.43E-03 |
| 2597867 | IKZF2 | 0.57 | 1.93E-06 | 4.45E-03 |
| 2671936 | SLC6A20 | 0.40 | 1.94E-06 | 4.48E-03 |
| 3892974 | COL9A3 | -0.62 | 1.98E-06 | 4.56E-03 |
| 3407849 | C12orf39 | -0.64 | 1.99E-06 | 4.58E-03 |
| 3156307 | PTK2 | 0.51 | 2.02E-06 | 4.65E-03 |
| 4011989 | CXCR3 | -0.46 | 2.03E-06 | 4.68E-03 |
| 3861948 | GMFG | -1.05 | 2.04E-06 | 4.68E-03 |

(continued)

| Transcript cluster ID | Gene symbol | LogFC | P.Value | FDR adj.P.Val |
|---|---|---|---|---|
| 2946194 | HIST1H1A | 0.93 | 2.05E-06 | 4.71E-03 |
| 2348792 | CCDC76 | -0.33 | 2.09E-06 | 4.79E-03 |
| 3079005 | RARRES2 | 0.55 | 2.13E-06 | 4.88E-03 |
| 3572209 | PGF | -0.64 | 2.14E-06 | 4.92E-03 |
| 2625907 | FLNB | 0.46 | 2.15E-06 | 4.93E-03 |
| 3246888 | PRKG1 | -0.85 | 2.15E-06 | 4.94E-03 |
| 2999485 | STK17A | -0.87 | 2.19E-06 | 5.02E-03 |
| 4013460 | CYSLTR1 | -1.12 | 2.22E-06 | 5.10E-03 |
| 2729667 | STAP1 | -1.32 | 2.23E-06 | 5.11E-03 |
| 2902407 | LTA | -0.50 | 2.27E-06 | 5.18E-03 |
| 2701049 | GPR87 | 0.33 | 2.32E-06 | 5.30E-03 |
| 3442785 | CLEC4C | -0.52 | 2.34E-06 | 5.36E-03 |
| 3230397 | LCN10,LCN6 | 0.48 | 2.39E-06 | 5.47E-03 |
| 2474568 | KRTCAP3 | 0.49 | 2.43E-06 | 5.56E-03 |
| 3443464 | PZP | -0.36 | 2.47E-06 | 5.63E-03 |
| 2484552 | AHSA2 | -0.49 | 2.49E-06 | 5.68E-03 |
| 3691326 | SALL1 | 0.78 | 2.49E-06 | 5.68E-03 |
| 3839910 | FPR2 | -1.40 | 2.49E-06 | 5.68E-03 |
| 3369931 | RAG2 | -1.44 | 2.55E-06 | 5.82E-03 |
| 3360417 | HBB,HBD | -0.94 | 2.60E-06 | 5.92E-03 |
| 3010503 | CD36 | -1.44 | 2.62E-06 | 5.97E-03 |
| 3263555 | ADD3 | -0.48 | 2.63E-06 | 5.99E-03 |
| 4011889 | ZMYM3 | 0.27 | 2.64E-06 | 6.02E-03 |
| 2748830 | GUCY1A3 | 0.73 | 2.65E-06 | 6.04E-03 |
| 3356115 | APLP2 | 0.44 | 2.71E-06 | 6.15E-03 |

(continued)

| Transcript cluster ID | Gene symbol | LogFC | P.Value | FDR adj.P.Val |
|---|---|---|---|---|
| 3382861 | PAK1 | -0.38 | 2.77E-06 | 6.30E-03 |
| 3649890 | ABCC1 | -0.38 | 2.78E-06 | 6.31E-03 |
| 3862661 | BLVRB | -0.78 | 2.81E-06 | 6.37E-03 |
| 3884324 | CTNNBL1 | -0.42 | 2.83E-06 | 6.43E-03 |
| 3417201 | IKZF4 | 0.30 | 2.84E-06 | 6.44E-03 |
| 2978026 | FBXO30 | -0.46 | 2.84E-06 | 6.45E-03 |
| 2822407 | HISPPD1 | -0.42 | 2.85E-06 | 6.45E-03 |
| 2461037 | PCNXL2 | 0.36 | 2.95E-06 | 6.70E-03 |
| 2400322 | HP1BP3 | -0.25 | 3.02E-06 | 6.83E-03 |
| 2554975 | BCL11A | -0.93 | 3.23E-06 | 7.31E-03 |
| 3388673 | MMP7 | 1.39 | 3.31E-06 | 7.50E-03 |
| 3633794 | ETFA,TYRO3,TYRO3P | -0.40 | 3.34E-06 | 7.57E-03 |
| 3824993 | GDF15 | 0.78 | 3.39E-06 | 7.67E-03 |
| 3343202 | EED | -0.43 | 3.49E-06 | 7.88E-03 |
| 3572235 | MLH3 | -0.32 | 3.54E-06 | 8.00E-03 |

| Transcript cluster ID | Gene symbol | LogFC | P.Value | FDR adj.P.Val |
|---|---|---|---|---|
| 3256192 | C10orf116,KIAA1975 | 0.65 | 3.57E-06 | 8.05E-03 |
| 3329649 | DDB2 | 0.32 | 3.60E-06 | 8.12E-03 |
| 3057955 | FGL2 | -1.07 | 3.60E-06 | 8.12E-03 |
| 2766289 | TMEM156 | -1.15 | 3.60E-06 | 8.12E-03 |
| 2779335 | RG9MTD2 | -0.41 | 3.61E-06 | 8.14E-03 |
| 3982689 | TBX22 | -0.69 | 3.61E-06 | 8.14E-03 |
| 2468622 | ID2 | 0.48 | 3.66E-06 | 8.24E-03 |
| 3913483 | TCFL5 | -0.54 | 3.70E-06 | 8.32E-03 |
| 3665722 | PARD6A | -0.37 | 3.70E-06 | 8.32E-03 |

(continued)

| Transcript cluster ID | Gene symbol | LogFC | P.Value | FDR adj.P.Val |
|---|---|---|---|---|
| 2633256 | ST3GAL6 | -0.72 | 3.75E-06 | 8.44E-03 |
| 3415320 | KRT7 | 1.11 | 3.76E-06 | 8.46E-03 |
| 2842561 | HIGD2A | -0.58 | 3.77E-06 | 8.47E-03 |
| 2932508 | TIAM2 | -0.51 | 3.80E-06 | 8.53E-03 |
| 3844470 | PPAP2C | 0.37 | 3.83E-06 | 8.60E-03 |
| 2451043 | LMOD1 | -0.41 | 3.83E-06 | 8.60E-03 |
| 3888721 | PTPN1 | -0.44 | 3.92E-06 | 8.78E-03 |
| 2518272 | ITGA4 | -1.14 | 3.92E-06 | 8.80E-03 |
| 2913694 | CD109 LOC100134294,PRSS1,PRSS2,PRSS3,TR | 1.11 | 3.93E-06 | 8.80E-03 |
| 3028744 | Y6 | 1.06 | 3.95E-06 | 8.85E-03 |
| 2586227 | FASTKD1 | -0.41 | 3.99E-06 | 8.93E-03 |
| 3662774 | GPR114 | -0.39 | 4.00E-06 | 8.96E-03 |
| 2713382 | BDH1 | -0.39 | 4.02E-06 | 8.99E-03 |
| 3558418 | STXBP6 | 0.93 | 4.05E-06 | 9.06E-03 |
| 3600212 | LRRC49 | 0.52 | 4.11E-06 | 9.18E-03 |
| 3635198 | BCL2A1 | -1.21 | 4.11E-06 | 9.18E-03 |
| 3634811 | CTSH | 0.88 | 4.12E-06 | 9.19E-03 |
| 3730322 | MRC2 | 0.35 | 4.14E-06 | 9.24E-03 |
| 3753568 | SLFN13 | 0.52 | 4.15E-06 | 9.26E-03 |
| 3462693 | KRR1 | -0.30 | 4.17E-06 | 9.29E-03 |
| 2648677 | MME | -1.39 | 4.17E-06 | 9.29E-03 |
| 2778856 | TSPAN5 | -0.97 | 4.17E-06 | 9.29E-03 |
| 3421118 | hCG_1757335,RAP1B | -0.49 | 4.18E-06 | 9.31E-03 |
| 3138464 | PDE7A | -0.68 | 4.29E-06 | 9.55E-03 |
| 3046681 | TRGV3 | -0.88 | 4.30E-06 | 9.57E-03 |

(continued)

| Transcript cluster ID | Gene symbol | LogFC | P.Value | FDR adj.P.Val |
|---|---|---|---|---|
| 2606643 | MYEOV2 | -0.27 | 4.35E-06 | 9.67E-03 |
| 3079722 | CRYGN | 0.49 | 4.35E-06 | 9.68E-03 |
| 2347132 | FNBP1L | 0.88 | 4.42E-06 | 9.82E-03 |
| 3860229 | CLIP3 | 0.33 | 4.46E-06 | 9.90E-03 |
| 2840002 | CCDC99 | -0.68 | 4.62E-06 | 1.03E-02 |
| 3784208 | DTNA | 0.61 | 4.71E-06 | 1.04E-02 |
| 3642654 | HBM | -0.90 | 4.76E-06 | 1.06E-02 |
| 4041923 | CCNL2 | -0.30 | 4.80E-06 | 1.06E-02 |
| 3282601 | MPP7 | 0.63 | 4.83E-06 | 1.07E-02 |
| 3336906 | SSH3 | 0.25 | 4.83E-06 | 1.07E-02 |
| 3130211 | PPP2CB | 0.52 | 4.83E-06 | 1.07E-02 |
| 3204680 | SIT1 | -1.00 | 4.88E-06 | 1.08E-02 |
| 3560527 | C14orf147 | 0.39 | 4.91E-06 | 1.09E-02 |
| 2688499 | ZBED2 | 0.96 | 4.93E-06 | 1.09E-02 |
| 3039791 | AGR2 | 1.25 | 5.00E-06 | 1.10E-02 |
| 2363852 | FCRLA | -0.86 | 5.00E-06 | 1.10E-02 |
| 3790361 | ZNF532 | 0.37 | 5.12E-06 | 1.13E-02 |
| 2417390 | CTBP2,GPR177 | 0.61 | 5.13E-06 | 1.13E-02 |
| 3677969 | SRL | 0.54 | 5.47E-06 | 1.21E-02 |
| 3070712 | WASL | 0.46 | 5.58E-06 | 1.23E-02 |
| 2726542 | FLJ21511 | -1.22 | 5.64E-06 | 1.24E-02 |
| 3609592 | MCTP2 | 0.63 | 5.68E-06 | 1.25E-02 |
| 2428796 | PTPN22 | -0.91 | 5.78E-06 | 1.27E-02 |
| 2696379 | ANAPC13 | -0.38 | 5.83E-06 | 1.28E-02 |
| 2730714 | DCK | -0.67 | 5.92E-06 | 1.30E-02 |

(continued)

| Transcript cluster ID | Gene symbol | LogFC | P.Value | FDR adj.P.Val |
|---|---|---|---|---|
| 2350840 | GNAI3 | -0.40 | 6.11E-06 | 1.34E-02 |
| 3742627 | C17orf87 | -1.23 | 6.28E-06 | 1.38E-02 |
| 3795942 | YES1 | 0.65 | 6.33E-06 | 1.39E-02 |
| 3205293 | PAX5 | -0.99 | 6.36E-06 | 1.40E-02 |
| 3167511 | GALT | -0.23 | 6.60E-06 | 1.45E-02 |
| 2801526 | CCT5 | -0.31 | 6.61E-06 | 1.45E-02 |
| 3465409 | BTG1,LOC256021 | -0.66 | 6.79E-06 | 1.49E-02 |
| 3729419 | CA4 | -0.44 | 6.83E-06 | 1.50E-02 |
| 2319550 | RBP7 | -0.83 | 6.84E-06 | 1.50E-02 |
| 2412668 | TXNDC12 | -0.37 | 6.88E-06 | 1.51E-02 |
| 2937144 | SMOC2 | -1.08 | 6.93E-06 | 1.52E-02 |
| 2835440 | TCOF1 | -0.27 | 7.01E-06 | 1.53E-02 |
| 2422035 | GBPS | -1.14 | 7.11E-06 | 1.55E-02 |
| 3380065 | FLJ42258 | 0.32 | 7.22E-06 | 1.58E-02 |
| 3452970 | SENP1 | -0.36 | 7.22E-06 | 1.58E-02 |
| 3105600 | CA2 | -0.91 | 7.26E-06 | 1.58E-02 |
| 3353867 | OR10G4,OR10G7,OR10G8,OR10G9 | 0.35 | 7.31E-06 | 1.60E-02 |
| 3452865 | COL2A1 | -0.25 | 7.33E-06 | 1.60E-02 |
| 2375795 | LAX1 | -0.91 | 7.43E-06 | 1.62E-02 |
| 2583374 | PLA2R1 | -0.98 | 7.44E-06 | 1.62E-02 |
| 2702307 | CCNL1 | -0.37 | 7.45E-06 | 1.62E-02 |
| 3852832 | EMR3 | -1.22 | 7.56E-06 | 1.65E-02 |
| 3018696 | DLD | -0.35 | 7.69E-06 | 1.67E-02 |
| 2750627 | CPE | 1.28 | 7.73E-06 | 1.68E-02 |
| 2390050 | NLRP3 | -0.52 | 7.82E-06 | 1.70E-02 |

(continued)

| Transcript cluster ID | Gene symbol | LogFC | P.Value | FDR adj.P.Val |
|---|---|---|---|---|
| 2378662 | TRAF5 | -0.87 | 7.87E-06 | 1.71E-02 |
| 2351572 | CD53 | -1.15 | 7.92E-06 | 1.72E-02 |
| 2699564 | PLOD2 | 1.05 | 7.92E-06 | 1.72E-02 |
| 2353669 | CD2,LOC100128308 | -1.19 | 7.94E-06 | 1.72E-02 |
| 3075778 | HIPK2 | 0.34 | 8.04E-06 | 1.74E-02 |
| 3225398 | HSPA5 | -0.38 | 8.04E-06 | 1.74E-02 |
| 3741547 | P2RX5 | -0.67 | 8.09E-06 | 1.75E-02 |
| 3929931 | ATPSO,LOC440258 | -0.26 | 8.10E-06 | 1.76E-02 |
| 2914693 | SH3BGRL2 | 0.87 | 8.15E-06 | 1.76E-02 |
| 3447022 | ST8SIA1 | -0.72 | 8.21E-06 | 1.78E-02 |
| 4019486 | SEPT6 | -0.97 | 8.26E-06 | 1.79E-02 |
| 3813198 | FBXO15 | -0.51 | 8.29E-06 | 1.79E-02 |
| 3597338 | TPM1 | 0.45 | 8.29E-06 | 1.79E-02 |
| 3145980 | HRSP12 | -0.49 | 8.48E-06 | 1.83E-02 |
| 3415668 | TENC1 | 0.28 | 8.63E-06 | 1.87E-02 |
| 3861413 | MAP4K1 | -0.65 | 8.64E-06 | 1.87E-02 |
| 3079803 | PRKAG2 | -0.38 | 8.65E-06 | 1.87E-02 |
| 4045676 | S100A13 | 0.78 | 8.70E-06 | 1.88E-02 |
| 3113133 | COLEC10 | -0.59 | 8.94E-06 | 1.93E-02 |
| 4011844 | IL2RG,LOC158830 | -1.22 | 9.12E-06 | 1.96E-02 |
| 3733275 | KCNJ2 | 0.98 | 9.20E-06 | 1.98E-02 |
| 2876046 | PPP2CA | -0.24 | 9.50E-06 | 2.05E-02 |
| 3672489 | IRF8 | -1.08 | 9.85E-06 | 2.12E-02 |
| 2860178 | CD180 | -1.33 | 9.98E-06 | 2.15E-02 |
| 3456732 | ITGA5 | -0.58 | 1.02E-05 | 2.20E-02 |

(continued)

| Transcript cluster ID | Gene symbol | LogFC | P.Value | FDR adj.P.Val |
|---|---|---|---|---|
| 2822492 | C5orf30 | -0.40 | 1.02E-05 | 2.20E-02 |
| 3558012 | TINF2 | -0.31 | 1.04E-05 | 2.24E-02 |
| 3212919 | C9orf153,ISCA1,ISCA1L | -0.45 | 1.05E-05 | 2.25E-02 |
| 2878726 | HDAC3 | -0.27 | 1.06E-05 | 2.26E-02 |
| 3868768 | KLK6 | 0.37 | 1.07E-05 | 2.29E-02 |
| 2919669 | PRDM1 | 0.56 | 1.07E-05 | 2.30E-02 |
| 3701384 | C16orf61 | -0.46 | 1.08E-05 | 2.32E-02 |
| 3665288 | E2F4 | -0.39 | 1.11E-05 | 2.37E-02 |
| 3925473 | SAMSN1 | -1.04 | 1.11E-05 | 2.37E-02 |
| 3454892 | GALNT6 | -0.41 | 1.11E-05 | 2.38E-02 |
| 3175494 | GCNT1 | 0.45 | 1.13E-05 | 2.42E-02 |
| 3519309 | SPRY2 | 0.69 | 1.14E-05 | 2.44E-02 |
| 2673181 | PLXNB1 | 0.26 | 1.17E-05 | 2.50E-02 |
| 2440327 | SLAMF1 | -0.81 | 1.19E-05 | 2.54E-02 |
| 2638676 | EAF2 | -1.10 | 1.21E-05 | 2.58E-02 |
| 2704441 | EVI1,MDS1 | 0.70 | 1.21E-05 | 2.58E-02 |
| 3989089 | ZBTB33 | 0.27 | 1.21E-05 | 2.58E-02 |
| 3902552 | FOXS1 | -0.30 | 1.22E-05 | 2.61E-02 |
| 3632298 | ADPGK | -0.43 | 1.23E-05 | 2.62E-02 |
| 2912416 | BAI3 | 0.34 | 1.23E-05 | 2.62E-02 |
| 2907730 | SRF | -0.27 | 1.25E-05 | 2.67E-02 |
| 2985781 | THBS2 | 0.39 | 1.27E-05 | 2.71E-02 |
| 2475407 | CLIP4 | 0.53 | 1.35E-05 | 2.87E-02 |
| 2412312 | C1orf34 | 0.69 | 1.36E-05 | 2.89E-02 |
| 3834502 | CD79A | -1.34 | 1.36E-05 | 2.89E-02 |

| Transcript cluster ID | Gene symbol | LogFC | P.Value | FDR adj.P.Val |
|---|---|---|---|---|

(continued)

| Transcript cluster ID | Gene symbol | LogFC | P.Value | FDR adj.P.Val |
|---|---|---|---|---|
| 2351872 | RAP1A | -0.36 | 1.41E-05 | 3.00E-02 |
| 3099750 | SDCBP | -0.49 | 1.43E-05 | 3.03E-02 |
| 3945651 | APOBEC3F,APOBEC3G hCG_1998957,HLA-DQB1,HLA-DQB2,HLA-DRB1,HLA-DRB2,HLA-DRB3,HLA-DRB4,HLA-DRB5,LOC100133484,LOC100133583,LOC100133661,LOC100133811,LOC730415, | -0.70 | 1.44E-05 | 3.05E-02 |
| 2950125 | RNASE2,ZNF749 | 1.07 | 1.44E-05 | 3.06E-02 |
| 3250146 | SRGN | -0.88 | 1.45E-05 | 3.07E-02 |
| 3400236 | B4GALNT3 | 0.29 | 1.45E-05 | 3.08E-02 |
| 3731543 | RGS9 | -0.38 | 1.45E-05 | 3.08E-02 |
| 3797561 | LAMA1 | 0.43 | 1.46E-05 | 3.10E-02 |
| 2974671 | C6orf192 | -0.77 | 1.47E-05 | 3.11E-02 |
| 3905875 | MAFB | -0.64 | 1.47E-05 | 3.11E-02 |
| 2376548 | MFSD4 | 0.32 | 1.53E-05 | 3.22E-02 |
| 2687739 | CD47,LOC151657 | 0.29 | 1.53E-05 | 3.24E-02 |
| 2708922 | IGF2BP2 | 0.72 | 1.54E-05 | 3.25E-02 |
| 3107828 | PLEKHF2 | -0.65 | 1.56E-05 | 3.30E-02 |
| 3674848 | RHBDF1 | 0.28 | 1.60E-05 | 3.37E-02 |
| 4015884 | ARMCX2 | 0.53 | 1.60E-05 | 3.37E-02 |
| 3086206 | FDFT1 | -0.34 | 1.60E-05 | 3.37E-02 |
| 2701071 | P2RY13 | -1.17 | 1.60E-05 | 3.37E-02 |
| 3512874 | LCP1 | -0.91 | 1.67E-05 | 3.51E-02 |
| 3046708 | TARP,TRGV3 | -1.22 | 1.68E-05 | 3.55E-02 |
| 3013054 | COL1A2 | 0.56 | 1.69E-05 | 3.56E-02 |
| 3390860 | POU2AF1 | -0.83 | 1.70E-05 | 3.57E-02 |
| 2549092 | SOS1 | -0.33 | 1.71E-05 | 3.59E-02 |
| 2480961 | TACSTD1 | 1.12 | 1.71E-05 | 3.60E-02 |

| Transcript cluster ID | Gene symbol | LogFC | P.Value | FDR adj.P.Val |
|---|---|---|---|---|
| 3259253 | ENTPD1,LOC100127889 | 0.69 | 1.74E-05 | 3.65E-02 |
| 3351675 | CXCR5 | -0.91 | 1.75E-05 | 3.67E-02 |
| 3513549 | RCBTB2 | -0.41 | 1.76E-05 | 3.70E-02 |
| 2739308 | EGF | -0.43 | 1.79E-05 | 3.76E-02 |
| 2665572 | SGOL1 | -0.76 | 1.80E-05 | 3.78E-02 |
| 2434178 | MTMR11 | 0.31 | 1.85E-05 | 3.87E-02 |
| 3089215 | BMP1 | 0.24 | 1.86E-05 | 3.90E-02 |
| 2608309 | LRRN1 | 1.05 | 1.88E-05 | 3.93E-02 |
| 2732942 | BMP2K | -0.61 | 1.96E-05 | 4.10E-02 |
| 3018309 | PIK3CG | -0.97 | 1.99E-05 | 4.15E-02 |
| 3336680 | RHOD | 0.36 | 1.99E-05 | 4.16E-02 |
| 3644810 | C16orf59 | -0.23 | 2.00E-05 | 4.17E-02 |
| 3450861 | ABCD2 | -0.85 | 2.06E-05 | 4.31E-02 |
| 3462094 | CCDC131 | -0.32 | 2.07E-05 | 4.33E-02 |
| 3213847 | SHC3 | 0.30 | 2.09E-05 | 4.35E-02 |
| 3745525 | LOC388335,MAGOH2 | -0.47 | 2.09E-05 | 4.35E-02 |
| 3759137 | ITGA2B | -0.43 | 2.13E-05 | 4.43E-02 |
| 2413519 | C1orf41 | -0.51 | 2.13E-05 | 4.44E-02 |
| 3848243 | INSR,LOC100128567,LOC100131165 | 0.40 | 2.14E-05 | 4.47E-02 |
| 2651835 | GPR160 | -0.56 | 2.16E-05 | 4.49E-02 |
| 3129588 | KIF13B | 0.29 | 2.17E-05 | 4.52E-02 |
| 3962145 | TNFRSF13C | -0.55 | 2.19E-05 | 4.55E-02 |
| 3759006 | SLC4A1 | -1.03 | 2.23E-05 | 4.64E-02 |
| 2878273 | HBEGF | 0.53 | 2.31E-05 | 4.80E-02 |
| 3467351 | ANKS1B | -0.51 | 2.36E-05 | 4.91E-02 |

| Transcript cluster ID | Gene symbol | LogFC | P.Value | FDR adj.P.Val |
|---|---|---|---|---|
| 3582745 | LOC90925 | -0.84 | 2.39E-05 | 4.95E-02 |
| 2739714 | C4orf32 | -0.26 | 2.46E-05 | 5.10E-02 |
| 2486811 | PLEK | -1.19 | 2.51E-05 | 5.19E-02 |
| 3205586 | EXOSC3,SHB | -0.41 | 2.52E-05 | 5.23E-02 |
| 2434746 | FAM63A | -0.43 | 2.56E-05 | 5.30E-02 |
| 3018605 | SLC26A4 | -1.40 | 2.56E-05 | 5.30E-02 |
| 2806468 | IL7R | -1.46 | 2.61E-05 | 5.39E-02 |
| 3642687 | HBQ1 | -0.50 | 2.64E-05 | 5.46E-02 |
| 3657193 | TGFB1I1 | 0.26 | 2.66E-05 | 5.50E-02 |
| 3114832 | SQLE | -0.50 | 2.68E-05 | 5.54E-02 |
| 2334602 | TSPAN1 | 1.23 | 2.72E-05 | 5.61E-02 |
| 3404436 | CLEC2D,NPM1 | -1.05 | 2.78E-05 | 5.74E-02 |
| 3713794 | EPN2,LOC100128851 | 0.24 | 2.80E-05 | 5.79E-02 |
| 3921933 | BACE2 | 0.49 | 2.81E-05 | 5.79E-02 |
| 3161167 | KIAA1432 | -0.38 | 2.85E-05 | 5.87E-02 |
| 3197955 | GLDC | -0.84 | 2.89E-05 | 5.96E-02 |
| 3840164 | ZNF610 | 0.34 | 2.91E-05 | 6.00E-02 |
| 3140478 | RPESP | -0.39 | 3.00E-05 | 6.18E-02 |
| 2542795 | SDC1 | 0.49 | 3.02E-05 | 6.21E-02 |
| 3590014 | CASC5 | -0.57 | 3.07E-05 | 6.31E-02 |
| 3290746 | LOC100129721,SLC16A9 | 1.00 | 3.10E-05 | 6.36E-02 |
| 3447863 | KRAS | -0.27 | 3.12E-05 | 6.40E-02 |
| 3937787 | CRKL | -0.25 | 3.18E-05 | 6.53E-02 |
| 3227696 | RAPGEF1 | -0.49 | 3.24E-05 | 6.64E-02 |
| 2455933 | ESRRG | -0.52 | 3.26E-05 | 6.69E-02 |

(continued)

| Transcript cluster ID | Gene symbol | LogFC | P.Value | FDR adj.P.Val |
|---|---|---|---|---|
| 3439256 | RPS11 | -0.32 | 3.26E-05 | 6.69E-02 |
| 3685051 | USP31 | 0.27 | 3.27E-05 | 6.71E-02 |
| 3834046 | AXL | 0.48 | 3.41E-05 | 6.98E-02 |
| 3333942 | RTN3 | -0.27 | 3.42E-05 | 6.99E-02 |
| 3294959 | C10orf55 | 0.39 | 3.43E-05 | 7.01E-02 |
| 3221135 | C9orf80 | -0.29 | 3.50E-05 | 7.16E-02 |
| 3847112 | PTPRS | 0.36 | 3.51E-05 | 7.18E-02 |
| 3105777 | WWP1 | -0.40 | 3.54E-05 | 7.24E-02 |
| 2955638 | CLIC5 | 0.91 | 3.56E-05 | 7.27E-02 |
| 3064689 | MYLC2PL | -0.23 | 3.58E-05 | 7.31E-02 |
| 3161261 | MLANA | -0.36 | 3.65E-05 | 7.44E-02 |
| 2638988 | PARP15 | -0.70 | 3.65E-05 | 7.45E-02 |
| 3864646 | KCNN4 | 0.27 | 3.66E-05 | 7.46E-02 |
| 2566848 | AFF3 | -0.59 | 3.69E-05 | 7.51E-02 |
| 3952762 | CLDN5 | -0.23 | 3.74E-05 | 7.61E-02 |
| 3747324 | SFRS6,ZNF624 | -0.27 | 3.76E-05 | 7.65E-02 |
| 2603960 | KCNJ13 | -0.53 | 3.80E-05 | 7.74E-02 |
| 3839346 | SPIB | -0.74 | 3.95E-05 | 8.03E-02 |
| 2341387 | LRRC7 | -0.42 | 3.96E-05 | 8.04E-02 |
| 3019793 | FOXP2 | -0.43 | 4.01E-05 | 8.14E-02 |
| 3947863 | PARVB | -0.43 | 4.02E-05 | 8.16E-02 |
| 3657219 | SLC5A2 | -0.21 | 4.08E-05 | 8.27E-02 |
| 3225855 | ANGPTL2 | 0.35 | 4.09E-05 | 8.29E-02 |
| 3770029 | CDC42EP4 | 0.24 | 4.13E-05 | 8.37E-02 |
| 3204744 | TLN1 | -0.37 | 4.17E-05 | 8.45E-02 |

(continued)

| Transcript cluster ID | Gene symbol | LogFC | P.Value | FDR adj.P.Val |
|---|---|---|---|---|
| 3569374 | VTI1B | -0.27 | 4.23E-05 | 8.58E-02 |
| 3803120 | B4GALT6 | 0.54 | 4.26E-05 | 8.63E-02 |
| 4008011 | FOXP3 | -0.25 | 4.27E-05 | 8.64E-02 |
| 3761054 | COPZ2 | -0.69 | 4.28E-05 | 8.66E-02 |
| 2883609 | CLINT1,LOC100131045 | -0.25 | 4.30E-05 | 8.69E-02 |
| 2473571 | RAB10 | -0.29 | 4.31E-05 | 8.70E-02 |
| 3591365 | ADAL | -0.48 | 4.33E-05 | 8.74E-02 |
| 3560617 | RPS19,SNX6 | -0.38 | 4.35E-05 | 8.77E-02 |
| 3870990 | GP6 | -0.32 | 4.40E-05 | 8.87E-02 |
| 3345940 | CNTN5 | -0.43 | 4.41E-05 | 8.89E-02 |
| 2401493 | ID3 | -0.65 | 4.42E-05 | 8.92E-02 |
| 3416577 | NCKAP1L | -0.99 | 4.43E-05 | 8.92E-02 |
| 3822049 | CALR | -0.28 | 4.43E-05 | 8.93E-02 |
| 3598959 | SMAD3 | 0.35 | 4.44E-05 | 8.93E-02 |
| 3682028 | MYH11 | -0.27 | 4.50E-05 | 9.05E-02 |
| 3614534 | GABRB3 | -0.74 | 4.51E-05 | 9.06E-02 |
| 2445982 | ANGPTL1 | -0.71 | 4.59E-05 | 9.23E-02 |
| 3126504 | CSGALNACT1 | -0.63 | 4.62E-05 | 9.28E-02 |
| 3089192 | SFTPC | -0.26 | 4.62E-05 | 9.28E-02 |
| 3518455 | FBXL3 | -0.26 | 4.66E-05 | 9.34E-02 |
| 3169331 | ALDH1B1 | -0.60 | 4.67E-05 | 9.36E-02 |
| 3456805 | GTSF1 | -1.00 | 4.67E-05 | 9.36E-02 |
| 3306984 | GPAM | -0.69 | 4.69E-05 | 9.38E-02 |
| 3894228 | CSNK2A1,CSNK2A1P | -0.30 | 4.70E-05 | 9.41E-02 |
| 3955940 | CRYBB1 | -0.35 | 4.77E-05 | 9.55E-02 |

(continued)

| Transcript cluster ID | Gene symbol | LogFC | P.Value | FDR adj.P.Val |
|---|---|---|---|---|
| 3200982 | MLLT3 | -0.47 | 4.98E-05 | 9.96E-02 |
| 3824963 | PGPEP1 | 0.30 | 4.98E-05 | 9.96E-02 |
| 3601229 | CD276 | 0.59 | 5.00E-05 | 9.98E-02 |

**Example 4: Detection of Blood Contamination**

In this example, a system to detect expression levels as contributed by blood contaminants is developed. In some cases this is referred to as the "blood statistic." In one case, the blood statistic may reflect expression values of various genes known from the literature to be detectable in red blood cells. In one version of the blood statistic, 6 molecular markers (Affymetrix Exon/Afirma Transcript Cluster IDs, Table 11) are selected. Expression values of these markers are averaged to produce a 1-dimensional statistic characterizing a sample.

**Table 11. List of blood statistic markers**

| TCID | GENE Symbol | Description |
|---|---|---|
| 3360401 | HBB | hemoglobin, beta |
| 3360417 | HBB | hemoglobin, beta |
| 3360456 | HBG2 | hemoglobin, gamma G |
| 3642654 | HBM | hemoglobin, mu |
| 3642687 | HBQ1 | hemoglobin, theta 1 |
| 3642643 | HBZ | hemoglobin, zeta |

As an alternative to using the literature markers of red blood cells as in Table 11, a data-driven approach is also used to define a marker set sensitive to contamination of thyroid samples with whole blood. This marker set is identified by comparing expression levels in fresh blood samples with that in thyroid tissue samples. Specifically, differential expression analysis between these two sample types are carried out using LIMMA methodology. Top markers identified in this analysis are then checked for sensitivity to histopathological subtypes of thyroid malignancies and filtered down to a small set used subsequently to characterize unknown blood proportion in the test samples.

Specifically, the method comprised steps to:

1. Compare pure blood samples with tissue controls and analyze markers showing differential gene expression between these two sample types by LIMMA;
2. Identify markers that show consistently high expression in blood samples and no expression in surgical thyroid tissues (LIMMA)
3. Verify in the large thyroid tissue data set that these markers are not active across the entire universe of thyroid malignancies
4. Use top up-regulated blood markers to estimate proportion of blood in each sample.

In some cases down-regulated markers may be associated with a lack of thyroid follicular cells. In some cases, lowered expression of these markers may not be directly used to estimate blood proportion.

In some cases, up-regulated markers may be saturated at high blood levels, and this may result in under-estimation of blood at high blood proportions.

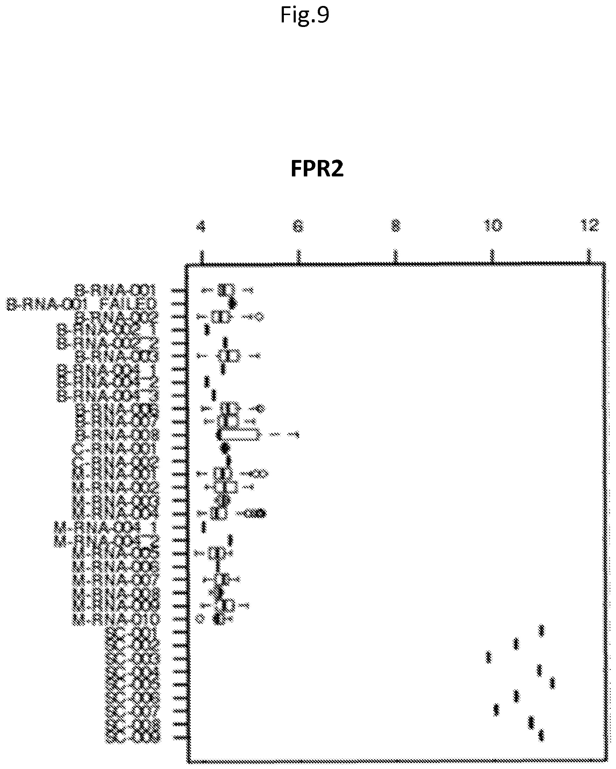

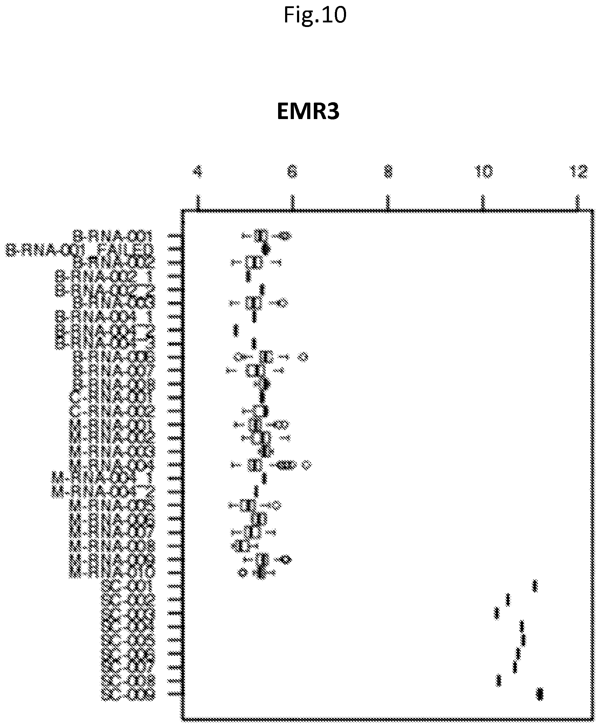

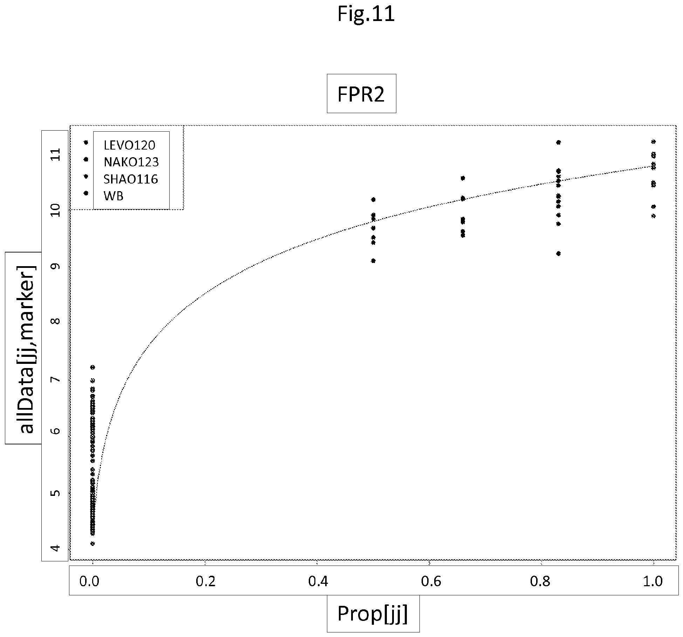

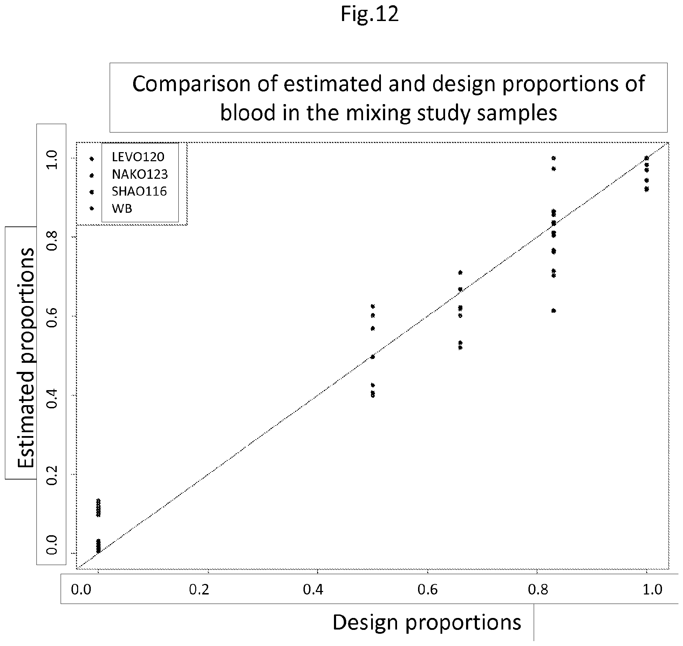

Some of the top markers identified using this approach (Table 12) are known to be exclusively expressed in blood from the literature (Su et al 2004). To further confirm this, expression levels of these markers are evaluated using a Gene Atlas tissue-specific expression data set. The expression levels in thyroid tissue and whole blood for representative markers are shown in Figures 8-10. In vitro mixtures of whole blood samples mixed with thyroid samples correlate well with the predicted expression derived from pure blood and pure thyroid tissue samples using standard in silico modeling (Figures 11 and 12).

**Table 12. List of heme statistic markers (version 2)**

| TCID | GENE Symbol | Description |
|---|---|---|
| 3759006 | SLC4A1 | solute carrier family 4, anion exchanger, member 1 (erythrocyte membrane protein band 3, Diego blood group) |
| 3839910 | FPR2 | formyl peptide receptor 2 |
| 3852832 | EMR3 | egf-like module containing, mucin-like, hormone receptor-like 3 |
| 2327677 | EPB41 | erythrocyte membrane protein band 4.1 (elliptocytosis 1, RH-linked) |
| 3564210 | PYGL | phosphorylase, glycogen, liver |
| 3976766 | WAS | Wiskott-Aldrich syndrome (eczema-thrombocytopenia) |
| 3360417 | HBB | hemoglobin, beta |

Having identified these markers, expression levels of these markers are used in thyroid tissue samples and fresh blood samples to estimate the mixing proportion of whole blood in the sample of interest in the following manner:

1. Find the proportion of blood that results in the smallest error between observed expression and predicted expression, based on linear interpolation in raw intensity space between thyroid tissue expression (used as a surrogate for pure thyroid sample with no blood contamination) and fresh whole blood expression.
2. Let $Y_{i,mTH}$ denote the median expression of marker $i$ within thyroid tissue, and let $Y_{i,mWB}$ denote the median expression of marker $i$ in whole fresh blood sample.
3. Then the proportion of whole blood cells $\alpha$ in some test sample $Y$ can be found as the one minimizing total error between observed and expected intensity values for these markers:

$$\alpha = \arg\min \sum_{i=1}^{Nm} (\log2(\alpha \times 2^{Y_{i,mWB}} + (1-\alpha) \times 2^{Y_{i,mTH}}) - Y_i)^{\wedge}2$$

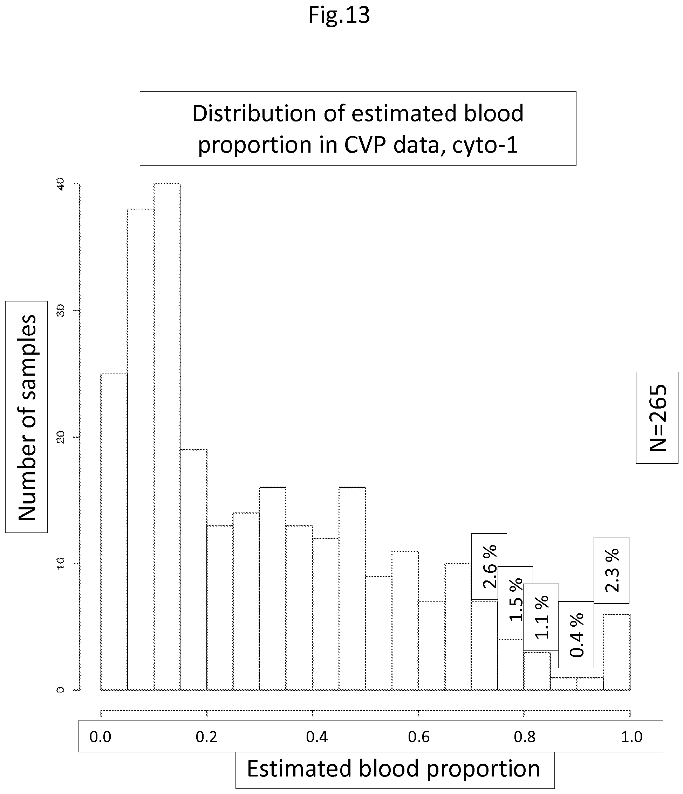

The median expression value for markers of interest in pure whole blood and thyroid tissue samples are provided in Table 3. Applying this methodology to estimate the mixture proportion in known mixtures, empirical estimates are compared with the design proportions in the in-vitro mixing experiments. The results of this comparison are shown in Figure 13. While there is good correlation, estimates may be unreliable at the high end due to saturation of blood-specific markers on the array. In addition, this analysis (as well as the analysis carried out using the blood statistic as previously described) indicates that some of the original FNA samples used for in-vitro mixing experiments contain some non-negligible blood proportion prior to mixing with blood [ green data points at zero blood proportion by design].

**Table 13: Median expression values of Blood Stat markers in pure whole blood and thyroid tissue samples.**

| Transcription cluster | GENE Symbol | Median intensity, whole blood samples, $Y_{i,mWB}$ | Median intensity, thyroid tissue samples, $Y_{i,mTH}$ |
|---|---|---|---|
| 3759006 | SLC4A1 | 10.650715 | 6.304225 |
| 3839910 | FPR2 | 10.78089 | 4.41854 |
| 3852832 | EMR3 | 10.74911 | 5.25297 |
| 2327677 | EPB41 | 12.044105 | 7.775005 |
| 3564210 | PYGL | 11.02537 | 7.52884 |
| 3976766 | WAS | 9.72518 | 5.99617 |
| 3360417 | HBB | 8.991625 | 5.07795 |

**Example 5: Detection of Follicular Content**

In this example, a system to detect expression levels as contributed by follicular cells are developed to detect the amount of follicular tissue content in a sample. In some cases this is referred to as the "follicular statistic." In this example, the follicular statistic consists of a set of 10 molecular markers (Affymetrix Exon/Afirma Transcript Cluster IDs) developed to assist in estimating the amount of follicular content present in a given FNA. These are derived following a two-step procedure whereby the first step consisted of using a large list of follicular markers published in scientific literature, examining their differential gene expression within a highly curated thyroid FNA sample cohort and choosing only those that changed the least between all thyroid histopathology subtypes (PTC, FC, BFN, etc). This seed marker list is shown in the Table 14. In some cases, some of these markers may show saturation on the microarray, even when follicular cells are present in very low quantity. Thus, these markers may not accurately track the follicular content within a mixture (for example, TG). Others may be affected by malignant cell transformation (upregulated) and have altered expression levels in the malignant state (such as TPO), while being non-informative in benign samples. Thus examination of expression patterns within a large and highly curated thyroid tissue sample cohort is conducted. Only those markers showing stable, unsaturated expression, across all thyroid histopathology subtypes (PTC, FC, BFN, etc) are retained for further evaluation. Expression level changes are evaluated using a LIMMA approach.

The next step in the process used a seed gene list (Table 14) as a "fishing pole" to identify novel markers with correlated expression (negative or positive) which are also insensitive to the histopathology subtype of the samples. This is evaluated using Pearson correlation coefficient, using thyroid FNA and samples of non-follicular histology (such as parathyroid adenoma, medullary cancer, cancer metastasis into the thyroid samples). These non-follicular samples are included in the analysis to represent signals from non-follicular cell populations and increase the dynamic range of expression for the markers of interest. This correlation search is done to identify other potential markers not known from the literature, but which show consistent expression across thyroid subtypes and correlate well with known markers.

The marker with strongest undifferentiated signal is KRT7, one of the genes in the seed set. The ten most highly correlated markers are shown below in Table 15. Their average normalized expression level is used as the follicular statistic to extrapolate the relative strength of follicular cell signals in any set of thyroid FNA samples. In this heterogeneous FNA example, averaging the normalized expression levels are sufficient to arrive at a useful Follicular Statistic.

The follicular statistic allows extrapolation of the the relative strength of gene signals arising from follicular cells. Although the follicular statistic cannot be directly interpreted as the proportion of follicular cells in the mixture, it is characterized as a monotone function of the amount of follicular cells. With lower amount of follicular cells, it may be harder to differentiate malignant from benign conditions using a gene expression classifier (GEC) or other machine learning predictive methods. Thus, empirical cut-offs for the statistic can be used as either (a) quality control mechanism to remove samples with insufficient follicular content from GEC analysis, or (b) modify estimates of post-test risk of malignancy using the information about the follicular content of the sample and effectively establish classifier decision cut-off boundaries as a function of follicular content may are developed.

In addition, the follicular statistic is used to adjust expression levels for genes whose expression correlates with the amount of follicular cells in the mixture using a linear modeling approach. This aids in searching for genes that are differentially expressed across a variable of interest (such as BRAF+ and BRAF- samples, for example) after adjusting for the effects of follicular content on gene expression. Using a standard linear modeling approach, follicular statistic is added as a covariate to the equation as in:

$$Y \sim Phenotype + folStat$$

where Y is expression intensity of a given marker. Standard approaches such as LIMMA are then used to identify genes differentially expressed by phenotype after adjusting for differences in follicular content. In addition, intensity profiles for new samples can be adjusted for the observed level of follicular statistic to restore true expression profiles characterizing the expression intensities of a given sample at a given target value of the follicular stat representing a pure sample state.

This can be done using models for tech factor removal as previously described in U.S. Patent Application Serial No. 12/964,666, filed 12/9/10. Specifically, expression levels for a marker of interest are previously modeled as $Y \sim Phenotype + folStat$ using training data, and the coefficients of this model are treated as known and fixed. In the real data sets generated by FNA samples, thousands of markers show significant dependence on the *folStat* variable. Coefficient for the dependence on follicular stat is $\beta$. Samples have a "target" follicular stat value of $F_t$ in the 'pure' non-contaminated state. For an incoming test sample with follicular stat value of F, the predicted intensity value for this marker at the target follicular stat level is $Y_{adj} = Y + (F_t - F) * \beta$.

**Table 14. Follicular markers reported in scientific literature and used as a seed set to fish-out more in order to arrive at the methods of the invention.**

| TCID | Marker | Specific Cell type | Generic Cell type |
|---|---|---|---|
| 2336891 | DIO1 | Follicular | Follicular |
| 3573870 | DIO2 | Follicular | Follicular |
| 3002640 | EGFR | Follicular | Follicular |
| 2387483 | KRT18 | Follicular | Follicular |
| 2663326 | KRT18 | Follicular | Follicular |
| 2698434 | KRT18 | Follicular | Follicular |
| 3211115 | KRT18 | Follicular | Follicular |
| 3415576 | KRT18 | Follicular | Follicular |
| 3469238 | KRT18 | Follicular | Follicular |
| 3668077 | KRT18 | Follicular | Follicular |
| 3953556 | KRT18 | Follicular | Follicular |
| 3983549 | KRT18 | Follicular | Follicular |
| 4028716 | KRT18 | Follicular | Follicular |
| 3757108 | KRT19 | Follicular | Follicular |
| 3415320 | KRT7 | Follicular | Follicular |
| 3455186 | KRT7 | Follicular | Follicular |
| 2697231 | KRT8 | Follicular | Follicular |
| 2873168 | KRT8 | Follicular | Follicular |
| 3100563 | KRT8 | Follicular | Follicular |
| 3455516 | KRT8 | Follicular | Follicular |
| 2437118 | MUC1 | Follicular | Follicular |
| 3561381 | NKX2-1 | Follicular | Follicular |
| 3824623 | SLC5A5 | Follicular | Follicular |
| 3116614 | TG | Follicular | Follicular |
| 2466554 | TPO | Follicular | Follicular |
| 3236958 | VIM | Follicular | Follicular |

**Table 15. List of follicular statistic markers**

| TCID | GENE Symbol | GeneID | Description |
|---|---|---|---|
| 3415320 | KRT7 | 3855 | keratin 7 |
| 3666409 | CDH1 | 999 | cadherin 1, type 1, E-cadherin (epithelial) |
| 3113180 | MAL2 | 114569 | mal, T-cell differentiation protein 2 |
| 3107548 | ESRP1 | 54845 | epithelial splicing regulatory protein 1 |
| 4045676 | S100A1 | 6271 | S100 calcium binding protein A1 |
| 4045676 | S100A13 | 6284 | S100 calcium binding protein A13 |
| 2480961 | EPCAM | 4072 | epithelial cell adhesion molecule |

(continued)

| TCID | GENE Symbol | GeneID | Description |
|---|---|---|---|
| 3615579 | TJP1 | 7082 | tight junction protein 1 (zona occludens 1) |
| 3987996 | PLS3 | 5358 | plastin 3 (T isoform) |
| 2699564 | PLOD2 | 5352 | procollagen-lysine, 2-oxoglutarate 5-dioxygenase 2 |
| 2700585 | PFN2 | 5217 | profilin 2 |

**Example 6: In silico Modeling**

In this example, in silico modeling is performed to improve selectivity of FNA analysis. As described herein, mixing models are developed. Mixing proportion of known components are specified, as described in Table 16. After choosing an analytical model for mixture samples, *in silico* mixing of profiles for normal adjacent tissues and prior clinical FNAs profiled as a part of another study are performed to characterize the tolerance of classifier calls to varying proportions of nodule cells.

CEL files for additional adjacent normal tissue samples profiled following identical laboratory protocols may be used to supplement the source of normal adjacent tissue in the in silico simulation.

The sample selection for this study is based on the quantity and quality of total RNA from normal thyroid FNA. Simulations using pilot data and data simulated from the two alternative models indicate sufficient sample size to discriminate between two alternative models based on the marginal likelihood (assuming model correctness).

Experimental design for this study (including mixture proportions) is chosen in a way that (a) allowed evaluation of classifier performance at high dilution levels and (b) maximized the ability to disambiguate two alternative models described above. In addition to the mixture of pre-operative FNA and ex-vivo normal adj acent tissue FNA from the same patient, 80% Normal Thyroid FNA / 20% nodule FNA samples mixes from two different patients are added to investigate the region of largest expected discrepancy in linear classifier scores between two alternative models.

**Table 16. *In vitro* mixture experiment design.**

| Sample ID Normal Thyroid | Sample ID nodule FNA | Percentage normal thyroid | normal thyroid RNA (ng) | nodule RNA (ng) | # Replicates |
|---|---|---|---|---|---|
| - | C1A181P | 0% | 0 ng | 15.00 ng | 1 |
| C1A231X | C1A181P | 40% | 6.00 ng | 9.00 ng | 1 |
| C1A231X | C1A181P | 80% | 12.00 ng | 3.00 ng | 1 |
| C1A231X | - | 100% | 15 ng | 0 ng | 1 |
| C1A231X | C1A231P | 40% | 6.00 ng | 9.00 ng | 1 |
| C1A231X | C1A231P | 60% | 9.00 ng | 6.00 ng | 1 |
| C1A231X | C1A231P | 80% | 12.00 ng | 3.00 ng | 1 |
|  | C1A231P | 0% | 0 ng | 15.00 ng | 1 |
| C1A231X | C1A381P | 80% | 12.00 ng | 3.00 ng | 1 |
| C1A231X | C1A301P | 80% | 12.00 ng | 3.00 ng | 1 |
|  | B-RNA-003 | 0 | 0 | 15 | 1 |
|  | M-RNA-001 | 0 | 0 | 15 | 1 |
|  | NTC* | 0 | 0 | 0 | 1 |
| **TOTAL** |  |  |  |  | **12 arrays** |
| *note: no template control (NTC) is not run on arrays | | | | | |

Models $M_0$ and $M_1$ are compared here in their (a) ability to predict observed expression intensity values and (b) ability to predict observed classifier scores for the *in vitro* mixed samples, assuming profiles of pure unmixed samples are known.

To compare quality of predictions for intensity values, the predictive distribution of intensities for the markers of interest is constructed using the generative models $M_0$ and $M_1$. The standardized residuals are then estimated as the predicted intensities minus the observed intensity, normalized by the standard deviation of the predictive distribution. Figures 14a and 14b show the standardized residuals across all mixture samples for 142 markers that are a part of the Afirma-T classifier, identifying model $M_0$ as the one with the better fit to the observed data. A single mixture (231P with 231X, center of each panel) with a poor fit to the model predictions had low post-hybridization quality control metric (HAAUC ~ 0.87), which explains the discrepancy.

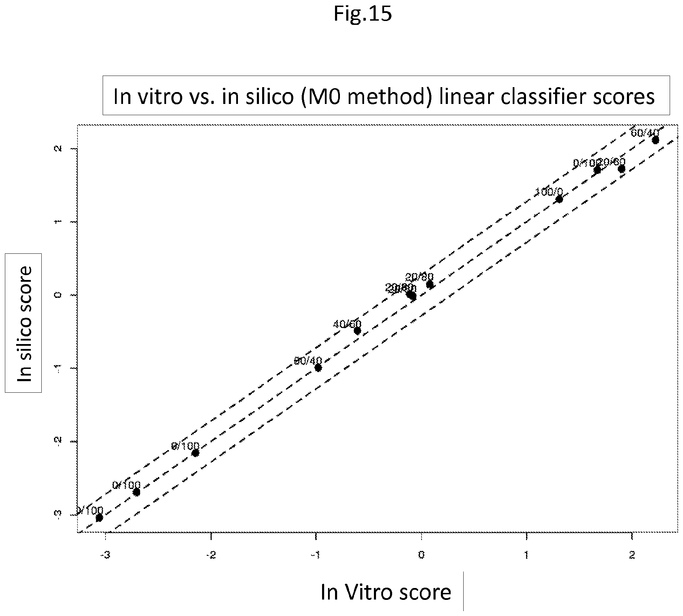

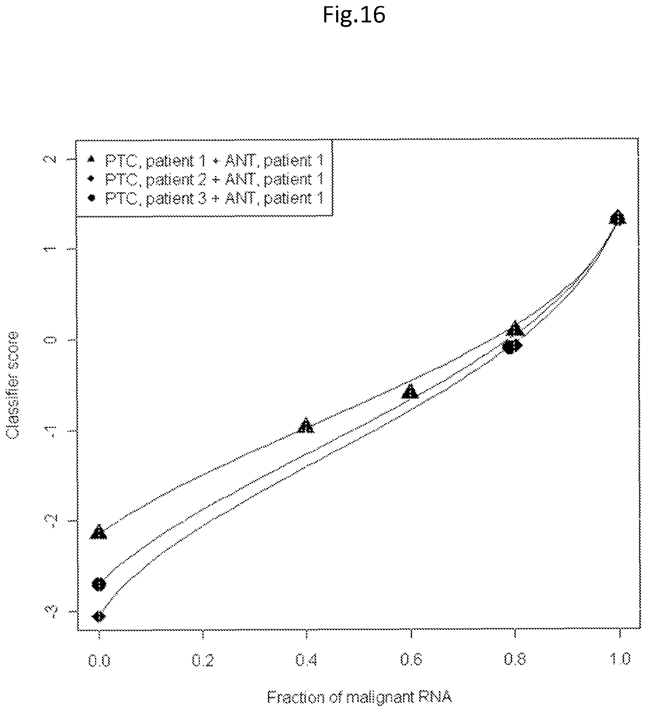

The classifier scores are then compared to predictions from both models. The results are shown in Figures 15-17; Figure 15 shows results for model $M_0$ across all samples; Figure 16 shows results across mixing proportions for mixtures of malignant and normal thyroid tissue; Figure 17 compares predictions of two models for one of the mixtures. These results also suggest an accurate score approximation by the model across all mixture samples. Although the predicted classifier scores for the mixtures using the $M_0$ model are not linearly explained by the mixture proportion, *in silico* simulations using this model approximate the in vitro GEC scores with precision.

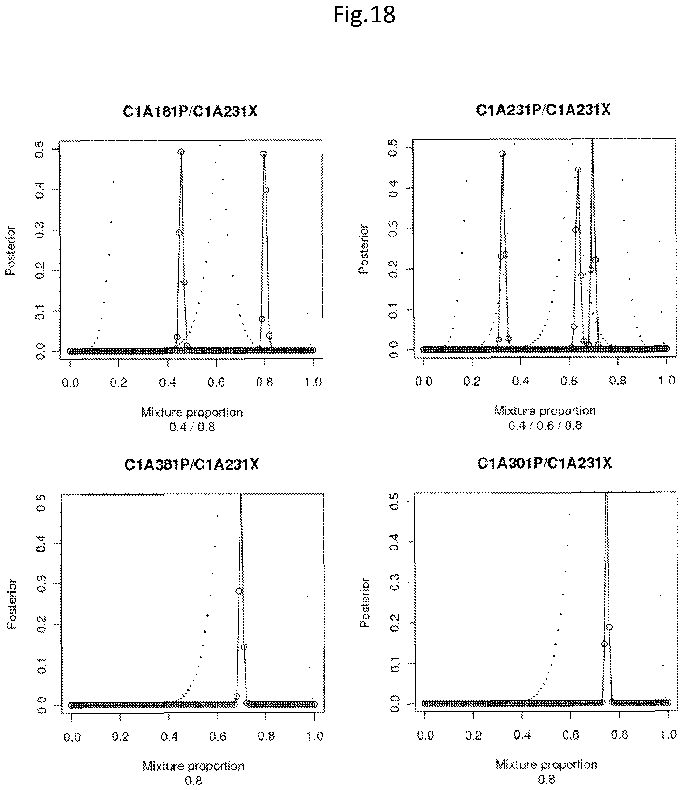

Finally, the model $M_0$ is used to estimate posterior distribution of the mixing proportions for all mixtures and demonstrate that these estimates are able to recover actual design proportions with high precision. These results are shown in Figure 18 and suggest low variance of the mixing proportion estimates given observed data, suggesting that this number of markers is sufficient to restore the proportion from the data with high precision. In essence the methods of the invention and Figure 18 demonstrate that the *in silico* modeling framework can be used to estimate the posterior probability of the mixing proportion variable in the model (alpha) and accurately infer the mixing proportion used in the experimental design. This can be applied to the 142-marker panel in the Afirma GEC.

**Table 17. *In vitro* and *in silico* mixture results.**

| Sample Mix | Mix Proportion | AUC | In vitro score | In silico mean score |
|---|---|---|---|---|
| C1A381P | 100% | 0.935 | -2.702 | -2.685 |
| C1A301P | 100% | 0.938 | -3.055 | -3.087 |
| C1A181P | 100% | 0.939 | 1.673 | 1.669 |
| C1A181P/C1A231X | 60/40 | 0.936 | 2.224 | 2.093 |
| C1A181P/C1A231X | 20/80 | 0.942 | 1.903 | 1.762 |
| C1A231X | 100% | 0.942 | 1.313 | 1.326 |
| C1A231P/C1A231X | 60/40 | 0.942 | -0.977 | -0.966 |
| C1A231P/C1A231X | 40/60 | 0.875 | -0.605 | -0.455 |
| C1A231P/C1A231X | 20/80 | 0.944 | 0.08 | 0.13 |
| C1A231P | 100% | 0.94 | -2.143 | -2.147 |
| C1A381P/C1A231X | 20/80 | 0.944 | -0.11 | 0.105 |
| C1A301P/C1A231X | 20/80 | 0.946 | -0.083 | 0.036 |

**Example 7: Example genes in the gene expression classifier, or "main classifier"**

In this example, a list of genes representing the gene expression classifier, or "main classifier" is provided (as previously described in US. Appl. 13/708,439).

**Table 18: List of 167 Transcript cluster identification numbers (TCID) in the gene expression classifier and their gene annotations.**

| Main Classifier | | |
|---|---|---|
| **TCID** | **GENE** | **Description** |
| 3450861 | ABCD2 | ATP-binding cassette, sub-family D (ALD), member 2 |
| 3341061 | ACER3 | alkaline ceramidase 3 |
| 2796553 | ACSL1 | acyl-CoA synthetase long-chain family member 1 |
| 2566848 | AFF3 | AF4/FMR2 family, member 3 |
| 3375735 | AHNAK | AHNAK nucleoprotein |

(continued)

| Main Classifier | | |
|---|---|---|
| **TCID** | **GENE** | **Description** |
| 2439554 | AIM2 | absent in melanoma 2 |
| 2988882 | AIMP2 | aminoacyl tRNA synthetase complex-interacting multifunctional protein 2 |
| 3169331 | ALDH1B1 | aldehyde dehydrogenase 1 family, member B1 |
| 3768474 | ARSG | arylsulfatase G |
| 3214845 | ASPN | asporin |
| 3006572 | AUTS2 | autism susceptibility candidate 2 |
| 3902489 | BCL2L1 | BCL2-like 1 |
| 2984616 | BRP44L | brain protein 44-like |
| 2688717 | BTLA | B and T lymphocyte associated |
| 2730303 | C4orf7 | chromosome 4 open reading frame 7 |
| 2822492 | C5orf30 | chromosome 5 open reading frame 30 |
| 3259367 | CC2D2B | coiled-coil and C2 domain containing 2B |
| 3204285 | CCL 19 | chemokine (C-C motif) ligand 19 |
| 3338192 | CCND 1 | cyclin D1 |
| 3010503 | CD36 | CD36 molecule (thrombospondin receptor) |
| 3326635 | CD44 | CD44 molecule (Indian blood group) |
| 2326463 | CD52 | CD52 molecule |
| 2635741 | CD96 | CD96 molecule |
| 2373336 | CFH | complement factor H |
| 2373336 | CFHR1 | complement factor H-related 1 |
| 2710599 | CLDN1 | claudin 1 |
| 2657808 | CLDN16 | claudin 16 |
| 2750627 | CPE | carboxypeptidase E |
| 2377283 | CR2 | complement component (3d/Epstein Barr virus) receptor 2 |
| 3242353 | CREM | cAMP responsive element modulator |
| 2490351 | CTNNA2 | catenin (cadherin-associated protein), alpha 2 |
| 2732508 | CXCL13 | chemokine (C-X-C motif) ligand 13 |
| 3042001 | CYCS | cytochrome c, somatic |
| 2854445 | DAB2 | disabled homolog 2, mitogen-responsive phosphoprotein (Drosophila) |
| 2321911 | DDI2 | DNA-damage inducible 1 homolog 2 (S. cerevisiae) |
| 3122678 | DEFB1 | defensin, beta 1 |
| 2642791 | DNAJC13 | DnaJ (Hsp40) homolog, subfamily C, member 13 |
| 2584018 | DPP4 | dipeptidyl-peptidase 4 |
| 3032647 | DPP6 | dipeptidyl-peptidase 6 |
| 2981874 | DYNLT1 | dynein, light chain, Tctex-type 1 |
| 2638676 | EAF2 | ELL associated factor 2 |
| 2739308 | EGF | epidermal growth factor |
| 2988882 | EIF2AK1 | eukaryotic translation initiation factor 2-alpha kinase 1 |
| 3852832 | EMR3 | egf-like module containing, mucin-like, hormone receptor-like 3 |
| 3142381 | FABP4 | fatty acid binding protein 4, adipocyte |
| 3603932 | FAH | fumarylacetoacetate hydrolase (fumarylacetoacetase) |
| 2396750 | FBXO2 | F-box protein 2 |
| 2396750 | FBXO44 | F-box protein 44 |
| 2526806 | FN1 | fibronectin 1 |
| 2598261 | FN1 | fibronectin 1 |
| 3839910 | FPR2 | formyl peptide receptor 2 |

(continued)

| Main Classifier | | |
|---|---|---|
| **TCID** | **GENE** | **Description** |
| 3486096 | FREM2 | FRAS1 related extracellular matrix protein 2 |
| 2970897 | FRK | fyn-related kinase |
| 3212008 | FRMD3 | FERM domain containing 3 |
| 3393479 | FXYD6 | FXYD domain containing ion transport regulator 6 |
| 2378068 | G0S2 | G0/G1switch 2 |
| 2884845 | GABRB2 | gamma-aminobutyric acid (GABA) A receptor, beta 2 |
| 3063795 | GAL3ST4 | galactose-3-O-sulfotransferase 4 |
| 3031556 | GIMAP2 | GTPase, IMAP family member 2 |
| 3861948 | GMFG | glia maturation factor, gamma |
| 3302990 | GOT1 | glutamic-oxaloacetic transaminase 1, soluble (aspartate aminotransferase 1) |
| 3540862 | GPHN | gephyrin |
| 3982612 | GPR174 | G protein-coupled receptor 174 |
| 2809793 | GZMK | granzyme K (granzyme 3; tryptase II) |
| 2638676 | HCG11 | HLA complex group 11 |
| 3417703 | HSD 17B6 | hydroxysteroid (17-beta) dehydrogenase 6 homolog (mouse) |
| 2877508 | HSPA9 | heat shock 70kDa protein 9 (mortalin) |
| 2708922 | IGF2BP2 | insulin-like growth factor 2 mRNA binding protein 2 |
| 3375735 | IGHG 1 | immunoglobulin heavy constant gamma 1 (G1m marker) |
| 2806468 | IL7R | interleukin 7 receptor |
| 2604998 | IQCA1 | IQ motif containing with AAA domain 1 |
| 3852832 | ITGB1 | integrin, beta 1 (fibronectin receptor, beta polypeptide, antigen CD29 includes MDF2, MSK12) |
| 3724545 | ITGB3 | integrin, beta 3 (platelet glycoprotein IIIa, antigen CD61) |
| 2427619 | KCNA3 | potassium voltage-gated channel, shaker-related subfamily, member 3 |
| 3397774 | KCNJ1 | potassium inwardly-rectifying channel, subfamily J, member 1 |
| 3404030 | KLRG1 | killer cell lectin-like receptor subfamily G, member 1 |
| 3512874 | LCP1 | lymphocyte cytosolic protein 1 (L-plastin) |
| 2708855 | LIPH | lipase, member H |
| 3875642 | LOC100131599 | hypothetical protein LOC100131599 |
| 2526806 | LOC100507488 | histone demethylase UTY-like |
| 2638676 | LOC647979 | hypothetical LOC647979 |
| 3147985 | LRP12 | low density lipoprotein receptor-related protein 12 |
| 2578790 | LRP1B | low density lipoprotein receptor-related protein 1B |
| 2352609 | MAGI3 | membrane associated guanylate kinase, WW and PDZ domain containing 3 |
| 3111561 | MAPK6 | mitogen-activated protein kinase 6 |
| 3108526 | MATN2 | matrilin 2 |
| 3009299 | MDH2 | malate dehydrogenase 2, NAD (mitochondrial) |
| 3329343 | MDK | midkine (neurite growth-promoting factor 2) |
| 3768474 | MIR635 | microRNA 635 |
| 3367673 | MPPED2 | metallophosphoesterase domain containing 2 |
| 3662201 | MT1F | metallothionein 1F |
| 3692999 | MT1G | metallothionein 1G |
| 3662201 | MT1H | metallothionein 1H |
| 3622934 | MYEF2 | myelin expression factor 2 |
| 3341497 | NDUFC2 | NADH dehydrogenase (ubiquinone) 1, subcomplex unknown, 2, 14.5kDa |

(continued)

| Main Classifier | | |
|---|---|---|
| **TCID** | **GENE** | **Description** |
| 3067478 | NRCAM | neuronal cell adhesion molecule |
| 3654699 | NUPR1 | nuclear protein, transcriptional regulator, 1 |
| 4020655 | ODZ1 | odz, odd Oz/ten-m homolog 1(Drosophila) |
| 3353914 | OR10D1P | olfactory receptor, family 10, subfamily D, member 1 pseudogene |
| 3982560 | P2RY10 | purinergic receptor P2Y, G-protein coupled, 10 |
| 2701071 | P2RY13 | purinergic receptor P2Y, G-protein coupled, 13 |
| 3948047 | PARVG | parvin, gamma |
| 3606034 | PDE8A | phosphodiesterase 8A |
| 3970833 | PDHA1 | pyruvate dehydrogenase (lipoamide) alpha 1 |
| 2377094 | PFKFB2 | 6-phosphofructo-2-kinase/fructose-2,6-biphosphatase 2 |
| 3278198 | PHYH | phytanoyl-CoA 2-hydroxylase |
| 3811086 | PIGN | phosphatidylinositol glycan anchor biosynthesis, class N |
| 3744680 | PIK3R5 | phosphoinositide-3-kinase, regulatory subunit 5 |
| 3111561 | PKHD1L1 | polycystic kidney and hepatic disease 1 (autosomal recessive)-like 1 |
| 3376529 | PLA2G16 | phospholipase A2, group XVI |
| 3875642 | PLCB1 | phospholipase C, beta 1 (phosphoinositide-specific) |
| 2486811 | PLEK | pleckstrin |
| 2880051 | PPP2R2B | protein phosphatase 2, regulatory subunit B, beta |
| 3246888 | PRKG1 | protein kinase, cGMP-dependent, type I |
| 3874751 | PRNP | prion protein |
| 2685304 | PROS1 | protein S (alpha) |
| 2373842 | PTPRC | protein tyrosine phosphatase, receptor type, C |
| 3270270 | PTPRE | protein tyrosine phosphatase, receptor type, E |
| 3959862 | PVALB | parvalbumin |
| 2688499 | PVRL2 | poliovirus receptor-related 2 (herpesvirus entry mediator B) |
| 3564210 | PYGL | phosphorylase, glycogen, liver |
| 2362351 | PYHIN1 | pyrin and HIN domain family, member 1 |
| 3443464 | PZP | pregnancy-zone protein |
| 2372812 | RGS13 | regulator of G-protein signaling 13 |
| 3110395 | RIMS2 | regulating synaptic membrane exocytosis 2 |
| 3895795 | RNF24 | ring finger protein 24 |
| 2964231 | RRAGD | Ras-related GTP binding D |
| 2442008 | RXRG | retinoid X receptor, gamma |
| 3494629 | SCEL | sciellin |
| 2904485 | SCUBE3 | signal peptide, CUB domain, EGF-like 3 |
| 2798538 | SDHA | succinate dehydrogenase complex, subunit A, flavoprotein (Fp) |
| 3059667 | SEMA3D | sema domain, immunoglobulin domain (Ig), short basic domain, secreted, (semaphorin) 3D |
| 3365136 | SERGEF | secretion regulating guanine nucleotide exchange factor |
| 3577612 | SERPINA 1 | serpin peptidase inhibitor, clade A (alpha-1 antiproteinase, antitrypsin), member 1 |
| 3577612 | SERPINA2 | serpin peptidase inhibitor, clade A (alpha-1 antiproteinase, antitrypsin), member 2 |
| 2440258 | SLAMF6 | SLAM family member 6 |
| 2428501 | SLC16A1 | solute carrier family 16, member 1 (monocarboxylic acid transporter 1) |
| 3622934 | SLC24A5 | solute carrier family 24, member 5 |
| 3185522 | SLC31A1 | solute carrier family 31 (copper transporters), member 1 |

(continued)

| Main Classifier | | |
|---|---|---|
| **TCID** | **GENE** | **Description** |
| 2721959 | SLC34A2 | solute carrier family 34 (sodium phosphate), member 2 |
| 3761959 | SLC35B1 | solute carrier family 35, member B 1 |
| 3373845 | SLC43A3 | solute carrier family 43, member 3 |
| 3759006 | SLC4A1 | solute carrier family 4, anion exchanger, member 1 (erythrocyte membrane protein band 3, Diego blood group) |
| 2730746 | SLC4A4 | solute carrier family 4, sodium bicarbonate cotransporter, member 4 |
| 2777714 | SNCA | synuclein, alpha (non A4 component of amyloid precursor) |
| 2877508 | SNORD63 | small nucleolar RNA, C/D box 63 |
| 2562529 | ST3GAL5 | ST3 beta-galactoside alpha-2,3-sialyltransferase 5 |
| 2834282 | STK32A | serine/threonine kinase 32A |
| 3341497 | THRSP | thyroid hormone responsive |
| 3976341 | TIMP 1 | TIMP metallopeptidase inhibitor 1 |
| 3772661 | TIMP2 | TIMP metallopeptidase inhibitor 2 |
| 2491271 | TMSB 10 | thymosin beta 10 |
| 3648391 | TNFRSF17 | tumor necrosis factor receptor superfamily, member 17 |
| 3441849 | TNFRSF1A | tumor necrosis factor receptor superfamily, member 1A |
| 2412668 | TXNDC12 | thioredoxin domain containing 12 (endoplasmic reticulum) |
| 4027585 | unknown | |
| 3353914 | VWA5A | von Willebrand factor A domain containing 5A |
| 3976766 | WAS | Wiskott-Aldrich syndrome (eczema-thrombocytopenia) |
| 3768474 | WIPI1 | WD repeat domain, phosphoinositide interacting 1 |
| 2688499 | ZBED2 | zinc finger, BED-type containing 2 |
| 2817731 | ZFYVE 16 | zinc finger, FYVE domain containing 16 |
| **Medullary Carcinoma Cassette** | | |
| 3364127 | CALCA | calcitonin-related polypeptide alpha |
| 3834341 | CEACAM5 | carcinoembryonic antigen-related cell adhesion molecule 5 |
| 3594003 | SCG3 | secretogranin III |
| 2585400 | SCN9A | sodium channel, voltage-gated, type IX, alpha subunit |
| 3805614 | SYT4 | synaptotagmin IV |
| **Renal Carcinoma Cassette** | | |
| 2923928 | FABP7 | fatty acid binding protein 7, brain |
| 3393446 | FXYD2 | FXYD domain containing ion transport regulator 2 |
| 2883317 | HAVCR1 | hepatitis A virus cellular receptor 1 |
| 2883317 | LOC100101266 | hepatitis A virus cellular receptor 1 pseudogene |
| 3428225 | NR1H4 | nuclear receptor subfamily 1, group H, member 4 |
| 2479698 | PREPL | prolyl endopeptidase-like |
| 2479698 | SLC3A1 | solute carrier family 3 (cystine, dibasic and neutral amino acid transporters, activator of cystine, dibasic and neutral amino acid transport), member 1 |
| **Parathyroid Cassette** | | |
| 3159754 | DMRT2 | doublesex and mab-3 related transcription factor 2 |
| 2941690 | GCM2 | glial cells missing homolog 2 (Drosophila) |
| 3363686 | KIDINS220 | kinase D-interacting substrate, 220kDa |
| 3484895 | KL | klotho |
| 3363686 | PTH | parathyroid hormone |
| 2894790 | SYCP2L | synaptonemal complex protein 2-like |

(continued)

| Parathyroid Cassette | | |
|---|---|---|
| 2894790 | TMEM14B | transmembrane protein 14B |
| **Breast Carcinoma Cassette** | | |
| 3039830 | AGR3 | anterior gradient homolog 3 (Xenopus laevis) |
| 3264997 | C10orf81 | chromosome 10 open reading frame 81 |
| 2926802 | MYB | v-myb myeloblastosis viral oncogene homolog (avian) |
| 3912079 | SYCP2 | synaptonemal complex protein 2 |
| 2430163 | VTCN1 | V-set domain containing T cell activation inhibitor 1 |
| **Melanoma Cassette** | | |
| 3811949 | CDH19 | cadherin 19, type 2 |
| 3161261 | MLANA | melan-A |
| 3935486 | S100B | S100 calcium binding protein B |
| 3457336 | SILV | silver homolog (mouse) |
| 3343832 | TYR | tyrosinase (oculocutaneous albinism IA) |
| 3343832 | TYRL | tyrosinase-like (pseudogene) |
| **Hiirthle Cassette** | | |
| 2566848 | AFF3 | AF4/FMR2 family, member 3 |
| 2988882 | AIMP2 | aminoacyl tRNA synthetase complex-interacting multifunctional protein 2 |
| 3169331 | ALDH1B1 | aldehyde dehydrogenase 1 family, member B 1 |
| 2984616 | BRP44L | brain protein 44-like |
| 2822492 | C5orf30 | chromosome 5 open reading frame 30 |
| 3326635 | CD44 | CD44 molecule (Indian blood group) |
| 2750627 | CPE | carboxypeptidase E |
| 3042001 | CYCS | cytochrome c, somatic |
| 3122678 | DEFB1 | defensin, beta 1 |
| 2739308 | EGF | epidermal growth factor |
| 2988882 | EIF2AK1 | eukaryotic translation initiation factor 2-alpha kinase 1 |
| 3603932 | FAH | fumarylacetoacetate hydrolase (fumarylacetoacetase) |
| 2970897 | FRK | fyn-related kinase |
| 3212008 | FRMD3 | FERM domain containing 3 |
| 3302990 | GOT1 | glutamic-oxaloacetic transaminase 1, soluble (aspartate aminotransferase 1) |
| 3417703 | HSD 17B6 | hydroxysteroid (17-beta) dehydrogenase 6 homolog (mouse) |
| 2877508 | HSPA9 | heat shock 70kDa protein 9 (mortalin) |
| 2708922 | IGF2BP2 | insulin-like growth factor 2 mRNA binding protein 2 |
| 2604998 | IQCA1 | IQ motif containing with AAA domain 1 |
| 3724545 | ITGB3 | integrin, beta 3 (platelet glycoprotein IIIa, antigen CD61) |
| 3397774 | KCNJ1 | potassium inwardly-rectifying channel, subfamily J, member 1 |
| 3009299 | MDH2 | malate dehydrogenase 2, NAD (mitochondrial) |
| 3654699 | NUPR1 | nuclear protein, transcriptional regulator, 1 |
| 4020655 | ODZ1 | odz, odd Oz/ten-m homolog 1(Drosophila) |
| 3970833 | PDHA1 | pyruvate dehydrogenase (lipoamide) alpha 1 |
| 2377094 | PFKFB2 | 6-phosphofructo-2-kinase/fructose-2,6-biphosphatase 2 |
| 3278198 | PHYH | phytanoyl-CoA 2-hydroxylase |
| 2880051 | PPP2R2B | protein phosphatase 2, regulatory subunit B, beta |
| 3959862 | PVALB | parvalbumin |
| 2688499 | PVRL2 | poliovirus receptor-related 2 (herpesvirus entry mediator B) |

(continued)

| Hiirthle Cassette | | |
|---|---|---|
| 2964231 | RRAGD | Ras-related GTP binding D |
| 2798538 | SDHA | succinate dehydrogenase complex, subunit A, flavoprotein (Fp) |
| 2428501 | SLC16A1 | solute carrier family 16, member 1 (monocarboxylic acid transporter 1) |
| 2877508 | SNORD63 | small nucleolar RNA, C/D box 63 |
| 2562529 | ST3GAL5 | ST3 beta-galactoside alpha-2,3-sialyltransferase 5 |
| 2688499 | ZBED2 | zinc finger, BED-type containing 2 |
| **Additional Genes Analyzed** | | |
| 3116614 | TG | |
| 3415320 | KRT7 | |
| 3757108 | KRT19 | |
| 4012178 | CITED 1 | |
| 3546213 | TSHR | |
| 3561381 | TFF1 | |

## Example 8: Examples of BRAF markers

In this example, a list of genes representing BRAF markers is provided (as previously described in US. Appl. 13/708,439).

**Table 19. BRAF signature biomarkers. PTC hetmut vs. PTC wild type, no covariates.**

The results from a LIMMA analysis (without adjusting for additional confounding covariates) are filtered based on FDR p-value ($\leq 0.0001$). Listed below are the 477 genes that passed the filter.

| Table 9: BRAF markers, no covariates | | | | |
|---|---|---|---|---|
| **TCID na30hg19** | **GENE** | **Description** | **Effect Size (log scale) no covariates** | **FDR adjusted p-value no covariates** |
| 3417249 | ERBB3 | v-erb-b2 erythroblastic leukemia viral oncogene homolog 3 (avian) | 1.56 | 4.25E-08 |
| 2560625 | FAM176A | family with sequence similarity 176, member A | 0.59 | 9.66E-08 |
| 2828441 | PDLIM4 | PDZ and LIM domain 4 | 1.14 | 9.66E-08 |
| 3678462 | PPL | periplakin | 0.98 | 1.32E-07 |
| 2414958 | TACSTD2 | tumor-associated calcium signal transducer 2 | 1.48 | 1.32E-07 |
| 2358949 | CGN | cingulin | 0.58 | 2.55E-07 |
| 2378256 | SYT14 | synaptotagmin XIV | 2.34 | 2.55E-07 |
| 2622970 | DOCK3 | dedicator of cytokinesis 3 | 0.94 | 3.28E-07 |
| 3040518 | MACC1 | metastasis associated in colon cancer 1 | 1.89 | 3.28E-07 |
| 2973376 | PTPRK | protein tyrosine phosphatase, receptor type, K | 1.28 | 3.28E-07 |
| 2560076 | RTKN | rhotekin | 0.51 | 3.28E-07 |
| 2648535 | SGEF | Src homology 3 domain-containing guanine nucleotide exchange factor | 1.00 | 3.28E-07 |
| 2991860 | ITGB8 | integrin, beta 8 | 1.60 | 3.37E-07 |
| 3110608 | TM7SF4 | transmembrane 7 superfamily member 4 | 2.72 | 3.44E-07 |

(continued)

The results from a LIMMA analysis (without adjusting for additional confounding covariates) are filtered based on FDR p-value (≤ 0.0001). Listed below are the 477 genes that passed the filter.

| TCID na30hg19 | GENE | Description | Effect Size (log scale) no covariates | FDR adjusted p-value no covariates |
|---|---|---|---|---|
| | | **Table 9: BRAF markers, no covariates** | | |
| 2333318 | PTPRF | protein tyrosine phosphatase, receptor type, F | 0.99 | 3.56E-07 |
| 3352438 | POU2F3 | POU class 2 homeobox 3 | 0.60 | 3.91E-07 |
| 2738664 | SGMS2 | sphingomyelin synthase 2 | 1.57 | 4.15E-07 |
| 2622121 | DAG1 | dystroglycan 1 (dystrophin-associated glycoprotein 1) | 0.75 | 5.98E-07 |
| 2903782 | ITPR3 | inositol 1,4,5-triphosphate receptor, type 3 | 1.03 | 5.98E-07 |
| 3890333 | TFAP2C | transcription factor AP-2 gamma (activating enhancer binding protein 2 gamma) | 0.66 | 6.08E-07 |
| 2809245 | ITGA2 | integrin, alpha 2 (CD49B, alpha 2 subunit of VLA-2 receptor) | 2.17 | 6.13E-07 |
| 2371139 | LAMC2 | laminin, gamma 2 | 1.44 | 7.90E-07 |
| 3109687 | GRHL2 | grainyhead-like 2 (Drosophila) | 1.15 | 1.03E-06 |
| 3868783 | KLK7 | kallikrein-related peptidase 7 | 1.66 | 1.03E-06 |
| 2452478 | LEMD1 | LEM domain containing 1 | 1.61 | 1.03E-06 |
| 3154002 | KCNQ3 | potassium voltage-gated channel, KQT-like subfamily, member 3 | 0.84 | 1.06E-06 |
| 2611779 | TMEM43 | transmembrane protein 43 | 0.70 | 1.06E-06 |
| 3636391 | HOMER2 | homer homolog 2 (Drosophila) | 0.96 | 1.10E-06 |
| 3636391 | LOC100131860 | hypothetical protein LOC100131860 | 0.96 | 1.10E-06 |
| 2423829 | ARHGAP29 | Rho GTPase activating protein 29 | 1.80 | 1.14E-06 |
| 3529908 | NFATC4 | nuclear factor of activated T-cells, cytoplasmic, calcineurin-dependent 4 | 0.46 | 1.14E-06 |
| 2360677 | EFNA1 | ephrin-A1 | 0.77 | 1.14E-06 |
| 2344888 | CYR61 | cysteine-rich, angiogenic inducer, 61 | 0.86 | 1.20E-06 |
| 2910680 | LRRC1 | leucine rich repeat containing 1 | 0.87 | 1.20E-06 |
| 3390195 | EXPH5 | exophilin 5 | 1.22 | 1.21E-06 |
| 3269694 | FANK1 | fibronectin type III and ankyrin repeat domains 1 | 1.20 | 1.21E-06 |
| 2323899 | UBXN 10 | UBX domain protein 10 | 1.06 | 1.21E-06 |
| 2451309 | COX7C | cytochrome c oxidase subunit VIIc | 0.70 | 1.42E-06 |
| 2451309 | KDM5B | lysine (K)-specific demethylase 5B | 0.70 | 1.42E-06 |
| 2783596 | PDE5A | phosphodiesterase 5A, cGMP-specific | 2.06 | 1.44E-06 |
| 3198974 | MPDZ | multiple PDZ domain protein | 1.36 | 1.54E-06 |
| 2759582 | AFAP 1 | actin filament associated protein 1 | 0.64 | 2.00E-06 |
| 2468811 | ASAP2 | ArfGAP with SH3 domain, ankyrin repeat and PH domain 2 | 1.21 | 2.00E-06 |

The results from a LIMMA analysis (without adjusting for additional confounding covariates) are filtered based on FDR p-value ($\leq$ 0.0001). Listed below are the 477 genes that passed the filter.

| | | | | |
|---|---|---|---|---|
| | | **Table 9: BRAF markers, no covariates** | | |
| **TCID na30hg19** | **GENE** | **Description** | **Effect Size (log scale) no covariates** | **FDR adjusted p-value no covariates** |
| 2484970 | EHBP1 | EH domain binding protein 1 | 1.00 | 2.00E-06 |
| 3696226 | ESRP2 | epithelial splicing regulatory protein 2 | 0.51 | 2.00E-06 |
| 2759582 | LOC389199 | hypothetical LOC389199 | 0.64 | 2.00E-06 |
| 3183111 | SLC44A1 | solute carrier family 44, member 1 | 1.09 | 2.00E-06 |
| 3104698 | ZBTB10 | zinc finger and BTB domain containing 10 | 0.60 | 2.00E-06 |
| 2356818 | BCL9 | B-cell CLL/lymphoma 9 | 0.89 | 2.15E-06 |
| 3040967 | RAPGEF5 | Rap guanine nucleotide exchange factor (GEF) 5 | 1.05 | 2.15E-06 |
| 3456081 | RARG | retinoic acid receptor, gamma | 0.49 | 2.15E-06 |
| 4045643 | S100A16 | S100 calcium binding protein A16 | 1.58 | 2.15E-06 |
| 2437118 | MUC1 | mucin 1, cell surface associated | 1.38 | 2.21E-06 |
| 3868828 | KLK10 | kallikrein-related peptidase 10 | 1.56 | 2.42E-06 |
| 2830861 | EGR1 | early growth response 1 | 1.44 | 2.59E-06 |
| 2582562 | ACVR1 | activin A receptor, type I | 1.04 | 2.66E-06 |
| 2385873 | KCNK1 | potassium channel, subfamily K, member 1 | 0.90 | 2.74E-06 |
| 3807595 | LOC441420 | similar to KIAA1119 protein | 1.12 | 2.79E-06 |
| 3807595 | MYOSB | myosin VB | 1.12 | 2.79E-06 |
| 3523318 | NALCN | sodium leak channel, non-selective | 0.71 | 2.79E-06 |
| 2453881 | IRF6 | interferon regulatory factor 6 | 1.03 | 2.88E-06 |
| 3556990 | JUB | jub, ajuba homolog (Xenopus laevis) | 1.14 | 2.88E-06 |
| 3628832 | DAPK2 | death-associated protein kinase 2 | 1.39 | 2.89E-06 |
| 3020273 | CAV2 | caveolin 2 | 1.71 | 2.92E-06 |
| 2685304 | PROS 1 | protein S (alpha) | 1.92 | 2.92E-06 |
| 2525533 | LOC648149 | hypothetical protein LOC648149 | 1.35 | 2.96E-06 |
| 2525533 | MAP2 | microtubule-associated protein 2 | 1.35 | 2.96E-06 |
| 3173880 | LOC100289287 | similar to tight junction protein 2 (zona occludens 2) | 1.02 | 2.98E-06 |
| 3173880 | TJP2 | tight junction protein 2 (zona occludens 2) | 1.02 | 2.98E-06 |
| 3183757 | RAD23B | RAD23 homolog B (S. cerevisiae) | 0.61 | 3.08E-06 |
| 3705491 | FAM57A | family with sequence similarity 57, member A | 0.70 | 3.13E-06 |
| 3795942 | YES1 | v-yes-1 Yamaguchi sarcoma viral oncogene homolog 1 | 0.76 | 3.28E-06 |
| 2742109 | FGF2 | fibroblast growth factor 2 (basic) | 0.97 | 3.44E-06 |
| 3108489 | LAPTM4B | lysosomal protein transmembrane 4 beta | 1.08 | 3.44E-06 |

(continued)

The results from a LIMMA analysis (without adjusting for additional confounding covariates) are filtered based on FDR p-value ($\leq$ 0.0001). Listed below are the 477 genes that passed the filter.

Table 9: BRAF markers, no covariates

| TCID na30hg19 | GENE | Description | Effect Size (log scale) no covariates | FDR adjusted p-value no covariates |
|---|---|---|---|---|
| 2742109 | NUDT6 | nudix (nucleoside diphosphate linked moiety X)-type motif 6 | 0.97 | 3.44E-06 |
| 3863640 | CXCL17 | chemokine (C-X-C motif) ligand 17 | 1.93 | 3.56E-06 |
| 2976360 | PERP | PERP, TP53 apoptosis effector | 1.59 | 3.64E-06 |
| 2405284 | TMEM54 | transmembrane protein 54 | 0.94 | 3.66E-06 |
| 3056264 | ABHD 11 | abhydrolase domain containing 11 | 0.57 | 3.83E-06 |
| 2593407 | PGAP 1 | post-GPI attachment to proteins 1 | 1.16 | 3.84E-06 |
| 3726154 | ITGA3 | integrin, alpha 3 (antigen CD49C, alpha 3 subunit of VLA-3 receptor) | 1.45 | 3.92E-06 |
| 3783529 | DSG2 | desmoglein 2 | 1.77 | 4.41E-06 |
| 2700365 | TM4SF1 | transmembrane 4 L six family member 1 | 2.20 | 4.41E-06 |
| 3973692 | PRRG1 | proline rich Gla (G-carboxyglutamic acid) 1 | 1.68 | 4.44E-06 |
| 3401217 | TULP3 | tubby like protein 3 | 0.81 | 4.44E-06 |
| 2875454 | SEPT8 | septin 8 | 0.85 | 4.65E-06 |
| 3110272 | FZD6 | frizzled homolog 6 (Drosophila) | 1.61 | 4.65E-06 |
| 3110272 | LOC100131102 | hypothetical protein LOC100131102 | 1.61 | 4.65E-06 |
| 3928415 | CLDN8 | claudin 8 | 1.49 | 4.77E-06 |
| 3653123 | PRKCB | protein kinase C, beta | -1.44 | 4.96E-06 |
| 3368940 | ABTB2 | ankyrin repeat and BTB (POZ) domain containing 2 | 0.43 | 5.09E-06 |
| 2351787 | C1orf88 | chromosome 1 open reading frame 88 | 1.34 | 5.09E-06 |
| 2327310 | SMPDL3B | sphingomyelin phosphodiesterase, acid-like 3B | 0.89 | 5.79E-06 |
| 3408831 | SSPN | sarcospan (Kras oncogene-associated gene) | 1.26 | 6.08E-06 |
| 3385951 | NOX4 | NADPH oxidase 4 | 0.71 | 6.12E-06 |
| 2434178 | MTMR11 | myotubularin related protein 11 | 0.44 | 6.20E-06 |
| 3473750 | FLJ20674 | hypothetical protein FLJ20674 | 0.66 | 6.24E-06 |
| 3580791 | BAGS | BCL2-associated athanogene 5 | 0.57 | 6.34E-06 |
| 2632453 | ARL 13B | ADP-ribosylation factor-like 13B | 0.98 | 6.38E-06 |
| 3235516 | CAMK1D | calcium/calmodulin-dependent protein kinase ID | -0.75 | 6.38E-06 |
| 2708817 | TMEM41A | transmembrane protein 41A | 0.63 | 6.54E-06 |
| 3050609 | COBL | cordon-bleu homolog (mouse) | 0.60 | 6.66E-06 |
| 2567167 | LONRF2 | LON peptidase N-terminal domain and ring finger 2 | 1.61 | 8.04E-06 |
| 2590582 | PDE1A | phosphodiesterase 1A, calmodulin-dependent | 1.76 | 8.82E-06 |

(continued)

The results from a LIMMA analysis (without adjusting for additional confounding covariates) are filtered based on FDR p-value (≤ 0.0001). Listed below are the 477 genes that passed the filter.

| Table 9: BRAF markers, no covariates | | | | |
|---|---|---|---|---|
| TCID na30hg19 | GENE | Description | Effect Size (log scale) no covariates | FDR adjusted p-value no covariates |
| 2734270 | CDS1 | CDP-diacylglycerol synthase (phosphatidate cytidylyltransferase) 1 | 1.13 | 8.89E-06 |
| 3590164 | SPINT1 | serine peptidase inhibitor, Kunitz type 1 | 0.78 | 8.89E-06 |
| 2341083 | GADD45A | growth arrest and DNA-damage-inducible, alpha | 0.84 | 9.03E-06 |
| 3757108 | KRT19 | keratin 19 | 1.26 | 9.13E-06 |
| 3994710 | MAMLD1 | mastermind-like domain containing 1 | 0.68 | 9.13E-06 |
| 2412312 | TTC39A | tetratricopeptide repeat domain 39A | 1.04 | 9.13E-06 |
| 3975893 | PHF 16 | PHD finger protein 16 | 0.72 | 9.57E-06 |
| 3056292 | CLDN3 | claudin 3 | 1.04 | 9.58E-06 |
| 2346625 | EPHX4 | epoxide hydrolase 4 | 1.00 | 1.02E-05 |
| 3389976 | SLC35F2 | solute carrier family 35, member F2 | 1.02 | 1.02E-05 |
| 2548776 | ATL2 | atlastin GTPase 2 | 1.12 | 1.05E-05 |
| 2635906 | PHLDB2 | pleckstrin homology-like domain, family B, member 2 | 1.28 | 1.05E-05 |
| 2511820 | PKP4 | plakophilin 4 | 1.23 | 1.05E-05 |
| 3351200 | TMPRSS4 | transmembrane protease, serine 4 | 1.40 | 1.05E-05 |
| 2457842 | TP53BP2 | tumor protein p53 binding protein, 2 | 0.70 | 1.07E-05 |
| 3012019 | CLDN12 | claudin 12 | 1.35 | 1.07E-05 |
| 3012019 | PFTK1 | PFTAIRE protein kinase 1 | 1.35 | 1.07E-05 |
| 3522398 | AIDA | axin interactor, dorsalization associated | 1.51 | 1.07E-05 |
| 3522398 | DOCK9 | dedicator of cytokinesis 9 | 1.51 | 1.07E-05 |
| 2649609 | MLF1 | myeloid leukemia factor 1 | 1.24 | 1.07E-05 |
| 3757329 | JUP | junction plakoglobin | 0.90 | 1.09E-05 |
| 3679959 | EMP2 | epithelial membrane protein 2 | 1.43 | 1.10E-05 |
| 3219885 | PTPN3 | protein tyrosine phosphatase, non-receptor type 3 | 1.01 | 1.10E-05 |
| 2732844 | ANXA3 | annexin A3 | 1.44 | 1.10E-05 |
| 2408499 | SCMH1 | sex comb on midleg homolog 1 (Drosophila) | 0.62 | 1.11E-05 |
| 2931090 | PPP 1R14C | protein phosphatase 1, regulatory (inhibitor) subunit 14C | 1.11 | 1.13E-05 |
| 3453252 | ADCY6 | adenylate cyclase 6 | 0.31 | 1.13E-05 |
| 3020302 | CAV1 | caveolin 1, caveolae protein, 22kDa | 1.97 | 1.13E-05 |
| 3007960 | CLDN4 | claudin 4 | 1.60 | 1. 13E-05 |
| 2686023 | DCBLD2 | discoidin, CUB and LCCL domain containing 2 | 1.30 | 1.13E-05 |

(continued)

The results from a LIMMA analysis (without adjusting for additional confounding covariates) are filtered based on FDR p-value (≤ 0.0001). Listed below are the 477 genes that passed the filter.

### Table 9: BRAF markers, no covariates

| TCID na30hg19 | GENE | Description | Effect Size (log scale) no covariates | FDR adjusted p-value no covariates |
|---|---|---|---|---|
| 2625907 | FLNB | filamin B, beta | 0.81 | 1.13E-05 |
| 3079005 | RARRES2 | retinoic acid receptor responder (tazarotene induced) 2 | 0.76 | 1.13E-05 |
| 3034027 | DNAJB6 | DnaJ (Hsp40) homolog, subfamily B, member 6 | -0.57 | 1. 14E-05 |
| 3034027 | TMEM135 | transmembrane protein 135 | -0.57 | 1.14E-05 |
| 2708855 | C11orf72 | chromosome 11 open reading frame 72 | 2.07 | 1. 14E-05 |
| 2708855 | LIPH | lipase, member H | 2.07 | 1.14E-05 |
| 3600283 | THSD4 | thrombospondin, type I, domain containing 4 | 0.63 | 1. 19E-05 |
| 2827525 | KDELC1 | KDEL (Lys-Asp-Glu-Leu) containing 1 | 1.10 | 1.19E-05 |
| 2539607 | MBOAT2 | membrane bound O-acyltransferase domain containing 2 | 1.29 | 1. 19E-05 |
| 2827525 | SLC12A2 | solute carrier family 12 (sodium/potassium/ chloride transporters), member 2 | 1.10 | 1.19E-05 |
| 2936857 | MLLT4 | myeloid/lymphoid or mixed-lineage leukemia (trithorax homolog, Drosophila); translocated to, 4 | 1.29 | 1.26E-05 |
| 4024373 | CDR1 | cerebellar degeneration-related protein 1, 34kDa | 1.97 | 1.29E-05 |
| 3351498 | TMEM25 | transmembrane protein 25 | 0.48 | 1.29E-05 |
| 3351498 | TTC36 | tetratricopeptide repeat domain 36 | 0.48 | 1.29E-05 |
| 4024373 | YTHDC2 | YTH domain containing 2 | 1.97 | 1.29E-05 |
| 2450798 | LAD 1 | ladinin 1 | 0.43 | 1.29E-05 |
| 3044129 | GGCT | gamma-glutamyl cyclotransferase | 1.09 | 1.30E-05 |
| 2594951 | ALS2CR4 | amyotrophic lateral sclerosis 2 (juvenile) chromosome region, candidate 4 | 0.83 | 1.31E-05 |
| 2881860 | CCDC69 | coiled-coil domain containing 69 | -0.94 | 1.31E-05 |
| 2643901 | PPP2R3A | protein phosphatase 2 (formerly 2A), regulatory subunit B", alpha | 0.68 | 1.31E-05 |
| 4018454 | AMOT | angiomotin | 1.09 | 1.32E-05 |
| 3581221 | AHNAK2 | AHNAK nucleoprotein 2 | 1.45 | 1.34E-05 |
| 3683377 | GPRC5B | G protein-coupled receptor, family C, group 5, member B | 1.37 | 1.34E-05 |
| 2790823 | MAP9 | microtubule-associated protein 9 | 0.71 | 1.34E-05 |
| 2402431 | PAQR7 | progestin and adipoQ receptor family member VII | 0.56 | 1.34E-05 |

(continued)

The results from a LIMMA analysis (without adjusting for additional confounding covariates) are filtered based on FDR p-value (≤ 0.0001). Listed below are the 477 genes that passed the filter.

Table 9: BRAF markers, no covariates

| TCID na30hg19 | GENE | Description | Effect Size (log scale) no covariates | FDR adjusted p-value no covariates |
|---|---|---|---|---|
| 3284596 | PARD3 | par-3 partitioning defective 3 homolog (C. elegans) | 1.11 | 1.34E-05 |
| 3911217 | PMEPA1 | prostate transmembrane protein, androgen induced 1 | 0.47 | 1.34E-05 |
| 2662087 | SRGAP3 | SLIT-ROBO Rho GTPase activating protein 3 | 0.45 | 1.34E-05 |
| 2653114 | NAALADL2 | N-acetylated alpha-linked acidic dipeptidase-like 2 | 0.77 | 1.36E-05 |
| 2590736 | NCKAP1 | NCK-associated protein 1 | 1.49 | 1.36E-05 |
| 3217361 | ANKS6 | ankyrin repeat and sterile alpha motif domain containing 6 | 0.72 | 1.39E-05 |
| 3832280 | C19orf33 | chromosome 19 open reading frame 33 | 1.13 | 1.39E-05 |
| 4045665 | S100A14 | S100 calcium binding protein A14 | 1.41 | 1.39E-05 |
| 3832280 | YIF 1B | Yip1 interacting factor homolog B (S. cerevisiae) | 1.13 | 1.39E-05 |
| 2370123 | XPR1 | xenotropic and polytropic retrovirus receptor | 1.07 | 1.41E-05 |
| 2750594 | SC4MOL | sterol-C4-methyl oxidase-like | 0.90 | 1.42E-05 |
| 3154263 | SLA | Src-like-adaptor | -1.17 | 1.42E-05 |
| 2608469 | ITPR1 | inositol 1,4,5-triphosphate receptor, type 1 | -1.06 | 1.44E-05 |
| 3320944 | TEAD 1 | TEA domain family member 1 (SV40 transcriptional enhancer factor) | 1.34 | 1.44E-05 |
| 3087167 | TUSC3 | tumor suppressor candidate 3 | 1.84 | 1.44E-05 |
| 3335894 | CST6 | cystatin E/M | 2.04 | 1.45E-05 |
| 2610707 | HRH1 | histamine receptor H1 | 0.77 | 1.45E-05 |
| 2617188 | ITGA9 | integrin, alpha 9 | 1.32 | 1.45E-05 |
| 2807359 | OSMR | oncostatin M receptor | 1.49 | 1.45E-05 |
| 2400177 | CAMK2N1 | calcium/calmodulin-dependent protein kinase II inhibitor 1 | 1.76 | 1.48E-05 |
| 3044072 | NOD 1 | nucleotide-binding oligomerization domain containing 1 | 0.97 | 1.51E-05 |
| 2822215 | PAM | peptidylglycine alpha-amidating monooxygenase | 1.38 | 1.51E-05 |
| 2645906 | PLS1 | plastin 1 (I isoform) | 1.03 | 1.51E-05 |
| 2853642 | CSorf42 | chromosome 5 open reading frame 42 | 0.86 | 1.52E-05 |
| 2783099 | TRAM1L1 | translocation associated membrane protein 1-like 1 | 1.12 | 1.52E-05 |
| 2945440 | DCDC2 | doublecortin domain containing 2 | 1.23 | 1.55E-05 |
| 2945440 | KAAG 1 | kidney associated antigen 1 | 1.23 | 1.55E-05 |

(continued)

The results from a LIMMA analysis (without adjusting for additional confounding covariates) are filtered based on FDR p-value (≤ 0.0001). Listed below are the 477 genes that passed the filter.

| Table 9: BRAF markers, no covariates | | | | |
|---|---|---|---|---|
| TCID na30hg19 | GENE | Description | Effect Size (log scale) no covariates | FDR adjusted p-value no covariates |
| 2520138 | MFSD6 | major facilitator superfamily domain containing 6 | 0.65 | 1.57E-05 |
| 3703665 | ZCCHC14 | zinc finger, CCHC domain containing 14 | 0.68 | 1.57E-05 |
| 3048886 | PURB | purine-rich element binding protein B | 0.43 | 1.60E-05 |
| 2734421 | ARHGAP24 | Rho GTPase activating protein 24 | -0.98 | 1.61E-05 |
| 2893794 | DSP | desmoplakin | 1.50 | 1.62E-05 |
| 2820925 | RHOBTB3 | Rho-related BTB domain containing 3 | 1.26 | 1.63E-05 |
| 3159483 | KANK1 | KN motif and ankyrin repeat domains 1 | 0.53 | 1.64E-05 |
| 3159483 | LOC100133062 | similar to Uncharacterized protein C6orf146 | 0.53 | 1.64E-05 |
| 2816298 | IQGAP2 | IQ motif containing GTPase activating protein 2 | -1.35 | 1.66E-05 |
| 3020343 | MET | met proto-oncogene (hepatocyte growth factor receptor) | 2.11 | 1.66E-05 |
| 2373336 | CFH | complement factor H | 1.96 | 1.67E-05 |
| 2373336 | CFHR1 | complement factor H-related 1 | 1.96 | 1.67E-05 |
| 2773545 | BTC | betacellulin | 0.94 | 1.70E-05 |
| 2858592 | DEPDC1B | DEP domain containing 1B | 1.20 | 1.89E-05 |
| 3751002 | RAB34 | RAB34, member RAS oncogene family | 0.90 | 1.94E-05 |
| 3717870 | TMEM98 | transmembrane protein 98 | 1.73 | 2.02E-05 |
| 2326327 | CNKSR1 | connector enhancer of kinase suppressor of Ras 1 | 0.47 | 2.03E-05 |
| 3585905 | APBA2 | amyloid beta (A4) precursor protein-binding, family A, member 2 | -0.50 | 2.04E-05 |
| 2819044 | RASA1 | RAS p21 protein activator (GTPase activating protein) 1 | 0.73 | 2.11E-05 |
| 3110395 | RIMS2 | regulating synaptic membrane exocytosis 2 | 1.10 | 2.15E-05 |
| 2451931 | GOLT1A | golgi transport 1 homolog A (S. cerevisiae) | 1.03 | 2.17E-05 |
| 2768654 | OCIAD2 | OCIA domain containing 2 | 0.98 | 2.17E-05 |
| 2872848 | LOX | lysyl oxidase | 1.53 | 2.19E-05 |
| 3321150 | ARNTL | aryl hydrocarbon receptor nuclear translocator-like | 1.17 | 2.22E-05 |
| 3839206 | MYH14 | myosin, heavy chain 14 | 0.39 | 2.26E-05 |
| 2954355 | CUL7 | cullin 7 | 0.39 | 2.29E-05 |
| 2954355 | CUL9 | cullin 9 | 0.39 | 2.29E-05 |
| 2954355 | KLC4 | kinesin light chain 4 | 0.39 | 2.29E-05 |
| 3046197 | ELMO 1 | engulfment and cell motility 1 | -1.07 | 2.29E-05 |

133

(continued)

The results from a LIMMA analysis (without adjusting for additional confounding covariates) are filtered based on FDR p-value (≤ 0.0001). Listed below are the 477 genes that passed the filter.

| TCID na30hg19 | GENE | Description | Effect Size (log scale) no covariates | FDR adjusted p-value no covariates |
|---|---|---|---|---|
| | | **Table 9: BRAF markers, no covariates** | | |
| 2350596 | CELSR2 | cadherin, EGF LAG seven-pass G-type receptor 2 (flamingo homolog, Drosophila) | 0.38 | 2.30E-05 |
| 3755323 | CISD3 | CDGSH iron sulfur domain 3 | 0.81 | 2.31E-05 |
| 3099566 | FAM110B | family with sequence similarity 110, member B | 0.80 | 2.31E-05 |
| 3755323 | PCGF2 | polycomb group ring finger 2 | 0.81 | 2.31E-05 |
| 2827057 | GRAMD3 | GRAM domain containing 3 | 1.35 | 2.33E-05 |
| 4001223 | RAI2 | retinoic acid induced 2 | 0.64 | 2.33E-05 |
| 3412345 | TMEM 117 | transmembrane protein 117 | 1.04 | 2.33E-05 |
| 2327817 | PTPRU | protein tyrosine phosphatase, receptor type, U | 0.56 | 2.48E-05 |
| 3336486 | C11orf80 | chromosome 11 open reading frame 80 | 0.63 | 2.49E-05 |
| 3336486 | RCE1 | RCE1 homolog, prenyl protein peptidase (S. cerevisiae) | 0.63 | 2.49E-05 |
| 3087501 | ZDHHC2 | zinc finger, DHHC-type containing 2 | 0.77 | 2.49E-05 |
| 2601287 | AP1S3 | adaptor-related protein complex 1, sigma 3 subunit | 0.72 | 2.51E-05 |
| 3238962 | KIAA1217 | KIAA1217 | 1.48 | 2.51E-05 |
| 3238962 | PRINS | psoriasis associated RNA induced by stress (non-protein coding) | 1.48 | 2.51E-05 |
| 2583465 | ITGB6 | integrin, beta 6 | 1.40 | 2.55E-05 |
| 3815116 | PALM | paralemmin | 0.36 | 2.56E-05 |
| 3942350 | MTP18 | mitochondrial protein 18 kDa | 0.69 | 2.63E-05 |
| 3942350 | SEC14L2 | SEC14-like 2 (S. cerevisiae) | 0.69 | 2.63E-05 |
| 3338552 | CTTN | cortactin | 0.91 | 2.81E-05 |
| 3494137 | LMO7 | LIM domain 7 | 1.21 | 2.81E-05 |
| 3188883 | OLFML2A | olfactomedin-like 2A | 0.48 | 2.81E-05 |
| 3463522 | PAWR | PRKC, apoptosis, WT1, regulator | 1.07 | 2.81E-05 |
| 3850457 | AP1M2 | adaptor-related protein complex 1, mu 2 subunit | 1.06 | 2.85E-05 |
| 3062868 | BAIAP2L1 | BAI1-associated protein 2-like 1 | 0.73 | 2.94E-05 |
| 2675171 | HYAL2 | hyaluronoglucosaminidase 2 | 0.72 | 2.94E-05 |
| 2339139 | INADL | InaD-like (Drosophila) | 0.93 | 2.94E-05 |
| 2958670 | RAB23 | RAB23, member RAS oncogene family | 1.22 | 2.94E-05 |
| 3654956 | LAT | linker for activation of T cells | -0.82 | 2.96E-05 |
| 3654956 | LOC100288332 | similar to acyl-CoA synthetase medium-chain family member 2 | -0.82 | 2.96E-05 |

(continued)

The results from a LIMMA analysis (without adjusting for additional confounding covariates) are filtered based on FDR p-value (≤ 0.0001). Listed below are the 477 genes that passed the filter.

| Table 9: BRAF markers, no covariates | | | | |
|---|---|---|---|---|
| TCID na30hg19 | GENE | Description | Effect Size (log scale) no covariates | FDR adjusted p-value no covariates |
| 3654956 | LOC100288442 | hypothetical LOC100288442 | -0.82 | 2.96E-05 |
| 3654956 | LOC100289169 | hypothetical protein LOC100289169 | -0.82 | 2.96E-05 |
| 3654956 | LOC728734 | similar to NPIP-like protein ENSP00000283050 | -0.82 | 2.96E-05 |
| 3654956 | LOC728741 | hypothetical LOC728741 | -0.82 | 2.96E-05 |
| 3654956 | LOC728888 | similar to acyl-CoA synthetase medium-chain family member 2 | -0.82 | 2.96E-05 |
| 3654956 | LOC729602 | NPIP-like protein ENSP00000283050 | -0.82 | 2.96E-05 |
| 3654956 | LOC730153 | NPIP-like protein ENSP00000346774 | -0.82 | 2.96E-05 |
| 2363248 | LY9 | lymphocyte antigen 9 | -0.83 | 2.96E-05 |
| 3654956 | NPIPL2 | nuclear pore complex interacting protein-like 2 | -0.82 | 2.96E-05 |
| 3654956 | NPIPL3 | nuclear pore complex interacting protein-like 3 | -0.82 | 2.96E-05 |
| 3654956 | SPIN1 | spindlin 1 | -0.82 | 2.96E-05 |
| 3654956 | SPNS1 | spinster homolog 1 (Drosophila) | -0.82 | 2.96E-05 |
| 2781736 | CFI | complement factor I | 1.87 | 2.98E-05 |
| 3922793 | LOC100132338 | hypothetical protein LOC100132338 | 0.69 | 2.99E-05 |
| 3922793 | PDE9A | phosphodiesterase 9A | 0.69 | 2.99E-05 |
| 3459120 | LRIG3 | leucine-rich repeats and immunoglobulin-like domains 3 | 1.46 | 3.06E-05 |
| 2673181 | PLXNB1 | plexin B 1 | 0.38 | 3.07E-05 |
| 3088213 | SH2D4A | SH2 domain containing 4A | 1.32 | 3.10E-05 |
| 2555830 | TMEM17 | transmembrane protein 17 | 1.08 | 3.10E-05 |
| 2329041 | KIAA1522 | KIAA1522 | 0.50 | 3.12E-05 |
| 2455418 | AP3S1 | adaptor-related protein complex 3, sigma 1 subunit | 1.02 | 3.14E-05 |
| 2455418 | LOC643454 | adaptor-related protein complex 3, sigma 1 subunit pseudogene | 1.02 | 3.14E-05 |
| 2455418 | PTPN14 | protein tyrosine phosphatase, non-receptor type 14 | 1.02 | 3.14E-05 |
| 2659039 | MUC20 | mucin 20, cell surface associated | 0.70 | 3.19E-05 |
| 2659039 | SDHA | succinate dehydrogenase complex, subunit A, flavoprotein (Fp) | 0.70 | 3.19E-05 |
| 2659039 | SDHALP1 | succinate dehydrogenase complex, subunit A, flavoprotein pseudogene 1 | 0.70 | 3.19E-05 |
| 2659039 | SDHALP2 | succinate dehydrogenase complex, subunit A, flavoprotein pseudogene 2 | 0.70 | 3.19E-05 |

(continued)

The results from a LIMMA analysis (without adjusting for additional confounding covariates) are filtered based on FDR p-value (≤ 0.0001). Listed below are the 477 genes that passed the filter.

| | | Table 9: BRAF markers, no covariates | | |
|---|---|---|---|---|
| **TCID na30hg19** | **GENE** | **Description** | **Effect Size (log scale) no covariates** | **FDR adjusted p-value no covariates** |
| 2452977 | FAIM3 | Fas apoptotic inhibitory molecule 3 | -1.55 | 3.23E-05 |
| 2751936 | GALNT7 | UDP-N-acetyl-alpha-D-galactosamine: polypeptide N-acetylgalactosaminyltransferase 7 (GalNAc-T7) | 0.92 | 3.23E-05 |
| 3031573 | GIMAP5 | GTPase, IMAP family member 5 | -1.36 | 3.28E-05 |
| 2342904 | ST6GALNAC5 | ST6 (alpha-N-acetyl-neuraminyl-2,3-beta-galactosyl-1,3)-N-acetylgalactosaminide alpha-2,6-sialyltransferase 5 | 0.45 | 3.28E-05 |
| 2348437 | SNX7 | sorting nexin 7 | 1.00 | 3.29E-05 |
| 2407786 | LOC100130627 | hypothetical LOC100130627 | 0.74 | 3.33E-05 |
| 2407786 | RHBDL2 | rhomboid, veinlet-like 2 (Drosophila) | 0.74 | 3.33E-05 |
| 3630668 | CALML4 | calmodulin-like 4 | -0.79 | 3.52E-05 |
| 2603987 | NGEF | neuronal guanine nucleotide exchange factor | 0.43 | 3.60E-05 |
| 2451870 | ETNK2 | ethanolamine kinase 2 | 1.26 | 3.64E-05 |
| 3535628 | GNG2 | guanine nucleotide binding protein (G protein), gamma 2 | -1.34 | 3.64E-05 |
| 3329343 | MDK | midkine (neurite growth-promoting factor 2) | 1.09 | 3.64E-05 |
| 3464417 | MGAT4C | mannosyl (alpha-1,3-)-glycoprotein beta-1,4-N-acetylglucosaminyltransferase, isozyme C (putative) | 1.60 | 3.64E-05 |
| 3997825 | MXRA5 | matrix-remodelling associated 5 | 1.18 | 3.64E-05 |
| 2378121 | TRAF3IP3 | TRAF3 interacting protein 3 | -1.18 | 3.64E-05 |
| 2325002 | KDM1 | lysine (K)-specific demethylase 1 | 0.59 | 3.65E-05 |
| 2424102 | CNN3 | calponin 3, acidic | 1.48 | 3.69E-05 |
| 3346453 | YAP 1 | Yes-associated protein 1, 65kDa | 0.94 | 3.69E-05 |
| 2951500 | TEAD3 | TEA domain family member 3 | 0.56 | 3.88E-05 |
| 3067478 | NRCAM | neuronal cell adhesion molecule | 1.55 | 4.09E-05 |
| 2649113 | LOC100287227 | hypothetical LOC100287227 | 0.92 | 4.16E-05 |
| 2649113 | TIP ARP | TCDD-inducible poly(ADP-ribose) polymerase | 0.92 | 4.16E-05 |
| 3753860 | CCL5 | chemokine (C-C motif) ligand 5 | -1.22 | 4.23E-05 |
| 2986825 | C7orf20 | chromosome 7 open reading frame 20 | 0.61 | 4.45E-05 |
| 2397025 | DHRS3 | dehydrogenase/reductase (SDR family) member 3 | 1.18 | 4.45E-05 |
| 3759587 | LOC100129115 | hypothetical protein LOC100129115 | 0.55 | 4.45E-05 |
| 3842264 | NAT 14 | N-acetyltransferase 14 (GCN5-related, putative) | 0.30 | 4.45E-05 |

(continued)

The results from a LIMMA analysis (without adjusting for additional confounding covariates) are filtered based on FDR p-value (≤ 0.0001). Listed below are the 477 genes that passed the filter.

| TCID na30hg19 | GENE | Description | Effect Size (log scale) no covariates | FDR adjusted p-value no covariates |
|---|---|---|---|---|
| | | **Table 9: BRAF markers, no covariates** | | |
| 3759587 | PLCD3 | phospholipase C, delta 3 | 0.55 | 4.45E-05 |
| 2986825 | UNC84A | unc-84 homolog A (C. elegans) | 0.61 | 4.45E-05 |
| 3092415 | LOC100129846 | hypothetical protein LOC100129846 | 1.07 | 4.52E-05 |
| 3092415 | RBPMS | RNA binding protein with multiple splicing | 1.07 | 4.52E-05 |
| 3092415 | SDHALP2 | succinate dehydrogenase complex, subunit A, flavoprotein pseudogene 2 | 1.07 | 4.52E-05 |
| 2523689 | ABI2 | abl-interactor 2 | 0.90 | 4.52E-05 |
| 3518086 | TBC1D4 | TBC1 domain family, member 4 | -0.54 | 4.58E-05 |
| 2708610 | MAGEF1 | melanoma antigen family F, 1 | 0.55 | 4.61E-05 |
| 2656146 | MAP3K13 | mitogen-activated protein kinase kinase kinase 13 | 0.93 | 4.70E-05 |
| 3107342 | PDP1 | pyruvate dehyrogenase phosphatase catalytic subunit 1 | 0.70 | 4.70E-05 |
| 3720402 | ERBB2 | v-erb-b2 erythroblastic leukemia viral oncogene homolog 2, neuro/glioblastoma derived oncogene homolog (avian) | 0.75 | 4.72E-05 |
| 3415320 | KRT7 | keratin 7 | 1.12 | 4.72E-05 |
| 3389273 | CASP4 | caspase 4, apoptosis-related cysteine peptidase | -1.32 | 4.73E-05 |
| 2458338 | ENAH | enabled homolog (Drosophila) | 1.21 | 4.73E-05 |
| 3104323 | FAM164A | family with sequence similarity 164, member A | 1.17 | 4.73E-05 |
| 3389273 | LOC643733 | hypothetical LOC643733 | -1.32 | 4.73E-05 |
| 3219621 | CTNNAL1 | catenin (cadherin-associated protein), alpha-like 1 | 1.29 | 4.77E-05 |
| 3361381 | CYB5R2 | cytochrome b5 reductase 2 | 0.62 | 4.77E-05 |
| 3610804 | IGF1R | insulin-like growth factor 1 receptor | 0.78 | 4.77E-05 |
| 3113180 | MAL2 | mal, T-cell differentiation protein 2 | 1.41 | 4.77E-05 |
| 2721959 | ROS1 | c-ros oncogene 1 , receptor tyrosine kinase | 2.41 | 4.77E-05 |
| 2721959 | SLC34A2 | solute carrier family 34 (sodium phosphate), member 2 | 2.41 | 4.77E-05 |
| 2611122 | TSEN2 | tRNA splicing endonuclease 2 homolog (S. cerevisiae) | 0.44 | 4.77E-05 |
| 3876245 | SNAP25 | synaptosomal-associated protein, 25kDa | 0.54 | 4.79E-05 |
| 2420832 | DDAH1 | dimethylarginine dimethylaminohydrolase 1 | 1.50 | 4.80E-05 |
| 3784344 | MAPRE2 | microtubule-associated protein, RP/EB family, member 2 | -0.75 | 4.80E-05 |
| 3495076 | NDFIP2 | Nedd4 family interacting protein 2 | 1.01 | 4.80E-05 |

(continued)

The results from a LIMMA analysis (without adjusting for additional confounding covariates) are filtered based on FDR p-value (≤ 0.0001). Listed below are the 477 genes that passed the filter.

**Table 9: BRAF markers, no covariates**

| TCID na30hg19 | GENE | Description | Effect Size (log scale) no covariates | FDR adjusted p-value no covariates |
|---|---|---|---|---|
| 2871896 | CDO1 | cysteine dioxygenase, type I | 1.14 | 4.82E-05 |
| 3818547 | VAV1 | vav 1 guanine nucleotide exchange factor | -1.08 | 4.85E-05 |
| 2417272 | GNG12 | guanine nucleotide binding protein (G protein), gamma 12 | 1.45 | 4.85E-05 |
| 3417809 | NAB2 | NGFI-A binding protein 2 (EGR1 binding protein 2) | 0.56 | 4.85E-05 |
| 2673873 | IMPDH2 | IMP (inosine monophosphate) dehydrogenase 2 | 0.61 | 4.92E-05 |
| 2948790 | CDSN | comeodesmosin | 0.78 | 4.97E-05 |
| 2615892 | CMTM8 | CKLF-like MARVEL transmembrane domain containing 8 | 0.70 | 4.97E-05 |
| 3780981 | KIAA 1772 | KIAA1772 | 0.72 | 4.97E-05 |
| 2371065 | LAMC 1 | laminin, gamma 1 (formerly LAMB2) | 1.14 | 4.97E-05 |
| 3765689 | LOC100129112 | hypothetical protein LOC100129112 | 0.64 | 4.97E-05 |
| 3765689 | MED 13 | mediator complex subunit 13 | 0.64 | 4.97E-05 |
| 3355733 | EWSR1 | Ewing sarcoma breakpoint region 1 | -1.26 | 5.09E-05 |
| 3355733 | FLI1 | Friend leukemia virus integration 1 | -1.26 | 5.09E-05 |
| 2402517 | SLC30A2 | solute carrier family 30 (zinc transporter), member 2 | 0.62 | 5.16E-05 |
| 2924330 | TPD52L1 | tumor protein D52-like 1 | 1.42 | 5.16E-05 |
| 2870964 | EPB41L4A | erythrocyte membrane protein band 4.1 like 4A | 1.05 | 5.18E-05 |
| 3564919 | FERMT2 | fermitin family homolog 2 (Drosophila) | 1.19 | 5.18E-05 |
| 2519229 | ITGAV | integrin, alpha V (vitronectin receptor, alpha polypeptide, antigen CD51) | 1.19 | 5.18E-05 |
| 2435218 | TDRKH | tudor and KH domain containing | 0.92 | 5.19E-05 |
| 2361257 | RAB25 | RAB25, member RAS oncogene family | 1.44 | 5.22E-05 |
| 2347132 | FNBP1L | formin binding protein 1-like | 1.28 | 5.27E-05 |
| 3175494 | GCNT1 | glucosaminyl (N-acetyl) transferase 1, core 2 (beta-1,6-N-acetylglucosaminyltransferase) | 0.75 | 5.31E-05 |
| 3326461 | EHF | ets homologous factor | 1.32 | 5.38E-05 |
| 3638204 | MFGE8 | milk fat globule-EGF factor 8 protein | 1.49 | 5.38E-05 |
| 3638204 | QTRT1 | queuine tRNA-ribosyltransferase 1 | 1.49 | 5.38E-05 |
| 3267382 | INPP5F | inositol polyphosphate-5-phosphatase F | 0.84 | 5.41E-05 |
| 3471327 | HVCN1 | hydrogen voltage-gated channel 1 | -0.91 | 5.41E-05 |
| 2580802 | RND3 | Rho family GTPase 3 | 1.53 | 5.41E-05 |
| 4024685 | SLITRK4 | SLIT and NTRK-like family, member 4 | 0.98 | 5.41E-05 |

(continued)

| The results from a LIMMA analysis (without adjusting for additional confounding covariates) are filtered based on FDR p-value (≤ 0.0001). Listed below are the 477 genes that passed the filter. | | | | |
| --- | --- | --- | --- | --- |
| **Table 9: BRAF markers, no covariates** | | | | |
| **TCID na30hg19** | **GENE** | **Description** | **Effect Size (log scale) no covariates** | **FDR adjusted p-value no covariates** |
| 3471327 | TCTN1 | tectonic family member 1 | -0.91 | 5.41E-05 |
| 3456805 | GTSF1 | gametocyte specific factor 1 | -1.37 | 5.52E-05 |
| 2881607 | LOC134466 | zinc finger protein 300 pseudogene | 0.88 | 5.52E-05 |
| 3424442 | TMTC2 | transmembrane and tetratricopeptide repeat containing 2 | 0.49 | 5.52E-05 |
| 2881607 | ZNF300 | zinc finger protein 300 | 0.88 | 5.52E-05 |
| 3842675 | LOC283788 | FSHD region gene 1 pseudogene | 0.67 | 5.54E-05 |
| 3211938 | RASEF | RAS and EF-hand domain containing | 1.38 | 5.54E-05 |
| 3842675 | ZNF542 | zinc finger protein 542 | 0.67 | 5.54E-05 |
| 2364189 | UAP1 | UDP-N-acteylglucosamine pyrophosphorylase 1 | 0.83 | 5.56E-05 |
| 3656223 | ITGAL | integrin, alpha L (antigen CD11A (p180), lymphocyte function-associated antigen 1; alpha polypeptide) | -1.04 | 5.59E-05 |
| 4024420 | CXorf18 | chromosome X open reading frame 18 | 1.13 | 5.64E-05 |
| 4024420 | LDOC1 | leucine zipper, down-regulated in cancer 1 | 1.13 | 5.64E-05 |
| 3397877 | RICS | Rho GTPase-activating protein | 0.56 | 5.73E-05 |
| 3577612 | SERPINA 1 | serpin peptidase inhibitor, clade A (alpha-1 antiproteinase, antitrypsin), member 1 | 0.70 | 5.73E-05 |
| 3577612 | SERPINA2 | serpin peptidase inhibitor, clade A (alpha-1 antiproteinase, antitrypsin), member 2 | 0.70 | 5.73E-05 |
| 4013018 | ZDHHC15 | zinc finger, DHHC-type containing 15 | 0.65 | 5.88E-05 |
| 2622912 | MAPKAPK3 | mitogen-activated protein kinase-activated protein kinase 3 | 0.59 | 5.90E-05 |
| 2337716 | PRKAA2 | protein kinase, AMP-activated, alpha 2 catalytic subunit | 1.29 | 5.91E-05 |
| 3070712 | WASL | Wiskott-Aldrich syndrome-like | 0.72 | 5.91E-05 |
| 2524016 | PARD3B | par-3 partitioning defective 3 homolog B (C. elegans) | 0.52 | 6.14E-05 |
| 3547696 | TTC8 | tetratricopeptide repeat domain 8 | 0.71 | 6.14E-05 |
| 2358993 | TUFT1 | tuftelin 1 | 0.46 | 6.14E-05 |
| 3710870 | RICH2 | Rho-type GTPase-activating protein RICH2 | 0.64 | 6.21E-05 |
| 3959350 | APOL3 | apolipoprotein L, 3 | -0.62 | 6.37E-05 |
| 3407096 | PLEKHA5 | pleckstrin homology domain containing, family A member 5 | 1.09 | 6.37E-05 |
| 3497195 | CLDN10 | claudin 10 | 1.15 | 6.39E-05 |

(continued)

The results from a LIMMA analysis (without adjusting for additional confounding covariates) are filtered based on FDR p-value (≤ 0.0001). Listed below are the 477 genes that passed the filter.

| | | Table 9: BRAF markers, no covariates | | |
|---|---|---|---|---|
| **TCID na30hg19** | **GENE** | **Description** | **Effect Size (log scale) no covariates** | **FDR adjusted p-value no covariates** |
| 3497195 | DZIP1 | DAZ interacting protein 1 | 1.15 | 6.39E-05 |
| 3696142 | DPEP2 | dipeptidase 2 | -1.07 | 6.50E-05 |
| 2792127 | NPY1R | neuropeptide Y receptor Y 1 | 1.31 | 6.50E-05 |
| 3615579 | TJP 1 | tight junction protein 1 (zona occludens 1) | 1.28 | 6.50E-05 |
| 3409211 | PPFIBP 1 | PTPRF interacting protein, binding protein 1 (liprin beta 1) | 1.04 | 6.53E-05 |
| 2949038 | ATP6V1G2 | ATPase, H+ transporting, lysosomal 13kDa, V1 subunit G2 | 0.30 | 6.57E-05 |
| 2949038 | BAT1 | HLA-B associated transcript 1 | 0.30 | 6.57E-05 |
| 3838385 | CD37 | CD37 molecule | -1.41 | 6.57E-05 |
| 2949038 | SNORD117 | small nucleolar RNA, C/D box 117 | 0.30 | 6.57E-05 |
| 2949038 | SNORD84 | small nucleolar RNA, C/D box 84 | 0.30 | 6.57E-05 |
| 3752709 | MYO1D | myosin ID | 1.02 | 6.67E-05 |
| 3031466 | GIMAP8 | GTPase, IMAP family member 8 | -0.91 | 6.77E-05 |
| 3031466 | LOC285972 | hypothetical protein LOC285972 | -0.91 | 6.77E-05 |
| 2962026 | LCA5 | Leber congenital amaurosis 5 | 1.42 | 6.90E-05 |
| 3357397 | GLB1L2 | galactosidase, beta 1-like 2 | 0.81 | 6.93E-05 |
| 3795184 | LOC100127994 | hypothetical protein LOC 100127994 | -0.35 | 6.93E-05 |
| 3795184 | NFATC1 | nuclear factor of activated T-cells, cytoplasmic, calcineurin-dependent 1 | -0.35 | 6.93E-05 |
| 3670918 | PLCG2 | phospholipase C, gamma 2 (phosphatidylinositol-specific) | -0.98 | 6.93E-05 |
| 3648306 | SNN | stannin | -0.40 | 6.93E-05 |
| 3648306 | TXNDC11 | thioredoxin domain containing 11 | -0.40 | 6.93E-05 |
| 2769346 | FIP1L1 | FIP1 like 1 (S. cerevisiae) | 0.75 | 6.94E-05 |
| 2769346 | LNX1 | ligand of numb-protein X 1 | 0.75 | 6.94E-05 |
| 3445786 | ARHGDIB | Rho GDP dissociation inhibitor (GDI) beta | -0.60 | 7.00E-05 |
| 2673830 | DALRD3 | DALR anticodon binding domain containing 3 | 0.28 | 7.24E-05 |
| 3870533 | TMC4 | transmembrane channel-like 4 | 0.72 | 7.24E-05 |
| 2673830 | WDR6 | WD repeat domain 6 | 0.28 | 7.24E-05 |
| 3871935 | ZNF667 | zinc finger protein 667 | 0.72 | 7.24E-05 |
| 3457891 | GLS2 | glutaminase 2 (liver, mitochondrial) | 0.35 | 7.26E-05 |
| 2991233 | AHR | aryl hydrocarbon receptor | 0.88 | 7.27E-05 |
| 3624513 | LOC 100 129973 | hypothetical protein LOC 100129973 | 1.10 | 7.29E-05 |

(continued)

The results from a LIMMA analysis (without adjusting for additional confounding covariates) are filtered based on FDR p-value (≤ 0.0001). Listed below are the 477 genes that passed the filter.

| TCID na30hg19 | GENE | Description | Effect Size (log scale) no covariates | FDR adjusted p-value no covariates |
|---|---|---|---|---|
| 3624513 | MYOSC | myosin VC | 1.10 | 7.29E-05 |
| 3294576 | USP54 | ubiquitin specific peptidase 54 | 0.81 | 7.35E-05 |
| 3345427 | ENDOD1 | endonuclease domain containing 1 | 0.61 | 7.47E-05 |
| 2438458 | CRABP2 | cellular retinoic acid binding protein 2 | 1.43 | 7.51E-05 |
| 2827645 | SLC27A6 | solute carrier family 27 (fatty acid transporter), member 6 | 2.18 | 7.66E-05 |
| 3307939 | ABLIM1 | actin binding LIM protein 1 | 0.68 | 7.68E-05 |
| 3151607 | FBXO32 | F-box protein 32 | 0.80 | 7.68E-05 |
| 3450234 | PKP2 | plakophilin 2 | 0.71 | 7.74E-05 |
| 2469157 | GRHL1 | grainyhead-like 1 (Drosophila) | 0.55 | 7.74E-05 |
| 3781124 | MIB1 | mindbomb homolog 1 (Drosophila) | 0.59 | 7.74E-05 |
| 3279982 | PTPLA | protein tyrosine phosphatase-like (proline instead of catalytic arginine), member A | 0.85 | 7.74E-05 |
| 3097152 | MCM4 | minichromosome maintenance complex component 4 | 0.74 | 7.83E-05 |
| 3289235 | SGMS 1 | sphingomyelin synthase 1 | 0.70 | 7.87E-05 |
| 3107548 | ESRP1 | epithelial splicing regulatory protein 1 | 1.52 | 7.92E-05 |
| 2839543 | WWC1 | WW and C2 domain containing 1 | 0.63 | 7.92E-05 |
| 3493543 | KLF5 | Kruppel-like factor 5 (intestinal) | 0.54 | 7.99E-05 |
| 3868998 | NKG7 | natural killer cell group 7 sequence | -1.29 | 7.99E-05 |
| 2706297 | TBL1XR1 | transducin (beta)-like 1 X-linked receptor 1 | 0.58 | 8.17E-05 |
| 2966193 | C6orf168 | chromosome 6 open reading frame 168 | 0.92 | 8.19E-05 |
| 2914070 | MYO6 | myosin VI | 1.35 | 8.19E-05 |
| 3394660 | TRIM29 | tripartite motif-containing 29 | 0.51 | 8.26E-05 |
| 2598261 | FN1 | fibronectin 1 | 1.52 | 8.35E-05 |
| 3420713 | CAND1 | cullin-associated and neddylation-dissociated 1 | 0.62 | 8.36E-05 |
| 3227574 | FAM78A | family with sequence similarity 78, member A | -0.89 | 8.37E-05 |
| 2720584 | SLIT2 | slit homolog 2 (Drosophila) | 1.52 | 8.41E-05 |
| 2700585 | PFN2 | profilin 2 | 1.39 | 8.48E-05 |
| 3143643 | MMP16 | matrix metallopeptidase 16 (membrane-inserted) | 1.58 | 8.56E-05 |
| 3610958 | IGF1R | insulin-like growth factor 1 receptor | 1.03 | 8.64E-05 |
| 2462160 | NID 1 | nidogen 1 | 0.50 | 8.64E-05 |
| 3622934 | MYEF2 | myelin expression factor 2 | 0.91 | 8.65E-05 |

(continued)

The results from a LIMMA analysis (without adjusting for additional confounding covariates) are filtered based on FDR p-value (≤ 0.0001). Listed below are the 477 genes that passed the filter.

| Table 9: BRAF markers, no covariates | | | | |
|---|---|---|---|---|
| TCID na30hg19 | GENE | Description | Effect Size (log scale) no covariates | FDR adjusted p-value no covariates |
| 3622934 | SLC24A5 | solute carrier family 24, member 5 | 0.91 | 8.65E-05 |
| 2600689 | EPHA4 | EPH receptor A4 | 1.47 | 8.67E-05 |
| 2380055 | KCTD3 | potassium channel tetramerisation domain containing 3 | 0.93 | 8.67E-05 |
| 2927255 | PEX7 | peroxisomal biogenesis factor 7 | 0.62 | 8.67E-05 |
| 3645555 | TNFRSF12A | tumor necrosis factor receptor superfamily, member 12A | 1.24 | 8.67E-05 |
| 2960955 | SLC17A5 | solute carrier family 17 (anion/sugar transporter), member 5 | 0.97 | 8.76E-05 |
| 3753568 | SLFN11 | schlafen family member 11 | 0.85 | 8.81E-05 |
| 3753568 | SLFN13 | schlafen family member 13 | 0.85 | 8.81E-05 |
| 2377229 | CD55 | CD55 molecule, decay accelerating factor for complement (Cromer blood group) | 0.68 | 8.89E-05 |
| | | | 0.44 | 8.94E-05 |
| 2829542 | C5orf24 | chromosome 5 open reading frame 24 | 0.64 | 9.06E-05 |
| 3319937 | WEE 1 | WEE1 homolog (S. pombe) | 0.70 | 9.06E-05 |
| 2582701 | CCDC148 | coiled-coil domain containing 148 | 1.43 | 9.16E-05 |
| 3079103 | GIMAP6 | GTPase, IMAP family member 6 | -0.84 | 9.16E-05 |
| 2820394 | NR2F1 | nuclear receptor subfamily 2, group F, member 1 | 0.32 | 9.16E-05 |
| 2420521 | SSX2IP | synovial sarcoma, X breakpoint 2 interacting protein | 0.56 | 9.16E-05 |
| 3025545 | CALD1 | caldesmon 1 | 1.03 | 9.20E-05 |
| 3604287 | IL 16 | interleukin 16 (lymphocyte chemoattractant factor) | -0.54 | 9.40E-05 |
| 3402506 | CD27 | CD27 molecule | -0.93 | 9.41E-05 |
| 3621728 | FRMD5 | FERM domain containing 5 | 0.79 | 9.41E-05 |
| 3621728 | hCG_1789710 | protein (peptidylprolyl cis/trans isomerase) NIMA-interacting, 4 (parvulin) pseudogene | 0.79 | 9.41E-05 |
| 3402506 | LOC678655 | hypothetical locus LOC678655 | -0.93 | 9.41E-05 |
| 3621728 | PIN4 | protein (peptidylprolyl cis/trans isomerase) NIMA-interacting, 4 (parvulin) | 0.79 | 9.41E-05 |
| 2338625 | HOOK1 | hook homolog 1 (Drosophila) | 1.15 | 9.42E-05 |
| 2523419 | ALS2CR8 | amyotrophic lateral sclerosis 2 (juvenile) chromosome region, candidate 8 | 0.61 | 9.43E-05 |
| 2900195 | ZNF165 | zinc finger protein 165 | 0.48 | 9.55E-05 |
| 3569754 | ZFP36L1 | zinc finger protein 36, C3H type-like 1 | 0.38 | 9.61E-05 |

(continued)

| | | | Effect Size (log scale) no covariates | FDR adjusted p-value no covariates |
|---|---|---|---|---|
| | The results from a LIMMA analysis (without adjusting for additional confounding covariates) are filtered based on FDR p-value (≤ 0.0001). Listed below are the 477 genes that passed the filter. |

**Table 9: BRAF markers, no covariates**

| TCID na30hg19 | GENE | Description | Effect Size (log scale) no covariates | FDR adjusted p-value no covariates |
|---|---|---|---|---|
| 2975385 | AHI1 | Abelson helper integration site 1 | 0.75 | 9.62E-05 |
| 3925639 | NRIP 1 | nuclear receptor interacting protein 1 | 0.82 | 9.63E-05 |
| 3301914 | PIK3AP1 | phosphoinositide-3-kinase adaptor protein 1 | -1.01 | 9.63E-05 |
| 3959953 | TMPRSS6 | transmembrane protease, serine 6 | 0.34 | 9.67E-05 |
| 4015397 | TSPAN6 | tetraspanin 6 | 1.43 | 9.67E-05 |

**Example 9: Primer Mixing: An Example of Implementation of 3'-5'-Amplification Bias Normalization**

Gene signal intensities in microarray assays may vary when identical RNA samples are run in temporally separated experiments using different reagents lots (Figure 29). In this example, an experiment is performed to observe 3' end amplification bias and apply a normalization procedure to correct for the bias.

In this example, two distinct technical factors contribute to 3' end bias including lot-to-lot variation during whole transcriptome RNA amplification (WTA) and lot-to-lot variation of the microarray chips used. Lot-to-lot differences in WTA amplification are directly observed when one signals measured across the entire length of all transcripts are observed. There is a distinct 3-prime transcript signal bias (increased signal) that can be largely traced to differential amplification within the length of the transcript due to variation in poly-dT and randomer priming activity of the WTA kit (Figure 30). Swapping primer mixes between two kits that produced very distinct 3-prime bias patterns providing evidence for the cause of this bias (Figure 31). While poly-dT primers may account for most of the observed variation, other complex interactions or factors involving all raw materials may contribute to measurable 3-prime bias. These intereations or factors may occur at any time, including before, during and after enzymatic reactions.

**Example 10: Detection of BRAF V600E Mutations in a Consecutive Cohort of 7,066 Thyroid Nodule - Fine Needle Aspirate Biopsies (FNABs) Using High-Dimensional RNA Expression Data**

BRAF V600E status may be assessed using DNA-based methods but immunohistochemical (IHC) staining-based approaches have also been developed. Interpretation of these stains may be qualitative and demonstrated to have imperfect inter-observer agreement and a high rate of indeterminate stain intensity. Gene expression signatures have been used to predict the presence or absence of point mutations or rearrangements in DNA in several cancers. A gene expression signature detecting BRAF V600E in a small cohort of PTC nodules is reported. The analytical and clinical validity of a gene expression signature in accurately classifying BRAF V600E mutation status in thyroid nodules is shown below.

Materials and Methods

FNABs were obtained prospectively from 716 patients as either part of a collection (n=360, VERA001) or from de-identified samples consecutively referred to the Veracyte CLIA-certified clinical laboratory for GEC testing (n=356, CLIA). Each patient had a slide prepared from an FNAB and read by a cytopathologist. A second FNAB for molecular testing was collected from the same nodule. RNA and DNA from FNABs were extracted. Total RNA was amplified, hybridized to a custom microarray, and gene expression measured.

A Competitive Allele-Specific TaqMan PCR (castPCT) assay specific to the BRAF thymine to adenine (T > A) transversion at nucleotide 1799 (V600E) was used to determine the percent mutation (% MUT) of BRAF V600E present in each DNA sample as previously reported. Classifier training labels were assigned such that samples with % MUT greater than 2.5% were labeled BRAF V600E-positive (BRAF-positive) and samples with % MUT of 2.5% or less were labeled BRAF V600E-negative (BRAF-negative). This threshold for the analytical sensitivity of the castPCR assay in FNAB-derived thyroid DNA was established previously and is implemented here to minimize unreliable training class labels due to stochastic (i.e. random) effects on amplification in low copy-number samples.

Classifier training and validation

All BRAF-positive Bethesda V and BRAF-negative Bethesda VI samples were randomly assigned to either the classifier training set or to the independent test set (Table 20) and an equivalent number of the more numerous BRAF-negative Bethesda V and BRAF-positive Bethesda VI samples were randomly selected into the respective sets to ensure cytology class-specific representation in both training and test performance evaluation. All BRAF-positive Bethesda III/IV nodules were randomly divided equally between training and test sets. Within Bethesda V and VI, patient age and gender, nodule size, cytology sub-type (PTC, etc.) and % MUT were evaluated after randomization to ensure homogeneity between training and test sets. Investigators responsible for test set scoring were not involved in randomization and were blind to test set castPCR results until after test set scoring.

Training of the Afirma BRAF RNA classifier was carried out using Robust Multichip Average (RMA) normalized transcript cluster-level gene expression summaries and 10-fold cross-validation (CV) across a variety of classification methods and gene counts. Gene selection occurred within each CV loop via limma to identify genes distinguishing BRAF-positive from BRAF-negative samples. Classifiers were evaluated for positive- (PPA) and negative percent agreement (NPA) with castPCR-derived training set labels. PPA and NPA are utilized when a surrogate comparison is made to results from a second test (in this case, castPCR) in lieu of a clinical reference standard (see Supplement). The highest scoring classification method and gene set were then used in a final round of training with all 181 training samples resulting in the Afirma BRAF RNA classifier.

The Afirma BRAF decision threshold was then adjusted via variability-based simulation to minimize the probability of test set false positives (Figure 32). The classifier and adjusted decision threshold were locked prior to scoring the test set and evaluating performance against castPCR. To strike a balance between assay analytical sensitivity and clinical relevance of predictions, the PPA and NPA of Afirma BRAF calls were evaluated with castPCR at % MUT thresholds ranging from 0% to 10%. Analytical verification studies characterized the accuracy, reproducibility (inter-laboratory and inter- and intra-run), and robustness of the Afirma BRAF classifier. For a subset (n=213) of FNABs in the test set for which GEC and castPCR results were previouslyreported and for which expert-derived histopathology was available, the histopathology was used to evaluate the clinical sensitivity and specificity of both Afirma BRAF and castPCR to detect malignancy in thyroid nodules via detection of the BRAF V600E mutation and associated gene expression signature.

Afirma BRAF was additionally used to predict the presence of V600E in a large (n=7,248) cohort of de-identified CLIA-derived FNABs consecutively reflexed to the Afirma GEC. Of these samples, 32 were removed from further consideration due to unsatisfactory cytology. 51 additional FNABs were removed after triggering Afirma GEC "cassettes" filtering out rare neoplasms. 93 FNABs were removed due to benign cytology as these samples are outside indication for both Afirma BRAF and Afirma GEC due to low prevalence of disease. This left 7,066 FNABs available for further study. For these samples, neither castPCR results nor histopathological truth was available but Afirma GEC test results were known. Results from these two tests were evaluated by Bethesda cytology category as well as by the source of cytology, i.e. from Thyroid Cytopathology Partners (TCP, n=4,824), or from a collection of mostly academic institutions each performing cytology on-site (Afirma-Enabled, A/E, n=2,242). Over/under-representation analyses (ORA) were performed using GeneTrail software with either Afirma BRAF genes or all genes differentially expressed between BRAF-negative and -positive samples (n=2,502, false discovery rate (FDR) < 0.1 by limma) as the ORA test sets. The ORA reference set included all human genes (n=44,829) and annotation in the KEGG pathways database (27). Significance was evaluated via Fisher's exact test with a corrected FDR threshold of p < 0.05.

Results: Classifier comparison to castPCR

We computed PPA and NPA under 10-fold CV (using the training set) and found that 128 transcripts (from 127 genes, Table S1) in a linear support vector machine (28) (SVM) maximized the area under the receiver-operator characteristic (ROC) curve (AUC) while minimizing run-to-run score variability (Figure 33). The locked Afirma BRAF classifier (and associated decision threshold) was then used to score the test set, and agreement between Afirma BRAF and castPCR was assessed across a range of castPCR label thresholds. Maximal PPA and NPA for all cytology categories were observed when the threshold for BRAF-positive status was ≥5% MUT (Figure 34, Figure 35). This result may be interpreted as demonstrating the effective analytical sensitivity of Afirma BRAF to be equivalent to 5% MUT by castPCR. This 5% threshold represents a conservative lower bound on the analytical sensitivity of Afirma BRAF given that no Afirma BRAF-positive samples were identified with castPCR %MUT values less than 5%, with the exception of the false positives (0% MUT) discussed below. At 5% analytical sensitivity, Afirma BRAF demonstrates a PPA with castPCR of 90.4% (95% exact binomial confidence interval [CI] 83.5-95.1%) and an NPA of 99% (95% CI 97.6-99.7%) (Table 21).

NPA was not significantly different across cytology categories but PPA was lower (approaching significance, p=0.059) in Bethesda V samples. Neither PPA nor NPA was significantly different between training and test sets overall or within each cytology category. Two samples in the training set and four in the test set (n=535) were identified that were Afirma BRAF positive but unambiguously 0% MUT by castPCR. This disagreement may have been due to technical variability in either assay (Figure 36, 37) or can be due to mutations other than the V600E mutation that cause similar gene expression changes. These samples were evaluated via deep, targeted DNA sequencing of the BRAF gene along with several other true BRAF-positive and BRAF-negative samples to serve as controls. One of these six discrepant samples was identified to have a double mutation at nucleotide positions 1798-1799, leading to the same valine to glutamate amino acid change found in the most common BRAF mutation. No BRAF mutations were identified in the other five discrepant samples.

## Clinical performance

We assessed the diagnostic value of BRAF V600E status for evaluation of nodules with Bethesda III-VI cytopathology using a subset of samples with associated gold-standard histopathology. Expert pathologists were unaware of the molecular results. Both Afirma BRAF and castPCR called all histopathologically benign samples as BRAF V600E-negative (specificity 100%, 95% CI 97.4%-100%), recapitulating the previously reported high specificity of the BRAF V600E mutation.

While both Afirma BRAF and castPCR identified 32 malignant samples as BRAF-positive, two samples called BRAF-positive by castPCR (with 4.2% and 20.2% MUT detected) were Afirma BRAF-negative. Two additional samples were called positive by Afirma BRAF but showed 0% MUT by castPCR. All four of these samples were malignant by histopathology.

## Afirma BRAF and Afirma GEC Results by Cytology

Afirma BRAF and Afirma GEC test results were evaluated on 7,066 de-identified FNABs from patients consecutively referred to the Veracyte CLIA laboratory for Afirma GEC testing. In 3,187 samples benign by Afirma GEC, none were Afirma BRAF positive (NPA 100%, 95% CI 99.9%-100%). In addition, while excluded from formal analysis, none of the cytologically benign nodules were called positive by Afirma BRAF. Afirma BRAF-positive call rates in Afirma GEC suspicious nodules varied by cytology category and were significantly higher in Bethesda III samples compared to Bethesda IV samples (2.4% versus 0.5%, p=0.004). In 4,809 Bethesda III and IV FNABs, an Afirma BRAF positive call rate of 1% (95% CI 0.8%-1.4%) was observed, while in the 2,684 (56%) of these FNABs called suspicious by the Afirma GEC, an Afirma BRAF positive call rate of 1.9% (95% CI 1.4%-2.5%) was observed, a statistically significant increase (p=0.004). The proportion of Afirma BRAF-positive nodules did not differ significantly (p=0.22) between cytology performed by TCP (55 of 4,824, 1.1%) and cytology performed by a collection of mostly academic Afirma-enabled institutions (34 of 2,242, 1.5%).

## Reproducibility & robustness to dilution

Intra- and inter-run reproducibility of the classifier was evaluated using 9 FNABs and three tissue controls selected from among training samples with high (BRAF-positive) or low (BRAF-negative) classifier scores and scores near the classifier decision boundary. Each FNAB and tissue was processed from total RNA in triplicate in each of three different runs across days, operators and reagent lots. The intra-assay standard deviation (SD) of Afirma BRAF scores is 0.171 (95% CI 0.146-0.204). Of the 106 Afirma BRAF calls produced, 106 resulted in concordant calls across all three runs (100% concordance). The inter-assay SD of scores is 0.204 (95% CI 0.178-0.237) for scores measured on a six point scale (Figure 37). FNABs often contain lymphocytes, blood or benign thyroid tissue that may interfere with or dilute BRAF-positive cells. To evaluate the impact of this dilution on Afirma BRAF signal, an Afirma BRAF-positive PTC sample was mixed in silico (using a previously reported mixture model) with increasing proportions of diluent samples. These in silico mixtures included dilution with samples of lymphocytic thyroiditis (LCT), pure blood, or benign thyroid tissue. BRAF-positive samples were called correctly at least 80% of the time in mixtures representing 36%, 38% and 42% BRAF-positive PTC content, respectively. Afirma BRAF results for the pure blood, LCT and benign thyroid tissue samples were all BRAF-negative and all BRAF-negative FNAB mixtures were correctly called BRAF-negative regardless of mixture proportion, thus the presence of diluents commonly encountered in thyroid FNABs does not result in Afirma BRAF false positives.

**Table 20: Sample counts by cytology, sample source and castPCR-derived BRAF label in training and test sets. All samples were prospectively collected either in a previous study (VERA001) or from consecutive patients referred to the Veracyte CLIA laboratory (CLIA).**

| Cytology | Source | Training Set | | | Test Set | | |
|---|---|---|---|---|---|---|---|
| | | BRAF- | BRAF+ | Prevalence | BRAF- | BRAF+ | Prevalence |
| Bethesda II | All Samples | 18 | 1 | 5.3% | 32 | 1 | 3.0% |
| | CLIA | 0 | 0 | - | 0 | 0 | - |
| | VERA001 | 18 | 1 | 5.3% | 32 | 1 | 3.0% |
| Bethesda III/IV | All Samples | 37 | 4 | 9.8% | 298 | 3 | 1.0% |
| | CLIA | 12 | 2 | 14.3% | 131 | 2 | 1.5% |
| | VERA001 | 25 | 2 | 7.4% | 167 | 1 | 0.6% |
| Bethesda V | All Samples | 34 | 27 | 44.3% | 61 | 28 | 31.5% |
| | CLIA | 17 | 14 | 45.2% | 41 | 21 | 33.9% |
| | VERA001 | 17 | 13 | 43.3% | 20 | 7 | 25.9% |
| Bethesda VI | All Samples | 25 | 35 | 58.3% | 29 | 83 | 74.1% |
| | CLIA | 17 | 19 | 52.8% | 20 | 60 | 75.0% |
| | VERA001 | 8 | 16 | 66.7% | 9 | 23 | 71.9% |
| Total | | 114 | 67 | 37.0% | 420 | 115 | 21.5% |
| | | 181 | | | 535 | | |

**Table 21: Positive percent agreement (PPA), negative percent agreement (NPA) and area under the ROC curve (AUC) for training (under cross validation (CV)) and test sets. AUCs for Bethesda II and III/IV cohorts were all equal to 1 in training and test but due to the small number of BRAF-positive samples, AUCs only for the remaining cytology cohorts are reported.**

| | Cytology | PPA | NPA | AUC |
|---|---|---|---|---|
| Training | Bethesda II | 100% [2.5%-100%] | 100% [81.5%-100%] | - |
| | Bethesda III/IV | 100% [39.8%-100%] | 100% [90.5%-100%] | - |
| | Bethesda V | 85.2% [66.3%-95.8%] | 100% [89.7%-100%] | 0.996 [0.987-1] |
| | Bethesda VI | 88.6% [73.3%-96.8%] | 96.0% [79.6%-99.9%] | 0.982 [0.958-1] |
| | Overall | 88.1% [77.8%-94.7%] | 99.1% [95.2%-100%] | 0.993 [0.986-1] |
| | | | | |
| Test | Bethesda II | 100% [2.5%-100%] | 100% [89.1%-100%] | - |
| | Bethesda III/IV | 100% [29.2%-100%] | 100% [98.8%-100%] | - |
| | Bethesda V | 75.0% [55.1%-89.3%] | 96.7% [88.7%-99.6%] | 0.975 [0.951-1] |

(continued)

| | Cytology | PPA | NPA | AUC |
|---|---|---|---|---|
| | Bethesda VI | 95.2% [88.1%-98.7%] | 93.1% [77.2%-99.2%] | 0.980 [0.955-1] |
| | Overall | 90.4% [83.5%-95.1%] | 99.0% [97.6%-99.7%] | 0.997 [0.994-0.999] |

**Table 22: Performance of Afirma BRAF and castPCR (at various thresholds in analytical sensitivity) in predicting malignancy (as defined by histology after resection) by cytology category. NPV and PPV are calculated using study prevalence (34.3%, 73 malignant nodules in 213 total nodules).**

| | Specificity | NPV | PPV | AUC |
|---|---|---|---|---|
| **Afirma BRAF** | 100% [97.4%-100%] | 77.30% | 100% | 0.840 [0.779-0.901] |
| **castPCR (0%)** | 100% [97.4%-100%] | 77.30% | 100% | 0.719 [0.662-0.776] |
| **castPCR (2.5%)** | 100% [97.4%-100%] | 77.30% | 100% | 0.719 [0.662-0.776] |
| **castPCR (5.0%)** | 100% [97.4%-100%] | 76.90% | 100% | 0.719 [0.662-0.776] |

**Example 11: Biomarkers used in the Afirma BRAF classifier**

In this example, 5 lists of gene markers are provided. The 128 transcripts (from 127 genes) used in the Afirma BRAF classifier along with RefSeq gene 57 symbols and Ensembl identifiers are listed in Table 23. Tables 24, 25, 26 and 27, provide for alternative lists of biomarkers that may be used in the BRAF classifier. Tables 24, 25, 26 and 27 are subsets of Table 23.

**Table 23: 128 transcript cluster IDs (TCIDs) derived from the Affymetrix exon array along with RefSeq and Ensembl IDs represented by each TCID**

| # | TCID | Gene Symbol | Ensembl |
|---|---|---|---|
| 1 | 3338192 | CCND1 | ENSG00000110092 |
| 2 | 2657808 | CLDN16 | ENSG00000113946 |
| 3 | 2598261 | FN1 | ENSG00000115414 |
| 4 | 2884845 | GABRB2 | ENSG00000145864 |
| 5 | 2708855 | LIPH | ENSG00000163898 |
| 6 | 3329343 | MDK | ENSG00000110492 |
| 7 | 3067478 | NRCAM | ENSG00000091129 |
| 8 | 2685304 | PROS1 | ENSG00000184500 |
| 9 | 2442008 | RXRG | ENSG00000143171 |
| 10 | 3494629 | SCEL | ENSG00000136155 |
| 11 | 2721959 | SLC34A2 | ENSG00000157765 |
| 12 | 2582562 | ACVR1 | ENSG00000115170 |
| 13 | 2759582 | AFAP1 | ENSG00000196526 |
| 14 | 2734421 | ARHGAP24 | ENSG00000138639 |
| 15 | 2423829 | ARHGAP29 | ENSG00000137962 |
| 16 | 3984945 | ARMCX3 | ENSG00000102401 |
| 17 | 4015838 | ARMCX6 | ENSG00000198960 |

(continued)

| # | TCID | Gene Symbol | Ensembl |
|---|------|-------------|---------|
| 18 | 3321150 | ARNTL | ENSG00000133794 |
| 19 | 2468811 | ASAP2 | ENSG00000151693 |
| 20 | 2711225 | ATP13A4 | ENSG00000127249 |
| 21 | 2711205 | ATP13A4 | ENSG00000127249 |
| 22 | 2356818 | BCL9 | ENSG00000116128 |
| 23 | 2381249 | C1orf115 | ENSG00000162817 |
| 24 | 2400177 | CAMK2N1 | ENSG00000162545 |
| 25 | 2582701 | CCDC148 | ENSG00000153237 |
| 26 | 3223425 | CDK5RAP2 | ENSG00000136861 |
| 27 | 2781736 | CFI | ENSG00000205403 |
| 28 | 4012178 | CITED1 | ENSG00000125931 |
| 29 | 3497195 | CLDN10 | ENSG00000134873 |
| 30 | 3743551 | CLDN7 | ENSG00000181885 |
| 31 | 2438458 | CRABP2 | ENSG00000143320 |
| 32 | 3335894 | CST6 | ENSG00000175315 |
| 33 | 3863640 | CXCL17 | ENSG00000189377 |
| 34 | 2686023 | DCBLD2 | ENSG00000057019 |
| 35 | 2397025 | DHRS3 | ENSG00000162496 |
| 36 | 3125116 | DLC1 | ENSG00000164741 |
| 37 | 3522398 | DOCK9 | ENSG00000088387 |
| 38 | 3263743 | DUSP5 | ENSG00000138166 |
| 39 | 2830861 | EGR1 | ENSG00000120738 |
| 40 | 3837431 | EHD2 | ENSG00000024422 |
| 41 | 3046197 | ELMO1 | ENSG00000155849 |
| 42 | 3679959 | EMP2 | ENSG00000213853 |
| 43 | 2600689 | EPHA4 | ENSG00000116106 |
| 44 | 3445908 | EPS8 | ENSG00000151491 |
| 45 | 3417249 | ERBB3 | ENSG00000065361 |
| 46 | 2560625 | FAM176A | ENSG00000115363 |
| 47 | 2523045 | FZD7 | ENSG00000155760 |
| 48 | 3044129 | GGCT | ENSG00000006625 |
| 49 | 3683377 | GPRC5B | ENSG00000167191 |
| 50 | 2827057 | GRAMD3 | ENSG00000155324 |
| 51 | 3187686 | GSN | ENSG00000148180 |
| 52 | 3250278 | HK1 | ENSG00000156515 |
| 53 | 2598828 | IGFBP5 | ENSG00000115461 |
| 54 | 3415744 | IGFBP6 | ENSG00000167779 |
| 55 | 2816298 | IQGAP2 | ENSG00000145703 |

(continued)

| # | TCID | Gene Symbol | Ensembl |
|---|------|-------------|---------|
| 56 | 2809245 | ITGA2 | ENSG00000164171 |
| 57 | 3726154 | ITGA3 | ENSG00000005884 |
| 58 | 2617188 | ITGA9 | ENSG00000144668 |
| 59 | 2991860 | ITGB8 | ENSG00000105855 |
| 60 | 2608469 | ITPR1 | ENSG00000150995 |
| 61 | 3556990 | JUB | ENSG00000129474 |
| 62 | 3154002 | KCNQ3 | ENSG00000184156 |
| 63 | 3238962 | KIAA1217 | ENSG00000120549 |
| 64 | 3868783 | KLK7 | ENSG00000169035 |
| 65 | 3757108 | KRT19 | ENSG00000171345 |
| 66 | 2371139 | LAMC2 | ENSG00000058085 |
| 67 | 2962026 | LCA5 | ENSG00000135338 |
| 68 | 2452478 | LEMD1 | ENSG00000186007 |
| 69 | 2567167 | LONRF2 | ENSG00000170500 |
| 70 | 3040518 | MACC1 | ENSG00000183742 |
| 71 | 2525533 | MAP2 | ENSG00000078018 |
| 72 | 3784344 | MAPRE2 | ENSG00000166974 |
| 73 | 3765689 | MED13 | ENSG00000108510 |
| 74 | 3020343 | MET | ENSG00000105976 |
| 75 | 3416895 | METTL7B | ENSG00000170439 |
| 76 | 3638204 | MFGE8 | ENSG00000140545 |
| 77 | 3464417 | MGAT4C | ENSG00000182050 |
| 78 | 2936857 | M LLT4 | ENSG00000130396 |
| 79 | 3393720 | M PZL2 | ENSG00000149573 |
| 80 | 3744463 | MYH10 | ENSG00000133026 |
| 81 | 3417809 | NAB2 | ENSG00000166886 |
| 82 | 3323052 | NAV2 | ENSG00000166833 |
| 83 | 3044072 | NOD1 | ENSG00000106100 |
| 84 | 2792127 | NPY1R | ENSG00000164128 |
| 85 | 3925639 | NRIP1 | ENSG00000180530 |
| 86 | 3815116 | PALM | ENSG00000099864 |
| 87 | 2822215 | PAM | ENSG00000145730 |
| 88 | 2783596 | PDE5A | ENSG00000138735 |
| 89 | 2828441 | PDLIM4 | ENSG00000131435 |
| 90 | 2976360 | PERP | ENSG00000112378 |
| 91 | 2511820 | PKP4 | ENSG00000144283 |
| 92 | 3136178 | PLAG1 | ENSG00000181690 |
| 93 | 3759587 | PLCD3 | ENSG00000161714 |

(continued)

| # | TCID | Gene Symbol | Ensembl |
|---|---|---|---|
| 94 | 3678462 | PPL | ENSG00000118898 |
| 95 | 2650393 | PPM1L | ENSG00000163590 |
| 96 | 3451375 | PRICKLE1 | ENSG00000139174 |
| 97 | 2994981 | PRR15 | ENSG00000176532 |
| 98 | 3126368 | PSD3 | ENSG00000156011 |
| 99 | 3126191 | PSD3 | ENSG00000156011 |
| 100 | 2455418 | PTPN14 | ENSG00000152104 |
| 101 | 2333318 | PTPRF | ENSG00000142949 |
| 102 | 3751002 | RAB34 | ENSG00000109113 |
| 103 | 3183757 | RAD23B | ENSG00000119318 |
| 104 | 3040967 | RAPGEF5 | ENSG00000136237 |
| 105 | 2819044 | RASA1 | ENSG00000145715 |
| 106 | 2580802 | RND3 | ENSG00000115963 |
| 107 | 4045643 | S100A16 | ENSG00000188643 |
| 108 | 3907234 | SDC4 | ENSG00000124145 |
| 109 | 2738664 | SGMS2 | ENSG00000164023 |
| 110 | 3088213 | SH2D4A | ENSG00000104611 |
| 111 | 2827645 | SLC27A6 | ENSG00000113396 |
| 112 | 3389976 | SLC35F2 | ENSG00000110660 |
| 113 | 2742224 | SPRY1 | ENSG00000164056 |
| 114 | 3408831 | SSPN | ENSG00000123096 |
| 115 | 2979871 | SYNE1 | ENSG00000131018 |
| 116 | 3973891 | SYTL5 | ENSG00000147041 |
| 117 | 2435218 | TDRKH | ENSG00000182134 |
| 118 | 2649113 | TIPARP | ENSG00000163659 |
| 119 | 3173880 | TJP2 | ENSG00000119139 |
| 120 | 3110608 | TM7SF4 | ENSG00000164935 |
| 121 | 3717870 | TMEM98 | ENSG00000006042 |
| 122 | 3645555 | TNFRSF12A | ENSG00000006327 |
| 123 | 2924330 | TPD52L1 | ENSG00000111907 |
| 124 | 4018327 | TRPC5 | ENSG00000072315 |
| 125 | 3087167 | TUSC3 | ENSG00000104723 |
| 126 | 3988596 | ZCCHC12 | ENSG00000174460 |
| 127 | 3987607 | ZCCHC16 | ENSG00000187823 |
| 128 | 2451870 | ETNK2 | ENSG00000143845 |

**Table 24: 39 transcript cluster IDs (TCIDs) derived from the Affymetrix exon array along with gene symbol and Ensembl IDs represented by each TCID (new biomarkers, a subset of Table 23)**

| # | TCID | Gene Symbol | Ensembl |
|---|------|-------------|---------|
| 1 | 3338192 | CCND1 | ENSG00000110092 |
| 2 | 2657808 | CLDN16 | ENSG00000113946 |
| 3 | 2884845 | GABRB2 | ENSG00000145864 |
| 4 | 2442008 | RXRG | ENSG00000143171 |
| 5 | 3494629 | SCEL | ENSG00000136155 |
| 6 | 3984945 | ARMCX3 | ENSG00000102401 |
| 7 | 4015838 | ARMCX6 | ENSG00000198960 |
| 8 | 2711225 | ATP13A4 | ENSG00000127249 |
| 9 | 2711205 | ATP13A4 | ENSG00000127249 |
| 10 | 2381249 | C1orf115 | ENSG00000162817 |
| 11 | 3223425 | CDK5RAP2 | ENSG00000136861 |
| 12 | 4012178 | CITED1 | ENSG00000125931 |
| 13 | 3743551 | CLDN7 | ENSG00000181885 |
| 14 | 3125116 | DLC1 | ENSG00000164741 |
| 15 | 3263743 | DUSP5 | ENSG00000138166 |
| 16 | 3837431 | EHD2 | ENSG00000024422 |
| 17 | 3445908 | EPS8 | ENSG00000151491 |
| 18 | 2523045 | FZD7 | ENSG00000155760 |
| 19 | 3187686 | GSN | ENSG00000148180 |
| 20 | 3250278 | HK1 | ENSG00000156515 |
| 21 | 2598828 | IGFBP5 | ENSG00000115461 |
| 22 | 3415744 | IGFBP6 | ENSG00000167779 |
| 23 | 3416895 | METTL7B | ENSG00000170439 |
| 24 | 3393720 | M PZL2 | ENSG00000149573 |
| 25 | 3744463 | MYH10 | ENSG00000133026 |
| 26 | 3323052 | NAV2 | ENSG00000166833 |
| 27 | 3136178 | PLAG1 | ENSG00000181690 |
| 28 | 2650393 | PPM1L | ENSG00000163590 |
| 29 | 3451375 | PRICKLE1 | ENSG00000139174 |
| 30 | 2994981 | PRR15 | ENSG00000176532 |
| 31 | 3126368 | PSD3 | ENSG00000156011 |
| 32 | 3126191 | PSD3 | ENSG00000156011 |
| 33 | 3907234 | SDC4 | ENSG00000124145 |
| 34 | 2742224 | SPRY1 | ENSG00000164056 |
| 35 | 2979871 | SYNE1 | ENSG00000131018 |
| 36 | 3973891 | SYTL5 | ENSG00000147041 |
| 37 | 4018327 | TRPC5 | ENSG00000072315 |

(continued)

| # | TCID | Gene Symbol | Ensembl |
|---|------|-------------|---------|
| 38 | 3988596 | ZCCHC12 | ENSG00000174460 |
| 39 | 3987607 | ZCCHC16 | ENSG00000187823 |

**Table 25: 119 transcript cluster IDs (TCIDs) derived from the Affymetrix exon array along with gene symbol and Ensembl IDs represented by each TCID (BRAF-V600E-specific biomarkers, a subset of Table 23)**

| # | TCID | Gene Symbol | Ensembl |
|---|------|-------------|---------|
| 1 | 3338192 | CCND1 | ENSG00000110092 |
| 2 | 2657808 | CLDN16 | ENSG00000113946 |
| 3 | 2884845 | GABRB2 | ENSG00000145864 |
| 4 | 2708855 | LIPH | ENSG00000163898 |
| 5 | 3329343 | MDK | ENSG00000110492 |
| 6 | 3067478 | NRCAM | ENSG00000091129 |
| 7 | 2685304 | PROS1 | ENSG00000184500 |
| 8 | 2442008 | RXRG | ENSG00000143171 |
| 9 | 3494629 | SCEL | ENSG00000136155 |
| 10 | 2582562 | ACVR1 | ENSG00000115170 |
| 11 | 2759582 | AFAP1 | ENSG00000196526 |
| 12 | 2734421 | ARHGAP24 | ENSG00000138639 |
| 13 | 2423829 | ARHGAP29 | ENSG00000137962 |
| 14 | 3984945 | ARMCX3 | ENSG00000102401 |
| 15 | 4015838 | ARMCX6 | ENSG00000198960 |
| 16 | 2468811 | ASAP2 | ENSG00000151693 |
| 17 | 2711225 | ATP13A4 | ENSG00000127249 |
| 18 | 2711205 | ATP13A4 | ENSG00000127249 |
| 19 | 2356818 | BCL9 | ENSG00000116128 |
| 20 | 2381249 | C1orf115 | ENSG00000162817 |
| 21 | 2400177 | CAMK2N1 | ENSG00000162545 |
| 22 | 2582701 | CCDC148 | ENSG00000153237 |
| 23 | 3223425 | CDK5RAP2 | ENSG00000136861 |
| 24 | 2781736 | CFI | ENSG00000205403 |
| 25 | 4012178 | CITED1 | ENSG00000125931 |
| 26 | 3497195 | CLDN10 | ENSG00000134873 |
| 27 | 3743551 | CLDN7 | ENSG00000181885 |
| 28 | 2438458 | CRABP2 | ENSG00000143320 |
| 29 | 3335894 | CST6 | ENSG00000175315 |
| 30 | 3863640 | CXCL17 | ENSG00000189377 |
| 31 | 2686023 | DCBLD2 | ENSG00000057019 |
| 32 | 2397025 | DHRS3 | ENSG00000162496 |

(continued)

| # | TCID | Gene Symbol | Ensembl |
|---|------|-------------|---------|
| 33 | 3125116 | DLC1 | ENSG00000164741 |
| 34 | 3522398 | DOCK9 | ENSG00000088387 |
| 35 | 3263743 | DUSP5 | ENSG00000138166 |
| 36 | 2830861 | EGR1 | ENSG00000120738 |
| 37 | 3837431 | EHD2 | ENSG00000024422 |
| 38 | 3046197 | ELMO1 | ENSG00000155849 |
| 39 | 3679959 | EMP2 | ENSG00000213853 |
| 40 | 2600689 | EPHA4 | ENSG00000116106 |
| 41 | 3445908 | EPS8 | ENSG00000151491 |
| 42 | 2560625 | FAM176A | ENSG00000115363 |
| 43 | 2523045 | FZD7 | ENSG00000155760 |
| 44 | 3044129 | GGCT | ENSG00000006625 |
| 45 | 3683377 | GPRC5B | ENSG00000167191 |
| 46 | 2827057 | GRAMD3 | ENSG00000155324 |
| 47 | 3187686 | GSN | ENSG00000148180 |
| 48 | 3250278 | HK1 | ENSG00000156515 |
| 49 | 2598828 | IGFBP5 | ENSG00000115461 |
| 50 | 3415744 | IGFBP6 | ENSG00000167779 |
| 51 | 2816298 | IQGAP2 | ENSG00000145703 |
| 52 | 2809245 | ITGA2 | ENSG00000164171 |
| 53 | 3726154 | ITGA3 | ENSG00000005884 |
| 54 | 2617188 | ITGA9 | ENSG00000144668 |
| 55 | 2991860 | ITGB8 | ENSG00000105855 |
| 56 | 2608469 | ITPR1 | ENSG00000150995 |
| 57 | 3556990 | JUB | ENSG00000129474 |
| 58 | 3154002 | KCNQ3 | ENSG00000184156 |
| 59 | 3238962 | KIAA1217 | ENSG00000120549 |
| 60 | 3868783 | KLK7 | ENSG00000169035 |
| 61 | 3757108 | KRT19 | ENSG00000171345 |
| 62 | 2371139 | LAMC2 | ENSG00000058085 |
| 63 | 2962026 | LCA5 | ENSG00000135338 |
| 64 | 2452478 | LEMD1 | ENSG00000186007 |
| 65 | 2567167 | LONRF2 | ENSG00000170500 |
| 66 | 3040518 | MACC1 | ENSG00000183742 |
| 67 | 2525533 | MAP2 | ENSG00000078018 |
| 68 | 3784344 | MAPRE2 | ENSG00000166974 |
| 69 | 3765689 | MED13 | ENSG00000108510 |
| 70 | 3416895 | METTL7B | ENSG00000170439 |

(continued)

| # | TCID | Gene Symbol | Ensembl |
|---|------|-------------|---------|
| 71 | 3638204 | MFGE8 | ENSG00000140545 |
| 72 | 3464417 | MGAT4C | ENSG00000182050 |
| 73 | 2936857 | M LLT4 | ENSG00000130396 |
| 74 | 3393720 | M PZL2 | ENSG00000149573 |
| 75 | 3744463 | MYH10 | ENSG00000133026 |
| 76 | 3417809 | NAB2 | ENSG00000166886 |
| 77 | 3323052 | NAV2 | ENSG00000166833 |
| 78 | 3044072 | NOD1 | ENSG00000106100 |
| 79 | 2792127 | NPY1R | ENSG00000164128 |
| 80 | 3925639 | NRIP1 | ENSG00000180530 |
| 81 | 3815116 | PALM | ENSG00000099864 |
| 82 | 2822215 | PAM | ENSG00000145730 |
| 83 | 2828441 | PDLIM4 | ENSG00000131435 |
| 84 | 2511820 | PKP4 | ENSG00000144283 |
| 85 | 3136178 | PLAG1 | ENSG00000181690 |
| 86 | 3759587 | PLCD3 | ENSG00000161714 |
| 87 | 3678462 | PPL | ENSG00000118898 |
| 88 | 2650393 | PPM1L | ENSG00000163590 |
| 89 | 3451375 | PRICKLE1 | ENSG00000139174 |
| 90 | 2994981 | PRR15 | ENSG00000176532 |
| 91 | 3126368 | PSD3 | ENSG00000156011 |
| 92 | 3126191 | PSD3 | ENSG00000156011 |
| 93 | 2455418 | PTPN14 | ENSG00000152104 |
| 94 | 2333318 | PTPRF | ENSG00000142949 |
| 95 | 3751002 | RAB34 | ENSG00000109113 |
| 96 | 3183757 | RAD23B | ENSG00000119318 |
| 97 | 3040967 | RAPGEF5 | ENSG00000136237 |
| 98 | 2819044 | RASA1 | ENSG00000145715 |
| 99 | 2580802 | RND3 | ENSG00000115963 |
| 100 | 4045643 | S100A16 | ENSG00000188643 |
| 101 | 3907234 | SDC4 | ENSG00000124145 |
| 102 | 2738664 | SGMS2 | ENSG00000164023 |
| 103 | 3088213 | SH2D4A | ENSG00000104611 |
| 104 | 2827645 | SLC27A6 | ENSG00000113396 |
| 105 | 3389976 | SLC35F2 | ENSG00000110660 |
| 106 | 2742224 | SPRY1 | ENSG00000164056 |
| 107 | 3408831 | SSPN | ENSG00000123096 |
| 108 | 2979871 | SYNE1 | ENSG00000131018 |

(continued)

| # | TCID | Gene Symbol | Ensembl |
|---|------|-------------|---------|
| 109 | 3973891 | SYTL5 | ENSG00000147041 |
| 110 | 2435218 | TDRKH | ENSG00000182134 |
| 111 | 2649113 | TIPARP | ENSG00000163659 |
| 112 | 3173880 | TJP2 | ENSG00000119139 |
| 113 | 3717870 | TMEM98 | ENSG00000006042 |
| 114 | 3645555 | TNFRSF12A | ENSG00000006327 |
| 115 | 4018327 | TRPC5 | ENSG00000072315 |
| 116 | 3087167 | TUSC3 | ENSG00000104723 |
| 117 | 3988596 | ZCCHC12 | ENSG00000174460 |
| 118 | 3987607 | ZCCHC16 | ENSG00000187823 |
| 119 | 2451870 | ETNK2 | ENSG00000143845 |

**Table 26: 100 transcript cluster IDs (TCIDs) derived from the Affymetrix exon array along with gene symbol and Ensembl IDs represented by each TCID (BRAF-V600E-specific biomarkers for thyroid nodule, a subset of Table 23)**

| # | TCID | Gene Symbol | Ensembl |
|---|------|-------------|---------|
| 1 | 2884845 | GABRB2 | ENSG00000145864 |
| 2 | 2708855 | LIPH | ENSG00000163898 |
| 3 | 3329343 | MDK | ENSG00000110492 |
| 4 | 3067478 | NRCAM | ENSG00000091129 |
| 5 | 2685304 | PROS1 | ENSG00000184500 |
| 6 | 2582562 | ACVR1 | ENSG00000115170 |
| 7 | 2759582 | AFAP1 | ENSG00000196526 |
| 8 | 2734421 | ARHGAP24 | ENSG00000138639 |
| 9 | 2423829 | ARHGAP29 | ENSG00000137962 |
| 10 | 3984945 | ARMCX3 | ENSG00000102401 |
| 11 | 4015838 | ARMCX6 | ENSG00000198960 |
| 12 | 2468811 | ASAP2 | ENSG00000151693 |
| 13 | 2711225 | ATP13A4 | ENSG00000127249 |
| 14 | 2711205 | ATP13A4 | ENSG00000127249 |
| 15 | 2356818 | BCL9 | ENSG00000116128 |
| 16 | 2381249 | C1orf115 | ENSG00000162817 |
| 17 | 2400177 | CAMK2N1 | ENSG00000162545 |
| 18 | 2582701 | CCDC148 | ENSG00000153237 |
| 19 | 3223425 | CDK5RAP2 | ENSG00000136861 |
| 20 | 2781736 | CFI | ENSG00000205403 |
| 21 | 3497195 | CLDN10 | ENSG00000134873 |
| 22 | 3743551 | CLDN7 | ENSG00000181885 |

(continued)

| # | TCID | Gene Symbol | Ensembl |
|---|---|---|---|
| 23 | 2438458 | CRABP2 | ENSG00000143320 |
| 24 | 3335894 | CST6 | ENSG00000175315 |
| 25 | 3863640 | CXCL17 | ENSG00000189377 |
| 26 | 2686023 | DCBLD2 | ENSG00000057019 |
| 27 | 2397025 | DHRS3 | ENSG00000162496 |
| 28 | 3125116 | DLC1 | ENSG00000164741 |
| 29 | 3522398 | DOCK9 | ENSG00000088387 |
| 30 | 2830861 | EGR1 | ENSG00000120738 |
| 31 | 3837431 | EHD2 | ENSG00000024422 |
| 32 | 3046197 | ELMO1 | ENSG00000155849 |
| 33 | 3679959 | EMP2 | ENSG00000213853 |
| 34 | 2600689 | EPHA4 | ENSG00000116106 |
| 35 | 2560625 | FAM176A | ENSG00000115363 |
| 36 | 3044129 | GGCT | ENSG00000006625 |
| 37 | 3683377 | GPRC5B | ENSG00000167191 |
| 38 | 2827057 | GRAMD3 | ENSG00000155324 |
| 39 | 3250278 | HK1 | ENSG00000156515 |
| 40 | 2816298 | IQGAP2 | ENSG00000145703 |
| 41 | 2809245 | ITGA2 | ENSG00000164171 |
| 42 | 3726154 | ITGA3 | ENSG00000005884 |
| 43 | 2617188 | ITGA9 | ENSG00000144668 |
| 44 | 2991860 | ITGB8 | ENSG00000105855 |
| 45 | 2608469 | ITPR1 | ENSG00000150995 |
| 46 | 3556990 | JUB | ENSG00000129474 |
| 47 | 3154002 | KCNQ3 | ENSG00000184156 |
| 48 | 3238962 | KIAA1217 | ENSG00000120549 |
| 49 | 3868783 | KLK7 | ENSG00000169035 |
| 50 | 3757108 | KRT19 | ENSG00000171345 |
| 51 | 2371139 | LAMC2 | ENSG00000058085 |
| 52 | 2962026 | LCA5 | ENSG00000135338 |
| 53 | 2452478 | LEMD1 | ENSG00000186007 |
| 54 | 2567167 | LONRF2 | ENSG00000170500 |
| 55 | 3040518 | MACC1 | ENSG00000183742 |
| 56 | 2525533 | MAP2 | ENSG00000078018 |
| 57 | 3784344 | MAPRE2 | ENSG00000166974 |
| 58 | 3765689 | MED13 | ENSG00000108510 |
| 59 | 3416895 | METTL7B | ENSG00000170439 |
| 60 | 3638204 | MFGE8 | ENSG00000140545 |

(continued)

| # | TCID | Gene Symbol | Ensembl |
|---|------|-------------|---------|
| 61 | 3464417 | MGAT4C | ENSG00000182050 |
| 62 | 2936857 | M LLT4 | ENSG00000130396 |
| 63 | 3417809 | NAB2 | ENSG00000166886 |
| 64 | 3323052 | NAV2 | ENSG00000166833 |
| 65 | 3044072 | NOD1 | ENSG00000106100 |
| 66 | 2792127 | NPY1R | ENSG00000164128 |
| 67 | 3925639 | NRIP1 | ENSG00000180530 |
| 68 | 3815116 | PALM | ENSG00000099864 |
| 69 | 2822215 | PAM | ENSG00000145730 |
| 70 | 2828441 | PDLIM4 | ENSG00000131435 |
| 71 | 2511820 | PKP4 | ENSG00000144283 |
| 72 | 3759587 | PLCD3 | ENSG00000161714 |
| 73 | 3678462 | PPL | ENSG00000118898 |
| 74 | 2650393 | PPM1L | ENSG00000163590 |
| 75 | 3451375 | PRICKLE1 | ENSG00000139174 |
| 76 | 2994981 | PRR15 | ENSG00000176532 |
| 77 | 2455418 | PTPN14 | ENSG00000152104 |
| 78 | 2333318 | PTPRF | ENSG00000142949 |
| 79 | 3751002 | RAB34 | ENSG00000109113 |
| 80 | 3183757 | RAD23B | ENSG00000119318 |
| 81 | 3040967 | RAPGEF5 | ENSG00000136237 |
| 82 | 2819044 | RASA1 | ENSG00000145715 |
| 83 | 2580802 | RND3 | ENSG00000115963 |
| 84 | 4045643 | S100A16 | ENSG00000188643 |
| 85 | 2738664 | SGMS2 | ENSG00000164023 |
| 86 | 3088213 | SH2D4A | ENSG00000104611 |
| 87 | 2827645 | SLC27A6 | ENSG00000113396 |
| 88 | 3389976 | SLC35F2 | ENSG00000110660 |
| 89 | 2742224 | SPRY1 | ENSG00000164056 |
| 90 | 3408831 | SSPN | ENSG00000123096 |
| 91 | 2435218 | TDRKH | ENSG00000182134 |
| 92 | 2649113 | TIPARP | ENSG00000163659 |
| 93 | 3173880 | TJP2 | ENSG00000119139 |
| 94 | 3717870 | TMEM98 | ENSG00000006042 |
| 95 | 3645555 | TNFRSF12A | ENSG00000006327 |
| 96 | 4018327 | TRPC5 | ENSG00000072315 |
| 97 | 3087167 | TUSC3 | ENSG00000104723 |
| 98 | 3988596 | ZCCHC12 | ENSG00000174460 |

(continued)

| # | TCID | Gene Symbol | Ensembl |
|---|---|---|---|
| 99 | 3987607 | ZCCHC16 | ENSG00000187823 |
| 100 | 2451870 | ETNK2 | ENSG00000143845 |

**Table 27: 20 transcript cluster IDs (TCIDs) derived from the Affymetrix exon array along with gene symbol and Ensembl IDs represented by each TCID (biomarkers, a subset of table 23)**

| # | TCID | Gene Symbol | Ensembl |
|---|---|---|---|
| 1 | 2884845 | GABRB2 | ENSG00000145864 |
| 2 | 3984945 | ARMCX3 | ENSG00000102401 |
| 3 | 4015838 | ARMCX6 | ENSG00000198960 |
| 4 | 2711225 | ATP13A4 | ENSG00000127249 |
| 5 | 2711205 | ATP13A4 | ENSG00000127249 |
| 6 | 2381249 | C1orf115 | ENSG00000162817 |
| 7 | 3223425 | CDK5RAP2 | ENSG00000136861 |
| 8 | 3743551 | CLDN7 | ENSG00000181885 |
| 9 | 3125116 | DLC1 | ENSG00000164741 |
| 10 | 3837431 | EHD2 | ENSG00000024422 |
| 11 | 3250278 | HK1 | ENSG00000156515 |
| 12 | 3416895 | METTL7B | ENSG00000170439 |
| 13 | 3323052 | NAV2 | ENSG00000166833 |
| 14 | 2650393 | PPM1L | ENSG00000163590 |
| 15 | 3451375 | PRICKLE1 | ENSG00000139174 |
| 16 | 2994981 | PRR15 | ENSG00000176532 |
| 17 | 2742224 | SPRY1 | ENSG00000164056 |
| 18 | 4018327 | TRPC5 | ENSG00000072315 |
| 19 | 3988596 | ZCCHC12 | ENSG00000174460 |
| 20 | 3987607 | ZCCHC16 | ENSG00000187823 |

**Example 12: Cancer Aggressiveness**

In this example, two distinct but slightly overlapping aggressiveness signatures have been identified using a highly curated cohort of BRAF V600E positive (BRAF+) and BRAF-mutation negative (BRAF-negative) samples and deep RNA sequencing. A cohort often BRAF+ samples from subjects with aggressive PTC phenotype was compared to a cohort of five BRAF+ samples from subjects without aggressive PTC. Similarly, samples from BRAF-negative subjects were also studied. A cohort of seven aggressive-BRAF-negative PTC samples was compared to eight not aggressive-BRAF-negative PTCs. Normalized and aligned RNAseq data was analyzed for differential expression using EdgeR (Bioconductor) and significance was established with an FDR p-value <0.1. The BRAF+ aggressiveness signature reveals 207 biomarker genes (Table 28, Figure 38) while the BRAF-negative aggressiveness signature has 162 genes (Table 29, Figure 39). Only eight genes are shared between both lists (Table 30). A recently developed 128 gene BRAF V600E classifier shares a single gene (CST6) with the genes found in the BRAF+ aggressiveness signature. While that classifier was developed to accurately classify samples into BRAF+ and BRAF-negative categories, the discovery of these aggressiveness signatures may be useful in prognosing thyroid disease regardless of BRAF mutation status. A third multivariate logistic regression analysis to using all 30 PTC samples and modeling malignancy, BRAF-status, and aggressive phenotype as independent variables compared to five benign samples, revealed an additional 32 biomarkers that are specifically correlated with the aggressive phenotype (List 1).

**Table 28. Biomarkers represented by gene symbols and Ensembl IDs of aggressive PTC in BRAF V600E positive samples.**

| Ensembl ID | Gene Symbol | Log2 Fold Change | ENSG00000156466 | GDF6 | -5.89 |
|---|---|---|---|---|---|
| ENSG00000205669 | ACOT6 | -4.79 | ENSG00000151892 | GFRA1 | 2.83 |
| | | | ENSG00000186417 | GLDN | 2.64 |
| ENSG00000143632 | ACTA1 | 5.52 | ENSG00000069122 | GPR116 | 2.36 |
| ENSG00000148926 | ADM | -2.30 | ENSG00000185038 | HEATR7B1 | 8.82 |
| ENSG00000187134 | AKR1C1 | -4.21 | ENSG00000069812 | HES2 | -2.41 |
| ENSG00000116748 | AMPD1 | 8.57 | ENSG00000004776 | HSPB6 | 5.11 |
| ENSG00000182492 | BGN | 2.55 | ENSG00000102468 | HTR2A | -6.48 |
| ENSG00000171722 | C1orf111 | 4.86 | ENSG00000132465 | IGJ | 4.16 |
| ENSG00000112936 | C7 | 3.27 | ENSG00000144847 | IGSF11 | -3.32 |
| ENSG00000108691 | CCL2 | -3.39 | ENSG00000136689 | IL1RN | -1.64 |
| ENSG00000108688 | CCL7 | -4.23 | ENSG00000169429 | IL8 | -4.03 |
| ENSG00000177455 | CD19 | 6.96 | ENSG00000077943 | ITGA8 | 3.81 |
| ENSG00000165556 | CDX2 | -11.74 | ENSG00000167749 | KLK4 | -3.56 |
| ENSG00000198848 | CES1 | -3.25 | ENSG00000125869 | LAMP5 | 2.85 |
| ENSG00000133063 | CHIT1 | -2.85 | ENSG00000174697 | LEP | -6.57 |
| ENSG00000108821 | COL1A1 | 2.05 | ENSG00000188992 | LIPI | -4.35 |
| ENSG00000164692 | COL1A2 | 2.65 | ENSG00000223648 | LOC100134256 | 6.48 |
| ENSG00000168542 | COL3A1 | 3.21 | | | |
| ENSG00000175315 | CST6 | 2.10 | ENSG00000211941 | LOC100291917 | 6.11 |
| ENSG00000160213 | CSTB | -1.93 | | | |
| ENSG00000143387 | CTSK | -3.21 | ENSG00000241351 | LOC100653210 | 5.75 |
| ENSG00000156234 | CXCL13 | 6.25 | | | |
| ENSG00000011465 | DCN | 2.94 | ENSG00000211953 | LOC100653245 | 5.34 |
| ENSG00000164330 | EBF1 | 3.44 | | | |
| ENSG00000136160 | EDNRB | -2.60 | ENSG00000211650 | LOC651536 | 7.58 |
| ENSG00000138792 | ENPEP | 4.77 | ENSG00000061337 | LZTS1 | 3.11 |
| ENSG00000157554 | ERG | 2.76 | ENSG00000196611 | MMP1 | -3.63 |
| ENSG00000143297 | FCRL5 | 3.53 | ENSG00000123342 | MMP19 | -3.33 |
| ENSG00000052795 | FNIP2 | -1.64 | ENSG00000181143 | MUC16 | 3.77 |
| ENSG00000167996 | FTH1 | -1.66 | ENSG00000125414 | MYH2 | 13.03 |
| ENSG00000087086 | FTL | -2.17 | ENSG00000168530 | MYL1 | 11.44 |
| ENSG00000130513 | GDF15 | -2.20 | ENSG00000111245 | MYL2 | 10.10 |

| | | | | | |
|---|---|---|---|---|---|
| ENSG00000170476 | MZB1 | 4.54 | ENSG00000211973 | NA | 6.33 |
| ENSG00000240138 | NA | -10.88 | ENSG00000211937 | NA | 6.31 |
| ENSG00000211949 | NA | 7.39 | ENSG00000211896 | NA | 6.27 |
| ENSG00000188101 | NA | -9.38 | ENSG00000211639 | NA | 10.21 |
| ENSG00000211625 | NA | 11.32 | ENSG00000211951 | NA | 7.40 |
| ENSG00000211968 | NA | 13.08 | ENSG00000211892 | NA | 5.28 |
| ENSG00000211648 | NA | 6.86 | ENSG00000253123 | NA | -3.73 |
| ENSG00000211895 | NA | 4.52 | ENSG00000240382 | NA | 7.46 |
| ENSG00000211947 | NA | 7.04 | ENSG00000211653 | NA | 5.81 |
| ENSG00000243264 | NA | 12.11 | ENSG00000242472 | NA | 9.96 |
| ENSG00000211959 | NA | 7.95 | ENSG00000224373 | NA | 6.66 |
| ENSG00000211677 | NA | 6.91 | ENSG00000243063 | NA | 6.62 |
| ENSG00000211666 | NA | 6.53 | ENSG00000244575 | NA | 7.33 |
| ENSG00000239951 | NA | 6.50 | ENSG00000211938 | NA | 6.14 |
| ENSG00000243466 | NA | 6.43 | ENSG00000226966 | NA | 3.99 |
| ENSG00000211935 | NA | 6.37 | ENSG00000251652 | NA | 4.81 |
| ENSG00000211654 | NA | 15.72 | ENSG00000211644 | NA | 4.65 |
| ENSG00000211976 | NA | 11.57 | ENSG00000211662 | NA | 5.37 |
| ENSG00000211664 | NA | 9.63 | ENSG00000211630 | NA | 10.14 |
| ENSG00000211668 | NA | 6.85 | ENSG00000214676 | NA | 8.51 |
| ENSG00000211966 | NA | 6.45 | ENSG00000243290 | NA | 6.93 |
| ENSG00000211897 | NA | 5.76 | ENSG00000211671 | NA | 6.66 |
| ENSG00000211945 | NA | 7.41 | ENSG00000211663 | NA | 7.06 |
| ENSG00000244437 | NA | 6.42 | ENSG00000241294 | NA | 6.91 |
| ENSG00000211660 | NA | 7.63 | ENSG00000240834 | NA | 7.65 |
| ENSG00000211673 | NA | 6.48 | ENSG00000253755 | NA | 6.08 |
| ENSG00000211679 | NA | 5.12 | ENSG00000211893 | NA | 6.02 |
| ENSG00000224650 | NA | 8.37 | ENSG00000211899 | NA | 4.06 |
| ENSG00000223350 | NA | 7.78 | ENSG00000225698 | NA | 7.78 |
| ENSG00000243238 | NA | 7.59 | ENSG00000211956 | NA | 5.84 |
| ENSG00000231475 | NA | 7.07 | ENSG00000211651 | NA | 6.29 |
| ENSG00000211659 | NA | 6.68 | ENSG00000211599 | NA | 10.55 |
| ENSG00000211598 | NA | 6.44 | ENSG00000211934 | NA | 5.66 |

| | | | | | |
|---|---|---|---|---|---|
| ENSG00000189039 | NA | 7.86 | ENSG00000236182 | NA | -4.17 |
| ENSG00000241158 | NA | 3.42 | ENSG00000223652 | NA | 9.93 |
| ENSG00000235385 | NA | -3.36 | ENSG00000239819 | NA | 6.68 |
| ENSG00000211665 | NA | 6.04 | ENSG00000240041 | NA | 6.66 |
| ENSG00000211632 | NA | 5.52 | ENSG00000255298 | NA | -4.03 |
| ENSG00000249912 | NA | 6.36 | ENSG00000022556 | NLRP2 | 3.37 |
| ENSG00000211933 | NA | 7.40 | ENSG00000171246 | NPTX1 | -5.38 |
| ENSG00000227839 | NA | 8.60 | ENSG00000197893 | NRAP | 11.78 |
| ENSG00000231292 | NA | 5.03 | ENSG00000021645 | NRXN3 | -4.51 |
| ENSG00000241755 | NA | 4.92 | ENSG00000176046 | NUPR1 | -2.10 |
| ENSG00000218730 | NA | 8.45 | ENSG00000115687 | PASK | 2.24 |
| ENSG00000224568 | NA | -2.54 | ENSG00000196092 | PAX5 | 4.86 |
| ENSG00000244445 | NA | 8.60 | ENSG00000162493 | PDPN | -2.09 |
| ENSG00000226608 | NA | -2.49 | ENSG00000139515 | PDX1 | -11.11 |
| ENSG00000211955 | NA | 6.04 | ENSG00000102174 | PHEX | 2.59 |
| ENSG00000211939 | NA | 6.68 | ENSG00000146070 | PLA2G7 | -2.68 |
| ENSG00000229751 | NA | 8.45 | ENSG00000104368 | PLAT | 4.56 |
| ENSG00000240864 | NA | 5.66 | ENSG00000120278 | PLEKHG1 | 2.05 |
| ENSG00000211946 | NA | 9.20 | ENSG00000147872 | PLIN2 | -2.57 |
| ENSG00000185168 | NA | 5.08 | ENSG00000123560 | PLP1 | 8.58 |
| ENSG00000211950 | NA | 5.90 | ENSG00000188783 | PRELP | 2.32 |
| ENSG00000248220 | NA | -3.38 | ENSG00000116132 | PRRX1 | 3.32 |
| ENSG00000239855 | NA | 5.50 | ENSG00000113319 | RASGRF2 | 2.33 |
| ENSG00000250144 | NA | -2.26 | ENSG00000025039 | RRAGD | -1.60 |
| ENSG00000232869 | NA | 3.88 | ENSG00000168079 | SCARA5 | 9.32 |
| ENSG00000238024 | NA | -3.75 | ENSG00000099194 | SCD | -2.36 |
| ENSG00000223092 | NA | 8.33 | ENSG00000167680 | SEMA6B | -2.90 |
| ENSG00000240671 | NA | 5.88 | ENSG00000170054 | SERPINA9 | 10.61 |
| ENSG00000254174 | NA | 8.77 | ENSG00000197632 | SERPINB2 | -3.75 |
| ENSG00000211965 | NA | 5.89 | ENSG00000122852 | SFTPA1 | 7.67 |
| ENSG00000211669 | NA | 7.44 | ENSG00000185303 | SFTPA1 | 6.81 |
| ENSG00000211900 | NA | 5.46 | ENSG00000112394 | SLC16A10 | -1.80 |
| ENSG00000211943 | NA | 5.25 | ENSG00000108932 | SLC16A6 | -2.48 |

| | | |
|---|---|---|
| ENSG00000112759 | SLC29A1 | -1.64 |
| ENSG00000011083 | SLC6A7 | -4.44 |
| ENSG00000185985 | SLITRK2 | -3.98 |
| ENSG00000118785 | SPP1 | -3.29 |
| ENSG00000184905 | TCEAL2 | -2.61 |
| ENSG00000182916 | TCEAL7 | 4.63 |
| ENSG00000145107 | TM4SF19 | -3.23 |
| ENSG00000101255 | TRIB3 | -2.70 |
| ENSG00000182463 | TSHZ2 | 2.86 |
| ENSG00000182612 | TSPAN10 | -2.47 |
| ENSG00000155657 | TTN | 3.19 |
| ENSG00000104833 | TUBB4A | -3.12 |
| ENSG00000036672 | USP2 | -1.98 |
| ENSG00000128218 | VPREB3 | 5.35 |

**Table 29. Biomarkers represented by gene symbols and Ensembl IDs of aggressive PTC in BRAF negative samples.**

| Ensembl ID | Gene Symbol | Log2 Fold Change | Ensembl ID | Gene Symbol | Log2 Fold Change |
|---|---|---|---|---|---|
| | | | ENSG00000157851 | DPYSL5 | 4.12 |
| | | | ENSG00000134765 | DSC1 | -2.88 |
| | | | ENSG00000134760 | DSG1 | -9.98 |
| ENSG00000159251 | ACTC1 | -8.11 | ENSG00000198692 | EIF1AY | 4.32 |
| ENSG00000078549 | ADCYAP1R1 | -8.66 | ENSG00000188833 | ENTPD8 | 4.60 |
| ENSG00000067842 | ATP2B3 | 8.18 | ENSG00000165566 | FAM123A | 4.06 |
| ENSG00000105929 | ATP6V0A4 | 5.85 | ENSG00000198797 | FAM5B | 3.22 |
| ENSG00000182492 | BGN | 5.34 | ENSG00000090512 | FETUB | -8.51 |
| ENSG00000125999 | BPIFB1 | 3.86 | ENSG00000143631 | FLG | -2.59 |
| ENSG00000184459 | BPIFC | -9.15 | ENSG00000143520 | FLG2 | -4.60 |
| ENSG00000124920 | C11orf9 | 3.01 | ENSG00000155816 | FMN2 | 4.96 |
| ENSG00000198854 | C1orf68 | -9.08 | ENSG00000169933 | FRMPD4 | 6.77 |
| ENSG00000214097 | C3orf43 | 8.21 | ENSG00000170820 | FSHR | 7.25 |
| ENSG00000164764 | C8orf84 | 3.48 | ENSG00000100626 | GALNTL1 | 5.70 |
| ENSG00000178538 | CA8 | 5.54 | ENSG00000156466 | GDF6 | -8.46 |
| ENSG00000107159 | CA9 | 8.59 | ENSG00000151892 | GFRA1 | 5.28 |
| ENSG00000163618 | CADPS | 6.74 | ENSG00000170075 | GPR37L1 | 4.17 |
| ENSG00000178372 | CALML5 | -8.06 | ENSG00000183840 | GPR39 | -4.72 |
| ENSG00000077274 | CAPN6 | 4.16 | ENSG00000116983 | HPCAL4 | -3.08 |
| ENSG00000073754 | CD5L | -5.24 | ENSG00000173641 | HSPB7 | -2.24 |
| ENSG00000138395 | CDK15 | 4.79 | ENSG00000204866 | IGFL2 | 4.68 |
| ENSG00000184984 | CHRM5 | 7.60 | ENSG00000204869 | IGFL4 | 6.85 |
| ENSG00000172752 | COL6A5 | 6.13 | ENSG00000163501 | IHH | 8.95 |
| ENSG00000121898 | CPXM2 | -3.01 | ENSG00000169306 | IL1RAPL1 | 7.26 |
| ENSG00000121904 | CSMD2 | 4.13 | ENSG00000123243 | ITIH5 | 3.50 |
| ENSG00000170373 | CST1 | 5.84 | ENSG00000184408 | KCND2 | 5.75 |
| ENSG00000166265 | CYYR1 | 3.54 | ENSG00000151704 | KCNJ1 | -3.26 |
| ENSG00000067048 | DDX3Y | 8.30 | ENSG00000012817 | KDM5D | 6.86 |
| ENSG00000050165 | DKK3 | 3.85 | ENSG00000167749 | KLK4 | 5.74 |
| ENSG00000104371 | DKK4 | 4.09 | ENSG00000203786 | KPRP | -6.35 |
| ENSG00000187957 | DNER | 6.45 | ENSG00000186395 | KRT10 | -4.55 |

| | | | | | |
|---|---|---|---|---|---|
| ENSG00000172867 | KRT2 | -7.82 | ENSG00000249346 | NA | 3.53 |
| ENSG00000186081 | KRT5 | -3.99 | ENSG00000223414 | NA | -3.77 |
| ENSG00000189182 | KRT77 | -8.64 | ENSG00000225698 | NA | -4.08 |
| ENSG00000188508 | KRTDAP | -4.78 | ENSG00000253407 | NA | 4.70 |
| ENSG00000132130 | LHX1 | 9.02 | ENSG00000231165 | NA | 6.02 |
| ENSG00000203782 | LOR | -6.51 | ENSG00000232760 | NA | -7.73 |
| ENSG00000171517 | LPAR3 | 3.25 | ENSG00000213574 | NA | -4.67 |
| ENSG00000161572 | LYZL6 | 4.34 | ENSG00000235612 | NA | 3.92 |
| ENSG00000162510 | MATN1 | 8.31 | ENSG00000173376 | NDNF | 5.64 |
| ENSG00000130675 | MNX1 | 9.03 | ENSG00000084628 | NKAIN1 | 8.54 |
| ENSG00000186732 | MPPED1 | 8.76 | ENSG00000140807 | NKD1 | 5.62 |
| ENSG00000125414 | MYH2 | -7.01 | ENSG00000089250 | NOS1 | 3.94 |
| ENSG00000204936 | NA | 5.80 | ENSG00000185269 | NOTUM | 7.53 |
| ENSG00000233864 | NA | 5.21 | ENSG00000171246 | NPTX1 | 6.30 |
| ENSG00000223561 | NA | 6.97 | ENSG00000116833 | NR5A2 | 5.66 |
| ENSG00000230938 | NA | -9.71 | ENSG00000122718 | OR2S2 | 7.68 |
| ENSG00000211630 | NA | -5.11 | ENSG00000164920 | OSR2 | 7.56 |
| ENSG00000099725 | NA | 3.83 | ENSG00000163982 | OTOP1 | 8.65 |
| ENSG00000256723 | NA | -8.10 | ENSG00000154553 | PDLIM3 | -2.58 |
| ENSG00000240661 | NA | -6.78 | ENSG00000121440 | PDZRN3 | 5.36 |
| ENSG00000211642 | NA | -4.68 | ENSG00000082175 | PGR | 5.28 |
| ENSG00000249780 | NA | 4.36 | ENSG00000165443 | PHYHIPL | 7.38 |
| ENSG00000236511 | NA | 6.26 | ENSG00000081277 | PKP1 | -2.16 |
| ENSG00000255509 | NA | 8.11 | ENSG00000180287 | PLD5 | 6.80 |
| ENSG00000231535 | NA | 8.84 | ENSG00000124429 | POF1B | -2.80 |
| ENSG00000253858 | NA | 4.92 | ENSG00000196834 | POTEI | 5.42 |
| ENSG00000219088 | NA | 6.55 | ENSG00000074211 | PPP2R2C | 6.13 |
| ENSG00000150276 | NA | -3.56 | ENSG00000126583 | PRKCG | 5.19 |
| ENSG00000183663 | NA | -9.13 | ENSG00000171864 | PRND | 5.02 |
| ENSG00000248995 | NA | 8.64 | ENSG00000105894 | PTN | 5.88 |
| ENSG00000241665 | NA | 3.22 | ENSG00000196090 | PTPRT | 6.78 |
| ENSG00000248329 | NA | -6.97 | ENSG00000106278 | PTPRZ1 | -3.72 |
| ENSG00000254098 | NA | 5.12 | ENSG00000115386 | REG1A | -8.36 |

| | | | | | | |
|---|---|---|---|---|---|---|
| ENSG00000186479 | RGS7BP | 7.60 | | ENSG00000163032 | VSNL1 | 3.98 |
| ENSG00000129824 | RPS4Y1 | 9.04 | | ENSG00000175121 | WFDC5 | -5.26 |
| ENSG00000169218 | RSPO1 | 6.26 | | ENSG00000156076 | WIF1 | 7.03 |
| ENSG00000189001 | SBSN | -4.91 | | ENSG00000067646 | ZFY | 7.07 |
| ENSG00000165953 | SERPINA12 | -7.08 | | | | |
| ENSG00000170099 | SERPINA6 | 10.43 | | | | |
| ENSG00000166634 | SERPINB12 | -6.61 | | | | |
| ENSG00000197641 | SERPINB13 | -8.92 | | | | |
| ENSG00000166396 | SERPINB7 | -5.36 | | | | |
| ENSG00000104332 | SFRP1 | 5.23 | | | | |
| ENSG00000141485 | SLC13A5 | 5.92 | | | | |
| ENSG00000196660 | SLC30A10 | 7.23 | | | | |
| ENSG00000188176 | SMTNL2 | 9.23 | | | | |
| ENSG00000168875 | SOX14 | 9.58 | | | | |
| ENSG00000141255 | SPATA22 | 8.69 | | | | |
| ENSG00000153820 | SPHKAP | -9.85 | | | | |
| ENSG00000133710 | SPINK5 | -3.17 | | | | |
| ENSG00000203785 | SPRR2E | -8.15 | | | | |
| ENSG00000139973 | SYT16 | 5.68 | | | | |
| ENSG00000011347 | SYT7 | -2.66 | | | | |
| ENSG00000137203 | TFAP2A | -2.52 | | | | |
| ENSG00000154096 | THY1 | 4.66 | | | | |
| ENSG00000181234 | TMEM132C | 7.20 | | | | |
| ENSG00000133687 | TMTC1 | 3.68 | | | | |
| ENSG00000105048 | TNNT1 | 4.11 | | | | |
| ENSG00000170893 | TRH | 7.69 | | | | |
| ENSG00000127324 | TSPAN8 | -9.36 | | | | |
| ENSG00000099749 | TXLNG2P | 9.48 | | | | |
| ENSG00000131002 | TXLNG2P | 6.48 | | | | |
| ENSG00000243566 | UPK3B | 3.75 | | | | |
| ENSG00000114374 | USP9Y | 6.69 | | | | |
| ENSG00000183878 | UTY | 6.83 | | | | |
| ENSG00000170162 | VGLL2 | 9.23 | | | | |

**Table 30. Biomarkers represented by gene symbols and Ensembl IDs of aggressive PTC shared in both BRAF-negative and BRAF+ samples samples.**

| Ensembl ID | Gene Symbol | Chromosome |
|---|---|---|
| ENSG00000182492 | BGN | chrX |
| ENSG00000156466 | GDF6 | chr8 |
| ENSG00000151892 | GFRA1 | chr10 |
| ENSG00000167749 | KLK4 | chr19 |
| ENSG00000125414 | MYH2 | chr17 |
| ENSG00000225698 | NA | chr14 |
| ENSG00000211630 | NA | chr2 |
| ENSG00000171246 | NPTX1 | chr17 |

**List 1. Additional biomarkers represented by Ennsembl IDs of aggressive phenotype**

ENSG00000065320, ENSG00000069188, ENSG00000081248, ENSG00000103546, ENSG00000104237, ENSG00000106689, ENSG00000114805, ENSG00000119698, ENSG00000130176, ENSG00000130540, ENSG00000132972, ENSG00000134533, ENSG00000149403, ENSG00000150594, ENSG00000152092, ENSG00000154165, ENSG00000158458, ENSG00000163638, ENSG00000164116, ENSG00000164946, ENSG00000166923, ENSG00000167105, ENSG00000171509, ENSG00000178031, ENSG00000204262, ENSG00000206755, ENSG00000213938, ENSG00000226738, ENSG00000227844, ENSG00000232680, ENSG00000238337, ENSG00000253168

The data generated in these experiments may be used to measure the amount of variation observed with distinct WTA kits and microarray lots in order to train an algorithm that calculates and systematically normalizes signal intensities such that independent experiments can be directly compared.

Devices, methods and systems of the present disclosure can be combined with and/or modified by other devices, methods and systems, such as those described in US2013/0231258.

It should be understood from the foregoing that, while particular implementations have been illustrated and described, various modifications can be made thereto and are contemplated herein. It is intended that the following claims define the scope of the invention.

**Claims**

1. A method of identifying a thyroid cancer in a subject by identifying a BRAFV600E mutation in a fine needle aspiration sample of a subject using a trained algorithm, wherein said trained algorithm was trained by a process comprising:

    (i) assaying a plurality of training samples for expression levels of a plurality of biomarkers comprising at least 5 biomarkers selected from Table 27 to obtain a training set comprising expression data, wherein said plurality of training samples comprises a first set of samples containing said BRAFV600E mutation and a second set of samples not containing said BRAFV600E mutation, wherein said plurality of training samples comprise fine needle aspirate (FNA) samples, and
    (ii) performing supervised learning on said expression data to yield said trained algorithm that is capable of classifying said fine needle aspiration sample as containing or not containing said BRAFV600E mutation at a statistical confidence level of at least about 95%;
    the method comprising:

    (a) assaying an expression level of a plurality of biomarkers in said fine needle aspiration sample from said subject, wherein said plurality of biomarkers comprises said at least 5 biomarkers selected from Table 27, wherein said fine needle aspiration sample is independent of said plurality of training samples; and
    (b) applying said trained algorithm to said expression level of (a) to classify said fine needle aspiration sample as containing or not containing said BRAFV600E mutation at a statistical confidence level of at least about 95%, to thereby predict a presence or absence of said thyroid cancer.

**2.** The method of claim 1, wherein said trained algorithm comprises a linear support vector machine classifier.

**3.** The method of claim 1, further comprising selecting a treatment for said subject based upon classification of said fine needle aspiration sample in (b).

**4.** The method of claim 1, further comprising classifying said fine needle aspiration sample as containing or not containing follicular tissue or cells using a clinical classifier comprising a plurality of genes selected from Table 14 or Table 15.

**5.** The method of claim 1, further comprising using a clinical classifier comprising a plurality of genes selected from Table 2, Table 9, or Table 10.

**6.** The method of claim 1, wherein (b) comprises an estimate of a proportion of said thyroid cancer in said fine needle aspiration sample.

**7.** The method of claim 1, wherein said fine needle aspiration sample is classified as containing or not containing said BRAFV600E mutation with an accuracy of at least 95%.

**8.** The method of claim 1, further comprising pre-screening said fine needle aspiration sample to identify a presence of a confounding condition, prior to (b), and wherein, based on said pre-screening, a rate of false positives returned by said trained algorithm is reduced.

**9.** The method of claim 1, wherein said at least 5 biomarkers further comprise one or more biomarkers selected from Table 24.


**Patentansprüche**

**1.** Verfahren zur Identifizierung eines Schilddrüsenkarzinoms in einem Individuum durch Identifizieren einer BRAFV600E-Mutation in einer Feinnadelaspirationsprobe eines Individuums unter Verwendung eines trainierten Algorithmus, wobei der trainierte Algorithmus durch einen Prozess trainiert wurde, der umfasst:

(i) Untersuchen einer Mehrzahl von Trainingsproben auf Expressionsstärken einer Mehrzahl von Biomarkern, die mindestens 5 aus Tabelle 27 ausgewählte Biomarker umfasst, um einen Trainingssatz zu erhalten, der Expressionsdaten umfasst, wobei die Mehrzahl von Trainingsproben einen ersten Satz von Proben, der die BRAFV600E-Mutation enthält, und einen zweiten Satz von Proben umfasst, der die BRAFV600E-Mutation nicht enthält, wobei die Mehrzahl von Trainingsproben Feinnadelaspirations-(FNA-)Proben umfasst, und
(ii) Durchführen überwachten Lernens an den Expressionsdaten, um den trainierten Algorithmus zu ergeben, der die Feinnadelaspirationsprobe mit einer statistischen Sicherheit von mindestens etwa 95 % als die BRAFV600E-Mutation enthaltend oder nicht enthaltend klassifizieren kann;
wobei das Verfahren umfasst:

(a) Untersuchen einer Expressionsstärke einer Mehrzahl von Biomarkern in der Feinnadelaspirationsprobe vom Individuum, wobei die Mehrzahl von Biomarkern die mindestens 5 aus Tabelle 27 ausgewählten Biomarkers umfasst, wobei die Feinnadelaspirationsprobe von der Mehrzahl von Trainingsproben unabhängig ist; und
(b) Anwenden des trainierten Algorithmus auf die Expressionsstärke von (a), um die Feinnadelaspirationsprobe mit einer statistischen Sicherheit von mindestens 95 % als die BRAFV600E-Mutation enthaltend oder nicht enthaltend zu klassifizieren, um dadurch ein Vorliegen oder Nichtvorliegen des Schilddrüsenkarzinoms vorherzusagen.

**2.** Verfahren nach Anspruch 1, wobei der trainierte Algorithmus einen linearen Support-Vector-Machine-Klassifikator umfasst.

**3.** Verfahren nach Anspruch 1, ferner umfassend ein Auswählen einer Behandlung für das Individuum basierend auf der Klassifizierung der Feinnadelaspirationsprobe in (b).

**4.** Verfahren nach Anspruch 1, ferner umfassend ein Klassifizieren der Feinnadelaspirationsprobe als Follikelgewebe oder Follikelzellen enthaltend oder nicht enthaltend unter Verwendung eines klinischen Klassifikators, der eine Mehrzahl von aus Tabelle 14 oder Tabelle 15 ausgewählten Genen umfasst.

5. Verfahren nach Anspruch 1, ferner umfassend ein Verwenden eines klinischen Klassifikators, der eine Mehrzahl von aus Tabelle 2, Tabelle 9 oder Tabelle 10 ausgewählten Genen umfasst.

6. Verfahren nach Anspruch 1, wobei (b) ein Schätzen eines Anteils des Schilddrüsenkarzinoms in der Feinnadelaspirationsprobe umfasst.

7. Verfahren nach Anspruch 1, wobei die Feinnadelaspirationsprobe mit einer Genauigkeit von mindestens 95 % als die BRAFV600E-Mutation enthaltend oder nicht enthaltend klassifiziert wird.

8. Verfahren nach Anspruch 1, ferner umfassend ein Durchführen von Prescreening an der Feinnadelaspirationsprobe vor (b), um ein Vorliegen eines Konfundierungszustands zu identifizieren, und wobei basierend auf dem Prescreening eine vom trainierten Algorithmus zurückgegebene Rate falsch positiver Ergebnisse reduziert wird.

9. Verfahren nach Anspruch 1, wobei die mindestens 5 Biomarker ferner einen oder mehrere aus Tabelle 24 ausgewählte Biomarker umfassen.

**Revendications**

1. Procédé d'identification d'un cancer de la thyroïde chez un sujet en identifiant une mutation BRAFV600E dans un échantillon d'aspiration à l'aiguille fine d'un sujet en utilisant un algorithme entraîné, où ledit algorithme entraîné a été entraîné par un processus comprenant :

(i) le dosage d'une pluralité d'échantillons d'entraînement pour les niveaux d'expression d'une pluralité de biomarqueurs comprenant au moins 5 biomarqueurs choisis dans le Tableau 27 pour obtenir un ensemble d'entraînement comprenant des données d'expression, où ladite pluralité d'échantillons d'entraînement comprend un premier ensemble d'échantillons contenant ladite mutation BRAFV600E et un deuxième ensemble d'échantillons ne contenant pas ladite mutation BRAFV600E, où ladite pluralité d'échantillons d'entraînement comprend des échantillons d'aspirat à l'aiguille fine (FNA), et
(ii) la réalisation d'un apprentissage supervisé sur lesdites données d'expression pour obtenir ledit algorithme entraîné qui est capable de classer ledit échantillon d'aspiration à l'aiguille fine comme contenant ou non ladite mutation BRAFV600E à un niveau de confiance statistique d'au moins environ 95% ;
le procédé, comprenant :

(a) le dosage d'un niveau d'expression d'une pluralité de biomarqueurs dans ledit échantillon d'aspiration à l'aiguille fine dudit sujet, où ladite pluralité de biomarqueurs comprend lesdits au moins 5 biomarqueurs choisis dans le Tableau 27, où ledit échantillon d'aspiration à l'aiguille fine est indépendant de ladite pluralité d'échantillons d'entraînement ; et
(b) l'application dudit algorithme entraîné audit niveau d'expression de (a) pour classer ledit échantillon d'aspiration à l'aiguille fine comme contenant ou non ladite mutation BRAFV600E à un niveau de confiance statistique d'au moins environ 95%, pour prédire ainsi la présence ou l'absence dudit cancer de la thyroïde.

2. Procédé de la revendication 1, dans lequel ledit algorithme entraîné comprend un classificateur de machine à vecteurs de support linéaire.

3. Procédé de la revendication 1, comprenant en outre la sélection d'un traitement pour ledit sujet sur la base de la classification dudit échantillon d'aspiration à l'aiguille fine dans (b).

4. Procédé de la revendication 1, comprenant en outre la classification dudit échantillon d'aspiration à l'aiguille fine comme contenant ou non un tissu ou des cellules folliculaire(s) en utilisant un classificateur clinique comprenant une pluralité de gènes choisis dans le Tableau 14 ou le Tableau 15.

5. Procédé de la revendication 1, comprenant en outre l'utilisation d'un classificateur clinique comprenant une pluralité de gènes choisis dans le Tableau 2, le Tableau 9, ou le Tableau 10.

**6.** Procédé de la revendication 1, dans lequel (b) comprend une estimation d'une proportion dudit cancer de la thyroïde dans ledit échantillon d'aspiration à l'aiguille fine.

**7.** Procédé de la revendication 1, dans lequel ledit échantillon d'aspiration à l'aiguille fine est classé comme contenant ou non ladite mutation BRAFV600E avec une précision d'au moins 95%.

**8.** Procédé de la revendication 1, comprenant en outre le pré-criblage dudit échantillon d'aspiration à l'aiguille fine pour identifier la présence d'une affection parasite, avant (b), et où, sur la base dudit pré-criblage, un taux de faux positifs renvoyés par ledit algorithme entraîné est réduit.

**9.** Procédé de la revendication 1, dans lequel lesdits au moins 5 biomarqueurs comprennent en outre un ou plusieurs biomarqueurs choisis dans le Tableau 24.

**FIGURE 1A**

Fig.1B

FIGURE 1B

# Fig.1C

Fig.2

**Gene Biomarker Panels for Diagnosing a Thyroid Condition**

1   Normal Thyroid (NML)
2   Lymphocytic, Autoimmune Thyroiditis (LCT)
3   Nodular Hyperplasia (NHP)
4   Follicular Thyroid Adenoma (FA)
5   Hurthle Cell Thyroid Adenoma (HA)
6   Parathyroid (non thyroid tissue) (PTA)
7   Anaplastic Thyroid Carcinoma (ATC)
8   Follicular Thyroid Carcinoma (FC)
9   Hurthle Cell Thyroid Carcinoma (HC)
10  Papillary Thyroid Carcinoma (PTC)
11  Follicular Variant of Papillary Carcinoma (FVPTC)
12  Medullary Thyroid Carcinoma (MTC)
13  Renal Carcinoma metastasis to the Thyroid (RCC)
14  Melanoma metastasis to the Thyroid (MMN)
15  B cell Lymphoma metastasis to the Thyroid (BCL)
16  Breast Carcinoma metastasis to the Thyroid (BCA)

Fig.3

FIGURE 3

**Optimized Gene Classification Panels of Diagnostic Utility in Thyroid FNA**

| Classification Panel | Subtype-specific Classifier Components | Number of Markers Per Panel |
|---|---|---|
| 1 | MTC | 5 |
| 2 | RCC | 5 |
| 3 | PTA | 5 |
| 4 | BCA | 5 |
| 5 | MMN | 5 |
| 6 | HA & HC[1] | 33 |
| 7 | Benign/Suspicious[2] | 142 |

[1] denotes the combination of two panels -HA and HC- into a single panel.

[2] denotes the combination of eight panels -FA, FC, NHP, PTC, FVPTC, LCT, HA, HC- into a single panel.

## Fig.4A

| TCID | GENE | Gene Description | Classification Panel |
|---|---|---|---|
| 3364127 | CALCA | calcitonin-related polypeptide alpha | MTC |
| 3834341 | CEACAM5 | carcinoembryonic antigen-related cell adhesion molecule 5 | MTC |
| 3594008 | SCG3 | secretogranin III | MTC |
| 2585400 | SCN2A | sodium channel, voltage-gated, type II, alpha subunit | MTC |
| 2585400 | SCN9A | sodium channel, voltage-gated, type IX, alpha subunit | MTC |
| 3803614 | SYT4 | synaptotagmin IV | MTC |
| 2923928 | FABP7 | fatty acid binding protein 7, brain | RCC |
| 3393446 | FXYD2 | FXYD domain containing ion transport regulator 2 | RCC |
| 2883317 | HAVCR1 | hepatitis A virus cellular receptor 1 | RCC |
| 2883317 | LOC100101266 | hepatitis A virus cellular receptor 1 pseudogene | RCC |
| 3428225 | NR1H4 | nuclear receptor subfamily 1, group H, member 4 | RCC |
| 2479698 | PREPL | prolyl endopeptidase-like | RCC |
| 2479698 | SLC3A1 | solute carrier family 3 (cystine, dibasic and neutral amino acid transporters, activator of cystine, dibasic and neutral amino acid transport), member 1 | RCC |
| 3159754 | DMRT2 | doublesex and mab-3 related transcription factor 2 | PTA |
| 2941699 | GCM2 | glial cells missing homolog 2 (Drosophila) | PTA |
| 3361686 | KIDINS220 | kinase D-interacting substrate, 220kDa | PTA |
| 3484893 | KL | klotho | PTA |
| 3363686 | PTH | parathyroid hormone | PTA |
| 2894799 | SYCP2L | synaptonemal complex protein 2-like | PTA |
| 3039830 | AGR3 | anterior gradient homolog 3 (Xenopus laevis) | BCA |
| 3264997 | C10orf81 | chromosome 10 open reading frame 81 | BCA |
| 2926802 | MYB | v-myb myeloblastosis viral oncogene homolog (avian) | BCA |
| 3912079 | SYCP2 | synaptonemal complex protein 2 | BCA |
| 2430163 | VTCN1 | V-set domain containing T cell activation inhibitor 1 | BCA |
| 3811949 | CDH19 | cadherin 19, type 2 | MMN |

Fig.4B

| TCID | GENE | Gene Description | Classification Panel |
|---|---|---|---|
| 3161261 | MLANA | melan-A | MMN |
| 3935486 | S100B | S100 calcium binding protein B | MMN |
| 3417336 | SILV | silver homolog (mouse) | MMN |
| 3343832 | TYR | tyrosinase (oculocutaneous albinism IA) | MMN |
| 3343832 | TYRL | tyrosinase-like (pseudogene) | MMN |
| 2566848 | AFF3 | AF4/FMR2 family, member 3 | HA & HC, Benign/Suspicious |
| 2988882 | AIMP2 | aminoacyl tRNA synthetase complex-interacting multifunctional protein 2 | HA & HC, Benign/Suspicious |
| 3169331 | ALDH1B1 | aldehyde dehydrogenase 1 family, member B1 | HA & HC, Benign/Suspicious |
| 2984616 | BRP44L | brain protein 44-like | HA & HC, Benign/Suspicious |
| 2822492 | C5orf30 | chromosome 5 open reading frame 30 | HA & HC, Benign/Suspicious |
| 3326635 | CD44 | CD44 molecule (Indian blood group) | HA & HC, Benign/Suspicious |
| 2750627 | CPE | carboxypeptidase E | HA & HC, Benign/Suspicious |
| 3042001 | CYCS | cytochrome c, somatic | HA & HC, Benign/Suspicious |
| 3122678 | DEFB1 | defensin, beta 1 | HA & HC, Benign/Suspicious |
| 2739308 | EGF | epidermal growth factor (beta-urogastrone) | HA & HC, Benign/Suspicious |
| 2988882 | EIF2AK1 | eukaryotic translation initiation factor 2-alpha kinase 1 | HA & HC, Benign/Suspicious |
| 3603932 | FAH | fumarylacetoacetate hydrolase (fumarylacetoacetase) | HA & HC, Benign/Suspicious |
| 2970897 | FRK | fyn-related kinase | HA & HC, Benign/Suspicious |
| 3212008 | FRMD3 | FERM domain containing 3 | HA & HC, Benign/Suspicious |
| 3302990 | GOT1 | glutamic-oxaloacetic transaminase 1, soluble (aspartate aminotransferase 1) | HA & HC, Benign/Suspicious |
| 3417703 | HSD17B6 | hydroxysteroid (17-beta) dehydrogenase 6 homolog (mouse) | HA & HC, Benign/Suspicious |
| 2877508 | HSPA9 | heat shock 70kDa protein 9 (mortalin) | HA & HC, Benign/Suspicious |

## Fig.4C

| TCID | GENE | Gene Description | Classification Panel |
|---|---|---|---|
| 2708922 | IGF2BP2 | insulin-like growth factor 2 mRNA binding protein 2 | HA & HC, Benign/Suspicious |
| 2604998 | IQCA1 | IQ motif containing with AAA domain 1 | HA & HC, Benign/Suspicious |
| 3724543 | ITGB3 | integrin, beta 3 (platelet glycoprotein IIIa, antigen CD61) | HA & HC, Benign/Suspicious |
| 3397774 | KCNJ1 | potassium inwardly-rectifying channel, subfamily J, member 1 | HA & HC, Benign/Suspicious |
| 2604998 | LOC100129258 | hypothetical protein LOC100129258 | HA & HC, Benign/Suspicious |
| 3005299 | MDH2 | malate dehydrogenase 2, NAD (mitochondrial) | HA & HC, Benign/Suspicious |
| 3654699 | NUPR1 | nuclear protein, transcriptional regulator, 1 | HA & HC, Benign/Suspicious |
| 4020655 | OD21 | odz, odd Oz/ten-m homolog 1(Drosophila) | HA & HC, Benign/Suspicious |
| 3970833 | PDHA1 | pyruvate dehydrogenase (lipoamide) alpha 1 | HA & HC, Benign/Suspicious |
| 2377094 | PFKFB2 | 6-phosphofructo-2-kinase/fructose-2,6-biphosphatase 2 | HA & HC, Benign/Suspicious |
| 3278198 | PHYH | phytanoyl-CoA 2-hydroxylase | HA & HC, Benign/Suspicious |
| 2860051 | PPP2R2B | protein phosphatase 2 (formerly 2A), regulatory subunit B, beta isoform | HA & HC, Benign/Suspicious |
| 3939862 | PVALB | parvalbumin | HA & HC, Benign/Suspicious |
| 2488499 | PVRL2 | poliovirus receptor-related 2 (herpesvirus entry mediator B) | HA & HC, Benign/Suspicious |
| 2604998 | RPL3 | ribosomal protein L3 | HA & HC, Benign/Suspicious |
| 2964231 | RRAGD | Ras-related GTP binding D | HA & HC, Benign/Suspicious |
| 2798538 | SDHA | succinate dehydrogenase complex, subunit A, flavoprotein (Fp) | HA & HC, Benign/Suspicious |
| 2798538 | SDHALP1 | succinate dehydrogenase complex, subunit A, flavoprotein pseudogene 1 | HA & HC, Benign/Suspicious |
| 2798538 | SDHALP2 | succinate dehydrogenase complex, subunit A, flavoprotein pseudogene 2 | HA & HC, Benign/Suspicious |

# Fig.4D

| TCID | GENE | Gene Description | Classification Panel |
|---|---|---|---|
| 2798538 | SDHAP3 | succinate dehydrogenase complex, subunit A, flavoprotein pseudogene 3 | HA & HC, Benign/Suspicious |
| 2428501 | SLC16A1 | solute carrier family 16, member 1 (monocarboxylic acid transporter 1) | HA & HC, Benign/Suspicious |
| 2877508 | SNORD63 | small nucleolar RNA, C/D box 63 | HA & HC, Benign/Suspicious |
| 2562529 | ST3GAL5 | ST3 beta-galactoside alpha-2,3-sialyltransferase 5 | HA & HC, Benign/Suspicious |
| 2688499 | ZBED2 | zinc finger, BED-type containing 2 | HA & HC, Benign/Suspicious |
| 3450861 | ABCD2 | ATP-binding cassette, sub-family D (ALD), member 2 | Benign/Suspicious |
| 3341061 | ACER3 | alkaline ceramidase 3 | Benign/Suspicious |
| 2796353 | ACSL1 | acyl-CoA synthetase long-chain family member 1 | Benign/Suspicious |
| 3373733 | AHNAK | AHNAK nucleoprotein | Benign/Suspicious |
| 2439354 | AIM2 | absent in melanoma 2 | Benign/Suspicious |
| 3768474 | ARSG | arylsulfatase G | Benign/Suspicious |
| 3214845 | ASPN | asporin | Benign/Suspicious |
| 3006572 | AUTS2 | autism susceptibility candidate 2 | Benign/Suspicious |
| 3902489 | BCL2L1 | BCL2-like 1 | Benign/Suspicious |
| 2688717 | BTLA | B and T lymphocyte associated | Benign/Suspicious |
| 2708851 | C11orf72 | chromosome 11 open reading frame 72 | Benign/Suspicious |
| 2730303 | C4orf7 | chromosome 4 open reading frame 7 | Benign/Suspicious |
| 3239367 | CC2D2B | coiled-coil and C2 domain containing 2B | Benign/Suspicious |
| 3204285 | CCL19 | chemokine (C-C motif) ligand 19 | Benign/Suspicious |
| 3338192 | CCND1 | cyclin D1 | Benign/Suspicious |
| 3810303 | CD36 | CD36 molecule (thrombospondin receptor) | Benign/Suspicious |
| 2326463 | CD52 | CD52 molecule | Benign/Suspicious |
| 2633741 | CD96 | CD96 molecule | Benign/Suspicious |
| 2373336 | CFH | complement factor H | Benign/Suspicious |
| 2373338 | CFHR1 | complement factor H-related 1 | Benign/Suspicious |
| 2710599 | CLDN1 | claudin 1 | Benign/Suspicious |
| 2657808 | CLDN16 | claudin 16 | Benign/Suspicious |

EP 2 971 164 B1

## Fig.4E

| TCID | GENE | Gene Description | Classification Panel |
|------|------|------------------|----------------------|
| 2377283 | CR2 | complement component (3d/Epstein Barr virus) receptor 2 | Benign Suspicious |
| 3242353 | CREM | cAMP responsive element modulator | Benign Suspicious |
| 2490351 | CTNNA2 | catenin (cadherin-associated protein), alpha 2 | Benign Suspicious |
| 2732508 | CXCL13 | chemokine (C-X-C motif) ligand 13 | Benign Suspicious |
| 2834445 | DAB2 | disabled homolog 2, mitogen-responsive phosphoprotein (Drosophila) | Benign Suspicious |
| 2321911 | DDI2 | DDI1, DNA-damage inducible 1, homolog 2 (S. cerevisiae) | Benign Suspicious |
| 2642791 | DNAJC13 | DnaJ (Hsp40) homolog, subfamily C, member 13 | Benign Suspicious |
| 2584018 | DPP4 | dipeptidyl-peptidase 4 | Benign Suspicious |
| 3032647 | DPP6 | dipeptidyl-peptidase 6 | Benign Suspicious |
| 2981874 | DYNLT1 | dynein, light chain, Tctex-type 1 | Benign Suspicious |
| 2638676 | EAF2 | ELL associated factor 2 | Benign Suspicious |
| 3852832 | EMR3 | egf-like module containing, mucin-like, hormone receptor-like 3 | Benign Suspicious |
| 3142381 | FABP4 | fatty acid binding protein 4, adipocyte | Benign Suspicious |
| 2396730 | FBXO2 | F-box protein 2 | Benign Suspicious |
| 3338192 | FLJ42258 | FLJ42258 protein | Benign Suspicious |
| 2526806 | FN1 | fibronectin 1 | Benign Suspicious |
| 2598261 | FN1 | fibronectin 1 | Benign Suspicious |
| 3839910 | FPR2 | formyl peptide receptor 2 | Benign Suspicious |
| 3486096 | FREM2 | FRAS1 related extracellular matrix protein 2 | Benign Suspicious |
| 3393479 | FXYD6 | FXYD domain containing ion transport regulator 6 | Benign Suspicious |
| 2378088 | G0S2 | G0/G1switch 2 | Benign Suspicious |
| 2894845 | GABRB2 | gamma-aminobutyric acid (GABA) A receptor, beta 2 | Benign Suspicious |
| 3063795 | GAL3ST4 | galactose-3-O-sulfotransferase 4 | Benign Suspicious |
| 3031556 | GIMAP2 | GTPase, IMAP family member 2 | Benign Suspicious |
| 3861948 | GMFG | glia maturation factor, gamma | Benign Suspicious |
| 3340862 | GPHN | gephyrin | Benign Suspicious |
| 3982612 | GPR174 | G protein-coupled receptor 174 | Benign Suspicious |
| 2809793 | GZMK | granzyme K (granzyme 3; tryptase II) | Benign Suspicious |
| 2638676 | HCG11 | HLA complex group 11 | Benign Suspicious |
| 2332609 | HNRNPA3 | heterogeneous nuclear ribonucleoprotein A3 | Benign Suspicious |
| 3375738 | IGHG1 | immunoglobulin heavy constant gamma 1 (G1m marker) | Benign Suspicious |
| 2806468 | IL7R | interleukin 7 receptor | Benign Suspicious |

180

## Fig.4F

| TCID | GENE | Gene Description | Classification Panel |
|---|---|---|---|
| 2377283 | CR2 | complement component (3d/Epstein Barr virus) receptor 2 | Benign Suspicious |
| 3242353 | CREM | cAMP responsive element modulator | Benign Suspicious |
| 2490351 | CTNNA2 | catenin (cadherin-associated protein), alpha 2 | Benign Suspicious |
| 2732508 | CXCL13 | chemokine (C-X-C motif) ligand 13 | Benign Suspicious |
| 2834445 | DAB2 | disabled homolog 2, mitogen-responsive phosphoprotein (Drosophila) | Benign Suspicious |
| 2321911 | DDI2 | DDI1, DNA-damage inducible 1, homolog 2 (S. cerevisiae) | Benign Suspicious |
| 2642791 | DNAJC13 | DnaJ (Hsp40) homolog, subfamily C, member 13 | Benign Suspicious |
| 2584018 | DPP4 | dipeptidyl-peptidase 4 | Benign Suspicious |
| 3032647 | DPP6 | dipeptidyl-peptidase 6 | Benign Suspicious |
| 2981874 | DYNLT1 | dynein, light chain, Tctex-type 1 | Benign Suspicious |
| 2638676 | EAF2 | ELL associated factor 2 | Benign Suspicious |
| 3852832 | EMR3 | egf-like module containing, mucin-like, hormone receptor-like 3 | Benign Suspicious |
| 3142381 | FABP4 | fatty acid binding protein 4, adipocyte | Benign Suspicious |
| 2396750 | FBXO2 | F-box protein 2 | Benign Suspicious |
| 3338192 | FLJ42258 | FLJ42258 protein | Benign Suspicious |
| 2526806 | FN1 | fibronectin 1 | Benign Suspicious |
| 2598281 | FN1 | fibronectin 1 | Benign Suspicious |
| 3839910 | FPR2 | formyl peptide receptor 2 | Benign Suspicious |
| 3486096 | FREM2 | FRAS1 related extracellular matrix protein 2 | Benign Suspicious |
| 2393479 | FXYD6 | FXYD domain containing ion transport regulator 6 | Benign Suspicious |
| 2378068 | G0S2 | G0/G1switch 2 | Benign Suspicious |
| 2884845 | GABRB2 | gamma-aminobutyric acid (GABA) A receptor, beta 2 | Benign Suspicious |
| 3063795 | GAL3ST4 | galactose-3-O-sulfotransferase 4 | Benign Suspicious |
| 3031556 | GIMAP2 | GTPase, IMAP family member 2 | Benign Suspicious |
| 3861948 | GMFG | glia maturation factor, gamma | Benign Suspicious |
| 3540862 | GPHN | gephyrin | Benign Suspicious |
| 3982612 | GPR174 | G protein-coupled receptor 174 | Benign Suspicious |
| 2809793 | GZMK | granzyme K (granzyme 3; tryptase II) | Benign Suspicious |
| 2638676 | HCG11 | HLA complex group 11 | Benign Suspicious |
| 2332609 | HNRNPA3 | heterogeneous nuclear ribonucleoprotein A3 | Benign Suspicious |
| 3375738 | IGHG1 | immunoglobulin heavy constant gamma 1 (G1m marker) | Benign Suspicious |
| 2806468 | IL7R | interleukin 7 receptor | Benign Suspicious |

## Fig.4G

| TCID | GENE | Gene Description | Classification Panel |
|---|---|---|---|
| 3875642 | PLCB1 | phospholipase C, beta 1 (phosphoinositide-specific) | Benign/Suspicious |
| 2486811 | PLEK | pleckstrin | Benign/Suspicious |
| 3246888 | PRKG1 | protein kinase, cGMP-dependent, type I | Benign/Suspicious |
| 3874731 | PRNP | prion protein | Benign/Suspicious |
| 2685304 | PROS1 | protein S (alpha) | Benign/Suspicious |
| 2373842 | PTPRC | protein tyrosine phosphatase, receptor type, C | Benign/Suspicious |
| 3270270 | PTPRE | protein tyrosine phosphatase, receptor type, E | Benign/Suspicious |
| 3564210 | PYGL | phosphorylase, glycogen, liver | Benign/Suspicious |
| 2362351 | PYHIN1 | pyrin and HIN domain family, member 1 | Benign/Suspicious |
| 3443464 | PZP | pregnancy-zone protein | Benign/Suspicious |
| 2372812 | RGS13 | regulator of G-protein signaling 13 | Benign/Suspicious |
| 3110395 | RIMS2 | regulating synaptic membrane exocytosis 2 | Benign/Suspicious |
| 3895795 | RNF24 | ring finger protein 24 | Benign/Suspicious |
| 2721959 | ROS1 | c-ros oncogene 1 , receptor tyrosine kinase | Benign/Suspicious |
| 2442308 | RXRG | retinoid X receptor, gamma | Benign/Suspicious |
| 3494629 | SCEL | sciellin | Benign/Suspicious |
| 2904485 | SCUBE3 | signal peptide, CUB domain, EGF-like 3 | Benign/Suspicious |
| 3059667 | SEMA3D | sema domain, immunoglobulin domain (Ig), short basic domain, secreted, (semaphorin) 3D | Benign/Suspicious |
| 3365136 | SERGEF | secretion regulating guanine nucleotide exchange factor | Benign/Suspicious |
| 3577612 | SERPINA1 | serpin peptidase inhibitor, clade A (alpha-1 antiproteinase, antitrypsin), member 1 | Benign/Suspicious |
| 3577612 | SERPINA2 | serpin peptidase inhibitor, clade A (alpha-1 antiproteinase, antitrypsin), member 2 | Benign/Suspicious |
| 3759006 | SHC1 | SHC (Src homology 2 domain containing) transforming protein 1 | Benign/Suspicious |
| 2440258 | SLAMF6 | SLAM family member 6 | Benign/Suspicious |
| 3622934 | SLC24A5 | solute carrier family 24, member 5 | Benign/Suspicious |
| 3185522 | SLC31A1 | solute carrier family 31 (copper transporters), member 1 | Benign/Suspicious |
| 2721959 | SLC34A2 | solute carrier family 34 (sodium phosphate), member 2 | Benign/Suspicious |
| 3761959 | SLC35B1 | solute carrier family 35, member B1 | Benign/Suspicious |
| 3373845 | SLC43A3 | solute carrier family 43, member 3 | Benign/Suspicious |
| 3759006 | SLC4A1 | solute carrier family 4, anion exchanger, member 1 (erythrocyte membrane protein band 3, Diego blood group) | Benign/Suspicious |
| 2730746 | SLC4A4 | solute carrier family 4, sodium bicarbonate cotransporter, member 4 | Benign/Suspicious |

Fig.4H

| TCID | GENE | Gene Description | Classification Panel |
|---|---|---|---|
| 2777714 | SNCA | synuclein, alpha (non A4 component of amyloid precursor) | Benign Suspicious |
| 2834282 | STK32A | serine/threonine kinase 32A | Benign Suspicious |
| 3341497 | THRSP | thyroid hormone responsive (SPOT14 homolog, rat) | Benign Suspicious |
| 3976341 | TIMP1 | TIMP metallopeptidase inhibitor 1 | Benign Suspicious |
| 3772661 | TIMP2 | TIMP metallopeptidase inhibitor 2 | Benign Suspicious |
| 2491271 | TMSB10 | thymosin beta 10 | Benign Suspicious |
| 3648391 | TNFRSF17 | tumor necrosis factor receptor superfamily, member 17 | Benign Suspicious |
| 3441849 | TNFRSF1A | tumor necrosis factor receptor superfamily, member 1A | Benign Suspicious |
| 2412668 | TXNDC12 | thioredoxin domain containing 12 (endoplasmic reticulum) | Benign Suspicious |
| 3353914 | VWA5A | von Willebrand factor A domain containing 5A | Benign Suspicious |
| 3976766 | WAS | Wiskott-Aldrich syndrome (eczema-thrombocytopenia) | Benign Suspicious |
| 3768474 | WIPI1 | WD repeat domain, phosphoinositide interacting 1 | Benign Suspicious |
| 2817731 | ZFYVE16 | zinc finger, FYVE domain containing 16 | Benign Suspicious |
| 4027385 | unknown | | Benign Suspicious |

Fig.5

Fig.6

Fig.7

Fig.8

SLC4A1

Fig.9

**FPR2**

# Fig.10

## EMR3

Fig.11

Fig.12

Comparison of estimated and design proportions of blood in the mixing study samples

Fig.13

Distribution of estimated blood proportion in CVP data, cyto-1

N=265

Number of samples

Estimated blood proportion

2.6 %  1.5 %  1.1 %  0.4 %  2.3 %

Fig.14A

# Fig.14B

Fig.15

In vitro vs. in silico (M0 method) linear classifier scores

In silico score

In Vitro score

# Fig.16

Fig.17

Mixing simulation, C1A231P/C1A231X

# Fig.18

## Fig.19

QUAL > 20, strandp <0.01, DP > 10

EP 2 971 164 B1

Fig.20

SNPs with QUAL > 20, DP > 10

# Fig.21

SNPs with QUAL > 100, DP > 20

## Fig.22

# Fig.23

# Fig.24A

Median expression by pos within transcript

Fig.24B

Median expression by pos within transcript

Fig.24C

Median expression by pos within transcript

Fig.24D

Fig.25A

Fig.25B

Fig.25C

Fig.25D

Fig.25E

## Fig.25F

## Fig.25H

Fig.26

Fig.27A

Fig. 27B

Fig.28

# Classification results: ROC curve

Training set excl 021 samples, updated labels

Fig.29

Fig.30

## Fig.31

| WTA kit lot | A1 primer lot | Experiment name |
|-------------|---------------|-----------------|
| Lot 11 | Lot 11 | Lot 11 Control |
| Lot 11 | Lot 12 | Lot 11 swap |
| Lot 12 | Lot 12 | Lot 12 Control |
| Lot 12 | Lot 11 | Lot 12 swap |

Fig.32

Fig.33

Fig.34

Fig.35

Fig.36

Fig.37

Fig.38A

Fig.38B

# Fig.38C

Fig.38D

Fig.38E

Fig.38F

Fig.39A

162 DE genes

Fig.39B

## Fig.39C

# Fig.39D

Fig.39E

Fig.39F

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61798941
- WO 2011143361 A2
- WO 2010056374 A2
- US 3645691 A
- US 7335762 B
- US 7323305 B
- US 7264929 B
- US 7244559 B
- US 7211390 B
- US 7361488 B
- US 7300788 B
- US 7280922 B
- US 7358061 B
- US 7319011 B
- US 5965360 A
- US 6436642 B
- US 20030186248 A
- US 20050042222 A
- US 20030190602 A
- US 20050048533 A
- US 20050266443 A
- US 20060035244 A
- US 2006083744 A
- US 20060088851 A
- US 20060105360 A
- US 20060127907 A
- US 20070020657 A
- US 20070037186 A
- US 20070065833 A
- US 20070161004 A
- US 20070238119 A
- US 20080044824 A
- US 20080131892 A
- US 2006019615 A
- US 2009006162 W
- WO 12964666 A
- US 13708439 B
- US 964666
- US 20130231258 A

### Non-patent literature cited in the description

- **GRIFFITH O. et al.** *J. clinical Oncology,* 2006, vol. 24, 5043-5051
- **DURAND S. et al.** *J. clinical Endocrinology,* 2008, vol. 93, 1195-1202
- **KEBEBEW E. et al.** *Annals of surgery,* 2007, vol. 246, 466-471
- *Bioinformatics,* 01 October 2007, vol. 23 (19), 2507-17
- *Cancer Inform.,* 2008, vol. 6, 77-97
- **COOPER et al.** *Thyroid,* 2006, vol. 16 (2
- **RAMZY, IBRAHIM.** *Clinical Cytopathology and Aspiration Biopsy,* 2001
- **SCHROEDER A et al.** *BMC Molecular Biology,* 2006, vol. 7, 3
- **SAMBROOK.** Molecular Cloning a Laboratory Manual. 2001
- **BALDI, P. ; HATFIELD, W.G.** *DNA Microarrays and Gene Expression,* 2002
- **IRIZARRY et al.** *Biostatistics,* April 2003, vol. 4 (2), 249-64
- **BOLSTAD et al.** *Bioinformatics,* 2003
- **TUKEY, J.W.** *Exploratory Data Analysis,* 1977
- Limma: linear models for microarray data. **SMYTH, G. K.** Bioinformatics and Computational Biology Solutions using Rand Bioconductor. Springer, 2005, 397-420
- **FISHEL ; KAUFMAN et al.** *Bioinformatics,* 2007, vol. 23 (13), 1599-606
- **SMYTH, G.K.** *Stat. Appl. Genet. Mol. Biol.,* 2004, vol. 3
- *Human Gene 1.0 ST Array performance,* 2007